# EUROPEAN PATENT APPLICATION

(11) **EP 4 700 073 A2**
(43) Date of publication of application: **25.02.2026**
(21) Application number: 26150683.6
(22) Date of filing: 17.09.2024
(51) Int. Cl.: C08J 3/24

(54) **COPOLYMERS OF N-VINYLPYRROLIDONE WITH FUNCTIONALIZED MALEIC ACID MOIETIES**

(30) Priority: 17.09.2023 EP 23197821; 17.09.2023 EP 23197822
(62) Divisional of application: 24771972.7
(71) Applicant: Hemoteq AG, 52146 Würselen (DE)
(72) Inventor: HOFFMANN, Michael, 52159 Roetgen (DE); HOFFMANN, Erika, 52159 Roetgen (DE); WIEMER, Katharina, 52223 Stolberg (DE); HORBACH, Helmut, 52066 Aachen (DE)
(74) Representative: Arth, Hans-Lothar

(57) **Abstract**

The present invention relates to vinylpyrrolidone copolymers composed of blocks of N-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent (BP) via a C₁-C₅ alkyl linker. The vinylpyrrolidone copolymers of the present invention are particularly suitable for use in coatings for medical devices and/or medical aids. The present invention therefore also relates to compositions comprising vinylpyrrolidone copolymers and optionally a crosslinker, as well as medical devices having a coating with crosslinked vinylpyrrolidone copolymers and optionally with a crosslinker.

## Description

The present invention relates to vinylpyrrolidone copolymers composed of blocks of N-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent (BP) via a C₁-C₅ alkyl linker. The vinylpyrrolidone copolymers of the present invention are particularly suitable for use in coatings for medical devices and/or medical aids. The present invention therefore also relates to compositions comprising vinylpyrrolidone copolymers and optionally a crosslinker, as well as medical devices having a coating with crosslinked vinylpyrrolidone copolymers and optionally with a crosslinker.

### Background of the invention

In general, particularly high demands are placed on medical devices that inevitably come into contact with a living organism during their intended use, since every medical intervention in the organism involves a potentially life-threatening situation to which the organism naturally reacts with the body's own protective measures. Medically necessary interventions should therefore be performed in such a way that traumatic consequences are reduced to a minimum and unavoidable level. In addition, the healing process following the intervention should be controlled so that no treatment-related complications occur in the short or long term. Medical devices should therefore have sufficient stability, biocompatibility, hemocompatibility, hydrophilicity, flexibility, biodegradation, pressure resistance, tear resistance or fit to ensure that no undesirable consequences occur, such as degenerative inflammatory processes, infections, thrombosis, bleeding, tissue proliferation, cell decay, necrosis, scarring, arthritis, bone proliferation, bone decay, cartilage damage, cartilage loss and connective tissue damage.

An essential aspect of avoiding complications - in addition to the gentlest possible surgical technique - is to ensure that the medical device is as easy to handle as possible and that it functions optimally without itself causing avoidable short-term or long-term damage. For example, a rough surface is not desirable in medical devices if surrounding tissue is not to be deliberately rubbed, injured or irritated during contact with the tissue. A rough surface would only unintentionally increase inflammatory processes and thus make healing processes more difficult or even prevent them. Degenerating inflammatory processes, overreactions of the tissue, the immune system and the coagulation system, infections, thromboses, bleeding, tissue proliferation that can be traced back to the presence of the medical device as a foreign body, and medical devices that do not do justice to their task and lead to reactions that may be manifested by persistent and permanent pain or only as late effects (e.g. in the skeletal region, bandages, etc.) may result in a severe injury. Therefore, medical devices that do not fulfill their purpose and lead to reactions that may be indicated by persistent and permanent pain or only as late effects (e.g. in the skeletal area, intervertebral discs, joints, spine, arthritis, bone growths, bone decay, cartilage damage, loss of cartilage) due to insufficient performance must be avoided.

The state of the art has long attempted to solve these complex problems with the aid of various materials. A key objective here is to ensure that no tissue is injured or irritated more than is absolutely necessary, that inflammatory processes do not become a greater burden than is already unavoidable as a result of the intervention itself, and that the healing process is not delayed but supported and the intended function of the medical device or medical aid is thus fulfilled as optimally as possible.

To date, only very few prior art medical devices are known that can fulfill these desired properties. This is particularly true in the vascular field. In the vascular field, hydrophilic substances are mostly used for coatings of e.g. stents or catheters. These products are advertised as having a low-friction and well-adherent coating, which improves implantation conditions in the long term. Even though these products already have improved properties, they still do not adequately meet the high demands placed on such products. However, it is not only in the vascular field, but also in catheters that have to be used long-term, e.g. in the urinary tract, or where access has to be established in the short or long term (drip, cannulas of any kind such as ventilation through a tracheotomy, artificial intestinal outlet, etc.) that it is crucial that no inflammation and infection occur. Thus, depending on the area of application, there are different requirements that are placed on the medical device or aid.

Therefore, there is still an urgent need for medical devices or medical aids with improved and optimized properties that meet these high requirements, because only if all necessary parameters are taken into account and sufficiently fulfilled, a medical device or medical aid can optimally support the organism. The properties - matched to the intended use - such as flexibility, stretchability, deformability, degradation behavior, duration of use, storability, sterilization stability, smoothness, hydrophilicity, frictional resistance, etc. are therefore regarded as prerequisites for the optimum benefit of medical devices or aids.

An optimum coating for medical devices used for long or short periods should in particular exhibit good adhesion to the medical device surface and at the same time be as flexible, stretchable and deformable as the uncoated material. Furthermore, it is particularly desirable that the coating exhibits low frictional resistance so that the tissue is not unnecessarily irritated during and also after implantation and use is facilitated, e.g. for the surgeon, nurse, so that the medical device can reach the target site without loss and can develop its intended function there optimally and without undesirable side effects.

It is also desirable that the coating can act as a transport medium or elution coordinator for active ingredients, or that other therapeutically active additives such as polymers, salts, hydrates, solvates, halogens, minerals, metals, metal salts, etc. can be present in the coating without impairing adhesion to the medical device surface or increasing frictional resistance.

The prior art describes medical devices having coatings with polyvinylpyrrolidone copolymers that are intended to exhibit improved biocompatibility and lubricity. One approach from the prior art to provide improved biocompatibility represents the provision of polyvinylpyrrolidone copolymers with covalently bound photoreactive groups. Another prior art approach is to add a crosslinker to a polyvinylpyrrolidone copolymer. After application of the coating to the medical device surface, crosslinking of the polyvinylpyrrolidone copolymers can be achieved in both cases, e.g. by UV irradiation.

Even if such coatings already exhibit improved properties, such as improved biocompatibility or lubricity, they still do not sufficiently meet the requirements for medical devices. There is therefore still an urgent need for suitable materials for use in coatings for medical devices or medical aids.

Therefore, the objective of the present invention is to provide polyvinylpyrrolidone copolymers which have improved physical and mechanical properties with respect to flexibility, stretchability, deformability, degradation, duration of use, storability and hydrophilicity and which can be used in particular for coating medical devices or medical aids, so as to provide coating with significantly reduced frictional resistance with stable adhesion to the surface. The task of the present invention therefore also relates to medical devices with a coating comprising polyvinylpyrrolidone copolymers.

The objective of the present invention is solved by the teaching of the independent claims. Further advantageous features, aspects and details of the invention are evident from the dependent claims, the description, the figures, and the examples of the present application.

### Brief description of the invention

Surprisingly, it has been found that a medical device having a coating of a cross-linked vinylpyrrolidone copolymer composed of blocks of N-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent (BP) via a C₁-C₅ alkyl linker, wherein the coating contains at least one anti-inflammatory, cytostatic, cytotoxic, antiproliferative, antimicrotubule, antiangiogenic, antiviral, antibacterial, antibiotic, antimitotic, antirestenotic, antifungal, antineoplastic, antimigrative, anticancerogenic, vasodilator, athrombogenic, and antithrombogenic agent, or a mixture thereof, solve the above-mentioned objective.

Thus, the present invention relates to medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups
(-COO-), or two carbamoyl groups (-CONH-), and 1% to 50% of the functional groups of all maleic acid moieties in the vinylpyrrolidone copolymer are functionalized with

   **-L-Y-BP**
wherein
   **L** represents a C₁-C₅ alkyl linker;
   **BP** represents a benzophenone substituent; and
   **Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
   with the proviso that when **Y** is -O-, the C₁-C₅ alkyl linker **(L)** is not ethylene (-CH₂CH₂-),
   wherein the coating contains at least one anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agent, or a mixture thereof.

Preferred is a medical device having a coating of a **cross-linked** vinylpyrrolidone copolymer as defined herein, wherein the anti-inflammatory, cytostatic, cytotoxic, antiproliferative, antimicrotubule, antiangiogenic, antiviral, antibacterial, antibiotic, antimitotic, antirestenotic, antifungal, antineoplastic, antimigrative, anticancerogenic, vasodilator, athrombogenic, and antithrombogenic agent is selected from the group comprising or consisting of:
sirolimus (rapamycin), everolimus, biolimus, zotarolimus, temsirolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoboside, teniposide, nimustine, carmustine, lomustine, cyclophosphamide, 4-hydroxyoxycyclophosphamide, estramustine, melphalan, ifosfamide, tropfosfamide, chlorambucil, bendamustine, dacarbazine, busulfan, procarbazine, treosulfan, tremozolomide, thiotepa, daunorubicin, doxorubicin, aclarubicin, epirubicin, Mitoxantrone, idarubicin, bleomycin, mitomycin, dactinomycin, methotrexate, fludarabine, fludarabine 5'-dihydrogen phosphate, cladribine, mercaptopurine, thioguanine, cytarabine, fluorouracil, gemcitabine, capecitabine, docetaxel, Carboplatin, cisplatin, oxaliplatin, amsacrine, irinotecan, topotecan, hydroxycarbamide, miltefosine, pentostatin, aldesleukin, tretinoin, asparaginase, pegasparase, anastrozole, exemestane, letrozole, formestane, aminoglutethemide, adriamycin, Azithromycin, spiramycin, cepharantine, 8-ergolines, dimethylergolines, agroclavin, 1-allylisuride, 1-allylterguride, bromerguride, bromocriptine (ergotaman-3',6',18-trione, 2-bromo-12'-hydroxy-2'-(1-methylethyl)-5'-(2-methylpropyl)-,(5'alpha)-), elymoclavine, ergocristine (ergotaman-3', 6', 18-trione, 12'-hydroxy-2'-(1-methylethyl)-5'-(phenylmethyl)-, (5'-alpha)-), ergocristinine, ergocornine (ergotaman-3',6',18-trione, 12'-hydroxy-2',5'-bis(1-methylethyl)-, (5'-alpha)-), ergocorninine, ergocryptin (ergotaman-3',6',18-trione, 12'-hydroxy-2'-(1-methylethyl)-5'-(2-methylpropyl)-, (5'alpha)- (9CI)), ergocryptinine, ergometrine, ergonovine (ergobasin, INN: Ergometrine, (8beta(S))-9,10-didehydro-N-(2-hydroxy-1-methylethyl)-6-methyl-ergoline-8-carboxamide), ergosine, ergosinine, ergotmetrinine, ergotamine (ergotaman-3', 6', 18-trione, 12'-hydroxy-2'-methyl-5'-(phenylmethyl)-, (5'-alpha)-(9CI)), ergotaminin, ergovalin (ergotaman-3',6',18-trione, 12'-hydroxy-2'-methyl-5'-(1-methylethyl)-, (5'alpha)-), lergotril, lisuride (CAS no.: 18016-80-3, 3-(9,10-didehydro-6-methylergolin-8alpha-yl)-1,1-diethylurea), lysergol, lysergic acid (D-lysergic acid), lysergic acid amide (LSA, D-lysergic acid amide), lysergic acid diethylamide (LSD, D-lysergic acid diethylamide, INN: lysergamide, (8β)-9,10-didehydro-N,N-diethyl-6-methyl-ergoline-8-carboxamide), isolysergic acid (D-isolysergic acid), isolysergic acid amide (D-isolysergic acid amide), isolysergic acid diethylamide (D-isolysergic acid diethylamide), mesulergin, metergoline, methergine (INN: methylergometrin, (8beta(S))-9,10-didehydro-N-(1-(hydroxymethyl)propyl)-6-methyl-ergoline-8-carboxamide), methylergometrin, methysergide (INN: methysergide, (8beta)-9,10-didehydro-N-(1-(hydroxymethyl)propyl)-1,6-dimethyl-ergoline-8-carboxamide), pergolide ((8β)-8-((methylthio)methyl)-6-propyl-ergoline), proterguride and terguride, celecoxip, thalidomide, Fasudil^{®,} cyclosporine, SMC proliferation inhibitor-2w, epothilone A and B, mitoxanthrone, azathioprine, mycophenolate mofetil, c-myc antisense, b-myc antisense, betulinic acid, camptothecin, PI-88 (sulfated oligosaccharide), melanocyte-stimulating hormone (α -MSH), activated protein C, IL1-β inhibitor, thymosinα -1, fumaric acid and its esters, calcipotriol, tacalcitol, lapachol, β-lapachone,podophyllotoxin, betulin, podophyllic acid-2-ethylhydrazide, molgramostim (rhuGM-CSF), peginterferon α-2b, lanograstim (r-HuG-CSF), filgrastim, macrogol, dacarbazine, basiliximab, daclizumab, Selectin (cytokine antagonist), CETP inhibitor, cadherins, cytokine inhibitors, COX-2 inhibitor, NFkB, angiopeptin, ciprofloxacin, camptothecin, fluroblastine, monoclonal antibodies that inhibit muscle cell proliferation, bFGF antagonists, probucol, prostaglandins, 1,11-dimethoxycanthin-6-one, 1-hydroxy-11-methoxycanthin-6-one, scopolectin, colchicine, NO donors such as pentaerythrityl tetranitrate, and syndnoeimines, S-nitroso derivatives, tamoxifen, staurosporine, β-estradiol,α -estradiol, estriol, estrone, ethinylestradiol, fosfestrol, medroxyprogesterone, estradiolcypionate, estradiolbenzoate, tranilast, camebacaurine and other terpenoids used in cancer therapy, verapamil, tyrosine kinase inhibitors (tyrphostins), cyclosporine A and B, paclitaxel and its derivatives such as 6-α -hydroxy-paclitaxel, baccatin, taxotere, synthetically produced as well as macrocyclic oligomers of carbon suboxide (MCS) obtained from native sources and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, auranofin, sodium aurothiomalate, oxaceprol, celecoxib, β-sitosterol, ademetionin, myrtecaine, polidocanol, nonivamide, levomenthol, benzocaine, aescin, ellipticin, D-24851 (Calbiochem), colcemid, cytochalasin A-E, indanocins, nocadazoles, S 100 protein, bacitracin, vitronectin receptor antagonists, azelastine, guanidyl cyclase stimulator tissue inhibitor of metalloproteinase-1 and 2, free nucleic acids, Nucleic acids incorporated into viral carriers, DNA and RNA fragments, plaminogen activator inhibitor-1, plasminogen activator inhibitor-2, antisense oligonucleotides, VEGF inhibitors, IGF-1, Agents from the group of antibiotics such as cefadroxil, cefazolin, cefaclor, cefotixin, tobramycin, gentamycin, penicillins such as dicloxacillin, oxacillin, sulfonamides, metronidazole, antithrombotics such as argatroban, aspirin, abciximab, synthetic antithrombin, bivalirudin, coumadin, enoxoparin, desulfated and N-reacetylated heparin, tissue plasminogen activator, Gpllb/llla platelet membrane receptor, factor Xₐ inhibitor antibody, interleukin inhibitors, heparin, hirudin, r-hirudin, PPACK, protamine, sodium salt of 2-methylthiazolidine-2,4-dicarboxylic acid prourokinase, streptokinase, warfarin, urokinase, Vasodilators such as dipyramidol, trapidil, nitroprusside, PDGF antagonists such as triazolopyrimidine and seramine, ACE inhibitors such as captopril, cilazapril, lisinopril, enalapril, losartan, thioprotease inhibitors, Prostacyclin, vasiprost, interferonα , β and γ, histamine antagonists, serotonin blockers, apoptosis inhibitors, apoptosis regulators such as p65, NF-kB, or Bcl-xL antisense oligonucleotides, halofuginone, nifedipine, tocopherol, vitamins B1, B2, B6, and B12, folic acid, tranilast, molsidomine, tea polyphenols, epicatechin gallate, epigallocatechin gallate, boswellic acids and their derivatives, leflunomide, Anakinra, etanercept, sulfasalazine, etoposide, dicloxacylline, tetracycline, triamcinolone, mutamycin, procainimide, D24851, SC-58125, retinoic acid, quinidine, disopyrimide, flecainide, propafenone, sotolol, amidoron, natural and synthetically produced steroids such as bryophyllin A, inotodiol, maquiroside A, ghalakinoside, mansonin, strebloside, hydrocortisone, betamethasone, dexamethasone, non-steroidal anti-inflammatory drugs (NSAIDS) such as fenoporfen, ibuprofen, indomethacin, naproxen, phenylbutazone and other antiviral agents such as acyclovir, ganciclovir and zidovudine, antifungal agents such as clotrimazole, flucytosine, griseofulvin, ketoconazole, miconazole, nystatin, terbinafine, antiprozoal agents like chloroquine, mefloquine, quinine, natural terpenoids like hippocaesculin, barringtogenol-C21-angelat, 14-dehydroagrostistachin, agroskerin, agroskerin, agrostistachin, 17-hydroxyagrostistachin, ovatodiolides, 4,7-oxycycloanisomelic acid, baccharinoids B1, B2, B3 and B7, tubeimoside, bruceanols A, B and C, bruceantinosides C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, furthermore cymarin, apocymarin, aristolochic acid, anopterin, hydroxyanopterin, anemonin, protoanemonin, berberin, cheliburin chloride, cictoxin, sinococulin, bombrestatin A and B, cudraisoflavone A, curcumin, dihydronitidine, nitidine chloride, 12-beta-hydroxypregnadiene-3,20-dione, bilobol, ginkgol, ginkgolic acid, helenalin, indicin, indicin-N-oxide, lasiocarpin, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetin, pancratistatin, liriodenin, oxoushinsunin, aristolactam-All, bisparthenolidin, periplocoside A, ghalakinoside, ursolic acid, deoxypsorospermine, psycorubin, ricin A, sanguinarin, manwuweizic acid, methylsorbifolin, sphatheliachromene, stizophyllin, mansonin, strebloside, akagerin, dihydrousambaraensin, hydroxyusambarin, strychnopentamine, strychnophyllin, usambarin, usambarensin, berberin, liriodenin, oxoushinsunin, daphnoretin, lariciresinol, methoxylariciresinol, syringaresinol, umbelliferon, afromoson, acetylvismion B, desacetylvismion A, vismion A and B, and sulfur-containing amino acids such as cysteine, as well as salts, hydrates, solvates, enantiomers, racemates, enantiomeric mixtures, diastereomeric mixtures; metabolites, prodrugs and mixtures of the active ingredients.

Preferred is a medical device having a coating of a **cross-linked** vinylpyrrolidone copolymer as defined herein, wherein the C₁-C₅ alkyl linker is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

Preferred is a medical device having a coating of a **cross-linked** vinylpyrrolidone copolymer as defined herein, wherein the benzophenone substituent **BP** is benzoylphenyl.

Preferred is a medical device having a coating of a **cross-linked** vinylpyrrolidone copolymer as defined herein, wherein Y represents -OCO-, -NHCO-, -COO-, - CONH-, -OCOCH₂-, -NHCOCH₂-, -COOCH₂-, or -CONHCH₂-.

Preferred is a medical device having a coating of a **cross-linked** vinylpyrrolidone copolymer as defined herein, wherein **Y** represents -NHCO-, -CONH-, - NHCOCH₂-, or -CONHCH₂-.

Preferred is a medical device having a coating of a **cross-linked** vinylpyrrolidone copolymer as defined herein, wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein q is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferred is a medical device having a coating of a **cross-linked** vinylpyrrolidone copolymer as defined herein, wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein q is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferred is a medical device having a coating of a **cross-linked** vinylpyrrolidone copolymer as defined herein, wherein the copolymer has the general formula (ii): 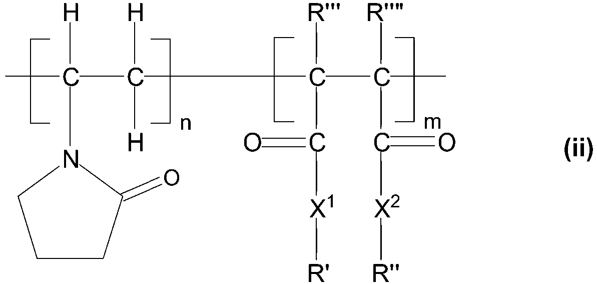 wherein
m = 0.1 - 10.0 mol% and
n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and
the remaining residues **R'** and **R"** are -H;
**R‴** and **Rʺʺ** represent independently of each other -H, -CH₃, -CH₂CH₃, or -F;
**L** represents -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
with the proviso that when **Y** is -O-, the C₁-C₅ alkyl linker **(L)** is not ethylene (-CH₂CH₂-);
**BP** represents 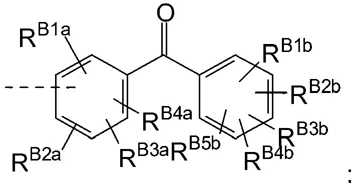
**R^{B1a}** - **R^{B4a}** and **R^{B1b}** - **R^{B5b}** represent independently of each other -H, -COOH, -CH₃ , -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50;
wherein **q** is an integer between 35 and 50.

Preferred is a medical device having a coating of a **cross-linked** vinylpyrrolidone copolymer of general formula **(ii)** as defined herein, wherein only one of X¹ and X² is -NH- and/or wherein **Y'** and **Y"** represent a bond.

Preferred is a medical device having a coating of a **cross-linked** vinylpyrrolidone copolymer of general formula **(ii)** as defined herein wherein up to **p%** of **R'** and **R"** are -(CH₂CH₂O)_{q}-H and **p** is a number from 1 - 50, wherein **q** is an integer between 35 and 50.

Preferred is a medical device having a coating of a cross-linked vinylpyrrolidone copolymer as defined herein, wherein the vinylpyrrolidone copolymer is selected from the group consisting of: 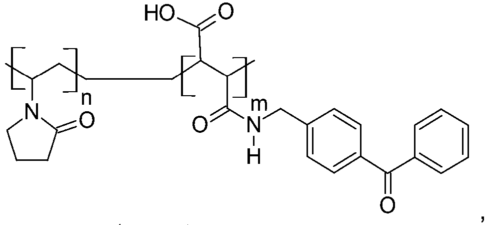 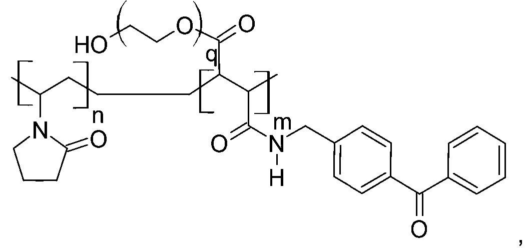 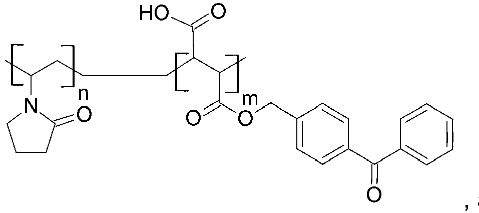 and 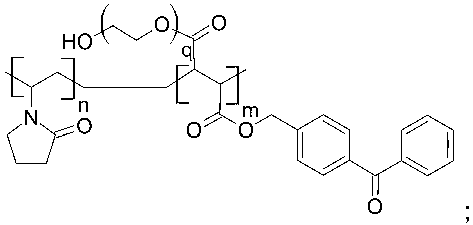 wherein
m = 0.1 - 10.0 mol% and
n = 100 mol% - m;
q is an integer between 35 and 50.

Preferred is a medical device having a coating of a **cross-linked** vinylpyrrolidone copolymer as defined herein, wherein the medical device is selected from the group comprising or consisting of balloon catheters, bladder catheters, catheter shafts, guide wires, stomach tubes, endoscopes, colonoscopes, needles, cannulas, endotracheal tubes, tracheal cannulas, implants such as stents, ocular lenses, prosthetic joints, screws, fixations, artificial exits, nasal implants, vocal cords, larynx, artificial and native valves such as cardiac or venous valves, esophageal sphincter, sphincter ani, bone graft substitutes, and ventilator tubes.

Preferred is a medical device having a coating of a **cross-linked** vinylpyrrolidone copolymer as defined herein, wherein the coating further comprises a crosslinker.

Preferred is a medical device having a coating of a **cross-linked** vinylpyrrolidone copolymer as defined herein, wherein the coating further comprises a crosslinker, and wherein the crosslinker has the general formula **(I):**

**BP¹-L¹-Z-L²-BP²** **(I)**

wherein **BP¹** and **BP²** are benzophenone substituents covalently linked to each other via a linker **Z,** wherein the linker **Z** is selected from an aryl linker, an alkyl linker and an amino acid linker, and wherein the linker **Z** has two linkers **L¹** and **L²** each functionalized with one of the two benzophenone substituents **BP¹** and **BP²,** wherein **L¹** and **L²** independently of each other include or consist of:
-O-CO-, -NH-CO-, -CO-, -O-, -NH-, -CO-O-, -CO-NH-, -NH-CO-O-, -O-CO-NH-, -O-CO-O-, and -NH-CO-NH-, or wherein the linker **Z** is -O-CO-, -NH-CO-, -CO-, -O-, -NH-, -CO-O-, -CO-NH-, -NH-CO-O-, -O-CO-NH-, -O-CO-O-, or -NH-CO-NH-.

It has been surprisingly found that coatings of medical devices or medical aids comprising a vinylpyrrolidone copolymer of the present invention exhibit significantly reduced frictional resistance and thus improved lubricity compared to the prior art, and at the same time adhere very well to the surface of the medical device or medical aid. Thus, compared to the prior art, the vinylpyrrolidone copolymers of the present invention provide improved physical and mechanical properties in terms of flexibility, stretchability, deformability, degradation behavior, service life, shelf life, suppleness, hydrophilicity, lubricity, frictional resistance and adhesion to the surface.

It has been surprisingly found that the presence of a C₁-C₅ alkyl linker, via which a benzophenone substituent **(BP)** is covalently bound to the vinylpyrrolidone copolymer, is essential for achieving the reduced frictional resistance and more stable adhesion to the surface of the medical device or the medical aid. The presence of the C₁-C₅ alkyl linker increases the flexibility of the covalently bound benzophenone substituent **(BP),** which is particularly beneficial to the crosslinking of the vinylpyrrolidone copolymer and to the binding to the surface of the medical device or medical aid.

Preferably, the C₁-C₅ alkyl linker is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-). Herein, the C₁-C₅ alkyl linker methylene (-CH₂-) is particularly preferred.

In preferred embodiments, up to 100% of all maleic acid moieties have a carboxy group (-COO-) and a carbamoyl group (-CONH-) as functional groups. Thus, herein preferred are vinylpyrrolidone copolymers composed of blocks of N-vinylpyrrolidone (NVP) moieties divided by maleic acid moieties, wherein each maleic acid moiety has as functional groups a carboxy group (-COO-) and a carbamoyl group (-CONH-), and 1% to 50% of the functional groups of all maleic acid moieties in the vinylpyrrolidone copolymer are functionalized with a benzophenone substituent **(BP)** via a C₁-C₅ alkyl linker. Herein, it is further preferred that the functional groups functionalized with a benzophenone substituent **(BP)** via the C₁-C₅ alkyl linker are carbamoyl groups (-CONH-). Thus, particularly preferred are herein vinylpyrrolidone copolymers composed of blocks of *N*-vinylpyrrolidone (NVP) moieties divided by maleic acid moieties, wherein each maleic acid moiety has as functional groups a carboxy group (-COO-) and a carbamoyl group (-CONH-), and 1 to 100%, preferably 100% of the carbamoyl groups (-CONH-) of all maleic acid moieties in the vinylpyrrolidone copolymer are functionalized with a benzophenone substituent **(BP)** via a C₁-C₅ alkyl linker.

In preferred embodiments, the benzophenone substituent **(BP)** is benzoylphenyl. In some preferred embodiments, the benzophenone substituent may further have one or more substituents selected from the group comprising or consisting of -OH, -NO₂, -COCH₃, -CONH₂, -SO₃H, -SO₂NH₂, -OCH₃, -OCF₃, -NH₂, -NH(CH)₃, -N(CH₃)₂, and -SOCH₃.

In preferred embodiments, up to 100% of the functional groups of all maleic acid moieties in the vinylpyrrolidone copolymer that are **not** functionalized with a benzophenone substituent **(BP)** via a C₁-C₅ alkyl linker may independently of each other have a non-benzophenone substituent which may be selected from the group comprising or consisting of polyethylene glycol (PEG), polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group, wherein the ratio of benzophenone substituent **(BP)** to non-benzophenone substituent is ≥ 1, and the remaining functional groups of all maleic acid moieties in the vinylpyrrolidone copolymer have the residue -H.

In preferred embodiments, up to 100% of the functional groups of all maleic acid moieties in the vinylpyrrolidone copolymer that are **not** functionalized with a benzophenone substituent **(BP)** via a C₁-C₅ alkyl linker may have a non-benzophenone substituent selected from the group comprising or consisting of polyethylene glycol, polylactide, polyglycolide, and polyacrylate, wherein the ratio of benzophenone substituent **(BP)** to the non-benzophenone substituent is ≥ 1, and the remaining functional groups of all maleic acid moieties in the vinylpyrrolidone copolymer have the residue -H.

In preferred embodiments, the vinylpyrrolidone copolymers composed of blocks of N-vinylpyrrolidone moieties divided by maleic acid moieties have a total amount of maleic acid moieties in the vinylpyrrolidone copolymer of 0.1 - 10.0 mol%, preferably 0.1 - 5.0 mol%, more preferably 0.1 - 2 mol%. In some particularly preferred embodiments, the vinylpyrrolidone copolymers have a total amount of maleic acid moieties in the vinylpyrrolidone copolymer of 1.5 - 2.5 mol%, more preferably 2 mol%. In some further preferred embodiments, the vinylpyrrolidone copolymers have a total amount of maleic acid moieties in the vinylpyrrolidone copolymer of less than 1 mol%, preferably 0.1 - 0.95 mol%, more preferably 0.1 - 0.90 mol%, more preferably 0.2 - 0.88 mol%, even more preferably 0.5 - 0.85 mol%.

In preferred embodiments, the molar ratio of N-vinylpyrrolidone moieties to maleic acid moieties in the vinylpyrrolidone copolymer is between 10:1 and 1000:1, preferably between 10:1 and 200:1, more preferably between 20:1 and 1000:1, more preferably between 20:1 and 200:1, more preferably between 50:1 and 150: 1, more preferably at 50:1, also preferably between 80:1 and 130:1.

The present invention further relates to coating compositions comprising a vinylpyrrolidone copolymer of the present invention. The present invention further relates to coating compositions comprising a vinylpyrrolidone copolymer of the present invention and a crosslinker.

The present invention further relates to coating compositions comprising a vinylpyrrolidone copolymer of the present invention (Component A), a crosslinker (Component B), and a first biostable and biodegradable polymer (Component C) and a second biostable and biodegradable polymer (Component D), wherein Component C is preferably PVP LMW, such as Kollidon^{®} 30, and Component D is preferably PVP HMW, such as Kollidon^{®}90F.

The present invention further relates to medical devices having a coating of a **crosslinked** vinylpyrrolidone copolymer composed of blocks of N-vinylpyrrolidone (NVP) moieties divided by maleic acid moieties, wherein each maleic acid moiety has as functional groups a carboxy group (-COO-) and a carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker.

### Description of the invention

The present invention relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L).**

It has been found that the properties of the residues on the carboxy groups of maleic acid and the distribution of the maleic acid monomers in the polymer chain advantageously determine the properties of crosslinked vinylpyrrolidone copolymers.

The vinylpyrrolidone copolymers of the present invention particularly exhibit excellent adhesion to various substrate materials relevant to the field of medical device and thus enable stable adhesion to medical device surfaces. Furthermore, a significantly reduced frictional resistance is observed for the vinylpyrrolidone copolymers of the present invention and thus these vinylpyrrolidone copolymers are particularly suitable for providing lubricious coatings on medical device surfaces. The coatings with the vinylpyrrolidone copolymers of the present invention also offer improved physical and mechanical properties. Due to the significantly reduced frictional resistance with stable adhesion, the vinylpyrrolidone copolymers of the present invention are thus particularly advantageous for use as components for coatings of surfaces, especially medical devices sufarces.

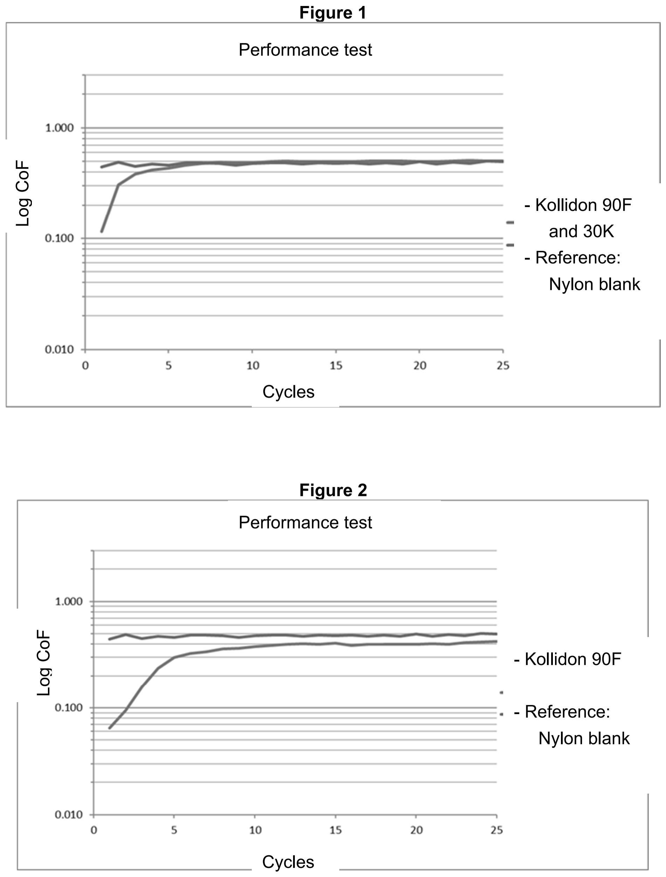

In comparison, the homopolymer of polyvinylpyrrolidone (PVP) alone shows no significant influence on the resulting frictional resistance when substrates are coated compared to uncoated substrate. To investigate the frictional resistance, nylon substrate was coated on one side with a mixture of Kollidon 90F (high molecular weight PVP) and PVP-30K (Fig.1) and once with Kollidon 90F alone (Fig. 2) and the coefficients of friction were determined.

Thus, the vinylpyrrolidone copolymers of the invention are particularly suitable for use for or in all medical devices and medical aids that can be used in or on the body for short or long periods of time. The vinylpyrrolidone copolymers of the present invention may be used, among other things, as components of coatings for tubing, catheters, or other materials that require insertion into the body. Examples of these include, but are not limited to, catheters of all kinds e.g. balloon catheters, bladder catheters, catheter shafts, guide wires, stomach tubes, endoscopes, colonoscopes, needles, cannulas, endotracheal tubes, tracheal tubes, implants such as stents, ocular lenses, joint prostheses, screws, fixations, artificial exits, nasal implants, vocal cords, larynx, artificial and native valves such as cardiac or venous valves, esophageal sphincter, sphincter ani, bone graft substitutes and ventilator tubes.

The surface or substrate to be coated can be made of a wide variety of common materials. Examples include, but are not limited to, polyamides (such as PA 12, PA 6, PA 66), stainless steel, nitinol, pebax, polyethylenes, polypropylenes, polyvinyls, polystyrenes, polyimides, polyamides, PTFE, silicone, polyurethanes, polyesters such as e.g., polymethacrylates, polyacrylates, polyacrylamides, polycarbonates, polylactides, polyglycolides, polylactic acids, polyacetates, polycarbonates etc. and their copolymers and derivatives. A different preference for binding to the material surface can be seen depending on the component mixture, which means that the optimum composition must be determined experimentally for different substrates, although the person skilled in the art is able to make this determination without excessive effort.

The vinylpyrrolidone copolymer according to the present invention is suitable for use as or in any medical device or medical aid that can be used in or on the body on a short-term or long-term basis. Consequently the vinylpyrrolidone copolymer of the present invention can be used, among other things, as coatings for tubes, catheters, or other materials that require insertion into the body. Examples of these include, but are not limited to, catheters of any kinds e.g. balloon catheters, bladder catheters, catheter shafts, guide wires, stomach tubes, endoscopes, colonoscopes, needles, cannulas, endotracheal tubes, tracheal cannulas, implants such as stents, ocular lenses, prosthetic joints, screws, fixations, artificial exits, nasal implants, vocal cords, larynx, artificial and native valves such as cardiac or venous valves, esophageal sphincter, sphincter ani, bone graft substitutes, and ventilator tubes. However, the molecule colloids of the present invention can also be used as cartilage substitutes, nerve sheaths, occlusion of venous valves, seals ex corpora and in corpora, subcutaneous or intramuscular implants, as moisturizing and/or active ingredient gels/ointments, micro- and macrocapsules, wound protectors, breast implants, generally as filling materials, plasters, pads, etc., or as components of the products mentioned herein. Further applications of the molecular colloids of the present invention include, but are not limited to cosmetics, personal care, hair and skin care, color cosmetics, pharmaceuticals, biomaterials for delivery systems, excipients and coatings, and industrial applications, paper coatings, textile coatings, ink additive, suspending aid and adhesives. Additional applications include, but are not limited to membrane applications, lubricious coatings, compatibilizers, adhesives, polymeric surfactants and antimicrobial and antifouling coatings. Further examples include skin lotions, moisturizers, eye creams, soaps, syndets, shower and bath preparations, sunscreen preparations, insect repellents, acne preparations, shaving products, etc. Thus, an enormously diverse product range of medical devices, medical aids and cosmetical and hygienical products, e.g. personal care compositions or cosmetical compositions and applications thereof is made possible.

### Definitions

### Linker

The term **"linker",** as used herein, refers to an organic molecule of low molecular weight that covalently links two or more organic compounds with each other. A linker generally represents a bifunctional chemical entity that binds a first compound to a second compound. A linker serves as a spacer for two or more organic compounds and can therefore advantageously increase flexibility. Linkers can have different lengths and polarities. Examples of linkers include, but are not limited to alkyl linkers, aryl linkers, aryl alkyl linkers, alkoxy linkers, aryloxy linkers, alkylhydroxy linkers, arylhydroxy linkers, alkylaryloxy linkers, alkylamino linkers, arylamino linkers, alkylarylamino linkers, each of which may be optionally substituted or unsubstituted. Polymers still having functional groups in the polymer chain can be functionalized with various organic compounds. These organic compounds can also be connected to the polymer chain via a linker, so that these organic compounds have a distance to the main chain of the polymer to increase the flexibility of these organic compounds.

### Crosslinking

The term **"crosslinking",** as used herein, refers to reactions in which one or more individual macromolecules are linked to form a three-dimensional network. The linkage can be achieved either directly during the assembly of the macromolecules or through reactions on preexisting polymers. The process of crosslinking changes the properties of polymers. In general, an increase in hardness, toughness, melting point and a decrease in solubility are observed. The changes increase with the degree of crosslinking, the proportion of linked sites relative to the total amount of polymer.

Crosslinking of already existing polymers can be accomplished either by functionalities already present in the polymer through clever choice of reaction conditions (self-crosslinking) or by the addition of multifunctional, low-molecular-weight substances. Crosslinking or UV curing are well known in the field of polymer chemistry. Examples of photo-reactive functional groups that can be used for crosslinking include, but are not limited to arylazides, azido-methyl coumarins, benzophenones, anthraquinones, certain diazo compounds, diazirines, and psoralen derivatives. Examples of direct crosslinking reactions include free radical polymerizations of monomers with two vinyl groups or polycondensation or polyaddition using monomers with two or more functional groups.

### Crosslinker

**A "crosslinker",** as used herein, refers to a bi- or multifunctional, low molecular weight compound characterized by at least two reactive groups and being capable of crosslinking individual macromolecules or polymers to form a three-dimensional network. The reactive groups of the crosslinkers according to the invention are benzophenones each of which may be optionally substituted or unsubstituted.

### Monomer

The term **"monomer",** as used herein, refers to an organic compound, generally an organic compound of low molecular weight, herein particularly an unsaturated (low molecular weight) organic compound, which has at least one chemically reactive group such as a double bond or an epoxide group, or has at least two chemically reactive groups such as two carboxy groups, two hydroxy groups or two amino groups, which can be reacted in a chemical reaction to form a long-chain unbranched or branched macromolecule consisting of monomers covalently coupled to each other.

Examples of hydrophilic monomers include, but are not limited to, hydroxyl-substituted lower alkyl acrylates and hydroxyl-substituted lower alkyl methacrylates such as hydroxyethyl acrylate, hydroxyethyl methacrylate, hydroxypropyl acrylate and hydroxypropyl methacrylate, acrylamide, methacrylamide, (lower alkyl) acrylamides and (lower alkyl) methacrylamides, N,N-dialkylacrylamides, ethoxylated acrylates, ethoxylated methacrylates such as polyethylene glycol monoacrylates, polyethylene glycol monomethacrylates, polyethylene glycol monomethyl ether acrylates, polyethylene glycol monomethyl ether acrylates, hydroxyl substituted (lower alkyl) acrylamides, hydroxyl substituted (lower alkyl) methacrylamides, hydroxyl substituted lower alkyl vinyl ethers, sodium vinyl sulfonate, sodium styrene sulfonate, 2-acrylamido-2-methyl-propanesulfonic acid, N-vinylpyrrole, N-vinyl-2-pyrrolidone, 2-vinyloxazoline, 2-vinyl-4,4'-dialkyloxazolin-5-one, 2-vinylpyridine, 4-vinylpyridine, allyl alcohol, 3-trimethylammonium-2-hydroxypropylmethacrylate chloride, vinyl alcohol, acrylonitrile, acryloyl chloride, ethylene glycol acrylate, methylolacrylamide, diacetone acrylamide, styrenesulfonic acid salt, dimethylaminoethyl methacrylate, dimethylaminoethyl methaarylamide,N-(1,1-dimethyl-3-oxobutyl)acrylamide, ethylene glycol vinyl ether, di(ethylene-glycol)vinyl ether, *N*-vinylpyrrolidone, 1-methyl-3-methylene-2-pyrrolidone, 1-methyl-5-methylene-2-pyrrolidone, 5-methyl-3-methylene-2-pyrrolidone, 1-ethyl-5-methylene-2-pyrrolidone, *N*-methyl-3-methylene-2-pyrrolidone, 5-ethyl-3-methylene-2-pyrrolidone, 1-n-propyl-3-methylene-2-pyrrolidone, 1-n-propyl-5-methylene-2-pyrrolidon, 1-isopropyl-3-methylene-2-pyrrolidone, 1-isopropyl-5-methylene-2-pyrrolidone, *N*-vinyl-*N*-methylacetamide, *N*-vinyl-*N*-ethylacetamide, *N*-vinyl-*N-*ethylformamide, *N*-vinylformamide, *N*-vinylacetamide, *N*-vinylisopropylamide, *N*-vinylcaprolactam, *N*-2-hydroxyethylvinylcarbamate, *N*-carboxy-β-alanine-*N*-vinyl ester, *N*-carboxyvinyl-β-alanine and *N*-carboxyvinyl-α-alanine.

Herein preferred monomers and/or oligomers are monomers as well as oligomers of known hydrophilic polymers. For example, preferred monomers are organic compounds that are at least monoolefinic unsaturated and can be polymerized. Examples of monoolefinic unsaturated monomers include, but are not limited to, acrylic acids, methacrylic acids, *N*-vinyl carbazoles, acrylic acid esters, acrylic acid amides, vinyls, vinylamines, vinylamides, *N*-vinylpyrrolidones, vinyl ethers, vinyl acetal, etc..

Examples of hydrophobic monomers include, but are not limited to, C₁-C₁₈ alkyl acrylates or C₁-C₁₈ methacrylates such as ethyl acrylate, butyl acrylate, isobutyl acrylate, hexyl acrylate, heptyl acrylate, methyl methacrylate, ethyl methacrylate, butyl methacrylate, isobutyl methacrylate, hexyl methacrylate, heptyl methacrylate, 1,3 butadiene, C₃-C₁₈ cycloalkyl acrylates or C₃-C₁₈ methacrylates such as cycloalkyl acrylate, isobornyl acrylate, isobornyl methacrylate, cycloalkyl methacrylate, C₃-C₁₈ alkyl acrylamides, C₃-C₁₈ methacrylamides, acrylonitrile, methacrylonitrile, vinyl C₁-C₁₈ alkanoates such as vinyl acetate, vinyl propionate, vinyl butyrate, vinyl valerate, C₂-C₁₈ alkenes, C₂-C₁₈ haloalkenes, styrene, (lower alkyl)styrene, d-methylstyrene, C₂-C₁₂ alkyl vinyl ethers, vinyl ethyl ethers, C₂-C₁₀ perfluoroalkyl acrylates, C₂-C₁₀ perfluoroalkyl methacrylates, partially fluorinated acrylates or partially fluorinated methacrylates, such as trifluoroethyl methacrylate, hexafluoroisopropyl methacrylate, hexafluorobutyl methacrylate, C₂-C₁₂ perfluoroalkylethylthiocarbonylaminoethyl acrylates and methacrylates, perfluorohexylethylthiocarbonylaminoethyl methacrylate, acryloxy and methacryloxyalkyl siloxanes, such as tristrimethylsilyloxysilylpropyl methacrylate (TRIS), 3-methacryloxy-propylpentamethyldisiloxane, N-vinylcarbazole, bis C₁-C₁₂ alkyl esters of maleic acid, fumaric acid, itaconic acid, mesaconic acid, such as e.g. dimethyl fumarate, dimethyl maleate, dibutyl maleate and dibutyl fumarate, chloroprene, vinyl chloride and vinylidene chloride.

### Polymer

The term **"polymer",** as used herein, refers to a macromolecule composed of long unbranched or branched chains of many covalently linked monomers. As used herein, macromolecules refer to molecules having an average molecular mass of at least 1,000 g/mol, preferably at least 5,000 g/mol, more preferably at least 10,000 g/mol.

Examples of polymers include, but are not limited to, polyacids, polyalcohols, polyethers, polyesters, polyesteramides, polyetherimines, polyetheramines, polyvinylcarbazoles, polyurethanes, polyacidesters, polyacidamines, polyacidamides, polyvinyls, polyvinylides, polyvinylalcohols, polyvinylamines, polyvinylamides, polyvinylpyrrolidones, polyvinylethers, polyhydroxycarboxylic acids, polylactams, polyethylene glycols, polyethylene glycol ethers, polyethylene oxides, polyoxazolines, polyacetals and polysiloxanes. Examples of known synthetic organic polymers include, but are not limited to, polyacrylamide, polyacrylic acid, polybutadiene, polymethacrylic acid, polyethyleneimine, polystyrene, polysulfonic acid, polytetrafluoroethylene, polyvinyl alcohol, polyvinyl chloride, polyethylene glycol, and polyvinyl pyrrolidone.

Particular examples of polymers include, but are not limited to, polylactonic acids, polyvalerolactones, poly-ε-decalactones, polyglycolic acids, polylactides, polyglycolides, copolymers of polylactides and polyglycolides, poly-ε-caprolactone, polypivotolactones, polycaprolactone glycolides, polygluconates, polylactic acid-polyethylene oxide copolymers, poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polycaprolactone (PCL), polycaprolactone-co-butyl acrylate, poly(phosphazene), poly(D,L-lactide-co-caprolactone) (PLA/PCL), Poly(glycolide-co-caprolactone) (PGA/PCL), polymaleic anhydrides, maleic anhydride copolymers, polymaleic acids, maleic acid copolymers, polylactams, poly(N-vinyl)-pyrrolidone (PVP), poly(N-vinyl)-pyrrolidone copolymers, N-dodecyl-pyrrolidone, N-decyl-pyrrolidone, N-octyl-pyrrolidone, N-alkyllactams, N-dodecylcaprolactam, N-decyl-caprolactam, N-octyl-caprolactam, N-dodecyl-valerolactam, N-decyl-valerolactam, N-octyl-valerolactam, polyvinyl alcohols, derivatives of polyvinyl alcohols, polyethers, polyethylene glycol (PEG), polyethylene oxide (PEO), polyethylene oxide propylene oxide, copolymers with polyethylene glycol and/or polypropylene glycol, polyoxyethylene sorbitan monolaurate, polyoxyethylene sorbitan monopalmitate, polyoxyethylene sorbitan monostearate, sorbitan monolaurate, sorbitan monopalmitate, sorbitan monostearate, polyoxyethylene oleyl ether, polyoxyethylene oleyl ether, bis-[alpha-methyl-(4-methylbenzyl)]-phenyl polyglycol ether, bis-[alpha-methyl-(4-n-dodecyl)]-phenyl-polyglycol ether, bis-(4-methyl-benzyl)-phenyl-polyglycol ether, Bis-(4-n-dodecyl-benzyl) phenyl-polyglycol ether, tris-[alpha-methyl-(4-methyl-benzyl)]-phenyl-polyglycol ether, Nonylphenol-polyglycol ether, nonylphenol diglycol ether, polyether ester multiblock polymers such as polyethylene glycol (PEG) and polybutylene terephthalate, polyhydroxybutyric acid, polyhydroxybutyrate, polyhydroxybutyrate-co-valerate, polydioxanone (PDS), poly(1,4-dioxane-2,3-dione), poly(1,3-dioxane-2-one), polyhydroxymethacrylates, polycyanoacrylates, poly-caprolactone dimethylacrylates, polycaprolactone butylacrylates, poly-g-ethylglutamate, multiblock polymers e. g. oligocaprolactone diol and oligodioxanone diol, polyglycolic acid trimethyl carbonates, poly(DTH-iminocarbonate), poly(DTE-co-DT-carbonate), poly(bisphenol A-iminocarbonate), polyesters, glycolated polyesters, polyesteramides, polyorthoesters, polytrimethyl carbonates, polyetherimines, poly[p-carboxyphenoxy)propane], polyether esters, polyalkenoxalates, copolymers of polyorthoesters, polytetrafluoroethylene (PTFE), polyvinyls, polyvinylides, polyvinylamines, polyvinylamides, polyvinyl ethers, polyvinyl chloride (PVC), polydimethylsiloxanes (PDMS), nylons, polyphosphoesters, polyhydroxypentanoic acid, polyacids, polyhydroxycarboxylic acids, polyacid esters, polyacid amines, polyacid amides, polyacrylic acids, polyacrylates, polymethyl methacrylates (PMMA), polybutyl methacrylates, polyacrylamides, polyacrylonitriles, polyamides, polyether amides, polyethylene amines, polyimides, polycarbonates, polycarbourethanes, polyvinyl ketones, polyvinyl halides, polyvinylidene halides, polyisobutylenes, polyvinylaromatics, polyvinyl esters, polyoxymethylene, polytetramethylene oxide, polyethylene, polypropylene, polyurethanes, soft polyurethanes, polyurethanes with amino acid residues in the backbone, polyetherurethanes, silicone polyetherurethanes, silicone polyurethanes, silicone polycarbonaturethanes, polyolefin elastomers, polyisobutylenes, polyvalerates, polyethylene terephthalates, polyetheretherketones, polyaryletheretherketones, lipids, fibrin, carrageenans, fibrinogen, starch, derivatives of starch, modified starch, collagen, protein-based polymers, polyamino acids, synthetic polyamino acids, zein, modified zein, polyhydroxyalkanoates, pectinic acid, actinic acid, hyaluronic acid, modified and unmodified fibrin and casein, carboxymethyl sulfates, albumin, heparan sulfate, heparin, chondroitin sulfate, dextrans, dextrins, α-cyclodextrin, β-cyclodextrin, γ-cyclodextrin, dimethyl-β-cyclodextrin, gum arabic, guar, collagen, collagen-N-hydroxysuccinimide, phospholipids, carboxymethyl chitosans, lanolin, gelatin, derivatives of gelatin, cellulose, derivatives of cellulose, modified cellulose, hydroxypropyl cellulose, ethyl cellulose, carboxymethyl cellulose, rayon, rayon triacetates, cellulose nitrates, cellulose acetates, cellulose butyrates, cellulose acetate butyrates, cellulose ethers, cellulose triacetates, ethyl vinyl acetate copolymers, polysulfones, epoxy resins, ABS resins, EPDM rubbers, agar, alginic acid, alginate, chicle, dammar, marshmallow extract, gellan (E 418), guar gum (E 412), gum arabic (E 414), gum from plantain seed husks, gum from spruce sap, locust bean gum (E 410), karaya (E 416), konjac flour (E 425), mastic, pectin, tara gum (E 417), tragacanth (E 413), xanthan gum (E 415), sago, shellac, silicones such as polysiloxanes, polydimethylsiloxanes, fluorosilicones, polyvinyl halogens, poly-para-xylylene (Parylene) such as Parylene N, Parylene C and/or Parylene D, as well as co-, block- or segment polymers and/or mixtures of the aforementioned polymers and derivatives thereof.

Examples of biocompatible polymers include, but are not limited to poly(esteramides), polyhydroxyalkanoates (PHA), poly(3-hydroxypropanoate), poly(3-hydroxybutyrate), poly(3-hydroxyvalerate), poly(3-hydroxyhexanoate), poly(3-hydroxyheptanoate), poly(3-hydroxyoctanoate), poly(4-hydroxybutyrate), poly(4-hydroxyvalerate), Poly(4-hydroxyhexanote), poly(4-hydroxyheptanoate), poly(4-hydroxyoctanoate), poly(dioxanone), poly(orthoester), poly(anhydride), poly(tyrosine carbonate), poly(tyrosine ester), poly(iminocarbonate), poly(glycolic acid-co-trimethylene carbonate), polyphosphoesters, polyphosphoesterurethanes, poly(amino acids), polycyanoacrylates, polyepoxyacrylates, polyurethane acrylates, Polyester acrylates, polyether acrylates, amine-modified polyether acrylates, polyacrylate acrylates, polycarbonate acrylates, polyepoxymethacrylates, polyurethane methacrylates, polyester methacrylates, polyether methacrylates, amine-modified polyether methacrylates, polyacrylate methacrylates, polycarbonate methacrylates, polyepoxyacrylamides, polyurethane acrylamides, polyester acrylamides, polyether acrylamides, amine-modified polyether acrylamides, polyacrylate acrylamides, polycarbonate acrylamides, polyepoxy methacrylamides, polyurethane methacrylamides, polyester methacrylamides, polyether methacrylamides, amine-modified polyether methacrylamides, polyacryl methacrylamides, polycarbonate methacrylamides, poly(trimethylene carbonate), poly(imino carbonate), polyurethanes, polyphosphazenes, silicones, polyesters, polyolefins, polyisobutylene, ethylene-alpha-olefin copolymers, acrylic polymers, acrylic copolymers, vinyl halide polymers, vinyl halide copolymers, polyvinyl chloride, polyvinyl ether, polyvinyl methyl ether, polyvinylidene halides, polyvinylidene chloride, polyacrylonitrile, polyvinyl ketones, polyvinyl aromatics, polystyrene, polyvinyl esters, polyvinyl acetate, ethylene-methyl methacrylate copolymers, acrylonitrile-styrene copolymers, ethylene-vinyl acetate copolymers, polyamides, polycaprolactam, polycarbonates, polyoxymethylenes, polyimides, polyethers, poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), polyurethanes, cellulose acetate, cellulose butyrate, cellulose acetate butyrate, cellophane, cellulose nitrate, cellulose propionate, cellulose ether, carboxymethyl cellulose, polyether, poly(ethylene glycol) (PEG), copoly(ether ester), polyalkylene oxides, poly(ethylene oxide), poloxamers, poloxamines, poly(propylene oxide), poly(ether ester), polyalkylene oxalates, polyphosphazenes, silane-modified polymers poly(aspirin), poly(styrene-isoprene-styrene)-PEG (SIS-PEG), polystyrene-PEG, polyisobutylene-PEG, Polycaprolactone-PEG (PCL-PEG), PLA-PEG, Poly(methyl methacrylate)-PEG (PMMA-PEG), polydimethylsiloxane-co-PEG (PDMS-PEG), Poly(vinylidene fluoride)-PEG (PVDF-PEG), (polypropylene oxide-co-polyethylene glycol), poly(tetra¬methylene glycol), hydroxy¬functionalized poly(vinylpyrrolidone), polypropylene oxide-co-polyethylene glycol, PEGylated heparin, glycosaminoglycan (GAG), GAG derivatives, polysaccharides, polymers and copolymers of hydroxyl-containing monomers such as HEMA, Hydroxypropyl methacrylate (HPMA), hydroxypropyl methacrylamide, PEG acrylate (PEGA), PEG methacrylate, 2-methacryloyloxyethylphosphorylcholine (MPC), N-vinylpyrrolidone (VP), polymers and copolymers of carboxylic acid-containing monomers such as methacrylic acid (MA), acrylic acid (AA), alkoxy methacrylate, alkoxy acrylate, 3-trimethylsilylpropyl methacrylate (TMSPMA), as well as co-, block- or segment polymers and/or mixtures of the aforementioned polymers and derivatives thereof.

Examples of biodegradable, or bioabsorbable polymers include, but are not limited to polyvalerolactones, poly-ε-decalactones, polylactonic acid, polyglycolic acid polylactides, polyglycolides, copolymers of polylactides and polyglycolides, poly ε-caprolactone, polyhydroxybutyric acid, polyhydroxybutyrates, polyhydroxyvalerates, Polyhydroxybutyrate-co-valerate, poly(1,4-dioxane-2,3-dione), poly(1,3-dioxane-2-one), poly-p-dioxanones, polyanhydrides such as polymaleic anhydrides, polyhydroxymethacrylates, fibrin, polycyanoacrylates, poly-b-maleic acid, polycaprolactone dimethylacrylates, polycaprolactone butyl acrylates, polypivotolactones, multiblock polymers such as those e.g., of oligocaprolactone diols and oligodioxanone diols, polyether ester multiblock polymers such as PEG and polybutylene terephthalate. Polyglycolic acid trimethyl carbonates, polycaprolactone glycolides, poly-g-ethylglutamate, poly(DTH-iminocarbonate), poly(DTE-co-DT-carbonate), poly(bisphenol A-iminocarbonate), polyorthoesters, polytrimethyl carbonates, polyglycolic acid trimethyl carbonates, polyiminocarbonates, poly(N-vinyl)-pyrolidone, polyvinyl alcohols, polyorthoesters and their copolymers, lipids, carrageenans, fibrinogen, starch, collagen, protein-based polymers, polyamino acids, synthetic polyamino acids, zein, modified zein, polyhydroxyalkanoates, pectinic acid, actinic acid, modified and unmodified fibrin and casein, carboxymethyl sulfate, albumin, furthermore hyaluronic acid, heparan sulfate, heparin, chondroitin sulfate, dextran, β-cyclodextrins, and copolymers with PEG and polypropylene glycol, gum arabic, guar, gelatin, collagen, collagen-N-hydroxysuccinimide, lipids, phospholipids, modifications and copolymers and/or mixtures of the aforementioned.

Examples of biostable polymers or extremely slowly degradable polymers include, but are not limited to polyacrylic acid, polyacrylates such as polymethylmethacrylate, polybutylmethacrylate, polyacrylamide, polyacrylonitrile, polyamides, polyetheramides, polyethyleneamine, polyimides, polycarbonates, polycarbourethanes, polyvinylketones, polyvinylhalides, polyvinylidenehalides, polyvinylethers, polyisobutylenes, polyvinyl aromatics, polyvinyl esters, polyvinyl pyrollidones, polyoxymethylenes, polytetramethylene oxide, polyethylene, polypropylene, polytetrafluoroethylene, polyurethanes, polyetherurethanes silicone-polyetherurethanes, silicone-polyurethanes, silicone-polycarbonate urethanes, polyolefin elastomers, polyisobutylenes, EPDM rubbers, fluorosilicones, carboxymethyl chitosans, polyarylether ether ketones, polyether ether ketones, polyethylene terephthalate, polyvalerates, carboxymethyl cellulose, cellulose, Rayon, Rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halogens and copolymers, cellulose ethers, cellulose triacetates,chitosans and copolymers and/or mixtures of these substances.

Examples of hydrophilic polymers include, but are not limited to, polyvinyl alcohol (PVA), polyethylene oxide (polyethylene glycol (PEG)), poly-*N*-vinyl-pyrrolidone, poly-*N*-vinyl-2-piperidone, poly-*N*-vinyl-2-caprolactam, poly-*N*-vinyl-3-methyl-2-caprolactam, poly-*N*-vinyl-3-methyl-2-piperidone, poly-*N*-vinyl-4-methyl-2-piperidone, poly-*N*-vinyl-4-methyl-2-caprolactam, poly-*N*-vinyl-3-ethyl-2-pyrrolidone, poly-*N*-vinyl-4,5-dimethyl-2-pyrrolidone, polyvinyl imidazole, poly-N-dimethyl acrylamide, polyacrylic acid, poly-2-ethyloxazoline, heparin polysaccharides, polysaccharides, polyoxyethylene derivatives as well as co-, block- or segment polymers thereof and derivatives thereof.

Examples of polymers obtainable through non-radical polymerization include, but are not limited to, polyvinyl alcohols, polyethers, polyorthoesters, polyetherimines, polyetheramines, polyurethanes, polyhydroxycarboxylic acids, polylactams, polyethylene glycols, polyethylene oxides, polyethylene imines, polyitaconic acids, polymesaconic acids, polyglutaconic acids, polycitraconic acids, polyaconitic acids, polyisocitronic acids, polyoxaloacetic acids, polymalic acids, polylactides, glucosaminoglycans, polyglycolides, cellulose, polyoxazolines, peptides, polysaccharides, polyelectrolytes, polyacetals, polysiloxanes, polymethylene malonic acids, as well as co-, block- or segment polymers thereof and derivatives thereof. Non-radical polymerization also yields polyethylene glycols, polyethylene oxides, polypropylene glycols or polyurethanes. Polyurethanes, for example, can be synthesized from diols and diisocyanates via polyaddition reaction. Polyethylene glycols / polyethylene oxides can be synthesized from ethylene oxide by means of alkaline catalysis.

A polymer may further comprise photosensitive and/or thermosensitive groups and may represent a photosensitive and/or thermosensitive polymer that can be activated via irradiation and/or heat and can bind to surfaces, for example, with the formation of covalent bonds.

### Hydrophilic Polymer

The term **"hydrophilic polymer",** as used herein, refers to a polymer having a partition coefficient between n-octanol and water of T_{ow} < 6.30 (log T_{ow} < 0.80), preferably T_{ow} < 1.80 (log T_{ow} < 0.26), more preferably T_{ow} < 0.63 (log T_{ow} < -0.20), and even more preferably T_{ow} < 0.40 (log T_{ow} < -0.40). A **"hydrophilic monomer"** is herein preferably a monomer with a solubility in water of more than 80 g/l, in particular of more than 100 g/l at 25° C and 1 bar.

### Hydrophobic Polymer

The term **"hydrophobic polymer"** or "lipophilic polymer", as used herein, refers to a polymer having a partition coefficient between n-butanol and water of ≥0.5, preferably ≥0.7, more preferably ≥0.9, and particularly preferably ≥1.1. A **"hydrophobic monomer"** as used herein is preferably a monomer having a solubility in water of not more than 80 g/l, particularly not more than 50 g/l at 25° C and 1 bar.

### Polymer Chain

The term **"polymer chain",** as used herein, refers to an unbranched or branched chain of a plurality of covalently linked monomers. In the production of polymers, many monomers are covalently linked to form a polymer chain consisting of repeating structural units (monomer moieties). Thus, polymers represent macromolecules composed of repeating, identical or different structural units (monomer moieties). An unbranched or linear polymer is a polymer that consists of one polymer chain, the main chain. Linear polymers are formed when the monomers can only be linked to two adjacent monomers. An example of a linear polymer is polyvinylpyrrolidone (PVP). A branched polymer has additional branching points in the polymer chain, via which a side chain can be formed in the polymer chain. For example, to prepare a branched polymer, a monomer can be used that has more than one chemically reactive group, such as a tri-, quaternary or polyfunctional monomer.

### Degree of Polymerization

The term **"degree of polymerization",** as used herein, refers to the number of covalently linked monomers in the polymer chain. Macromolecules having a degree of polymerization of at least 10 or macromolecules having a polymer chain of at least 10 covalently linked monomers are herein referred to as a polymer.

### Monomer Moiety

The term **"monomer moiety",** as used herein, refers to a covalently bound monomer incorporated in a polymer chain. Thus, the term "monomer moiety", refers to a structural unit of a polymer chain. Thus, a polymer chain or a polymer consists of many monomer moieties linked together through covalent bonds. If the monomer is an unsaturated organic compound of the general formula R-CH=CH₂, wherein R is any arbitrary residue, the monomer moiety has the following structure:

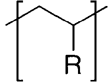

### Polymerization

The term **"polymerization"** or "polymerization reaction," as used herein, refers to a chemical reaction in which similar or different monomers are reacted to form polymers. Thus, in a polymerization reaction, linear, branched or crosslinked polymers are formed by successive reactions of monomers or oligomers. Herein, unsaturated organic compounds containing at least one double bond are preferably used as monomers:

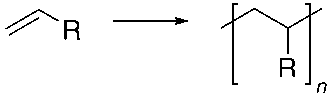

### Homopolymer and Homopolymerization

The term **"homopolymerization",** as used herein, refers to a polymerization, wherein only one type of monomer is used to produce a polymer. Thus, homopolymerization yields a polymer consisting of only one kind of monomer moiety. A polymer composed of the same monomer moieties is also referred to herein as a "homopolymer".

### Copolymer, Main Monomer, Comonomer and Copolymerization

The term **"copolymerization",** as used herein, refers to a polymerization. wherein two or more different monomers are used to produce a polymer. Thus, copolymerization yields a polymer, wherein the polymer chain is composed of different monomer moieties. A polymer that is built up from two or more different monomer moieties is therefore herein referred to as a **"copolymer".** The two or more different monomers may be present in the copolymer in equimolar ratio or in some other ratio. Generally, the monomer that primarily determines the physical and mechanical properties of the resulting copolymer is referred to as the **"main monomer".** The term **"comonomer",** as used herein, refers to a (second) monomer used in a copolymerization in addition to the main monomer. Copolymerization is commonly used in the prior art to tailor the physical and mechanical properties of a homopolymer to specific requirements, e.g., to control wetting properties or improve solubility. In this case, only small amounts or even substantial amounts of a comonomer can also be used. One advantage in using comonomers is that comonomers are also incorporated into the polymer chain and are covalently bound in the polymer and thus cannot be leached out. Comonomers can also be used to achieve crosslinking in order to obtain a crosslinked polymer. Since a copolymer consists of at least two types of monomers, copolymers can be classified according to how these monomers are arranged along the polymer chain. Linear copolymers consist of a single main chain and comprise alternating copolymers, random copolymers and block copolymers. Branched copolymers consist of a main chain with one or more polymeric side chains and may be grafted.

### Random Copolymer

The term **"random copolymer",** as used herein, refers to a copolymer in which the distribution of the main monomer and comonomer in the chain follows a statistical distribution. If the ratio of monomers in a section corresponds to the molar ratio, the distribution is said to be random.

### Block Copolymer

The term **"block copolymer",** as used herein, refers to a copolymer consisting of longer segments or blocks of each of two or more different monomers.

### Graft Copolymer

The term **"graft copolymer",** as used herein, refers to a copolymer in which polymer chains of a monomer are grafted onto the backbone of a polymer or copolymer.

### Alternating Copolymer

The term **"alternating copolymer",** as used herein, refers to a copolymer in which the two monomers are arranged alternately.

### Blocks

The term **"blocks",** as used herein, refers to segments in a polymer chain of a copolymer that are composed of only one type of a monomer and therefore have the structural characteristics of the homopolymer of said monomer. Herein, blocks preferably consist of at least two, more preferably at least three, further preferably at least four, even more preferably at least five, further preferably at least six, even more preferably at least seven, and further preferably at least eight identical monomers. A copolymer composed of blocks of monomer moieties of a first monomer divided by a second monomer moiety differs from an ordinary block polymer in that the polymer chain contains only blocks of the first monomer and no blocks of the second monomer are present. Thus, in a copolymer composed of blocks of a first monomer divided by a second monomer moiety, the second monomer moiety has only covalent linkages to the monomer moieties of the first monomer and no directly covalently linked moieties of the second monomer are present in the polymer chain. The first monomer is referred to herein as the main monomer and the second monomer is referred to as the comonomer. It should be understood that the size of the blocks, and thus the number of the main monomer in a block, also depends on the amount of comonomers in the polymer chain, i.e. the smaller the amount of comonomers the larger the number of the main monomer in the blocks. Thus, the size of the blocks decreases as more comonomer is used in the copolymerization. If the main monomer and the comonomer are ultimately present in a ratio of 1:1, an alternating copolymer is obtained in which the main monomer and the comonomer are arranged alternately in the polymer chain. In such an alternating copolymer, the blocks therefore consist of only one monomer moiety of the main monomer, but there are still no directly covalently linked comonomers in the polymer chain. It should be further understood that the size of the blocks, and thus the number of blocks of the main monomer of a block, also depends on the method of preparation of the copolymer. If the copolymer is prepared by copolymerization starting from the at least two monomers, the incorporation of the comonomer into the polymer chain can be statistically or random. Thereby, different sizes of the blocks are formed, depending on the number of blocks of the main monomer already incorporated after which a covalent linkage with a comonomer occurs. Preferred herein is that the blocks have substantially an equal number of blocks of the main monomer, that is, substantially equal sized blocks. Another possibility is to provide polymer chains of blocks of the main monomer already during copolymerization instead of the main monomer itself, i.e. oligomers of the main monomer. Chain extension can then be achieved by covalent linkage with a comonomer or with a further oligomer of the main monomer. This approach is advantageous in that a minimum number of blocks of the main monomer can be ensured in the blocks of the copolymer.

### Molecular Colloid

The term **"molecular colloid",** as used herein, generally refers to substances that are thermodynamically stable, hydrophilic systems whose particle size is determined by the linkage of 10³ to 10⁹ covalently bound atoms, preferably 10⁴ to 10⁷ covalently bound atoms. The colloid particles in the solutions of macromolecular compounds (e.g. polymers) are called molecular colloids. The molecular colloids are identical with the macromolecule (e.g. the polymer), whose structural units are connected by covalent bonds, wherein macromolecular compounds are compounds whose characteristic properties are essentially determined by the extraordinary size or length of their chain-like molecules, the molecular weights of most natural and synthetic compounds being between 10³ to 10⁹, preferably 10⁴ and 10⁷, wherein these macromolecular compounds give colloidal solutions in which the colloidal particles are generally identical with the individual macromolecules.

### Molecular Colloid Network

The term **"molecular colloid network",** as used herein, can be formed from a molecular colloid and a crosslinker through covalent bonds, i.e. via crosslinking. The bonds of the molecular colloid network in the solid phase are covalent bonds.Thus, "molecular colloid networks" refer to molecular colloids whose macromolecules or colloid particles are covalently bound to one another inter- and intramolecularly via bi- or multifunctional compounds (crosslinkers). Polymers suitable for preparing the molecular colloid network of the present invention include homopolymers as well as mixed polymers or copolymers. Crosslinking can be achieved either directly during the assembly of the polymers or by reactions on preexisting polymers. A crosslinker and at least one molecular colloid (i.e. polymer) are preferably crosslinked and together form the molecular colloid network.

### Coefficient of Friction

The term **"coefficient of friction",** as used herein, is also called the frictional coefficient and is also abbreviated herein as CoF. The coefficient of friction indicates the ratio of the frictional force to the contact force between two bodies. In sliding friction, the friction surfaces move relative to each other. Sliding friction occurs at the contact surfaces between bodies that move relative to each other. The "coefficient of friction", as used herein, therefore herein refers to the coefficient of sliding friction *µ*_{G}.

### Alkyl

The term **"alkyl",** as used herein, refers to saturated hydrocarbon groups having from 1 to 10 carbon atoms, either straight or branched, preferably having from 1 to 8 carbon atoms, and more preferably having from 1 to 6 carbon atoms. An alkyl having a certain number of carbon atoms is referred to as e.g. "C₁-C₈-alkyl" with -CH₃, -C₂H₅, -C₃H₇, -C₄H₉, -C₅H₁₁, -C₆H₁₃, -C₇H₁₅, and -C₈H₁₇, or " linear C₁-C₆-alkyl" with -CH₃, -CH₂CH₃, -CH₂CH₂CH₃, -CH₂CH₂CH₂CH₃, -CH₂CH₂CH₂CH₂CH₃, and -CH₂CH₂CH₂CH₂CH₂CH₃; or a "branched C₁-C₆ alkyl" with -CH(CH₃)₂, -CH₂CH(CH₃)₂, -CH(CH₃)C₂H₅, -C(CH₃)₃, -CH(CH₃)C₃H₇, -CH₂CH(CH₃)C₂H₅, -CH(CH₃)CH(CH₃)₂, -C(CH₃)₂C₂H₅, -CH₂C(CH₃)₃, -CH(C₂H₅)₂, -C₂H₄CH(CH₃)₂, -C₃H₆-CH(CH₃)₂, -C₂H₄CH(CH₃)C₂H₅, -CH(CH₃)C₄H₉, -CH₂CH(CH₃)-C₃H₇, -CH(CH₃)CH₂CH(CH₃)₂, -C(CH₃)₂C₃H₇, -CH(CH₃)CH(CH₃)C₂H₅, -CH₂-CH(CH₃)-CH(CH₃)₂, -CH₂-C(CH₃)₂C₂H₅, -CH(CH₃)C(CH₃)₃, -C(CH₃)₂CH(CH₃)₂, and -C₂H₄C(CH₃)₃.

### Alkylene

The term **"alkylene",** as used herein, refers to a straight or branched divalent hydrocarbon residue having 1 to 10 carbon atoms, preferably 1 to 8 carbon atoms, and more preferably 1 to 6 carbon atoms.

### Alkenyl

The term **"alkenyl",** as used herein, refers to groups having from 2 to 10 carbon atoms, preferably from 2 to 6 carbon atoms (C₂-C₆ alkenyl), which are either straight or branched and contain at least one double bond, but optionally more than one double bond. Examples of "straight or branched C₂-C₈ -alkenyl" include, but are not limited to: -CH=CH₂, -CH₂CH=CH₂, -C(CH₃)=CH₂, -CH=CHCH₃, -C₂H₄CH=CH₂, -CH₂CH=CH-CH₃, -CH=CHC₂H₅, -CH=C(CH₃)₂, -CH₂C(CH₃)=CH₂, -CH(CH₃)CH=CH, -C(CH₃)=CHCH₃, -CH=CHCH=CH₂, -C₃H₆CH=CH₂, -C₂H₄CH=CHCH₃, -CH₂CH=CHC₂H₅, -CH=CHC₃H₇, -CH₂CH=CHCH=CH₂, -CH=CHCH=CHCH₃, -C₂H₄CH=CHCH₃, -CH₂CH=CHC₂H₅, -CH₂CH=CHCH=CH₂, -CH=CHCH=CHCH₃, -CH=CHCH₂CH=CH₂, -C(CH₃)=CHCH=CH₂, -CH=C(CH₃)CH=CH₂, -CH=CHC(CH₃)=CH₂, -CH₂CH=C(CH₃)₂, -C(CH₃)=C(CH₃)₂, -C₂H₄CH=CH₂, -CH=CHC₂H₅, -CH=C(CH₃)₂, -CH₂CH=CH-CH₃, -CH=CHCH=CH₂, C₃H₆-CH=CH₂, -CH=CHC₃H₇, -C₄H₈CH=CH₂, -CH=CHC₄H₉, -C₃H₆CH=CHCH₃, -CH₂CH=CHC₃H₇, -C₂H₄CH=CHC₂H₅, -CH₂C(CH₃)=C(CH₃)₂, -C₂H₄CH=C(CH₃)₂, and -CH=CHPh.

### Alkenylene

The term **"alkenylene",** as used herein, refers to a straight or branched divalent hydrocarbon residue having from 2 to 12 carbon atoms and one or more carbon-carbon double bonds, preferably from 2 to 8 carbon atoms and more preferably from 2 to 6 carbon atoms.

### Alkynyl

The term **"alkynyl",** as used herein, refers to groups having from 2 to 10 carbon atoms, preferably from 2 to 8 carbon atoms, which are either straight or branched and contain at least one triple bond, but optionally more than one triple bond, and additionally optionally have one or more double bonds. Examples of "straight or branched C₂-C₈ alkynyl" include, but are not limited to: -CH₂C≡CH, -C=CH, -C≡CCH₃, -C₂H₄C≡CH, -C≡C-C₂H₅, -CH₂-C≡CCH₃, -C≡CCH=CH₂, -CH=CH-C=CH, -C≡CC≡CH, -C₃H₆C≡CH, -C≡CC₃H₇, -C₂H₄C≡CCH₃, -CH₂C≡CC₂H₅, -CH₂C≡CCH=CH₂, -CH₂CH=CHC≡CH, -CH₂C≡CC≡CH, -C≡CCH=CHCH₃, -CH=CHC≡CCH₃, -C≡CC≡CCH₃, -C≡CCH₂CH=CH₂, -CH=CHCH₂C≡CH, -C≡CCH₂C≡CH, -C(CH₃)=CHCH=CH₂, -CH=C(CH₃)CH=CH₂, -CH=CHC(CH₃)=CH₂, -C(CH₃)=CHC≡CH, -CH=C(CH₃)C≡CH, -C≡CC(CH₃)=CH₂, -C₄H₈C≡CH, -C≡CC₄H₉, -C₃H₆C≡CCH₃, -CH₂C≡CC₃H₇, and -C=CPh.

### Cycloalkyl

The term **"cycloalkyl",** as used herein, refers to cyclic alkyl groups having 3 to 12 carbon atoms, preferably 3 to 8 carbon atoms, having a single cyclic ring or multiple fused rings that may optionally be substituted with 1 to 3 alkyl groups. Examples of cycloalkyl groups include, but are not limited to, single ring structures such as cyclopropyl, cyclobutyl, cyclopentyl, cyclooctyl, 1-methylcyclopropyl, 2-methylcyclopentyl, 2-methylcyclooctyl, and the like, or multiple ring structures, including bridged ring systems, such as adamantyl. Examples of "C₃-C₈ cycloalkyl" include, but are not limited to, cyclo-C₃H₅, cyclo-C₄H₇, cyclo-C₅H₉, cyclo-C₆H₁₁, cyclo-C₇H₁₃, and cyclo-C₈H₁₅.

### Aryl. Diaryl, Arylalkyl

The term **"aryl",** as used herein, refers to an unsaturated aromatic carbocyclic group having 6 to 12 carbon atoms and having a single ring (e.g. phenyl) or multiple fused rings (e.g. naphthyl or anthryl). Examples of aryls include phenyl, pyridyl, naphthyl, and the like. The term **"diaryl",** as used herein, refers to an unsaturated aromatic carbocyclic group having at least 6 to 12 carbon atoms and having more than one ring, that are not fused e.g. diaryl compounds such as benzophenone or derivatives thereof.

The term **"arylalkyl",** as used herein, refers to an alkyl substituted with an aryl, i.e. arylalkyl groups preferably having 1 to 6 carbon atoms in the alkyl moiety and 6 to 12 carbon atoms in the aryl moiety. Such arylalkyl groups include benzyl, phenylethyl and the like. Examples of "C₆-C₈ arylalkyl" include, but are not limited to -CH₂Ph and -C₂H₄Ph. The term **"diarylalkyl",** as used herein, refers to an alkyl substituted with a diaryl, i.e. diarylalkyl groups preferably having 1 to 6 carbon atoms in the alkyl moiety and at least 6 to 12 carbon atoms in the diaryl moiety. Such diarylalkyl groups include benzoylbenzyl and the like.

### Oxy, Alkoxy, Aryloxy

The term "oxy", as used herein, refers to a divalent group -O-, which may have various substituents to form various oxy groups, including ethers and esters. The term **"alkoxy"** or "alkyloxy" as used herein are used interchangeably and refer to the group -OR^{α}, wherein R^{α} is an alkyl group, including optionally substituted alkyl groups. **"Aryloxy"** as used herein refers to the group -OR^{α}, wherein R^{α} is an aryl group, including optionally substituted aryl groups.

### Carbonyl

The term **"carbonyl",** as used herein, refers to the chemical group -C(O)- or C=O. Substituted carbonyl refers to the group R^{α}-C(O)-R^{α}, wherein each R^{α} is independently of each other selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical substituted carbonyl groups include acids, ketones, aldehydes, amides, esters, acyl halides, thioesters and the like.

### Amino, Aminoalkyl, Aminoaryl, Aminoarylalkyl, Aminocarbonyl, Aminocarbonylalkyl

The term **"amino",** as used herein, refers to the group -NH₂. Substituted amino refers to the group -NHR^{α}, NR^{α}R^{α}, and NR^{α}R^{α}R^{α}, wherein each R^{α} is independently of each other selected from optionally substituted alkyl, cycloalkyl, cycloheteroalkyl, alkoxy, carboxy, aryl, aryloxy, heteroaryl, heteroarylalkyl, acyl, alkoxycarbonyl, sulfanyl, sulfinyl, sulfonyl, and the like. Typical amino groups include dimethylamino, diethylamino, trimethylammonium, triethylammonium, methylysulfonylamino, furanyl-oxy-sulfamino and others. The term **"aminoalkyl",** as used herein, refers to an alkyl group in which one or more hydrogen atoms are replaced by an amino group, including a substituted amino group. The term **"aminoaryl",** as used herein, refers to an aryl group in which one or more hydrogen atoms are replaced by an amino group, including a substituted amino group. The term "**aminoarylalkyl**", as used herein, refers to an alkylaryl group in which one or more hydrogen atoms are replaced by an amino group, including a substituted amino group. The term **"aminocarbonyl",** as used herein, refers to a carbonyl group substituted with an amino group, including a substituted amino group as defined herein, and includes amides. The term **"aminocarbonylalkyl"** refers to an alkyl substituted with an aminocarbonyl group.

### Halogen, Haloalkyl

The term **"halogen",** as used herein, refers to fluorine, chlorine, bromine and iodine. The term **"haloalkyl",** as used herein, refers to an alkyl group in which one or more hydrogen atoms are replaced by a halogen. Thus, the term haloalkyl includes monohaloalkyl, dihaloalkyl, trihaloalkyl, etc., up to perhaloalkyl. The term "C₁-C₂ haloalkyl" comprises 1-fluoromethyl, difluoromethyl, trifluoromethyl, 1-fluoroethyl, 1,1-difluoroethyl, 1,2-difluoroethyl, 1,1,1-trifluoroethyl, perfluoroethyl, etc..

### Hydroxy, Hydroxyalkyl, Hydroxyaryl, Alkyaryloxy

The term **"hydroxy",** as used herein, refers to -OH. The term **"hydroxyalkyl",** as used herein, refers to an alkyl substituted with one or more hydroxy groups. The term **"hydroxyaryl",** as used herein, refers to an aryl substituted with one or more hydroxy groups. The term **"alkylaryloxy",** as used herein, refers to an arylalkyl substituted with one or more hydroxy groups.

### Heteroalkyl, Heteroalkenyl, Heteroalkynyl, Heteroaryl, Heteroarylalkyl, Heterocycloalkyl

The terms **"heteroalkyl", "heteroalkenyl",** and **"heteroalkynyl",** as used herein, refer to alkyl, alkenyl, and alkynyl residues, as defined herein, in which one or more carbon atoms are each independently replaced by the same or different heteroatoms or heteroatomic groups. Heteroatoms and/or heteroatomic groups that may replace the carbon atoms include, but are not limited to, -O-, -S-, -S-O-, -NR^{α}-, -PH-, -S(O)-, -S(O)₂-, -S(O)NR^{α}-, -S(O)₂NR^{α}-, and the like, including combinations thereof, wherein each R^{α} is independently selected from hydrogen, alkyl, heteroalkyl, cycloalkyl, heterocycloalkyl, aryl, and heteroaryl. The term **"heteroaryl"** refers to an aromatic heterocyclic group having 1 to 10 carbon atoms and 1 to 4 heteroatoms selected from oxygen, nitrogen and sulfur within the ring. Such heteroaryl groups may have a single ring (e.g., pyridyl or furyl) or multiple fused rings (e.g., indolizinyl or benzothienyl). The term "C₁-C₁₀ heteroaryl" refers to aromatic residues having one or more heteroatoms such as O, S, N. The term **"heteroarylalkyl"** refers to an alkyl substituted with a heteroaryl, i.e. heteroarylalkyl groups preferably having 1 to 6 carbon atoms in the alkyl moiety and 5 to 12 ring atoms in the heteroaryl moiety. Such heteroarylalkyl groups include pyridylmethyl and the like. The term **"heterocycloalkyl"** refers to a saturated or unsaturated group having a single ring or multiple fused rings, 2 to 9 carbon ring atoms and 1 to 4 hetero ring atoms selected from nitrogen, sulfur or oxygen within the ring. Such heterocyclic groups may have a single ring (e.g., piperidinyl or tetrahydrofuryl) or multiple fused rings (e.g., indolinyl, dihydrobenzofuran, or cuinuclidinyl). Examples of heterocycles include, but are not limited to, furan, thiophene, thiazole, oxazole, pyrrole, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, pyrrolidine, indoline and the like.

### Carboxy Group, Carboxylic Acid, Carboxylic Acid Ester

The term **"carboxy group",** as used herein, refers to the functional group -COOH. Organic compounds having one or more carboxy groups (-COOH) are referred to as **"carboxylic acids".** Carboxylic acids have the general formula R^{α}-COOH, wherein the residue R^{α} may represent a hydrogen atom or an organic residue. A carboxylic acid with only one carboxy group is referred to herein as a "monocarboxylic acid" and a carboxylic acid with two carboxy groups is referred to herein as a "dicarboxylic acid". Examples of carboxylic acids having one or more carboxy groups include, but are not limited to, acetic acid, citric acid, acrylic acid, benzoic acid, fumaric acid, maleic acid, oleic acid and salicylic acid. **"Carboxylic acid esters"** are esters of carboxylic acids with the functional group -COOR^{β}, wherein R^{β} represents an organic residue derived from an alcohol or a phenol. Therefore, carboxylic acid esters have the general formula R^{α}-COO-R^{β}. Examples of carboxylic acid esters include, but are not limited to, alkyl acetates such as ethyl ethanoate (ethyl acetate), triglycerides, lactones such as γ-butyrolactone and also polyesters such as polyethylene terephthalate (PET). The formula **(-COO-),** as used herein, is used to represent a carboxy group, which may be either a carboxyl group (-COOH) or an ester group (-COOR^{β}), wherein R^{β} represents an organic residue. Thus, the formula **(-COO-)** may be also used herein when a carboxy group may be optionally functionalized.

As used herein, -OCO- represents 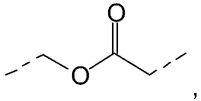 and -COO- represents 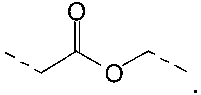 As used herein, a dashed line indicates connectivity and a solid line represents a bond.

### Carbamoyl Group, Carboxylic Acid Amides

The term **"carbamoyl group",** as used herein, refers to the functional group -CONH₂. Organic compounds having one or more carbamoyl groups (-CONH₂) are referred to as **"carboxylic acid amides"** in organic chemistry. Carboxylic acid amides are therefore derived from carboxylic acids. Carboxylic acid amides generally have the general formula R^{α}-CONH₂, wherein the residue R^{α} may represent a hydrogen atom or an organic residue. Carboxylic acid amides with the functional group -CONH₂ are also referred to as primary carboxylic acid amides. If one or both hydrogen atoms on the nitrogen are replaced by an organic residue, secondary carboxylic acid amides of the general formula R^{α}-CONHR^{β} or tertiary carboxylic acid amides of the general formula R^{α}-CONR^{β}R^{β'} are obtained, wherein the residues R^{β} and R^{β'} represent organic residues. Carboxylic acid amides can be prepared by reacting reactive carboxylic acid derivatives, such as carboxylic acid chlorides or carboxylic acid anhydrides with ammonia or primary or secondary amines. In this context, a carboxylic acid amide with only one carbamoyl group is referred to as a "monocarboxylic acid amide" and a carboxylic acid amide with two carbamoyl groups is referred to as a "dicarboxylic acid amide". Examples of carboxylic acid amides having one or more carbamoyl groups include, but are not limited to, formamide, *N,*N-dimethylformamide (DMF), acetamide, ε-caprolactam, *N*-vinylpyrrolidone, urea, polyamides such as peptides, proteins, or synthetic polyamides such as polyhexamethylene adipic acid amide (nylon). The formula **(-CONH-),** as used herein, is used to represent a carbamoyl group, which may be either a primary carbamoyl group (-CONH₂) or a secondary carbamoyl group (-CONHR^{β}), wherein R^{β} represents an organic residue. Thus, the formula **(-CONH-)** may be also used herein when a carbamoyl group may be optionally functionalized with an organic residue.

Herein, the functional group -NHCO- represents 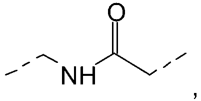 and -CONH-represents 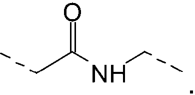 As used herein, a dashed line indicates connectivity and a solid line represents a bond.

### Carboxylic Acid Derivate

The term **"carboxylic acid derivative",** as used herein, refers to an organic compound having one or more functional groups formally derived from the carboxy group -COOH. In particular, the term carboxylic acid derivative, as used herein, refers to carboxylic acid esters of the general formula R^{α}-COO-R^{β}, primary carboxylic acid amides of the general formula R^{α}-CONH₂ and secondary carboxylic acid amides of the general R^{α}-CONH-Rβ, wherein the residues R^{α} and R^{β} represent organic residues. Particularly preferably, the term carboxylic acid derivative herein refers to carboxylic acid esters of the general formula R^{α}-COO-R^{β} and secondary carboxylic acid amides of the general formula R^{α}-CONH-R^{β}, wherein the residues R^{α} and R^{β} represent organic residues. In an organic compound with more than one carboxy group, one or more of these carboxy groups may be present as ester or amide. If a dicarboxylic acid is present, one or both carboxy groups may be functionalized with an organic residue or may have one or two carbamoyl groups instead of the carboxy groups.

### Carbamate Group

The term **"carbamate group",** as used herein, refers to the functional group -NHCOO-, or -OCONH-. Carbamates which are formally derived from carbamic acid (NH2COOH) generally have the general formula R^{β}R^{β'}NCOOR^{α}, wherein the residues R^{α}, R^{β} and R^{β'}represent organic residues.

Herein, the functional group -NHCOO- represents 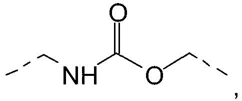 and -OCONH- represents 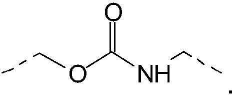 As used herein, a dashed line indicates connectivity and a solid line represents a bond.

### Urea Group

The term **"urea group",** as used herein, refers to the functional group -NHCONH-. Urea is an organic compound with chemical formula CO(NH₂)₂. This amide has two amino groups (-NH₂) joined by a carbonyl functional group (-C(=O)-). Ureas refer to a class of chemical compounds that share the same functional group, a carbonyl group attached to two organic amine residues: R^{α}R^{α'}N-C(=O)-NR^{β}R^{β'}, wherein the organic residues R^{α}, R^{α'}, R^{β} and R^{β'} are hydrogen, or represent organic residues.

Herein, the functional group -NHCONH- represents 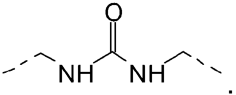 As used herein, a dashed line indicates connectivity and a solid line represents a bond.

### Carbonate Ester Group

The term **"carbonate ester group",** as used herein, refers to the functional group -OCOO-. Carbonate esters are esters of carbonic acid. This functional group consists of a carbonyl group flanked by two alkoxy groups. The general structure of these carbonates is R^{α}-O-C(=O)-O-R^{β}, wherein the residues R^{α} and R^{β} represent organic residues.

Herein, the functional group -OCOO- represents 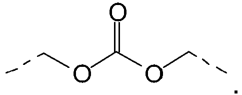 As used herein, a dashed line indicates connectivity and a solid line represents a bond.

### Organometallic Group

The term **"organometallic group",** as used herein, refers to chemical groups containing at least one carbon atom and at least one metal or electropositive element atom bound via a more or less polar covalent bond. In this context, the organic group can either be bound to the metal or element via a single, double or even triple bond, or can be linked multiple times with the metal or element atom.

### Silyl Group

The term **"silyl group",** as used herein, refers to chemical groups containing at least one carbon atom and at least one silicium atom bound via a more or less polar covalent bond. In this context, the silicium atom can either be bound to an oxygen atom. The term "silyl group" refers to the chemical group -SiR₃, wherein R represents hydrogen or an organic residue, e.g. -CH₃. For example, silyl ethers are a group of chemical compounds which contain a silicon atom covalently bonded to an alkoxy group. The general structure is R₁R₂R₃Si-O-R₄ wherein R₄ is an alkyl group or an aryl group.

### Optionally Substituted

The term **"optionally substituted",** as used herein, with respect to the aforementioned chemical groups means that the positions of the chemical group occupied by hydrogen may be substituted by another atom, such as carbon, oxygen, nitrogen or sulfur, or a chemical group, e.g. by hydroxy, oxo, nitro, methoxy, ethoxy, alkoxy, substituted alkoxy, trifluoromethoxy, haloalkoxy, fluorine, chlorine, bromine, iodine, halogen, methyl, ethyl, propyl, butyl, alkyl, alkenyl, alkynyl, substituted alkyl, trifluoromethyl, haloalkyl, hydroxyalkyl, alkoxyalkyl, thio, alkylthio, acyl, carboxy, alkoxycarbonyl, carboxamido, substituted carboxamido, alkylsulfonyl, alkylsulfinyl, alkylsulfonylamino, sulfonamido, substituted sulfonamido, cyano, amino, substituted amino, alkylamino, dialkylamino, aminoalkyl, acylamino, amidino, amidoximo, hydroxamoyl, phenyl, aryl, substituted aryl, aryloxy, arylalkyl, arylalkenyl, arylalkynyl, pyridyl, imidazolyl, heteroaryl, substituted heteroaryl, heteroaryloxy, heteroarylalkyl, heteroarylalkenyl, heteroarylalkynyl, cyclopropyl, cyclobutyl, cyclopentyl, cyclohexyl, cycloalkyl, cycloalkenyl, cycloalkylalkyl, substituted cycloalkyl, cycloalkyloxy, pyrrolidinyl, piperidinyl, morpholino, heterocyclic, (heterocyclyl)oxy, and (heterocyclyl)alkyl; wherein preferred heteroatoms are oxygen, nitrogen, and sulfur. However, it is clear to the person skilled in the art that the term "may be substituted" refers to the replacement of a hydrogen atom with any of the above chemical groups. The person skilled in the art understands that the term "optionally substituted" refers only to sterically useful and/or synthetically feasible chemical groups.

### Benzophenone

The term **"benzophenone",** as used herein, refers to an organic compound having the following structural formula:

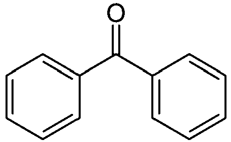

Benzophenone is often used as a photo-initiator for UV curing. Crosslinked polymer coatings or crosslinked materials can be prepared by adding benzophenone to a polymer composition and then irradiating the coating or material with UV light to effect crosslinking. Examples of benzophenones known in the prior art include, but are not limited to benzophenone, 4-phenylbenzophenone, 4-methoxybenzophenone, 4,4'-dimethoxybenzophenone, 4,4'-dimethylbenzophenone, 4,4'-dichlorobenzophenone, 4,4'-bis(di-methylamino)benzophenone, 4,4'-bis(diethylamino)benzophenone, 4,4'-bis(-methyl-ethylamino)benzophenone, 4,4'-bis(p-isopropylphenoxy)benzophenone, 4-methyl-benzophenone, 2,4,6-trimethylbenzophenone, 4-(4-methylthiophenyl)benzo-phenone, 3,3'-dimethyl-4-methoxybenzophenone, methyl-2-benzoylbenzoate, 4-(2-hydroxyethylthio)benzophenone, 4-(4-tolylthio)-benzophenone, 1-[4-(4-benzoyl-phenylsulfanyl)-phenyl]-2-methyl-2-(toluene-4-sulfonyl)-propan-1-one, 4-benzoyl-*N*,*N*,*N*-trimethylbenzene methanaminium chloride, 2-hydroxy-3-(4-benzoylphenoxy)-*N,N,N*-trimethyl-1-propanaminium chloride monohydrate, 4-(13-acryloyl1,4,7,10,13-pentaoxatridecyl)-benzophenone, 4-benzoyl-*N,N*-dimethyl-*N*-[2-(1-oxo-2-propenyl)oxy] ethylbenzene methanaminium chloride, benzophenone-3,3'-4,4'-tetracarboxylic acid dianhydride, 4-benzoylbiphenyl, 4,4'-bis(diethyl-amino)benzophenone, 4,4'-bis[2-(1-propenyl)phenoxy]benzophenone, 4-(diethyl-amino)benzophenone, 4,4'-dihydroxy benzophenone, 4-(dimethylamino)-benzophenone, 3,4-dimethylbenzophenone, 4-aminobenzophenone, 4,4'-di-aminobenzophenone, 3-hydroxybenzophenone, 4-hydroxybenzophenone, 4,4'-di-hydroxybenzophenone, 3,4-diaminobenzophenone, 2-methylbenzophenone, 3-methylbenzophenone, 4-methylbenzophenone and Michler ketone.

### Alkyl Linker

The term **"alkyl linker",** as used herein, refers to an alkylene capable of covalently linking two or more organic compounds. An "alkyl linker" represents a bifunctional chemical entity that binds a first compound to a second compound.

### C₁-C₅ Alkyl Linker

The term **"C₁-C₅ alkyl linker",** as used herein, preferably refers to a straight alkyl linker having 1 to 5 carbon atoms, i.e., a "C₁₋C₅ alkenylene". Particularly preferred C₁-C₅ alkyl linkers, as used herein, include, but are not limited to, methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

### Aromatic Linker

The term **"aromatic linker",** as used herein, refers to an aryl, diaryl or heteroaryl compound capable of covalently linking two or more organic compounds. An "aromatic linker" represents a bifunctional chemical entity that binds a first compound, herein a first benzophenone substituent, to a second compound, herein a second benzophenone substituent. An aromatic linker may have one or more substituents including, but not limited to -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, and -SOCH₃. Preferably aromatic linkes may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

### Benzophenone Substituent

The term **"benzophenone substituent",** as used herein, refers to a substituent present at positions of a chemical group occupied instead of hydrogen. Examples of benzophenone substituents include, but are not limited to benzoylphenyl 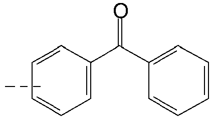 and benzoylbenzyl 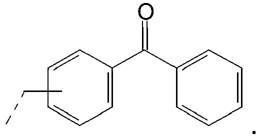 A benzophenone substituent may have one or more substituents including, but not limited to -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferably benzophenone substituent may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

### Benzophenone Linker

The term **"benzophenone linker",** as used herein, refers to a benzophenone molecule capable of covalently linking two or more organic compounds. A "benzophenone linker" represents a bifunctional chemical entity that binds a first compound, herein a first benzophenone substituent, to a second compound, herein a second benzophenone substituent. A benzophenone linker may have one or more substituents e.g. including, but not limited to -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferanbly a benzophenone linker may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

According to the present invention, benzophenone substituents, linkers such as aromatic linkers or aryl linkers, alkyl linkers, in particular benzophenone linkers may have one or more substituents selected from the group comprising or consisting of -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. Preferably, benzophenone substituents and benzophenone linkers may have one or more substituents selected from the group comprising or consisting of -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃. However, the benzophenone substituents or the benzophenone linker may also have substituents other than the substituents mentioned above. The skilled person is able to select further suitable substituents for benzophenone substituents or benzophenone linkers. Examples of substituents for benzophenone substituents or benzophenone linkers include, but are not limited to -F, -CI, -Br, -I, -CN, -N₃, -OCN, -NCO, -SCN, -NCS, -COOH, -COCN, -OH, -CHO, -NO₂, -NH₂, -COCH₃, -CONH₂, -SO₃H, -SO₂NH₂, -NHCONH₂, -NHC(=NH)NH₂, -OCONH₂ -CH₃ , alkyl substituents such as -CH₃, -C₂H₅, -C₃H₇, -CH(CH₃)₂, -cyclo-C₃H₅, -C₄H₉, -CH₂CH(CH₃)₂, -CH(CH₃)C₂H₅, -C(CH₃)₃, -cyclo-C₄H₇, -CH₂-cyclo-C₃H₅, -C₅H₁₁, -CH(CH₃)C₃H₇, -CH₂CH(CH₃)C₂H₅, -CH(CH₃)CH(CH₃)₂, -C(CH₃)₂-C₂H₅, -CH₂C(CH₃)₃, -CH(C₂H₅)₂, -C₂H₄CH(CH₃)₂, -cyclo-C₅H₉, -CH₂-cyclo-C₄H₇, -C₆H₁₃, -C₃H₆CH(CH₃)₂, -C₂H₄CH(CH₃)-C₂H₅, -CH(CH₃)C₄H₉, -CH₂CH(CH₃)-C₃H₇, -CH(CH₃)CH₂CH(CH₃)₂, -CH(CH₃)CH(CH₃)C₂H₅, -CH₂CH(CH₃)CH(CH₃)₂, -CH₂C(CH₃)₂C₂H₅, -C(CH₃)₂C₃H₇, -C(CH₃)₂CH(CH₃)₂, -C₂H₄C(CH₃)₃, -CH(CH₃)C(CH₃)₃, -cyclo-C₆H₁₁, -CH₂-cyclo-C₅H₉, -C₇H₁₅, -cyclo-C₇H₁₃, -CH₂-cyclo-C₆H₁₁, -C₈H₁₇, -cyclo-C₈H₁₅, haloalkyl substituents such as -CH₂F, -CHF₂, -CF₃, -CH₂Cl, -CH₂CH₂F, -CH₂CHF₂, -CH₂CF₃, -CH₂Br, -CH₂CH₂Cl, -CH₂CH₂Br, arylalkyl substituents such as -CH₂Ph, -Ph, -C₂H₄Ph, -PhPh, alkoxy substituents such as -OCH₃, -OC₂H₅, -OC₃H₇, -OCH(CH₃)₂, -OC₄H₉, -OCH₂CH(CH₃)₂, -CH(CH₃)C₂H₅, -OC(CH₃)₃, -OCH₂-cyclo-C₃H₅, -OC₅H₁₁, -OCH(CH₃)-C₃H₇, -OCH₂CH(CH₃)-C₂H₅, -OCH(CH₃)CH(CH₃)₂, -OC(CH₃)₂-C₂H₅, -OCH₂-C(CH₃)₃, -OCH(C₂H₅)₂, -OC₂H₄CH(CH₃)₂, -OCH₂-cyclo-C₄H₇, -OC₆H₁₃, -OC₃H₆CH(CH₃)₂, -OC₂H₄CH(CH₃)C₂H₅, -OCH(CH₃)C₄H₉, -OCH₂CH(CH₃)C₃H₇, -OCH(CH₃)CH₂CH(CH₃)₂, -OCH(CH₃)CH(CH₃)C₂H₅, -OCH₂CH(CH₃)CH(CH₃)₂, -OCH₂C(CH₃)₂C₂H₅, -OC(CH₃)₂C₃H₇, -OC(CH₃)₂CH(CH₃)₂, -OC₂H₄C(CH₃)₃, -OCH(CH₃)C(CH₃)₃, -OCH₂-cyclo-C₅H₉, -OC₇H₁₅, -OCH₂-cyclo-C₆H₁₁, -OC₈H₁₇, -OCF₃, -OC₂F₅, -OCH₂Ph, -OPh; -OC₂H₄Ph, -OC₂H₅OH, -OC₃H₇OH, -OC₄H₉OH, -OC₅H₁₁OH, -OC₆H₁₃OH, -OC₂H₅COOH, -OC₃H₇COOH, -OC₄H₉COOH, -OC₅H₁₁COOH, -OC₆H₁₃COOH, -OC₂H₅NH₂, -OC₃H₇ NH₂, -OC₄H₉NH₂, -OC₅H₁₁NH₂, -OC₆H₁₃NH₂, -OC₂H₅OC₂H₅OH, -OC₂H₅OC₂H₅OC₂H₅OH, -OC₂H₅OC₂H₅OC₂H₅OC₂H₅OH, alkylamine substituents such as -NHCH₃, -NHC₂H₅, -NHC₃H₇, -NHC₄H₉, -NH-cyclo-C₃H₅, -NH[CH(CH₃)₂], -NH[C(CH₃)₃], -N(CH₃)₂, -N(C₂H₅)₂, -N(C₃H₇)₂, -N(C₄H₉)₂, -N(cyclo-C₃H₅)₂, -N[CH(CH₃)₂]₂, -N[C(CH₃)₃]₂, ester substituents such as -COOC(CH₃)₃, -COOCH₃, -COOCH₂CH₃, -COOCH₂CH₂CH₃, -COOCH(CH₃)₂, -COOCH₂Ph, -OCOCH₃, -OCOC₂H₅, -OCOC₃H₇, -OCO-cyclo-C₃H₅, -OCOC₄H₉, -OCOCH(CH₃)₂, -OCOCH₂Ph, -OCOC(CH₃)₃, amide substituents such as -NHCOCH₃, -NHCOC₂H₅, -NHCOC₃H₇, -NHCOCH₃, -NHCOC₂H₅, -NHCOC₃H₇, -NHCOC₄H₉, -NHCO-cyclo-C₃H₅, -NHCO[CH(CH₃)₂], -NHCO[C(CH₃)₃], sulfonic substituents such as -SO₂CH₃, -SO₂C₂H₅, -SO₂C₃H₇, -SO₂Ph, -SO₂-cyclo-C₃H₅, -SO₂CH(CH₃)₂, -SO₂C₄H₉, -SO₂C(CH₃)₃, sulfonamide substituents such as -NHSO₂CH₃, -NHSO₂C₂H₅, -NHSO₂C₃H₇, -CH₂SO₂NH₂, -C₂H₄SO₂NH₂, -C₃H₆SO₂NH₂, sulfonoxide substituents such as -SOCH₃, -SOC₂H₅, -SOC₃H₇, -SO-cyclo-C₃H₅, -SOCH(CH₃)₂, -SOC(CH₃)₃, carbonic ester substituents such as -OCOOCH₃, -OCOOC₂H₅, -OCOOC₃H₇, -OCOO-cyclo-C₃H₅, -OCOOC₄H₉, -OCOOCH(CH₃)₂, -OCOOCH₂Ph, -OCOOC(CH₃)₃, alkyl urea substituents such as -NHCONHCH₃, -NHCONHC₂H₅, -NHCONHC₃H₇, -NHCONHC₄H₉, -NHCONH-cyclo-C₃H₅, -NHCONH[CH(CH₃)₂], -NHCONH[C(CH₃)₃], -NHCON(CH₃)₂, -NHCON(C₂H₅)₂, -NHCON(C₃H₇)₂, -NHCON(C₄H₉)₂, -NHCON(cyclo-C₃H₅)₂, -NHCON[CH(CH₃)₂]₂, -NHCON[C(CH₃)₃]₂, alkylguanidine substituents such as -NHC(=NH)NHCH₃, -NHC(=NH)NHC₂H₅, -NHC(=NH)NHC₃H₇, -NHC(=NH)NHC₄H₉, -NHC(=NH)NH-cyclo-C₃H₅, -NHC(=NH)NH[CH(CH₃)₂], -NHC(=NH)NH[C(CH₃)₃], -NHC(=NH)N(CH₃)₂, -NHC(=NH)N(C₂H₅)₂, -NHC(=NH)N(C₃H₇)₂, -NHC(=NH)-N(cyclo-C₃H₅)₂, -NHC(=NH)N(C₄H₉)₂, -NHC(=NH)N[CH(CH₃)₂]₂, -NHC(=NH)N[C(CH₃)₃]₂, alkyl carbamate substituents such as -OCONHCH₃, -OCONHC₂H₅, -OCONHC₃H₇, -OCONHC₄H₉, -OCONH-cyclo-C₃H₅, -OCONH[CH(CH₃)₂], -OCONH[C(CH₃)₃], -OCON(CH₃)₂, -OCON(C₂H₅)₂, -OCON(C₃H₇)₂, -OCON(C₄H₉)₂, -OCON(cyclo-C₃H₅)₂, -OCON[CH(CH₃)₂]₂, -OCON[C(CH₃)₃]₂, unsaturated alkoxy substituents such as -OC₂H₄CH=CH₂, -OCH₂-CH=CHCH₃, -OC₂H₄C≡CH, -OCH₂C≡CH, -OCH₂C≡CCH₃, -OC₃H₆CH=CH₂, -OC₂H₄CH=CH-CH₃, -OCH₂CH=CHC₂H₅, -OCH₂CH=CHCH=CH₂, -OC(CH₃)=CH-CH=CH₂, -OCH₂CH=C(CH₃)₂, -OC(CH₃)=C(CH₃)₂, -OC₃H₆C≡CH, -OC₂H₄C≡CCH₃, -OCH₂C≡CC₂H₅, -OCH₂C≡CCH=CH₂, -OCH₂CH=CHC≡CH, -OCH₂C≡CC≡CH, -OC₄H₈CH=CH₂, -CH=CHC₄H₉, -OC₃H₆CH=CHCH₃, -OCH₂CH=CHC₃H₇, -OC₂H₄-CH=CHC₂H₅, -OCH₂C(CH₃)=C(CH₃)₂, -OC₂H₄CH=C(CH₃)₂, -OC₄H₈C≡CH, -OC≡CC₄H₉, -OC₃H₆C≡CCH₃, -OCH₂C≡CC₃H₇, -OC₂H₄C≡CC₂H₅, alkenyl substituents and alkynyl substituents such as -C₂H₄CH=CH₂, -CH=CHC₂H₅, -CH=C(CH₃)₂, -CH₂CH=CHCH₃, -CH=CHCH=CH₂, -C₂H₄C≡CH, -C≡CC₂H₅, -CH₂C≡CCH₃, -C≡CCH=CH₂, -CH=CHC=CH, -C=CC=CH, -C₃H₆CH=CH₂, -CH=CHC₃H₇, -C₂H₄CH=CHCH₃, -CH₂CH=CHC₂H₅, -CH₂CH=CHCH=CH₂, -CH=CHCH=CHCH₃, -CH=CHCH₂CH=CH₂, -C(CH₃)=CHCH=CH₂, -CH=C(CH₃)-CH=CH₂, -CH=CHC(CH₃)=CH₂, -CH₂CH=C(CH₃)₂, -C(CH₃)=C(CH₃)₂, -C₃H₆C=CH, -C≡CC₃H₇, -C₂H₄C≡CCH₃, -CH₂C≡CC₂H₅, -CH₂C≡CCH=CH₂, -CH₂CH=CHC≡CH, -CH₂C≡CC≡CH, -C≡CCH=CHCH₃, -CH=CHC≡CCH₃, -C≡CC≡CCH₃, -C≡CCH₂CH=CH₂, -CH=CHCH₂C≡CH, -C≡CCH₂C≡CH, -C(CH₃)=CHCH=CH₂, -CH=C(CH₃)CH=CH₂, -CH=CHC(CH₃)=CH₂, -C(CH₃)=CHC≡CH, -CH=C(CH₃)C≡CH, -C≡CC(CH₃)=CH₂, -C₄H₈CH=CH₂, -CH=CHC₄H₉, -C₃H₆CH=CH-CH₃, -CH₂CH=CH-C₃H₇, -C₂H₄CH=CH-C₂H₅, -CH₂C(CH₃)=C(CH₃)₂, -C₂H₄CH=C(CH₃)₂, -C₄H₈C≡CH, -C≡CC₄H₉, -C₃H₆C≡CCH₃, -CH₂C≡CC₃H₇, -C₂H₄C≡CC₂H₅, -CH=CHPh and -C=CPh. As used herein, an optionally substituted benzophenone substituent or an optionally substituted linker, in particular an optionally substituted benzophenone linker means a benzophenone substituent or benzophenone linker, respectively, which may optionally have any of the above substituents. It is to be understood that, with respect to further use, the skilled person would consider organic compounds having one or more benzophenone substituents that do not undergo side reactions in further use.

### Lactam, N-Vinyllactam

The term **"lactam",** as used herein, refers to cyclic organic chemical compounds that have an amide bond in the ring. Example compounds include 2-pyrrolidone, also known as γ-butyrolactam, β-lactam δ-lactam or ε-caprolactam. The term **"N-vinyllactam"** as used herein, also referred to as "vinyllactam", refers to the aforementioned lactams, but having a vinyl group on the nitrogen atom. Examples of *N*-vinyllactams include, but are not limited to, *N*-vinyl-2-pyrrolidone (NVP) or *N*-vinylcaprolactam.

### Vinyl Group, Vinyl Compound

The term **"vinyl group",** as used herein, refers to the functional group -CH=CH₂. The term **"vinyl compound",** as used herein, refers to an organic compound having at least one vinyl group as a functional group. In the prior art vinyl compounds are commonly used as monomers in polymerization reactions.

### N-Vinylpyrrolidone (NVP)

The term **"N-vinylpyrrolidone",** also *N*-vinyl-2-pyrrolidone or NVP, as used herein, refers to a heterocyclic organic compound having the following structural formula:

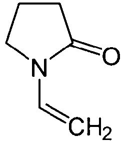

N-vinylpyrrolidone is widely used in the prior art in the production of polymers. *N-*vinylpyrrolidone is very reactive and can also polymerize spontaneously. Polymerization can also be initiated by UV irradiation. Due to its UV sensitivity, *N*-vinylpyrrolidone is used for the production of UV-curable inks, paints and coatings, and as a photoinitiator in other polymers. A variety of polymers can be prepared from *N*-vinylpyrrolidone, such as linear polyvinylpyrrolidones (PVP), crosslinked polyvinylpolypyrrolidone (PVPP) and numerous copolymers.

### N-Vinylpyrrolidone Moiety

The term **"*N*-vinylpyrrolidone moiety",** as used herein, refers to a covalently bound *N*-vinylpyrrolidone monomer incorporated into a polymer chain. In the case of polyvinylpyrrolidone (PVP), the monomer is *N*-vinylpyrrolidone and the monomer moiety is an *N*-vinylpyrrolidone moiety having the following structural formula:

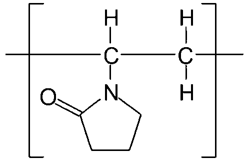

### Polyvinylpyrrolidon (PVP)

The term **"polyvinylpyrrolidone"** (PVP), as used herein, refers to a linear polymer that can be prepared by polymerization of *N*-vinylpyrrolidone (NVP). Thus, the polymer chain consists of many *N*-vinylpyrrolidone moieties linked together through covalent bonds. The *N*-vinylpyrrolidone moieties have the molecular formula C₆H₉NO and thus polyvinylpyrrolidone has the following general structural formula:

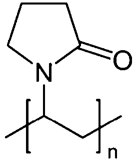

Alternative designations for polyvinylpyrrolidone include, but are not limited to, 1-ethenyl-2-pyrrolidone homopolymer, poly[1-(2-oxo-1-pyrrolidinyl)ethylene], povidone, polyvidone, 1-vinyl-2-pyrrolidinone polymer and additive E 1201. Polyvinylpyrrolidone is a hygroscopic, amorphous powder with a white to light yellow color and is readily soluble in water. Commercial designations are mainly povidone, PVP or Periston. A distinction is made here, for example, between Povidone K-30 and Povidone K-90. Povidone is also available under the trade name Kollidon^{®}. The K value is a classification commonly used in the plastics industry and is directly related to the average molar mass of the polymer. Thus, the K value can be used to indirectly infer the degree of polymerization and thus the chain length. Preferred povidones herein are povidone 90 and povidone 130. Also included herein as biodegradable polymers are the commercially available povidones Kollidon^{®} 90F with a molecular weight of 1000kDa - 1500kDa and Kollidon^{®} 30 with a molecular weight of 44kDa - 54 kDa.

Polyvinylpyrrolidone (PVP) is hydrophilic, biocompatible and non-toxic, dissolves in solvents of different polarity, exhibits excellent binding properties and shows a stabilizing effect in suspensions and emulsions. Polyvinylpyrrolidone (PVP) is used in the prior art for a variety of industrial applications, such as in personal care or cosmetic products e.g. as a thickening agent in shampoo or toothpaste, in pharmaceutical products as an excipient, binder or to increase viscosity e.g. in eye drops, as a coating component for medical products, in the food industry as a binding and thickening agent, stabilizer and coating agent and further e.g. as an additive in adhesives, in the production of batteries, ceramics, glass fibers, ink, for the production of membranes e.g. as dialysis or water treatment filters, as a binding and complexing agent in the agricultural industry or also for contact lenses and in contact lens solutions as a lubricant or wetting agent to reduce friction.

The polyvinylpyrrolidone homopolymer is a linear polymer and can be prepared by homopolymerization of *N*-vinylpyrrolidone (NVP). It is known in the art that the physical properties of the polyvinylpyrrolidone homopolymer can be varied by selective incorporation of one or more comonomers. Comonomers known from the prior art include, but are not limited to, *N*-vinylimidazole, vinyl acetate and vinylcaprolactam. For special applications, such as reducing hydrophilicity, hydrophobic comonomers, such as styrene, can also be used to provide a more hydrophobic and thus less water-soluble PVP copolymer.

### Vinylpyrrolidone Copolymer

The term **"vinylpyrrolidone copolymer",** as used herein, refers to a copolymer in which *N*-vinylpyrrolidone (NVP) is used as the main monomer. The properties of vinylpyrrolidone homopolymers (PVP) can be selectively varied by incorporating comonomers into the polymer chain. Examples of prior art comonomers include *N*-vinylimidazole, vinyl acetate and vinylcaprolactam. Vinylpyrrolidone/vinyl acetate copolymers can be used, for example, as thickeners and hydrophilic hot melt adhesives.

### Blocks of N-Vinylpyrrolidone moieties

The term **"blocks of N-vinylpyrrolidone moieties",** refers to segments in the polymer chain of a vinylpyrrolidone copolymer which are composed only of *N*-vinylpyrrolidone moieties and therefore locally exhibit the physical and mechanical properties of the polyvinylpyrrolidone homopolymer. Blocks of *N*-vinylpyrrolidone moieties herein preferably comprise at least two, more preferably at least three, further preferably at least four, even more preferably at least five, further preferably at least six, even more preferably at least seven, and further preferably at least eight identical monomer moieties. A copolymer of blocks of *N*-vinylpyrrolidone moieties divided by maleic acid moieties is characterized in that the polymer chain contains only blocks of *N*-vinylpyrrolidone moieties and no blocks of maleic acid moieties are present. Therefore, in a copolymer of blocks of *N*-vinylpyrrolidone moieties divided by maleic acid moieties, the maleic acid moieties have only covalent linkages to the *N*-vinylpyrrolidone moieties and no maleic acid moieties covalently linked to each other are present in the polymer chain of the vinylpyrrolidone copolymer. Preferably, the *N*-vinylpyrrolidone monomer is the main monomer and the maleic acid monomer is the comonomer.

### α,β-unsaturated carboxylic acid monomer

The term **"α,β-unsaturated carboxylic acid monomer",** as used herein, generally refers to α,β-unsaturated monocarboxylic acids, α,β-unsaturated dicarboxylic acids, α,β-unsaturated monocarboxylic acid amides, α,β-unsaturated monocarboxylic acid esters, α,β-unsaturated dicarboxylic acid amides, α,β-unsaturated dicarboxylic acid esters and α,β-unsaturated dicarboxylic acid anhydrides as well as derivatives of these compounds. Examples of α,β-unsaturated carboxylic acid monomers include, but are not limited to acrylic acid, methacrylic acid, ethacrylic acid, α-chloroacrylic acid, crotonic acid, maleic acid, itaconic acid, citraconic acid, mesaconic acid, cis/trans-glutaconic acid, aconitic acid, fumaric acid and mixtures thereof. The monomers also include the salts of the above acids, in particular the sodium, potassium and ammonium salts and the salts with amines. The monomers can be used as such or as mixtures of one another.

An α,β-unsaturated monocarboxylic acid monomer has the general formula: 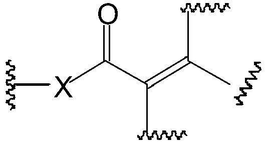 wherein X is -O-, -NH-, or -N-.

Suitable α,β-monoolefinically unsaturated monocarboxylic acids include, but are not limited to, acrylic acid, methacrylic acid, ethacrylic acid, α-chloroacrylic acid, and/or crotonic acid. In some embodiments, the α,β-unsaturated carboxylic acid monomer is preferably selected from an α,β-unsaturated monocarboxylic acid selected from the group consisting of or including acrylic acid, methacrylic acid, ethacrylic acid, α-chloroacrylic acid, crotonic acid, and derivatives thereof. Particularly preferred are α,β-unsaturated monocarboxylic acid selected from the group comprising or consisting of acrylic acid and/or methacrylic acid and derivatives thereof.

Suitable α,β-monoolefinically unsaturated monocarboxylic acid amides include, but are not limited to, methyl acrylamides, methyl methacrylamides, N-methylol acrylamides, N-methylol methacrylamides, N-butoxymethyl (meth)acrylamides, N-isobutoxymethyl(meth)acrylamides, tetrahydrofurfuryl-2-acrylamides, tetrahydrofurfuryl-2-methacrylamides, and derivatives thereof.

Suitable α,β-monoolefinically unsaturated monocarboxylic acid esters include, but are not limited to, alkoxy group-containing monomers such as 3-methoxybutyl methacrylate, 3-methoxybutyl acrylate, 2-methoxyethyl methacrylates, 2-methoxyethyl acrylate, 2-butoxyethyl acrylate, 2-butoxyethyl methacrylates, 2-ethoxyethyl acrylates, 2-ethoxyethyl methacrylate, tetrahydrofurfuryl-2-acrylate, tetrahydrofurfuryl-2-acrylate, tetrahydrofurfuryl-2-methacrylate, methyl acrylate, methyl methacrylate, methyl ethacrylate, ethyl methacrylate, ethyl acrylate, ethyl ethacrylate, n-propyl methacrylate, n-propyl acrylate, isopropyl methacrylate, isopropyl acrylate, n-butyl acrylate, n-butyl methacrylate, tert-butyl methacrylate, tert-butyl acrylate, n-pentyl methacrylate, n-pentyl acrylate, n-hexyl methacrylate, n-hexyl acrylate, n-heptyl methacrylate, n-heptyl acrylate, n-octyl methacrylate, n-octyl acrylate, 1,1,3,3-tetramethylbutyl acrylate, 1,1,3,3-tetramethylbutyl methacrylate, ethylhexyl acrylate, ethylhexyl methacrylate, n-nonyl methacrylate, n-nonyl acrylate, n-decyl methacrylate, n-decyl acrylate, n-undecyl acrylate, n-undecyl methacrylate, tridecyl methacrylate, tridecyl acrylate, myristyl methacrylate, myristyl acrylate, pentadecyl methacrylate, pentadecyl acrylate, palmityl methacrylate, palmityl acrylate, heptadecyl acrylate, heptadecyl methacrylate, nonadecyl methacrylate, nonadecyl acrylate, arrachinyl acrylate, arrachinyl methacrylate, behenyl methacrylate, behenyl acrylate, lignocerenyl methacrylate, lignocerenyl acrylate, cerotinyl acrylate, cerotinyl methacrylate, melissinyl (meth)acrylate, palmitoleinyl methacrylate, palmitoleinyl acrylate, oleyl methacrylate, oleyl acrylate, linolylacrylate, linolyl methacrylate, linolenyl methacrylate, linolenyl acrylate, stearyl methacrylate, stearyl acrylate, lauryl methacrylate and lauryl acrylate.

Suitable α,β-monoolefinically unsaturated secondary and tertiary monocarboxylic acid amides include, but are not limited to, N,N-dimethylacrylamides, N,N-dimethyl methacrylates, N,N-dimethylacrylates, N,N-diethylamino-ethyl-acrylamides, N,N-diethylamino-ethyl-methacrylamides, N,N-diisopropyl-acrylamides, N,N-diisopropyl-methacrylamides, N,N-dibutyl acrylates, N,N-dibutyl-acrylamides, N,N-dibutyl-methacrylamides, N,N-dibutyl methacrylates, diacetone acrylamides, N-tert-butyl methacrylamides, N-tert-butyl acrylamides, diacetone methacrylamides, N-(1-methylundecyl) methacrylamides, N-(1-methylundecyl)-acrylamides, N-isobornylacrylamides, N-isobornylmethacrylamides, N-benzyl-acrylamides, N-adamantyl-methacrylamides, acrylamidoacetic acids, N-adamantyl-acrylamides, N-benzyl-methacrylamides, N-4-methylphenylacrylamides, methylmethacrylamides, methylacrylamides, acrylamidoacetic esters, N-diphenylmethylacrylamides, phthalimidomethylmethacrylamides, phthalimidomethylacrylamides, acrylamidohydroxyacetic acids, methacrylamidohydroxyacetic acids, methacrylamidoacetic acids, methacrylamidoacetic esters, methyl methacrylamidoacetate, methyl acrylamidoacetate, 2-methacrylamido-2-methylbutyric acid, 2-acrylamido-2-methylbutyric acid, N-(2,2,2-trichloro-1-hydroxy)ethylacrylamide, N-(2,2,2-trichloro-1-hydroxy)¬ethylmethacrylamide, N,N-bis(2-cyanoethyl)methacrylamide, N-(3-hydroxy-2,2-di-methylpropyl)-acrylamide, N-(3-hydroxy-2,2-di-methylpropyl)-methacrylamide, dialkylmethacrylamide, dialkylacrylamide, p-hydroxyacrylic anilide and p-hydroxy-methacrylic anilide.

Other suitable α,β-monoolefinically unsaturated monocarboxylic acid monomers include, but are not limited to, 2-hydroxy-3-[N,N-di(2-hydroxyethyl)]-propyl methacrylate, 2-hydroxy-3-[N,N-di(2-hydroxyethyl)]-propyl acrylate, 2-methoxy-3-[N,N-di(2-hydroxyethyl)]-propyl acrylate, cyclohexyl methacrylate, 2-methoxy-3-[N,N-di(2-hydroxyethyl)]-propyl methacrylate, cyclohexyl acrylate 2-hydroxy-3-[N-hydroxyethyl-N-alkyl]-propyl methacrylate, furfuryl acrylate, 2-hydroxy-3-[N-hydroxyethyl-N-alkyl]-propyl acrylate, methacrylate, 2-hydroxy-3-[N-hydroxyethyl-N-methyl]-propyl methacrylate, furfuryl methacrylate, 2-hydroxy-3-[N-hydroxyethyl-N-methyl]-propyl acrylate, isobornyl acrylate, 2-hydroxy-3-[N-ethyl-N-methyl]-propyl methacrylate, 3-cyclohexylpropyl-1-acrylate, 3-cyclohexyl propyl-1-methacrylate, 4-tert-butyl-cyclohexyl methacrylate, 2-N-morpholinoethyl acrylate, 2-N-morpholinoethyl methacrylate, 4-tert-butylcyclohexyl acrylate, 2-N-morpholino-N-hexyl (meth)acrylate, N-cyclohexyl methacrylate, N-cyclohexyl acrylate, N-isobornyl acrylate, and N-isobornyl methacrylate.

Other suitable α,β-monoolefinically unsaturated monocarboxylic acid derivatives include, but are not limited to, butyl acrylate, methyl acrylate, ethyl acrylate, propyl acrylate, octyl acrylate, heptyl acrylate, nonyl acrylate, Hexyl acrylate, n-hexyl acrylate, isopropyl acrylate, isobutyl acrylate, decyl acrylate, isodecyl acrylate, isodecyl acrylate, lauryl acrylate, stearyl acrylate, behenyl acrylate, mellissyl acrylate, methoxyethyl acrylate, hydroxyethyl acrylate, hydroxyethyl acrylate, glycidyl acrylate, 2-ethylhexyl acrylate, 2-ethoxyethyl acrylate, ethylene glycol diacrylate, propylene glycol diacrylate, diethylene glycol diacrylate, polyethylene glycol diacrylate, 1,5-pentanediol diacrylate, neopentyl glycol diacrylate, 1,6-hexanediol acrylate, 1,6-hexanediol diacrylate, polyethylene glycol diacrylate, polypropylene glycol diacrylate, pentaerythritol triacrylate, trimethylolpropane triacrylate, trimethylolpropane dipentaerythritol pentaacrylate, n-butyl acrylate, benzoin acrylate, triethylene glycol diacrylate, pentaerythritol tetraacrylate, glyceryl propoxy triacrylate, 1,3-propylene glycol diacrylate, tripropylene glycol diacrylate, 1,3-butylene glycol diacrylate, 1,4-butanediol diacrylate, diethylene glycol acrylate, butyl methacrylate, triethylene glycol diacrylate, tetraethylene glycol diacrylate, methyl methacrylate, ethyl methacrylate, propyl methacrylate, octyl methacrylate, heptyl methacrylate, nonyl methacrylate, hexyl methacrylate, n-hexyl methacrylate, isopropyl methacrylate, isobutyl methacrylate, decyl methacrylate, isodecyl methacrylate, isodecyl methacrylate, stearyl methacrylate, glycidyl methacrylate, mellissyl methacrylate, lauryl methacrylate, hydroxyethyl methacrylate, methoxyethyl methacrylate, behenyl methacrylate, 2-ethylhexyl methacrylate, 2-ethoxyethyl methacrylate, ethylene glycol dimethacrylate, propylene glycol dimethacrylate, n-butyl methacrylate, diethylene glycol dimethacrylate, polyethylene glycol dimethacrylate, 1,5-pentanediol dimethacrylate, benzoin methacrylate, neopentyl glycol dimethacrylate, 1,6-hexanediol methacrylate, methyl ethacrylate, 1,6-hexanediol dimethacrylate, polyethylene glycol dimethacrylate, polypropylene glycol dimethacrylate, pentaerythritol trimethacrylate, trimethylolpropane trimethacrylate, trimethylolpropane dipentaerythritol pentamethacrylate, pentaerythritol tetramethacrylate, triethylene glycol dimethacrylate, glyceryl propoxy tri(meth)acrylate, 1,3-propylene glycol dimethacrylate, tripropylene glycol dimethacrylate, 1,3-butylene glycol dimethacrylate, 1,4-butanediol dimethacrylate, diethylene glycol dimethacrylate, triethylene glycol dimethacrylate, tetraethylene glycol dimethacrylate, ethoxylated bisphenol A-dimethacrylate, 1,12-dodecanediol diacrylate, 1,12-dodecanediol dimethacrylate, 1,3-butylene glycol diacrylate, 1,3-butylene glycol dimethacrylate, 1,4-butanediol diacrylate, 1,4-butanediol dimethacrylate, 1,6-hexanediol diacrylate, 1,6-hexanediol dimethacrylate, alkoxylated cyclohexanedimethanol diacrylates, alkoxylated cyclohexanedimethanol dimethacrylates, alkoxylated hexanediol diacrylates, alkoxylated hexanediol dimethacrylates, alkoxylated neopentyl glycol diacrylates, alkoxylated neopentyl glycol dimethacrylates, cyclohexane dimethanol diacrylate, cyclohexane dimethanol dimethacrylate, diethylene glycol diacrylate, diethylene glycol dimethacrylate, dipropylene glycol diacrylate, dipropylene glycol dimethacrylate, ethoxylated bisphenol A diacrylates, ethoxylated bisphenol A dimethacrylates, ethylene glycol diacrylate, ethylene glycol dimethacrylate, neopentyl glycol diacrylate, neopentyl glycol dimethacrylate, polyethylene glycol diacrylates, polyethylene glycol dimethacrylates, propylene glycol diacrylates, propylene glycol dimethacrylates, propoxylated neopentyl glycol diacrylates, propoxylated neopentyl glycol dimethacrylates, tetraethylene glycol diacrylate, tetraethylene glycol dimethacrylate, triethylene glycol diacrylate, triethylene glycol dimethacrylate, tripropylene glycol diacrylate, tripropylene glycol dimethacrylate, ethoxylated trimethylolpropane triacrylates, ethoxylated trimethylolpropane trimethacrylates, propoxylated glyceryl triacrylates, propoxylated glyceryl trimethacrylates, trimethylolpropane triacrylate, trimethylolpropane trimethacrylate, Pentaerythritol triacrylate, pentaerythritol trimethacrylate, propoxylated trimethylolpropane triacrylates, propoxylated trimethylolpropane trimethacrylates, tris(2-hydroxyethyl)iso-cyanurate triacrylate, tris(2-hydroxyethyl)isocyanurate trimethacrylate, caprolactone acrylates, caprolactone methacrylates, caprolactamacrylamides, caprolactamacrylamide, caprolactammethacrylamide, valerolactone acrylates, valerolactone methacrylates, valerolactamacrylamides, valerolactammethacrylamides, butyrolactone acrylates, butyrolactone methacrylates, butyrolactamacrylamides, butyrolactammethacrylamides, propiolactone acrylates, propiolactone methacrylates, propiolactamacrylamides, propio-lactamacrylamides, propio¬lactammethacrylamides, acrylic acid, methacrylic acid, 2-(2-ethoxyethoxy)ethyl acrylate, 2-(2-ethoxyethoxy)ethyl methacrylate, 2-phenoxyethyl acrylate, 2-phenoxyethyl methacrylate, 3,3,5-trimethylcyclohexyl acrylate, 3,3,5-tri-methylcyclohexyl methacrylate, alkoxylated lauryl acrylates, alkoxylated lauryl methacrylates, alkoxylated phenol acrylates, alkoxylated phenol methacrylates, alkoxylated tetrahydrofurfuryl acrylates, alkoxylated tetrahydrofurfuryl methacrylates, lauryl acrylate, lauryl methacrylate, cyclic trimethylolpropane formal acrylate, cyclic trimethylolpropane formal methacrylate, dicyclopentadienyl acrylate, dicyclopentadienyl methacrylate, diethylene glycol methyl ether acrylate, diethylene glycol methyl ether methacrylate, ethoxylated hydroxyethyl acrylates, ethoxylated hydroxyethyl methacrylates, ethoxylated nonylphenol acrylates, ethoxylated nonylphenol methacrylates, isobornyl acrylate, isobornyl methacrylate, isodecyl acrylate, isodecyl methacrylate, isoyl acrylate, isoocyl acrylate, isoocyl methacrylate, metal acrylates, metal methacrylates, methoxypolyethylene glycol acrylates, methoxypolyethylene glycol methacrylates, octyldecyl acrylate, octyldecyl methacrylate, polypropylene glycol acrylates, polypropylene glycol methacrylates, propoxylated allyl acrylates, propoxylated allyl methacrylates, stearyl acrylate, stearyl methacrylate, tetrahydrofurfuryl acrylate, tetrahydrofurfuryl methacrylate, tridecyl acrylate, tridecyl methacrylate, triethylene glycol ethyl ether acrylate, triethylene glycol ethyl thermethacrylate, 1,12-dodecanediol diacrylate, 1,12-dodecanediol dimethacrylate, 1,3-butylene glycol diacrylate, 1,3-butylene glycol dimethacrylate, 1,4-butanediol diacrylate, 1,4-butanediol dimethacrylate, 1,6-hexane diol diacrylate, 1,6-hexane diol dimethacrylate, alkoxylated cyclohexane dimethanol diacrylates, alkoxylated hexane diol diacrylates, alkoxylated hexane diol dimethacrylates, alkoxylated neopentyl glycol diacrylates, alkoxylated neopentyl glycol dimethacrylates, cyclohexane dimethanol diacrylate, cyclohexane dimethanol dimethacrylate, diethylene glycol diacrylate, diethylene glycol dimethacrylate, dipropylene glycol diacrylate, dipropylene glycol dimethacrylate, ethoxylated bisphenol A-diacrylates, ethoxylated bisphenol A-dimethacrylates, Ethylene glycol diacrylate, ethylene glycol dimethacrylate, neopentyl glycol diacrylate, neopentyl glycol dimethacrylate, polyethylene glycol diacrylates, polyethylene glycol dimethacrylates, propylene glycol diacrylates, propylene glycol di-methacrylates, propoxylated neopentyl glycol diacrylates, propoxylated neopentyl glycol dimethacrylates, tetraethylene glycol diacrylate, tetraethylene glycol dimethacrylate, triethylene glycol diacrylate, triethylene glycol dimethacrylate, tripropylene glycol diacrylate, tripropylene glycol dimethacrylate, ethoxylated trimethylolpropane triacrylates, ethoxylated trimethylolpropane trimethacrylates, propoxylated glyceryl triacrylates, propoxylated glyceryl trimethacrylates, trimethylolpropane triacrylate, trimethylolpropane trimethacrylate, pentaerythritol triacrylate, pentaerythritol trimethacrylate, propoxylated trimethylolpropane triacrylate, propoxylated trimethylolpropane trimethacrylate, tris(2-hydroxyethyl)isocyanurate triacrylate, tris(2-hydroxy-ethyl)isocyanurate trimethacrylate, di-trimethylolpropane tetraacrylate, dipentaerythritol pentaacrylate, ethoxylated pentaerythritol tetraacrylates, dipentaerythritol pentaacrylate and pentaerythritol tetraacrylate.

Examples of polymerizable monofunctional photo- and/or thermosensitive α,β-unsaturated carboxylic acid monomers, which can also be used as photoinitiators, are generally acrylic acids (AA), methacrylic acids (MA), methacrylates and acrylamides such as 2-hydroxymethyl methacrylate (HEMA), N,N-dimethylacrylamide (DMA), diisobutylacrylamide (DBA), aminopropyl methacrylamide (APMA), N,N-diiso¬butylacrylamide (DBA), 9-anthracenylmethyl methacrylate, N-[3-methacrylamido)-propyl]-2-(carboxymethyl)-hexadecanamide, N-[3-methacryl¬amido)-propyl]-3-carboxyheptadecanamide. Other examples of such polymerizable monofunctional photo- and/or thermosensitive α,β-unsaturated carboxylic acid monomers, which may also be used as photoinitiators, include, but are not limited to dipentaerythritol pentaacrylate (PPA), thoxylated trimethylolpropane triacrylates (TTA) N,N'-diethyl-1,3-propylene bisacrylamide (EPA), and 2,2,4-trimethyl-6-[2-(2-methylacryl-oyloxy)-ethoxycarbonylamino]-hexyl-carbamoyl-oxy-ethyldimethacrylate (UDMA).

An α,β-unsaturated dicarboxylic acid monomer has the general formula: 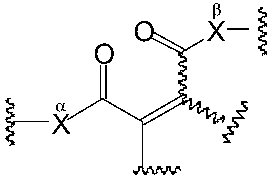 wherein X^{α} and X^{β} independently of each other represent -O-, -NH- or -N-.

Examples of α,β-unsaturated dicarboxylic acid monomers include, but are not limited to, maleic acid, itaconic acid, citraconic acid, mesaconic acid, glutaconic acid, aconitic acid, and/or fumaric acid. Monomers also include the salts of the above acids, in particular the sodium, potassium and ammonium salts and the salts with amines. The monomers can be used as such or as mixtures with one another.

Preferred α,β-monoolefinically unsaturated dicarboxylic acids include, but are not limited to, maleic acid, itaconic acid, citraconic acid, mesaconic acid, glutaconic acid, aconitic acid and/or fumaric acid. Particularly preferred herein are maleic acid and derivatives thereof. An α,β-unsaturated carboxylic acid monomer may be preferably selected from an α,β-unsaturated dicarboxylic acid or a derivative thereof, preferably selected from maleic acid, itaconic acid, citraconic acid, mesaconic acid, glutaconic acid, aconitic acid and/or fumaric acid and/or derivatives thereof.

Examples of α,β-monoolefinically unsaturated dicarboxylic acid esters include, but are not limited to, n-butyl maleic acid monoester, fumaric acid mono-ethyl ester, maleic acid monomethyl ester, maleic acid monoethyl ester, maleic acid monopropyl ester, maleic acid monobutyl ester, maleic acid monobenzyl ester, maleic acid mono(2-ethylhexyl) ester, maleic acid monohydroxyethyl ester, maleic acid monohydroxypropyl, fumaric acid monomethyl ester, fumaric acid mono-ethyl ester, fumaric acid monopropyl ester, fumaric acid monobutyl ester, fumaric acid monobenzyl ester, fumaric acid mono(2-ethylhexyl) ester, fumaric acid monohydroxyethyl ester, fumaric acid monohydroxypropyl, maleic acid dimethyl ester, maleic acid diethyl ester, maleic acid dipropyl ester, maleic acid dibutyl ester, maleic acid monobenzyl ester, maleic acid di(2-ethylhexyl) ester, maleic acid dihydroxyethyl ester, maleic acid dihydroxypropyl, fumaric acid dimethyl ester, fumaric acid monoethyl ester, fumaric acid dipropyl ester, fumaric acid monobutyl ester, fumaric acid dibenzyl ester, fumaric acid di(2-ethylhexyl) ester, fumaric acid dihydroxyethyl ester, fumaric acid monohydroxypropyl ester, itaconic acid monomethyl ester, itaconic acid monoethyl ester, itaconic acid monopropyl ester, itaconic acid monobutyl ester, itaconic acid monobenzyl ester, itaconic acid mono(2-ethylhexyl) ester, itaconic acid monohydroxyethyl ester, itaconic acid monohydroxypropyl, itaconic acid dimethyl ester, itaconic acid diethyl ester, itaconic acid dipropyl ester, itaconic acid dibutyl ester, itaconic acid dibenzyl ester, itaconic acid di(2-ethylhexyl) ester, itaconic acid dihydroxyethyl ester, itaconic acid dihydroxypropyl ester, citraconic acid monomethyl ester, citraconic acid monoethyl ester, citraconic acid monopropyl ester, citraconic acid monobutyl ester, citraconic acid monobenzyl ester, citraconic acid mono(2-ethylhexyl) ester, citraconic acid monohydroxyethyl ester, citraconic acid monohydroxypropyl, citraconic acid dimethyl ester, citraconic acid diethyl ester, citraconic acid dipropyl ester, citraconic acid dibutyl ester, citraconic acid dibenzyl ester, citraconic acid di(2-ethylhexyl) ester, citraconic acid dihydroxyethyl ester, citraconic acid dihydroxypropyl ester, mesaconic acid monomethyl ester, mesaconic acid monoethyl ester, mesaconic acid monopropyl ester, mesaconic acid monobutyl ester, mesaconic acid monobenzyl ester, mesaconic acid mono(2-ethylhexyl) ester, mesaconic acid monohydroxyethyl ester, mesaconic acid monohydroxypropyl, mesaconic acid dimethyl ester, mesaconic acid diethyl ester, citraconic acid dipropyl ester, mesaconic acid dibutyl ester, mesaconic acid dibenzyl ester, mesaconic acid di(2-ethylhexyl) ester, mesaconic acid dihydroxyethyl ester, mesaconic acid dihydroxypropyl ester, glutaconic acid monomethyl ester, glutaconic acid monoethyl ester, glutaconic acid monopropyl ester, glutaconic acid monobenzyl ester, glutaconic acid mono(2-ethylhexyl) ester, glutaconic acid monohydroxyethyl ester, glutaconic acid monohydroxypropyl, glutaconic acid dimethyl ester, glutaconic acid diethyl ester, glutaconic acid dipropyl ester, glutaconic acid dibutyl ester, glutaconic acid dibenzyl ester, glutaconic acid di(2-ethylhexyl) ester, glutaconic acid dihydroxyethyl ester, glutaconic acid dihydroxypropyl, aconitic acid monomethyl ester, aconitic acid monoethyl ester, aconitic acid monopropyl ester, aconitic acid monobutyl ester, aconitic acid mono(2-ethylhexyl) ester, aconitic acid monohydroxyethyl ester, aconitic acid monohydroxypropyl, aconitic acid dimethyl ester, aconitic acid diethyl ester, aconitic acid dipropyl ester, aconitic acid dibutyl ester, aconitic acid dibenzyl ester, aconitic acid di(2-ethylhexyl) ester, aconitic acid dihydroxyethyl ester, aconitic acid dihydroxypropyl ester, furthermore 3-methoxybutyl, 2-methoxyethyl, 2-butoxyethyl, 2-ethoxyethyl, tetrahydrofurfuryl-2, n-propyl, isopropyl, n-butyl, tert-butyl, n-pentyl, n-hexyl, n-heptyl, n-octyl, 1,1,3,3-tetramethylbutyl, ethylhexyl, n-nonyl, n-decyl, n-undecyl, tridecyl, myristyl, pentadecyl, palmityl, heptadecyl, nonadecyl, arrachinyl, behenyl, lignocerenyl, cerotinyl, melissinyl, palmitoleinyl, oleyl, linolyl, linolenyl, stearyl, lauryl mono and diesters of maleic acid, itaconic acid, citraconic acid, mesaconic acid, glutaconic acid, aconitic acid and fumaric acid.

Examples of α,β-monoolefinically unsaturated dicarboxylic acid anhydrides include, but are not limited to, maleic anhydride and maleic anhydride derivatives.

Preferred α,β-monoolefinically unsaturated dicarboxylic acid amides include, but are not limited to, maleic acid mono- and diamides, itaconic acid mono- and diamides, fumaric acid mono- and diamides, N,N-dimethylamides, N,N-diethylamino-ethyl-amides, N,N- diisopropyl-amides, N,N-diisopropyl-amides, N,N-dibutyl-amides, N,N-dibutyl-amides, N-tert-butyl-amides, diacetone-amides, N-(1-methylundecyl)-amides, N-(1-methylundecyl)-amides, N-isobornylamides, N-adamantyl-amides, N-benzyl-amides, N-4-methylphenylamides, methylamides, N-diphenylmethylamides, phthalimidomethylamides, phthalimidoamides, amidohydroxyacetic acidsAmidoacetic acids, amidoacetic acid esters, amidoacetic acid methyl esters, N-(2,2,2-trichloro-1-hydroxy)-ethylamides, N-(2,2,2-trichloro-1-hydroxy)¬ethylamides, N,N-bis(2-cyanoethyl)amides, N-(3-hydroxy-2,2-di-methylpropyl)amides, dialkylamides of maleic acid, itaconic acid, citraconic acid, mesaconic acid, glutaconic acid, aconitic acid and fumaric acid.

### Maleic Acid

The term **"maleic acid",** also referred to as cis-butenedioic acid or cis-ethylenedicarboxylic acid, as used herein, refers to a hydrophilic dicarboxylic acid that is readily soluble in water and has the chemical formula HO₂CCH=CHCO₂H:

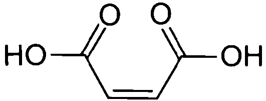

Maleic acid can undergo reactions at the carboxy groups on the one hand and reactions at the double bond on the other hand. Typical maleic acid derivatives are maleic acid esters and maleic acid amides. Maleic acid or maleic acid derivatives are used in the prior art to produce polymers, synthetic resins e.g. for refining and dyeing cotton.

The double bond of maleic acid can participate in polymerization reactions. For example, vinylpyrrolidone copolymers with maleic acid moieties can be obtained using *N*-vinylpyrrolidone (NVP) as the main monomer. For the copolymerization, in addition to maleic anhydride, an α,β-unsaturated maleic acid derivative can also be used as comonomer, e.g. a monoester, a diester, a monoamide or even a diamide of maleic acid, while retaining the double bond function to remain available for copolymerization with *N*-vinylpyrrolidone (NVP). Thus, maleic acid can first be converted to a suitable maleic acid derivative, which can be used as a comonomer in the copolymerization with *N*-vinylpyrrolidone (NVP). However, maleic anhydride can also be used first as a comonomer in the copolymerization with *N*-vinylpyrrolidone (NVP), in which case the carboxy groups can be further functionalized following the preparation of the vinylpyrrolidone copolymer.

Since maleic acid has functional groups with different reactivity - the carboxy groups and the double bond maleic acid can also serve as a linker to connect two or more compounds via covalent bonds.

Maleic acid homopolymers as well as maleic acid copolymers are known in the art, e.g. copolymers of maleic acid and styrene, styrene sulfonic acid, acrylic acid or methyl vinyl ether are commercially available. On average, these copolymers have a maleic acid content of 25-50 mol%. Since maleic acid has two carboxyl groups, such copolymers represent polyelectrolyte polymers that can be used, for example, to produce membranes. Copolymers functionalized at the carboxy group are also known in the prior art, such as poly-(styrene-co-maleic acid) with isobutyl ester or isobutyl/methyl ester functionalizations, e.g. poly-(styrene-co-maleic acid) with isobutyl ester functionalization is commercially available and has a molar ratio of 1.5:1 styrene/maleic acid with about half of the carboxyl groups present as isobutyl esters. The distribution of the isobutyl ester groups is not homogeneous, so that hydrophilic and hydrophobic regions are present in the polymer chain, which can lead to problems in forming a uniform film.

As comonomers, maleic acid and maleic acid derivatives offer the advantage that, due to steric hindrance, the ability to autopolymerize is limited and thus, advantageously, no local "maleic acid accumulations" occur in e.g. vinylpyrrolidone copolymers and, ultimately, a uniform distribution of the maleic acid moieties in the polymer chain is achieved. This provides, among other things, uniform and improved solubility in polar solvents. In addition, maleic acid offers the advantage that the carboxy groups can be functionalized with photoreactive benzophenone substituents, with which crosslinking of the polymer chain of the vinylpyrrolidone copolymer can be achieved after copolymerization, but also crosslinking to surfaces and thus binding to surfaces can be increased, resulting in improved and stable adhesion to various surfaces and materials.

### Maleic Acid Monomer

The term **"maleic acid monomer",** as used herein, refers to an α,β-unsaturated maleic acid derivative that can be used as a comonomer for copolymerization with N-vinylpyrrolidone as the main monomer to prepare vinylpyrrolidone copolymers, in particular vinylpyrrolidone copolymers composed of blocks of *N*-vinylpyrrolidone (NVP) moieties divided by maleic acid moieties. Examples maleic acid comonomers include, but are not limited to, maleic anhydride, maleic acid monoesters, maleic acid diesters, maleic acid monoamides, maleic acid monoamides and maleic acid esteramides: 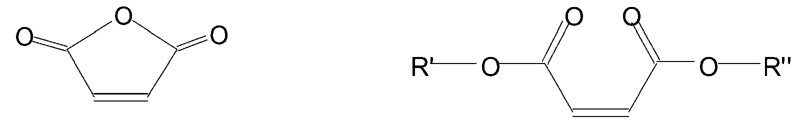 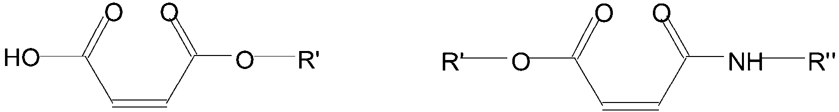 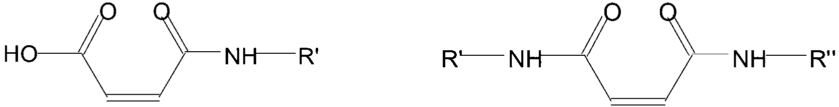 wherein R' and R" are as defined herein.

Furthermore, maleic acid derivatives substituted on the double bond, such as dimethylmaleic acid or dimethylmaleic anhydrides can also be used:

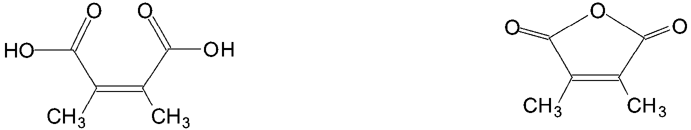

Thus, examples maleic acid derivatives that have subtituents at the double bond include, but are not limited to: 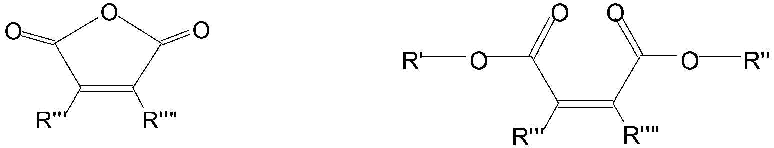 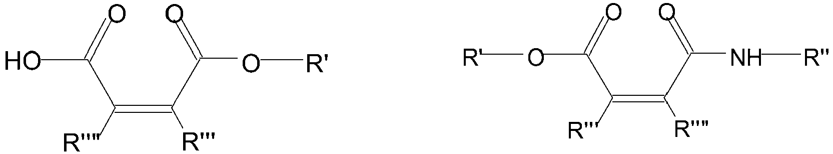 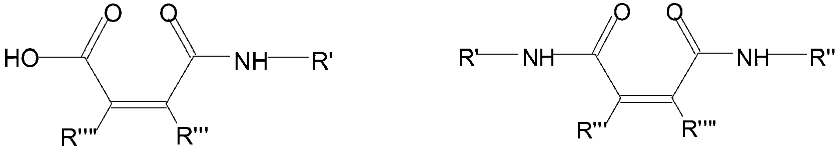 wherein R' and R" are as defined herein, and wherein R‴ and Rʺʺ may independently of each other represent -H, -CH₃, -CH₂CH₃, or -F.

### Maleic Acid Moiety

The term **"maleic acid moiety",** as used herein, refers to a maleic acid monomer covalently bound in a polymer chain. A maleic acid moiety having a carboxy group (-COO-) and a carbamoyl group (-CONH-) as functional groups may be represented as follows:

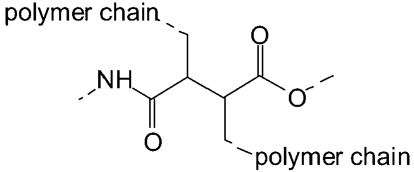

A maleic acid moiety having two carboxy groups (-COO-) or two carbamoyl groups (-CONH-) as functional groups can be represented as follows:

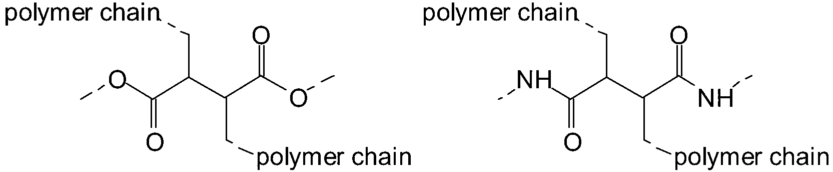

Maleic acid moieties may have as residues R' and R", a benzophenone substituent (BP), but non-benzophenone residues may be also present, which may be selected from polyethylene glycol, polactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl and an organometallic group:

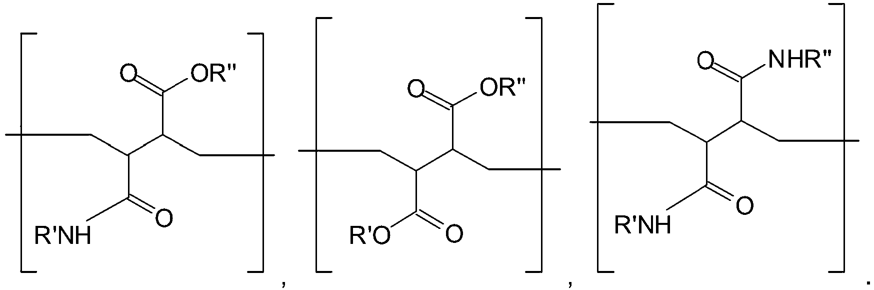

### Medical Device and Medical Aid

The term **"medical device",** as used herein, refers to "objects or substances that serve to detect, prevent, monitor, treat or alleviate diseases, but achieve this purpose primarily ("intended main effect") by physical means and not by pharmacological/immunological means or by metabolic action (Schorn G. MPG Medizinproduktegesetz, 4th edition, Wissenschaftliche Verlagsgesellschaft, Stuttgart, 2009). However, the physical effect of medical devices can certainly be supported by pharmacological, immunological or metabolic effects".

The term **"medical aid",** as used herein, refers to objects that are necessary to ensure the success of a medical treatment, to prevent an impending disability or to compensate for a disability, insofar as they are not to be regarded as general objects of daily use (Definition of the SGB V). Medical aids may mainly be used by the person concerned on his or her own responsibility.

Examples of medical devices and medical aids that can be used in or on the body for short or long periods of time, include, but are not limited to, catheters of any kinds e.g. balloon catheters, bladder catheters, catheter shafts, guide wires, stomach tubes, endoscopes, colonoscopes, needles, cannulas, endotracheal tubes, tracheal tubes, and the like, implants such as stents, eye lenses, joint prostheses, screws, fixations, artificial exits, nasal implants, vocal cords, larynx, artificial and native valves, bone substitutes, respiratory tubes, etc., but also cartilage substitutes, nerve sheaths, occlusion of venous valves, seals ex corpora and in corpora, subcutaneous or intramuscular implants, as moisturizing and/or active ingredient gel, micro- and macrocapsules, wound protection, breast implants, generally as filling material, plasters, pads, etc.

Examples of materials of medical device surfaces or medical aid surfaces include, but are not limited to polyamides (such as PA 12, PA 6, PA 66), stainless steel, nitinol, Pebax, polyethylenes, polypropylenes, polyvinyls, polystyrenes, polyimides, polyamides, PTFE, silicones, polyurethanes, polyesters such as polymethacrylates, polyacrylates, polyacrylamides, polycarbonates, polylactides, polyglycolic acids, polyglycolic acids, and many others.e.g. polymethacrylates, polyacrylates, polyacrylamides, polycarbonates, polylactides, polyglycolides, polylactic acids, polyacetates, polycarbonates etc. and their copolymers and derivatives.

### N-vinylpyrrolidone copolymers according to the invention

The present invention relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L).**

With other words, the present invention preferably relates to vinylpyrrolidone copolymers composed of blocks of *N*-vinylpyrrolidone moieties divided by maleic acid moieties, wherein each maleic acid moiety contains as functional groups a first functional group selected from a carboxy group (-COO-) and a carbamoyl group (-CONH-) and a second functional group selected from a carboxy group (-COO-) and a carbamoyl group (-CONH-), and 1% to 50% of the functional groups of all maleic acid moieties in the vinylpyrrolidone copolymer are functionalized with a benzophenone substituent **(BP)** via a C₁-C₅ alkyl linker **(L).**

The present invention preferably relates to vinylpyrrolidone copolymers composed of blocks of *N*-vinylpyrrolidone moieties divided by maleic acid moieties, wherein each maleic acid moiety contains as functional groups a first functional group selected from a carboxy group (-COO-) and a carbamoyl group (-CONH-) and a second functional group selected from a carboxy group (-COO-) and a carbamoyl group (-CONH-), and 1% to 100% of the first functional group of all maleic acid moieties in the vinylpyrrolidone copolymer are functionalized with a benzophenone substituent **(BP)** via a C₁-C₅ alkyl linker **(L).**

The present invention preferably relates to vinylpyrrolidone copolymers composed of blocks of *N*-vinylpyrrolidone moieties divided by maleic acid moieties, wherein each maleic acid moiety contains as functional groups a first functional group selected from a carboxy group (-COO-) and a carbamoyl group (-CONH-) and a second functional group selected from a carboxy group (-COO-) and a carbamoyl group (-CONH-), and 1% to 100% of the second functional group of all maleic acid moieties in the vinylpyrrolidone copolymer are functionalized with a benzophenone substituent **(BP)** via a C₁-C₅ alkyl linker **(L).**

The present invention preferably relates to vinylpyrrolidone copolymers composed of blocks of *N*-vinylpyrrolidone moieties divided by maleic acid moieties, wherein each maleic acid moiety has as functional groups a first functional group selected from a carboxy group (-COO-) and a carbamoyl group (-CONH-) and a second functional group selected from a carboxy group (-COO-) and a carbamoyl group (-CONH-), and 1% to 100% of the first functional group or the second functional group of all maleic acid moieties in the vinylpyrrolidone copolymer, but not both functional groups of the maleic acid moiety are functionalized with a benzophenone substituent **(BP)** via a C₁-C₅ alkyl linker **(L).**

The present invention preferably relates to vinylpyrrolidone copolymers composed of blocks of *N*-vinylpyrrolidone moieties divided by maleic acid moieties, wherein each maleic acid moiety has as functional groups a first functional group selected from a carboxy group (-COO-) and a carbamoyl group (-CONH-) and a second functional group selected from a carboxy group (-COO-) and a carbamoyl group (-CONH-), and the first functional group or the second functional group is functionalized with a benzophenone substituent **(BP)** via a C₁-C₅ alkyl linker **(L).**

In preferred embodiments, 10% to 50%, more preferably 20% to 50%, even more preferably 30% to 50%, even more preferably 40% to 50%, and particularly preferably 50% of the functional groups of all maleic acid moieties in the vinylpyrrolidone copolymer may be functionalized with a benzophenone substituent **(BP)** via a C₁-C₅ alkyl linker **(L).**

In preferred embodiments, 1%, more preferably 2%, more preferably 3%, more preferably 5%, more preferably 8%, more preferably 10%, more preferably 15%, more preferably 20%, more preferably 25%, more preferably 30%, more preferably 35%, more preferably 40%, more preferably 45%, and especially preferably 50% of the functional groups of all maleic acid moieties in the vinylpyrrolidone copolymer may be functionalized with a benzophenone substituent **(BP)** via a C₁-C₅ alkyl linker **(L).**

Thus, the present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-) or two carboxy groups (-COO-) or two carbamoyl groups (-CONH-), and 50% of the functional groups of all maleic acid moieties in the vinylpyrrolidone copolymer are functionalized with a benzophenone substituent **(BP)** via a C₁-C₅ alkyl linker **(L).**

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), and 1% to 50% of the functional groups of all maleic acid moieties in the vinylpyrrolidone copolymer are functionalized with a benzophenone substituent **(BP)** via a C₁-C₅ alkyl linker **(L).**

In preferred embodiments, 1%, more preferably 2%, more preferably 3%, more preferably 5%, more preferably 8%, more preferably 10%, more preferably 15%, more preferably 20%, more preferably 25%, more preferably 30%, more preferably 35%, more preferably 40%, more preferably 45%, and particularlypreferably 50% of the functional groups of all maleic acid moieties in the vinylpyrrolidone copolymer may be functionalized with a benzophenone substituent **(BP)** via a C₁-C₅ alkyl linker **(L).**

In preferred embodiments, 10% to 50%, more preferably 20% to 50%, even more preferably 30% to 50%, even more preferably 40% to 50%, and particularly preferably 50% of the functional groups of all maleic acid moieties in the vinylpyrrolidone copolymer may be functionalized with a benzophenone substituent **(BP)** via a C₁-C₅ alkyl linker **(L).**

The present invention further relates to a vinylpyrrolidone copolymer composed of blocks of *N*-vinylpyrrolidone moieties divided by maleic acid moieties, wherein each maleic acid moiety has two carboxy groups (-COO-) as functional groups, and 1% to 50% of the functional groups of all maleic acid moieties in the vinylpyrrolidone copolymer are functionalized with a benzophenone substituent **(BP)** via a C₁-C₅ alkyl linker **(L).**

In preferred embodiments, 10% to 50%, more preferably 20% to 50%, even more preferably 30% to 50%, even more preferably 40% to 50%, and particularly preferably 50% of the functional groups of all maleic acid moieties in the vinylpyrrolidone copolymer may be functionalized with a benzophenone substituent **(BP)** via a C₁-C₅ alkyl linker **(L).**

In preferred embodiments, 1%, more preferably 2%, more preferably 3%, more preferably 5%, more preferably 8%, more preferably 10%, more preferably 15%, more preferably 20%, more preferably 25%, more preferably 30%, more preferably 35%, more preferably 40%, more preferably 45%, and particularly preferably 50% of the functional groups of all maleic acid moieties in the vinylpyrrolidone copolymer may be functionalized with a benzophenone substituent **(BP)** via a C₁-C₅ alkyl linker **(L).**

The present invention further relates to a vinylpyrrolidone copolymer composed of blocks of *N*-vinylpyrrolidone moieties divided by maleic acid moieties, wherein each maleic acid moiety has two carbamoyl groups (-CONH-) as functional groups, and 1% to 50% of the functional groups of all maleic acid moieties in the vinylpyrrolidone copolymer are functionalized with a benzophenone substituent **(BP)** via a C₁-C₅ alkyl linker **(L).**

In preferred embodiments, 10% to 50%, more preferably 20% to 50%, still more preferably 30% to 50%, still more preferably 40% to 50%, and particularly preferably 50% of the functional groups of all maleic acid moieties in the vinylpyrrolidone copolymer may be functionalized with a benzophenone substituent **(BP)** via a C₁-C₅ alkyl linker **(L).**

In preferred embodiments, 1%, more preferably 2%, more preferably 3%, more preferably 5%, more preferably 8%, more preferably 10%, more preferably 15%, more preferably 20%, more preferably 25%, more preferably 30%, more preferably 35%, more preferably 40%, more preferably 45%, and particularly preferably 50% of the functional groups of all maleic acid moieties in the vinylpyrrolidone copolymer may be functionalized with a benzophenone substituent **(BP)** via a C₁- C₅ alkyl linker **(L).**

The vinylpyrrolidone copolymers of the present invention can be prepared by reacting *N*-vinylpyrrolidone with at least one maleic acid comonomer. Pre-functionalized maleic acid comonomers are herein particularly preferred, particularly preferably functionalized with a benzophenone substituent **(BP)** via a C₁-C₅ alkyl linker **(L).**

In preferred embodiments, the maleic acid is functionalized at the carboxy groups prior to copolymerization, as this ensures and offers the advantage that no non-functionalized maleic acid moieties are present in the copolymer. A maleic acid derivative functionalized in advance thus increases reproducibility in the preparation of the polymer and better control with respect to the properties of the resulting polymer.

It has been found that it is particularly advantageous if the polyvinylpyrrolidone copolymers composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** are completely soluble in water.

Polyvinylpyrrolidone homopolymers are generally readily soluble in water, and water-soluble homopolymers of maleic acid or maleic anhydride are known in the prior art. Copolymers of *N*-vinylpyrrolidones and maleic anhydride are also known in the prior art. These copolymers are also water-soluble, although hydrolysis of the maleic anhydride to maleic acid can occur on contact with water.

The vinylpyrrolidone copolymers of the present inventions preferably have an average molecular weight of 1,000 to 1,000,000 g/mol, preferably 10,000 to 700,000 g/mol and particularly preferably between 5,000 to 500,000 g/mol.

In addition to the *N*-vinylpyrrolidone and maleic acid monomers, one or more additional comonomers may be added to the copolymer, if desired. The additional comonomer may be added to the *N*-vinylpyrrolidone and maleic acid monomer, for example as part of the monomer mixture. The additional comonomer may be selected from vinyl acetate, an alpha-olefin, maleic anhydride, a vinyl ether, an acrylate, a methacrylate, an acrylamide, a methyl acrylamide, and styrene.

### Maleic Acid Comonomer

For preparing the vinylpyrrolidone copolymers of the present invention, maleic acid monomers are used as comonomers in the copolymerization reaction with N-vinylpyrrolidone as the main monomer. Maleic acid comonomers suitable for the present invention include, but are not limited to, maleic anhydride, maleic acid monoesters, maleic acid diesters, maleic acid monoamides, maleic acid monoamides and maleic acid esteramides:

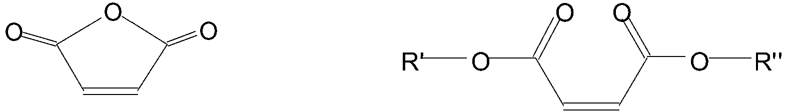

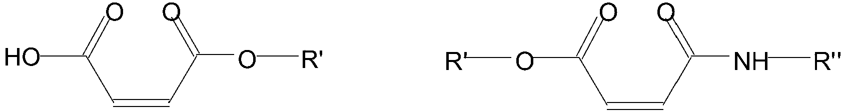

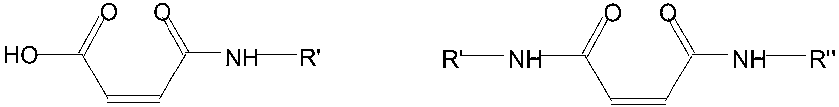

According to the present invention, the residue R' or one of the two residues R' or R" represents a C₁-C₅ alkyl linker (L) functionalized with a benzophenone substituent **(BP).** If two residues R' and R" are present, at least one of these residues represents a C₁-C₅-alkyl linker **(L)** functionalized with a benzophenone substituent **(BP).** Furthermore, the second of the residues R' or R" may have a non-benzophenone substituent selected from the group comprising or consisting of polyethylene glycol (PEG), polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group.

Maleic acid comonomers suitable for the present invention that have additional substituents at the double bound include, but are not limited to: 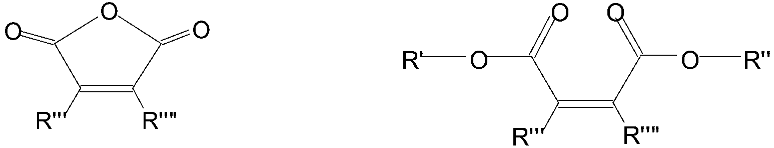 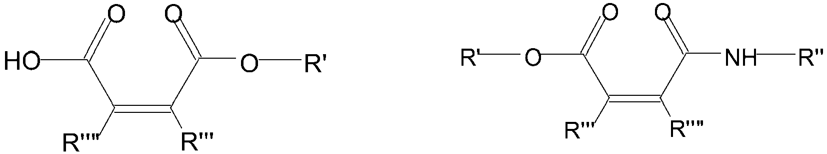 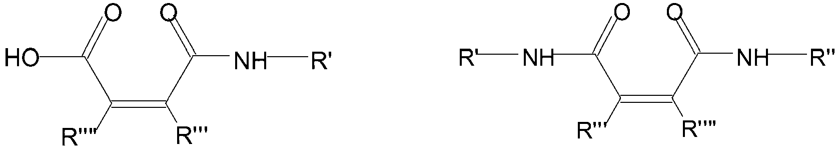 wherein the residue R' or one of the two residues R' or R" represents a C₁-C₅ alkyl linker (L) functionalized with a benzophenone substituent **(BP),** and wherein R‴ and Rʺʺ may independently of each other represent -H, -CH₃, -CH₂CH₃, or -F;

If two residues R' and R" are present, at least one of these residues represents a C₁-C₅-alkyl linker **(L)** functionalized with a benzophenone substituent **(BP).** Furthermore, the second of the residues R' or R" may have a non-benzophenone substituent selected from the group comprising or consisting of polyethylene glycol (PEG), polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group.

If maleic anhydride or a derivative thereof is used as comonomer in the copolymerization with *N*-vinylpyrrolidone, a vinylpyrrolidone copolymer composed of blocks of *N*-vinylpyrrolidone (NVP) divided by maleic anhydride moieties is obtained first. This vinylpyrrolidone copolymer can then be reacted with a C₁-C₅ alkyl linker **(L)** functionalized with a benzophenone substituent **(BP),** wherein the C₁-C₅ alkyl linker **(L)** has a hydroxyl group -OH or a primary amino group -NH₂ as a functional group at the respective end that is not functionalized with the benzophenone substituent **(BP),** to convert the maleic anhydride moieties into maleic acid moieties having as functional groups a carboxy group (-COO-) and a carbamoyl group (-CONH-), or two carboxy groups (-COO-), or two carbamoyl groups (-CONH-), wherein 1% to 50% of the functional groups of all maleic acid moieties in the vinylpyrrolidone copolymer are functionalized with a benzophenone substituent **(BP)** via the C₁-C₅ alkyl linker **(L).**

When maleic acid monoesters, maleic acid diesters, maleic acid monoamides, maleic acid diamides, maleic acid ester amides, and/or derivatives thereof are used as comonomers, in which at least one of the carboxy groups is functionalized with a benzophenone substituent **(BP)** via a C₁-C₅ alkyl linker **(L),** then copolymerization with *N*-vinylpyrrolidone directly produces vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 50% of the functional groups of all maleic acid moieties in the vinylpyrrolidone copolymer are functionalized with a benzophenone substituent (BP) via a C₁-C₅ alkyl linker **(L).**

In accordance with the present invention also a combination of maleic anhydride, maleic acid monoesters, maleic acid diesters, maleic acid monoamides, maleic acid diamides, maleic acid ester amides and/or derivatives thereof may be used as comonomers in the copolymerization with *N*-vinylpyrrolidone to provide vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L).**

The copolymers of the present invention can be prepared by polymerization reaction of *N*-vinylpyrrolidone (NVP) and at least one-maleic acid comonomer. The *N*-vinylpyrrolidone (NVP) is copolymerized with at least one maleic acid comonomer. 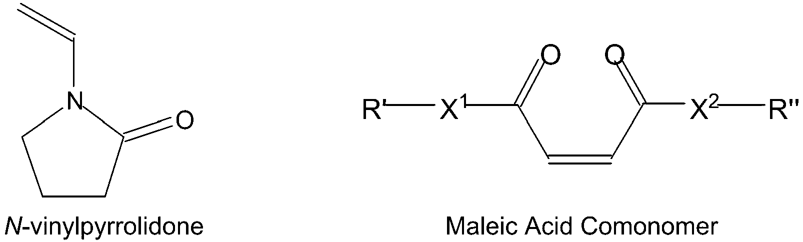 wherein
**X¹** and **X²** are independently of each other -O- or -NH-; and **R'** and **R'** are as defined herein.

Particularly preferably, the maleic acid comonomer is *N*-(4-benzoylbenzyl)-maleic acid monoamide:

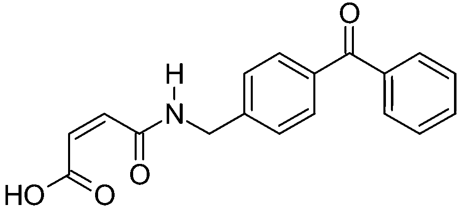

However, non-prefunctionalized maleic acid comonomers can also be used, such as maleic anhydride. Maleic anhydride can be converted into maleic acid by hydrolysis after reaction with NVP, and the vinylpyrrolidone copolymer can then be functionalized at the free carboxy groups of the maleic acid moieties with a benzophenone substituent **(BP)** via a C₁-C₅ alkyl linker **(L).** Thus, after performing copolymerization and after carrying out hydrolysis, each maleic acid moiety has two carboxy groups (-COOH), and 1% to 50% of the carboxy groups of all maleic acid moieties in the vinylpyrrolidone copolymer can be functionalized with a benzophenone substituent **(BP)** via a C₁-C₅ alkyl linker **(L),** wherein the C₁-C₅ alkyl linker **(L)** is an amino-C₁-C₅ alkyl linker or hydroxy-C₁-C₅ alkyl linker.

### C₁-C₅ Alkyl Linker L

The C₁-C₅ alkyl linker **(L)** may be selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), more preferably the C₁-C₅ alkyl linker may be selected from the group consisting of methylene (-CH₂-), ethylene (-CH₂CH₂-), propylene (-CH₂CH₂CH₂-), and butylene (-CH₂CH₂CH₂CH₂-), still more preferably, the C₁-C₅ alkyl linker (L) may be selected from the group consisting of methylene (-CH₂-), ethylene (-CH₂CH₂-), and propylene (-CH₂CH₂CH₂-), even more preferably, the C₁-C₅ alkyl linker **(L)** may be selected from the group consisting of methylene (-CH₂-) or ethylene (-CH₂CH₂-), and most preferably, the C₁-C₅ alkyl linker **(L)** is methylene (-CH₂-).

The C₁-C₅ alkyl linker **(L)** is covalently coupled to a maleic acid moiety, wherein the maleic acid moiety has as functional groups a carboxy group (-COO-) and a carbamoyl group (-CONH-), or two carboxy groups (-COO-), or two carbamoyl groups (-CONH-), wherein the C₁-C₅ alkyl linker **(L)** is covalently coupled via one of said functional groups. Furthermore, the C₁-C₅ alkyl linker **(L)** is covalently coupled to a benzophenone substituent **(BP).** This means that a benzophenone substituent **(BP)** is covalently coupled to a functional group of a maleic acid moiety via a C₁-C₅ alkyl linker **(L).**

According to the invention, a C₁-C₅ alkyl linker **(L)** is functionalized with a benzophenone substituent **(BP),** wherein the C₁-C₅ alkyl linker **(L)** may have a hydroxy group -OH or an amino group -NH₂ as a functional group at the respective end not functionalized with the benzophenone substituent **(BP)** to be covalently coupled to a functional group of a maleic acid moiety.

Preferably, the present invention relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer represent -COOCH₂BP, -COOCH₂CH₂BP, -COOCH₂CH₂CH₂BP, -COOCH₂CH₂CH₂CH₂BP, -COOCH₂CH₂CH₂CH₂CH₂-BP, -CONHCH₂BP, -CONHCH₂CH₂BP, -CONHCH₂CH₂CH₂BP, -CONHCH₂CH₂CH₂CH₂BP, or -CONHCH₂CH₂CH₂CH₂CH₂BP, wherein **BP** is a benzophenone substituent.

In particular preferred embodiments, the present invention relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer represent -COOCH₂BP, or -CONHCH₂BP, wherein **BP** is a benzophenone substituent.

Preferably, the present invention relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with -CH₂BP, -CH₂CH₂BP, -CH₂CH₂CH₂BP, -CH₂CH₂CH₂CH₂-BP, or -CH₂CH₂CH₂CH₂CH₂-BP, wherein **BP** is a benzophenone substituent.

In particular preferred embodiments, the present invention relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with -CH₂BP, wherein **BP** is a benzophenone substituent.

The present invention preferably relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-) or two carbamoyl groups (-CONH-), and 1% to 50% of the functional groups of all maleic acid moieties in the vinylpyrrolidone copolymer represent 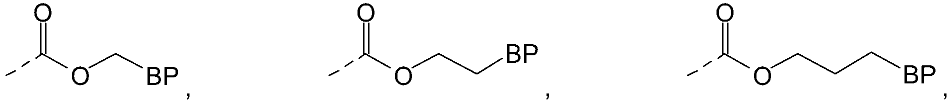 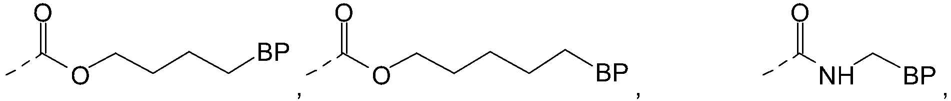 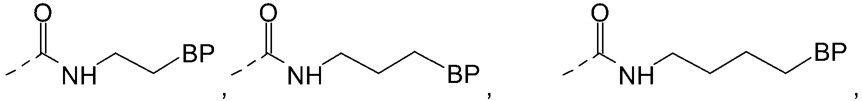 or 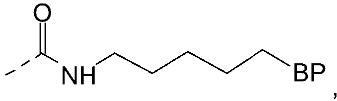 wherein **BP** is a benzophenone substituent.

In particular preferred embodiments, the present invention preferably relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-) or two carbamoyl groups (-CONH-), and 1% to 50% of the functional groups of all maleic acid moieties in the vinylpyrrolidone copolymer represent 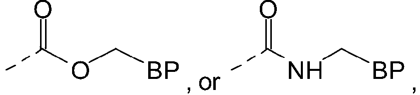 wherein **BP** is a benzophenone substituent.

Herein, the benzophenone substituent **BP** may be optionally substituted benzoylphenyl.

Thus, in some preferred embodiments, the present invention relates to a vinylpyrrolidone copolymer composed of blocks of *N*-vinylpyrrolidone (NVP) moietiesdivided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups have a residue selected from the group comprising or consisting of 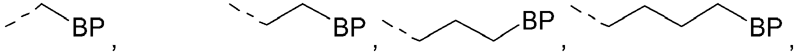 and 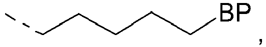 wherein **BP** is an optionally substituted benzoylphenyl.

In particularly preferred embodiments, the benzophenone substituent **BP** may represent 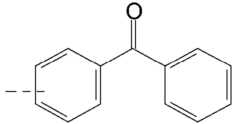 or preferably 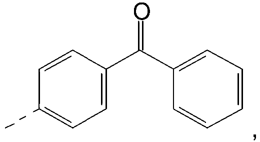 each of which may be optionally substituted or unsubstituted.

Preferably, the present invention relates to a vinylpyrrolidone copolymer composed of blocks of *N*-vinylpyrrolidone (NVP) moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups are functionalized with 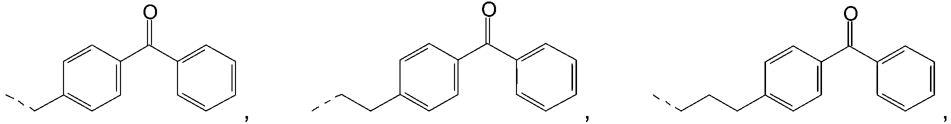 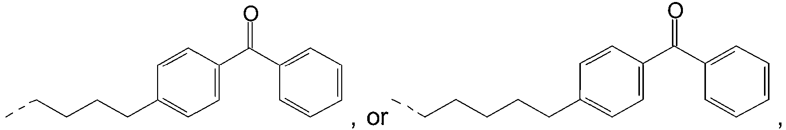 each of which may be optionally substituted or unsubstituted.

In particular preferred embodiments, the present invention relates to a vinylpyrrolidone copolymer composed of blocks of *N*-vinylpyrrolidone (NVP) moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups are functionalized with optionally substituted or unsubstituted

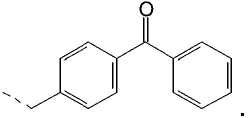

### C₁-C₅ Alkyl Linker L with functional group Y

In some preferred embodiments, the present invention relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the C₁-C₅ alkyl linker **(L)** not linked to the maleic acid moiety may have a functional group **Y,** wherein **Y** may be selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

In such embodiments, the benzophenone substituent is optionally substituted benzoylphenyl as further described in detail below.

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-.

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-);
**BP** represents an optionally substituted benzoylphenyl; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-.

Thus, the present invention preferably relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer represent 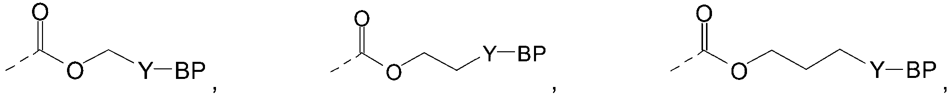 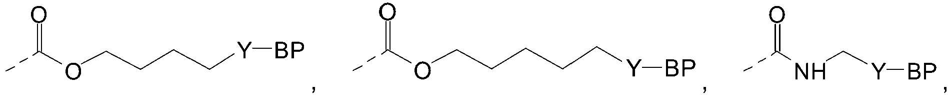 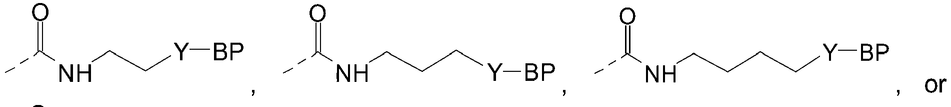 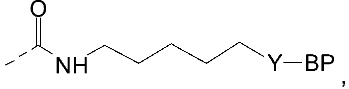 wherein **BP** is a benzophenone substituent, and wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-.

Thus, the present invention preferably relates to a vinylpyrrolidone copolymer composed of blocks of *N*-vinylpyrrolidone (NVP) moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with 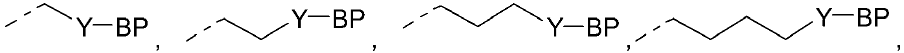 or 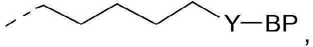
wherein **BP** is a benzophenone substituent, and
wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention preferably relates to vinylpyrrolidone copolymer composed of blocks of *N*-vinylpyrrolidone (NVP) divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with 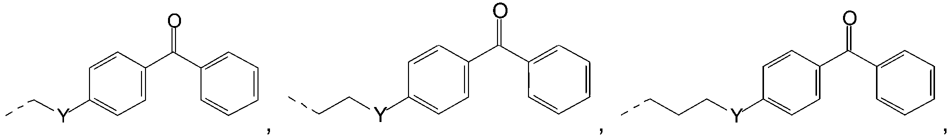 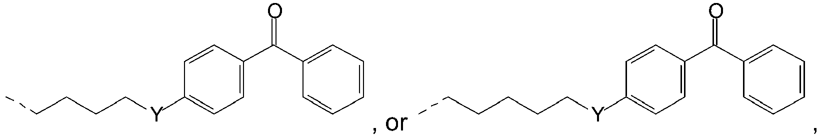 wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

In some preferred embodiments, **Y** may be selected from the group comprising or consisting of:
-OCO-, -NHCO-, -CO-, -O-, -NH-, -COO-, -CONH-, -NHCOO-, -OCONH-, -OCOO-, and -NHCONH-.

In some further preferred embodiments, **Y** may be selected from the group comprising or consisting of:
-OCO-, -NHCO-, -COO-, -CONH-, -NHCOO-, -OCONH-, -OCOO-, and -NHCONH-.

In some further preferred embodiments, **Y** may be selected from the group comprising or consisting of:
-OCO-, -NHCO-, -COO-, -CONH-, -NHCOO-, and -OCONH-.

In even more preferred embodiments, **Y** may be selected from the group comprising or consisting of: -OCO-, -NHCO-, -COO-, and -CONH-.

In some further preferred embodiments, **Y** may be selected from the group comprising or consisting of: -OCO-, and -COO-.

In some further preferred embodiments, **Y** may be selected from the group comprising or consisting of: -NHCO-, and -CONH-.

In some preferred embodiments, **Y** may be selected from the group comprising or consisting of:

-OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

Thus, in some preferred embodiments, the **Y-BP** may represent: 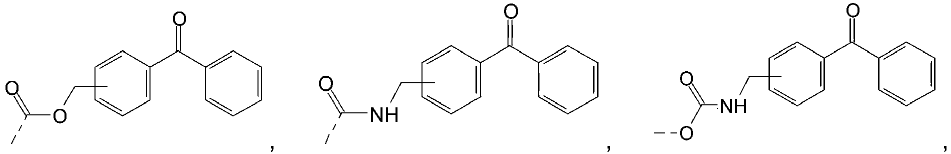 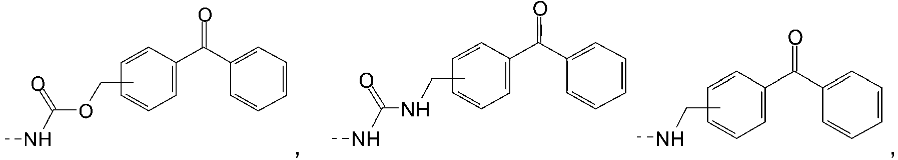 or 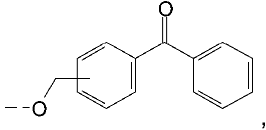 each of which may be optionally substituted or unsubstituted.

In some preferred embodiments, **Y** may be selected from the group consisting of or consisting of:
-OCOCH₂-, -NHCOCH₂-, -COOCH₂-, -CONHCH₂-, -NHCOOCH₂-, -OCONHCH₂-, -OCOOCH₂- and -NHCONHCH₂-.

In some further preferred embodiments, **Y** may be selected from the group consisting of or consisting of:
-COOCH₂-, -CONHCH₂-, -NHCOOCH₂-, -OCONHCH₂-, -OCOOCH₂- and -NHCONHCH₂-.

In some preferred embodiments, **Y** may be selected from the group consisting of or consisting of: -OCOCH₂-, -NHCOCH₂-, -COOCH₂-, -CONHCH₂-, -NHCOOCH₂-, and -OCONHCH₂-.

In some further preferred embodiments, **Y** may be selected from the group consisting of or consisting of:
-COOCH₂-, -CONHCH₂-, -NHCOOCH₂-, and -OCONHCH₂-.

In some further preferred embodiments, **Y** may be selected from the group comprising or consisting of: -OCOCH₂-, and -COOCH₂-.

In some further preferred embodiments, **Y** may be selected from the group comprising or consisting of:-NHCOCH₂-, and -CONHCH₂-.

In particular preferred embodiments, **Y** may be selected from the group comprising or consisting of: -COOCH₂-., and -CONHCH₂-.

In some preferred embodiments, **Y** may be selected from the group comprising or consisting of: -COO-, -CONH-, -COOCH₂-, and -CONHCH₂-.

Thus, in some preferred embodiments, the **Y-BP** may represent: 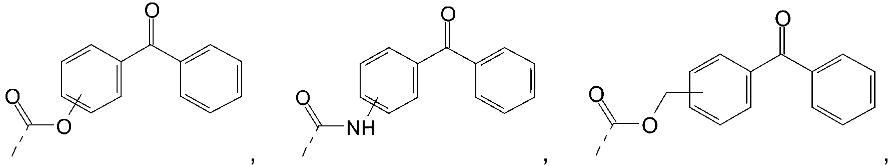 or 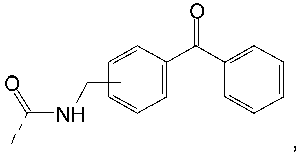 each of which may be optionally substituted or unsubstituted.

In further preferred embodiments, **Y** may be selected from the group comprising or consisting of -COO-, and -CONH-.

In further preferred embodiments, **Y** may be selected from the group consisting of or consisting of -COOCH₂-, and -CONHCH₂-.

In some even more preferred embodiments, the present invention relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the C₁-C₅ alkyl linker **(L)** not linked to the maleic acid moiety may have a functional group **Y,** wherein **Y** may be selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
with the proviso that when **Y** is -O-, the C₁-C₅ alkyl linker **(L)** is not ethylene (-CH₂CH₂-); or formulated differently
with the proviso that when **Y** is -O-, the C₁-C₅ alkyl linker **(L)** is selected from the group comprising or consisting of methylene (-CH₂-), propylene (-CH₂CH₂CH₂-), butylene (-CH₂CH₂CH₂CH₂-), and pentylene (-CH₂CH₂CH₂CH₂CH₂-).

In such embodiments, the benzophenone substituent is optionally substituted benzoylphenyl as further described in detail below.

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-,
with the proviso that when **Y** is -O-, the C₁-C₅ alkyl linker **(L)** is not ethylene (-CH₂CH₂-); or formulated differently
with the proviso that when **Y** is -O-, the C₁-C₅ alkyl linker **(L)** is selected from the group comprising or consisting of methylene (-CH₂-), propylene (-CH₂CH₂CH₂-), butylene (-CH₂CH₂CH₂CH₂-), and pentylene (-CH₂CH₂CH₂CH₂CH₂-).

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-);
**BP** represents an optionally substituted benzoylphenyl; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
with the proviso that when **Y** is -O-, the C₁-C₅ alkyl linker **(L)** is not ethylene (-CH₂CH₂-); or formulated differently
with the proviso that when **Y** is -O-, the C₁-C₅ alkyl linker **(L)** is selected from the group comprising or consisting of methylene (-CH₂-), propylene (-CH₂CH₂CH₂-), butylene (-CH₂CH₂CH₂CH₂-), and pentylene (-CH₂CH₂CH₂CH₂CH₂-).

In some of these further preferred embodiments, **Y** may be selected from the group comprising or consisting of:
-OCO-, -NHCO-, -COO-, -CONH-, -NHCOO-, -OCONH-, -OCOO-, and -NHCONH-.

In some further preferred embodiments, **Y** may be selected from the group comprising or consisting of:
-OCO-, -NHCO-, -COO-, -CONH-, -NHCOO-, and -OCONH-.

In even more preferred embodiments, **Y** may be selected from the group comprising or consisting of: -OCO-, -NHCO-, -COO-, and -CONH-.

In some further preferred embodiments, **Y** may be selected from the group comprising or consisting of: -OCO-, and -COO-.

In some further preferred embodiments, **Y** may be selected from the group comprising or consisting of: -NHCO-, and -CONH-.

In some preferred embodiments, **Y** may be selected from the group comprising or consisting of:
-OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

In some preferred embodiments, **Y** may be selected from the group consisting of or consisting of:
-OCOCH₂-, -NHCOCH₂-, -COOCH₂-, -CONHCH₂-, -NHCOOCH₂-, -OCONHCH₂-, -OCOOCH₂- and -NHCONHCH₂-.

In some further preferred embodiments, **Y** may be selected from the group consisting of or consisting of:
-COOCH₂-, -CONHCH₂-, -NHCOOCH₂-, -OCONHCH₂-, -OCOOCH₂- and -NHCONHCH₂-.

In some preferred embodiments, **Y** may be selected from the group consisting of or consisting of:
-OCOCH₂-, -NHCOCH₂-, -COOCH₂-, -CONHCH₂-, -NHCOOCH₂-, and -OCONHCH₂-.

In some further preferred embodiments, **Y** may be selected from the group consisting of or consisting of:
-COOCH₂-, -CONHCH₂-, -NHCOOCH₂-, and -OCONHCH₂-.

In some further preferred embodiments, **Y** may be selected from the group comprising or consisting of: -OCOCH₂-, and -COOCH₂-.

In some further preferred embodiments, **Y** may be selected from the group comprising or consisting of:-NHCOCH₂-, and -CONHCH₂-.

In particular preferred embodiments, **Y** may be selected from the group comprising or consisting of: -COOCH₂-., and -CONHCH₂-.

In some preferred embodiments, **Y** may be selected from the group comprising or consisting of: -COO-, -CONH-, -COOCH₂-, and -CONHCH₂-.

Thus, in some preferred embodiments, the **Y-BP** may represent: 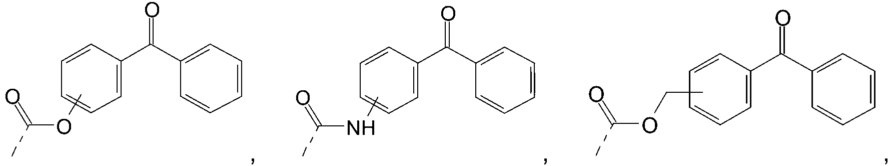 or 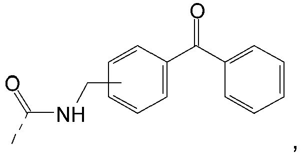 each of which is optionally substituted or unsubstituted.

In further preferred embodiments, **Y** may be selected from the group comprising or consisting of -COO-, and -CONH-.

In further preferred embodiments, **Y** may be selected from the group consisting of or consisting of -COOCH₂-, and -CONHCH₂-.

### Benzophenone Substituent BP

In the N-vinylpyrrolidone copolymers according to the invention, the benzophenone substituent **BP** is optionally substituted or unsubstituted

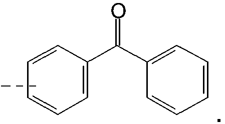

Thus, the benzophenone substituent **BP** may have one or more substituents including, but not limited to -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃. In some preferred embodiments, the benzophenone substituent may have one or more substituents including, but not limited to -OH, -NO₂, -COCH₃, -COOH, -CONH₂, -SO₃H, -SO₂NH₂, -OCH₃, -OCF₃, -NH₂, -NH(CH)₃, -N(CH₃)₂, and -SOCH₃.

Thus, **BP** may represent 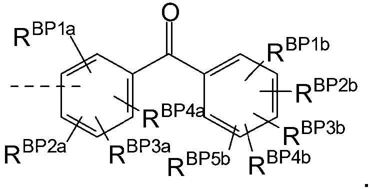 wherein **R^{BP1a}** - **R^{BP4a}** and **R^{BP1b}** - **R^{BP5b}** independently of each other represent -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

A benzophenone substituent **BP** represented by 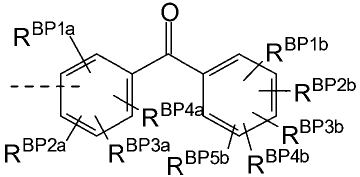 includes the following variations 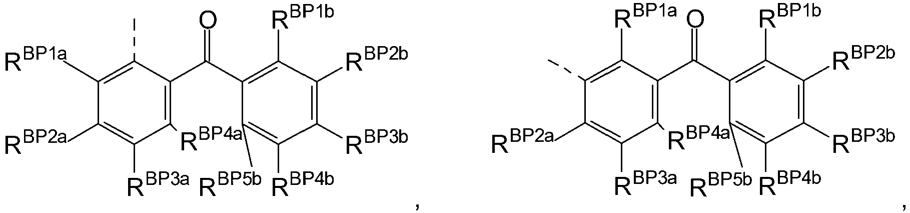 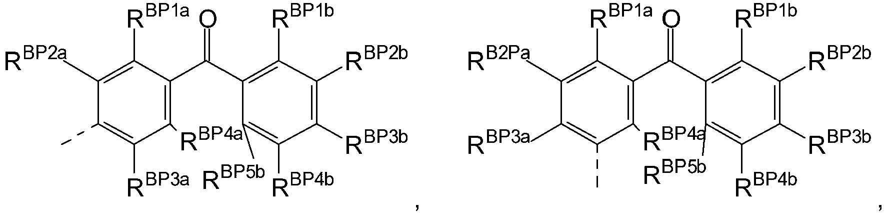 and 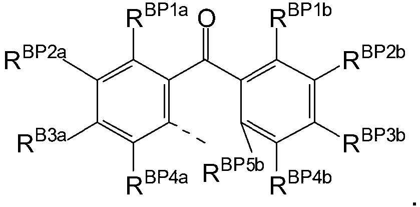 wherein **R^{BP1a}** - **R^{BP4a}** and **R^{BP1b}** - **R^{BP5b}** independently of each other represent -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, at least one of **R^{BP1a}** - **R^{BP4a},** and at least one of **R^{BP1b}** - **R^{BP5b}** is -H. Thus, in preferred embodiments, the benzophenone substituent **BP** representS 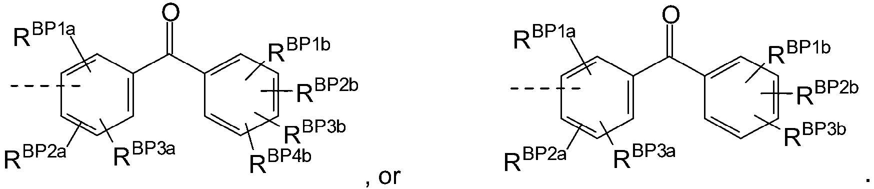 wherein **R^{BP1a}** - **R^{BP4a}** and **R^{BP1b}** - **R^{BP4b}** independently of each other represent -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituent **BP** represents 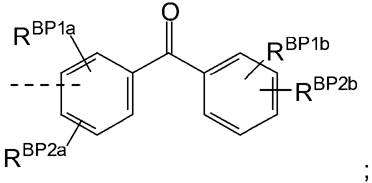 wherein **R^{BP1a}** - **R^{BP2a}** and **R^{BP1b}** - **R^{BP2b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In further preferred embodiments, the benzophenone substituent **BP** represents 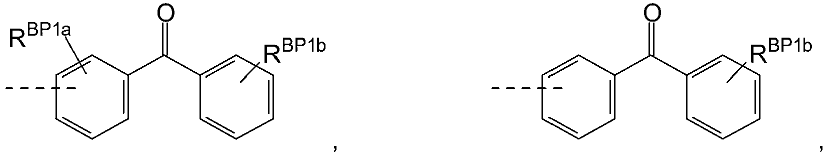 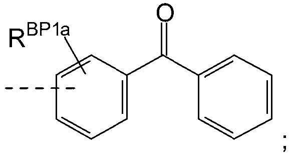 wherein **R^{BP1a}** and **R^{BP1b}** independently of each other represent -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

However, the benzophenone substituent **BP** is preferably an unsubstituted benzoylphenyl. Thus, benzoylphenyl is herein particularly preferred as benzophenone substituent **BP.** In particular preferred embodiments, the benzophenone substituent **BP** represents 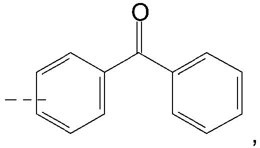 preferably

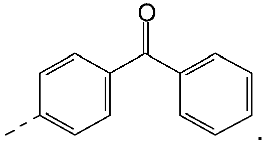

### Non-Benzophenone Substituent

PVP coatings are typically clear, transparent, hard and brittle. The negative mechanical properties can be improved with the use of plasticizers, e.g. one or more additional polymeric compounds can be blended into the PVP. Furthermore, the mechanical properties can also be improved by UV curing.

For example, polyethylene glycol (PEG) can be added to the PVP to improve the mechanical properties. PEG is a water-soluble non-toxic polymer with the general sum formula C₂ₙH₄ₙ₊₁Oₙ₊₁. The repeating unit of the linear polymer is -CH₂CH₂O-. PEGs with an average molecular mass of 200 to 35,000 g/mol are referred to in the prior art as polyethylene glycols, while PEGs with higher molecular mass from about 35,000 g/mol are referred to as polyethylene oxides. In the prior art, the term PEG is usually given together with a numerical value indicating the average relative molecular mass. Synonyms for polyethylene glycol (PEG) are polyethylene glycol, macrogol, PEO and E1521.

To improve the mechanical properties, the polyvinylpyrrolidone copolymer can be functionalized with, for example, polyethylene glycol (PEG), polylactide, polyglycolide or polyacrylate.

In some embodiments, vinylpyrrolidone copolymers are preferred, wherein up to 100% of the functional groups of all maleic acid moieties in the vinylpyrrolidone copolymer that are **not** functionalized with a benzophenone substituent (BP) via a C₁-C₅ alkyl linker **(L),** independently of each other have a non-benzophenone substitutent selected from the group comprising or consisting -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferabbly an integer between 35 and 50; wherein the ratio of benzophenone substituent **(BP)** to non-benzophenone substituent is ≥ 1, and the remaining functional groups of all maleic acid moieties in the vinylpyrrolidone copolymer have the residue -H.

In preferred embodiments, vinylpyrrolidone copolymers are preferred, wherein up to 100% of the functional groups of all maleic acid moieties in the vinylpyrrolidone copolymer that are **not** functionalized with a benzophenone substituent **(BP)** via a C₁-C₅ alkyl linker **(L),** have a non-benzophenone substituent selected from the group comprising or consisting of polyethylene glycol -(CH₂CH₂O)_{q}-H (polyethylene glycol), polylactide, polyglycolide and polyacrylate, wherein **q** is preferably an integer between 35 and 50; wherein the ratio of benzophenone substituent (BP) to non-benzophenone substituent is ≥ 1, and the remaining functional groups of all maleic acid moieties in the vinylpyrrolidone copolymer have the residue -H.

In some embodiments, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -CO-, -O-, -NH-, -COO-,
   -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-,
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, vinylpyrrolidone copolymers are further preferred, wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-);
**BP** represents an optionally substituted benzoylphenyl; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-,
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, vinylpyrrolidone copolymers are further preferred, wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In some embodiments, the present invention preferably relates to vinylpyrrolidone copolymers composed of blocks of N-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-,
with the proviso that when **Y** is -O-, the C₁-C₅ alkyl linker **(L)** is not ethylene (-CH₂CH₂-); or formulated differently
with the proviso that when **Y** is -O-, the C₁-C₅ alkyl linker **(L)** is selected from the group comprising or consisting of methylene (-CH₂-), propylene (-CH₂CH₂CH₂-), butylene (-CH₂CH₂CH₂CH₂-), and pentylene (-CH₂CH₂CH₂CH₂CH₂-),
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, vinylpyrrolidone copolymers are further preferred, wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

Thus, the present invention preferably relates to vinylpyrrolidone copolymers composed of blocks of *N*-vinylpyrrolidone moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-);
**BP** represents an optionally substituted benzoylphenyl; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-,
with the proviso that when **Y** is -O-, the C₁-C₅ alkyl linker **(L)** is not ethylene (-CH₂CH₂-); or formulated differently
with the proviso that when **Y** is -O-, the C₁-C₅ alkyl linker **(L)** is selected from the group comprising or consisting of methylene (-CH₂-), propylene (-CH₂CH₂CH₂-), butylene (-CH₂CH₂CH₂CH₂-), and pentylene (-CH₂CH₂CH₂CH₂CH₂-),
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, vinylpyrrolidone copolymers are further preferred, wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

### Molar Ratio of Main Monomer and Comonomer

To prepare hydrophilic polyvinylpyrrolidone copolymers with maleic acid moieties, the influence of the molar ratio of the two monomers *N*-vinylpyrrolidone (NVP) and maleic acid derivative to each other was investigated. Here, the synthesized polyvinylpyrrolidone copolymers composed of blocks of *N*-vinylpyrrolidone (NVP) moieties divided by maleic acid moieties, wherein each maleic acid moiety has as functional groups one carboxy group (-COO-) and one carbamoyl group (-CONH-), or two carboxy groups (-COO-), or two carbamoyl groups (-CONH-), and 1% to 50% of the functional groups of all maleic acidmoieties in the vinylpyrrolidone copolymer are functionalized with a benzophenone substituent **(BP)** via a C₁-C₅ alkyl linker **(L),** have a total content of maleic acid moieties in the copolymer of up to 5 mol%, exhibit very good properties in terms of flexibility, stretchability, deformability, degradation behavior, service life, shell life, suppleness, hydrophilicity and frictional resistance.

In preferred embodiments, the vinylpyrrolidone copolymer composed of blocks of *N*-vinylpyrrolidone moieties divided by maleic acid moieties has a total amount of maleic acid moieties in the vinylpyrrolidone copolymer of 0.1 - 10.0 mol%, preferably 0.1 - 5.0 mol%, more preferably 0.1 - 2 mol%. In some particularly preferred embodiments, the vinylpyrrolidone copolymers have a total amount of maleic acid moieties in the vinylpyrrolidone copolymer of 1.5 - 2.5 mol%, more preferably 2 mol%. In some further preferred embodiments, the vinylpyrrolidone copolymers have a total amount of maleic acid moieties in the vinylpyrrolidone copolymer of less than 1 mol%, preferably 0.1 - 0. 95 mol%, further preferably 0.1 - 0.90 mol%, more preferably 0.1 - 0.85 mol%, even more preferably 0.5 - 0.85 mol%.

In preferred embodiments, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

### Variant 1 of preferred N-vinylpyrrolidone copolymers according to the invention

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl.

Preferably, the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

In preferred embodiments, the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the linker **(L)** not linked to the maleic acid moiety has a functional group **Y,** wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

In further preferred embodiments, up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

In still further preferred embodiments, up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

Thus, the present invention preferably relates to a vinylpyrrolidone copolymer of the general formula (i): 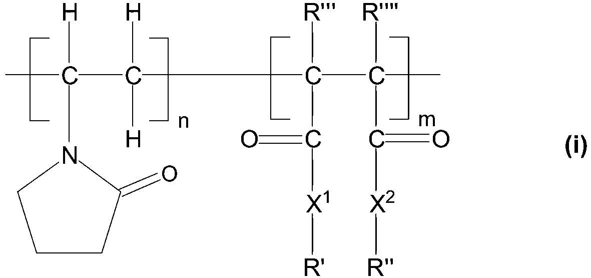
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 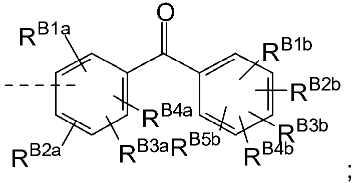
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein
**m** = 1.5 - 2.5 mol%, more preferably 2 mol%; and
**n** = 100 mol% - m.

In preferred embodiments, the present invention preferably relates to a vinylpyrrolidone copolymer of the general formula **(i'):** 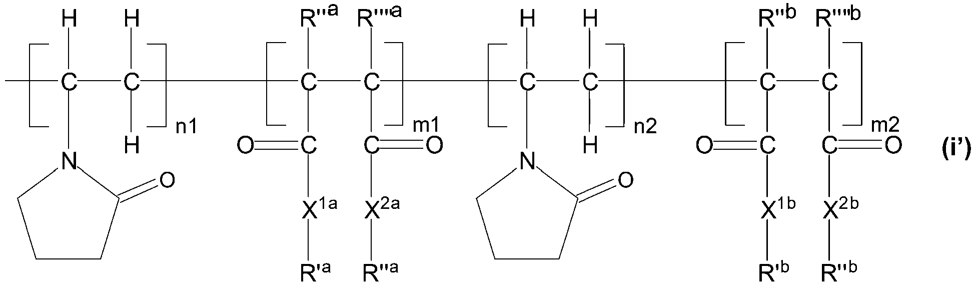
wherein **m1** + **m2 = m** = 0.1 - 10.0 mol%, and **n1** + **n2** = **n** = 100 mol% - m;
**X^{1a}**, **X^{2a}, X^{1b}** and **X^{2b}** independently of each are selected other from -O- and -NH-;
**p%** of the **R'^{a}, R"^{a}, R'^{b}** and **R"^{b}** independently of each other are selected from -L'-BP' and -L"-BP"; and
up to **p%** of **R'^{a}, R"^{a}, R'^{b}** and **R"^{b}** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'^{a}, R"^{a}, R'^{b} and R"^{b}** are -H;
**R‴^{a} , Rʺʺ^{a}, R‴^{b}** and **Rʺʺ^{b}** represent independently of each other -H, -CH₃, - CH₂CH₃, or -F; preferably, **R‴^{a}, Rʺʺ^{a}, R‴^{b}** and **Rʺʺ^{b}** represent-H;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**BP'** and **BP"** represent independently of each other 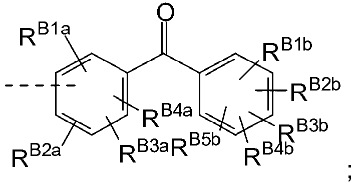
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 5 and 900, preferably **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is about 25. In most preferred embodiments **p** is about 50.

Preferred are vinylpyrrolidone copolymers of the general formula **(i),** wherein
**m1 + m2 = m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n1 + n2 = n** = 100 mol% - m.

Further preferred are vinylpyrrolidone copolymers of the general formula (i), wherein
**m1 + m2 = m** = 1.5 - 2.5 mol%, more preferably 2 mol%; and
**n1 + n2** = **n** = 100 mol% - m.

The present invention preferably relates to vinylpyrrolidone copolymers of the general formula **(i):** 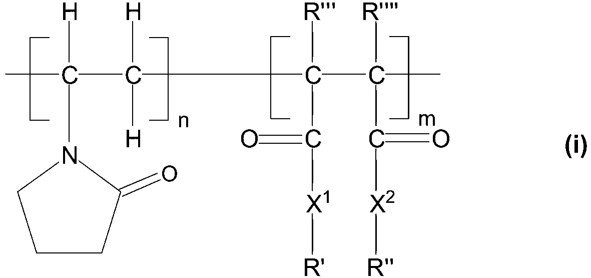 wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%;
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 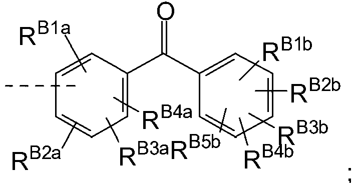
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein only one of **X¹** and **X²** is -NH-. Thus, preferred are vinylpyrrolidone copolymers of the general formula **(i),** wherein **X¹** and **X²** are not both -NH-.

Preferred are vinylpyrrolidone copolymers of the general formula **(i),** wherein **Y'** and **Y"** represent a bond.

Preferred are vinylpyrrolidone copolymers of the general formula **(i),** wherein **BP'** and **BP"** represent

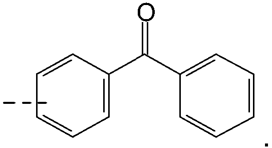

Preferred are vinylpyrrolidone copolymers of the general formula **(i),** wherein up to **p%** of **R'** and **R"** are -(CH₂CH₂O)_{q}-H, and **p** is a number from 1 - 50, wherein **q** is an integer between 35 and 50.

In preferred embodiments, the present invention preferably relates to a vinylpyrrolidone copolymer of the general formula **(i'):** 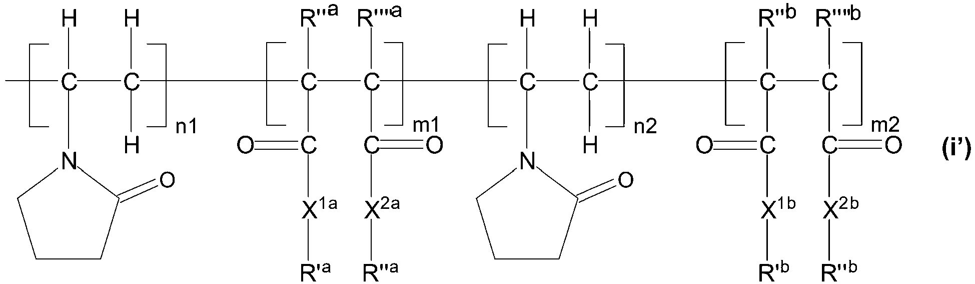
**m1 + m2 = m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n1 + n2 = n =** 100 mol% - m**.**
**X^{1a}, X^{2a}, X^{1b}** and **X^{2b}** independently of each are selected other from -O- and -NH-;
**p%** of the **R'^{a}, R"^{a}, R'^{b}** and **R"^{b}** independently of each other are selected from -L'-BP' and -L"-BP"; and
up to **p%** of **R'^{a}, R"^{a}, R'^{b}** and **R"^{b}** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'^{a}, R"^{a}, R'^{b} and R"^{b}** are -H;
**R‴^{a} , Rʺʺ^{a}, R‴^{b}** and **Rʺʺ^{b}** represent independently of each other -H, -CH₃, - CH₂CH₃, or -F; preferably, **R‴^{a}, Rʺʺ^{a}, R‴^{b}** and **Rʺʺ^{b}** represent-H;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**BP'** and **BP"** represent independently of each other 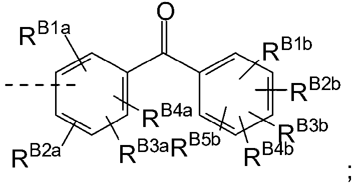
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 5 and 900, preferably **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i'), wherein only one of **X^{1a}** snf **X^{2a}** is -NH-, wherein only one of **X^{1b}** and **X^{2b}** is -NH-. Preferred are vinylpyrrolidone copolymers of the general formula (i), wherein only one of **X^{1a}, X^{2a}, X^{1b}** and **X^{2b}** is -NH-.

Preferred are vinylpyrrolidone copolymers of the general formula **(i'),** wherein **L'** and **L"** represent -CH₂-.

Preferred are vinylpyrrolidone copolymers of the general formula **(i'),** wherein **BP'** and **BP"** represent

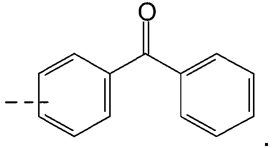

Preferred are vinylpyrrolidone copolymers of the general formula **(i),** wherein up to p% of **R'** and **R"** are -(CH₂CH₂O)_{q}-H, and **p** is a number from 1 - 50, wherein **q** is an integer between 35 and 50.

The present invention therefore also relates to vinylpyrrolidone copolymers of the general formula **(i):** 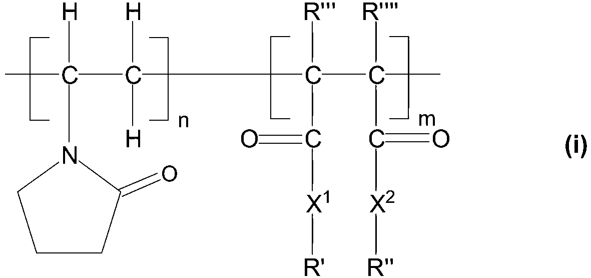
wherein **m** = 0.1 - 10.0 mol% and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **R‴′** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 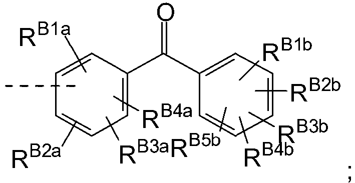
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein
**m** = 1.5 - 2.5 mol%, more preferably 2 mol%; and
**n** = 100 mol% - m.

The present invention therefore also relates to vinylpyrrolidone copolymers of the general formula (i): 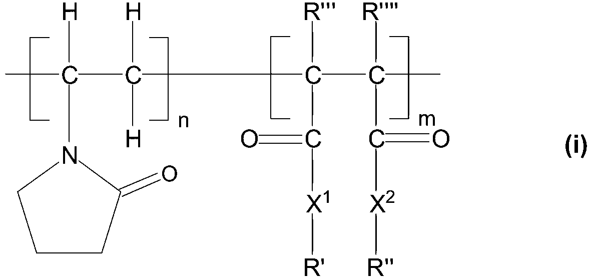
wherein **m** = 0.1 - 10.0 mol% and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **R‴′** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 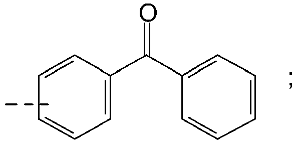
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(i),** wherein **m** = 1.5 - 2.5 mol%, more preferably 2 mol%; and **n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C₁-C₅ alkyl linker **(L)** not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein up to 100% of the functional groups of all maleic acid moieties in the vinylpyrrolidone copolymer that are **not** functionalized with a benzophenone substituent **(BP)** via a C₁-C₅ alkyl linker (L) have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50; wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
wherein up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula (I):
   formula (i): 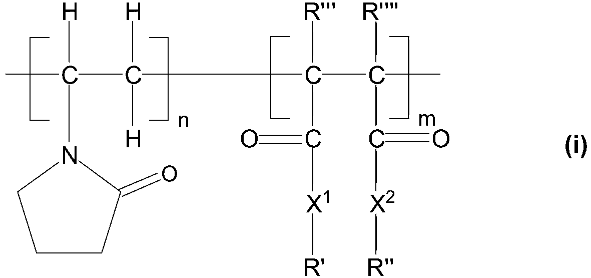
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and R" are -H;
**R‴** and **R‴′** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 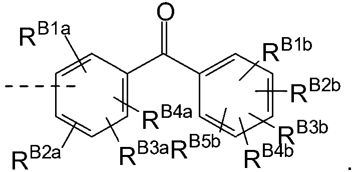
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments p is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(i),** wherein **m** = 1.5 - 2.5 mol%, more preferably 2 mol%; and **n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 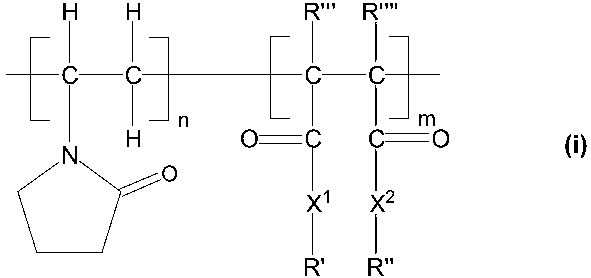
wherein **m** = 0.1 - 10.0 mol% and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 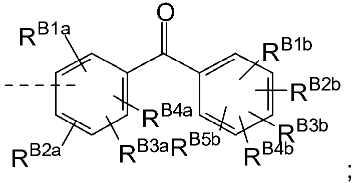
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 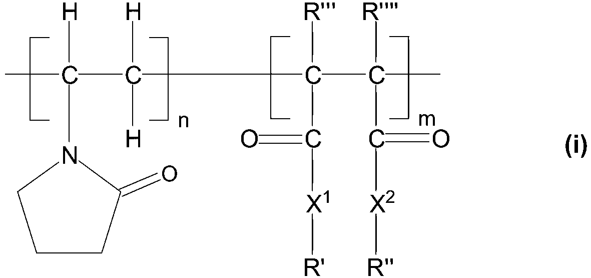
wherein **m** = 0.1 - 10.0 mol% and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **R‴′** represent independently of each other -H, -CH₃, -CH₂CH₃, or -F;
L' and L" represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 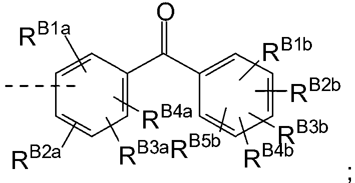
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein
**m** = 1.5 - 2.5 mol%, more preferably 2 mol%; and
**n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 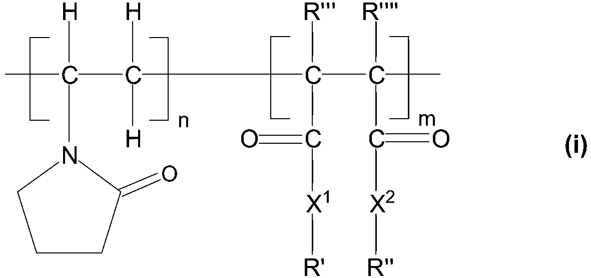
wherein **m** = 0.1 - 10.0 mol% and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **R′‴** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represents 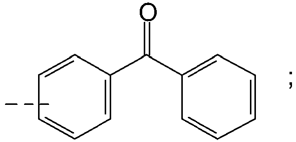
**p** represents a number from 1 - 50; and wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein
**m** = 1.5 - 2.5 mol%, more preferably 2 mol%; and
**n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 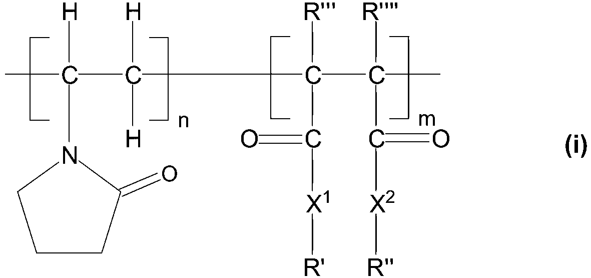
wherein **m** = 0.1 - 10.0 mol% and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **R′‴** represent -H; L' and L" represent -CH₂-; **Y'** and **Y"** represent a bond;
**BP'** and **BP"** represent independently of each other 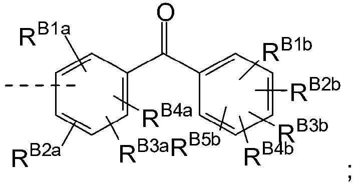
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula (i): 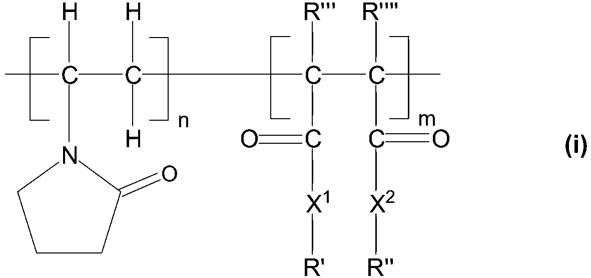
wherein **m** = 0.1 - 10.0 mol% and n = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **R′‴** represent -H; **L'** and **L"** represent -CH₂-; **Y'** and **Y"** represent a bond;
**BP'** and **BP"** represent 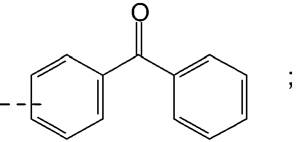
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(i),** wherein **m** = 1.5 - 2.5 mol%, more preferably 2 mol%; and **n** = 100 mol% - m.

### Variant 2 of preferred N-vinylpyrrolidone copolymers according to the invention

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.

Preferably, L is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

Preferably, the benzophenone substituent **BP** is optionally substituted benzoylphenyl, more preferably benzoylphenyl.

Preferably, **Y** represents a bond, -OCO-, -NHCO-, -COO-, -CONH-, -OCOCH₂-, -NHCOCH₂-, -COOCH₂-, or -CONHCH₂-.

More preferably, **Y** represents a bond, -NHCO-, -CONH-, -NHCOCH₂-, or -CONHCH₂-.

In preferred embodiments, up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

Thus, the present invention preferably relates to a vinylpyrrolidon copolymer of general formula **(ii):** 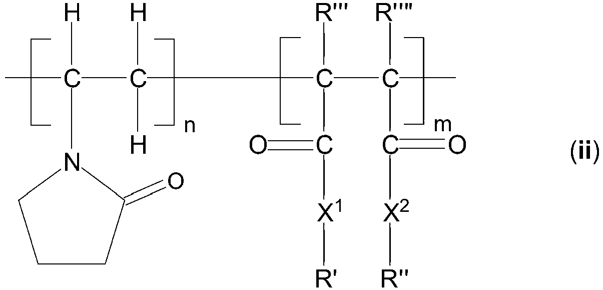
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and the remaining residues **R'** and **R"** are -H;
**R‴** and **R′‴** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, and -NHCONHCH₂-,
**BP'** and **BP"** represent independently of each other 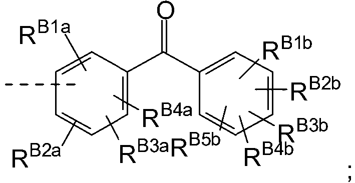
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferably, only one of **X¹** and **X²** is -NH-.

Preferred are vinylpyrrolidone copolymers of the general formula **(ii),** wherein only one of **X¹** and **X²** is -NH-. Thus, preferred are vinylpyrrolidone copolymers of the general formula **(ii),** wherein **X¹** and **X²** are not both -NH-.

Preferred are vinylpyrrolidone copolymers of the general formula **(ii),** wherein up to **p%** of **R'** and **R"** are -(CH₂CH₂O)_{q}-H and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein **Y'** and **Y"** represent a bond.

In preferred embodiments, the present invention preferably relates to a vinylpyrrolidone copolymer of the general formula **(ii'):** 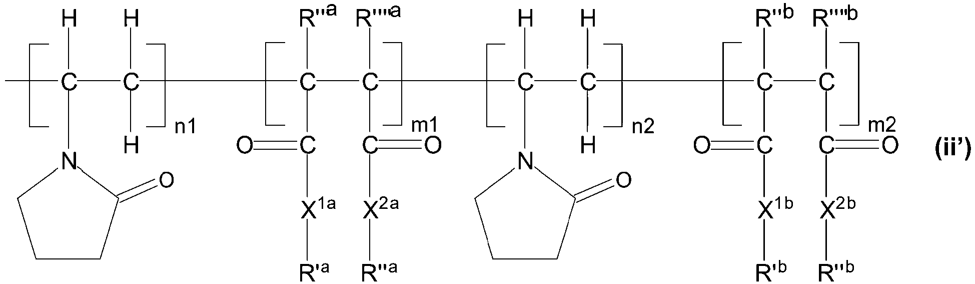
**m1 + m2 = m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n1 + n2 = n** = 100 mol% - m.
**X^{1a}**, **X^{2a}**, **X^{1b}** and **X^{2b}** independently of each are selected other from -O- and -NH-;
**p%** of the **R'^{a}**, **R"^{a}**, **R'^{b}** and **R"^{b}** independently of each other are selected from -L'-Y'-BP' and -L"-Y"-BP"; and
up to **p%** of **R'^{a}**, **R"^{a}**, **R'^{b}** and **R"^{b}** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'^{a}, R"^{a}**, **R'^{b}** and **R"^{b}** are -H;
**R‴^{a}**, **R′‴^{a}**, **R‴**^{b} and **R′‴^{b}** represent independently of each other -H, -CH₃, - CH₂CH₃, or -F; preferably, **R‴^{a}**, **R′‴^{a}**, **R‴^{b}** and **R‴^{b}** represent-H;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, and -NHCONHCH₂-,
**BP'** and **BP"** represent independently of each other 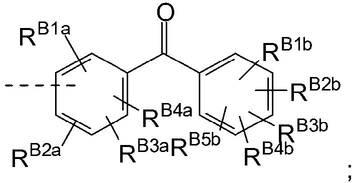
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 5 and 900, preferably **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein only one of **X^{1a}, X^{2a}, X^{1b}** and **X^{2b}** is -NH-.

Preferred are vinylpyrrolidone copolymers of the general formula **(ii),** wherein up to **p%** of **R'^{a}**, **R"^{a}**, **R'^{b}** and **R"^{b}** are -(CH₂CH₂O)_{q}-H and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein **Y'** and **Y"** represent a bond.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; and
wherein the benzophenone substituent **BP** is optionally substituted benzoylphenyl, preferably benzoylphenyl;
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H, ; and
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; and
wherein the benzophenone substituent **BP** is optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H, ; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of N̅-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 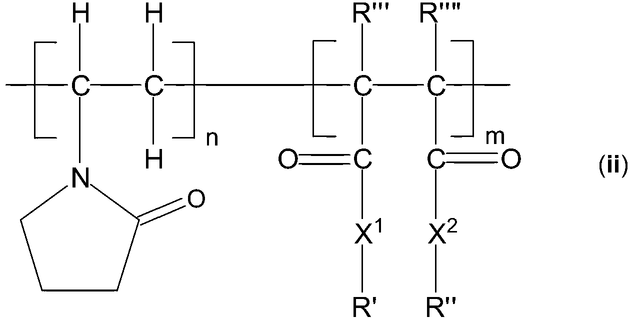
wherein **m** = 0.1 - 10.0 mol% and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R‴′** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
**BP'** and **BP"** represent independently of each other 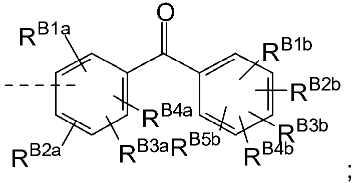
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 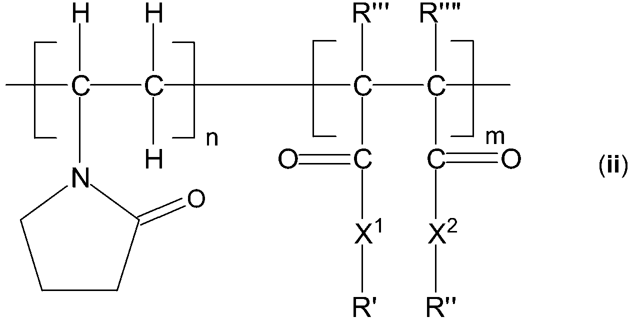
wherein m = 0.1 - 10.0 mol%, and n = 100 mol% - m;
**X¹** and **X²** independently of each are selected other from -O- and -NH-; wherein only one of **X¹** and **X²** is -NH-;
p% of the residues R' and R" independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and Rʺʺ represent independently of each other -H, -CH₃, -CH₂CH₃, or -F;
L' and L" represent -CH₂-, -CH₂CH₂-, -CH₂CH₂CH₂-, -CH₂CH₂CH₂CH₂-, or -CH₂CH₂CH₂CH₂CH₂-;
Y' and Y" independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
BP' and BP" represent independently of each other 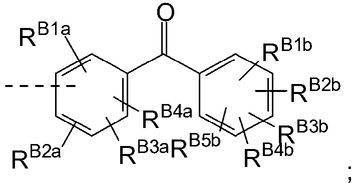
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 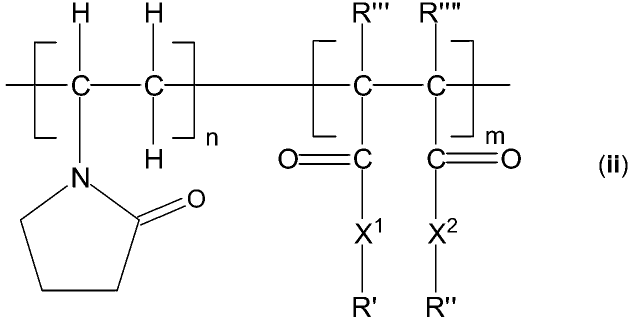
wherein m = 0.1 - 10.0 mol%, and n = 100 mol% - m;
**X¹** and **X²** independently of each are selected other from -O- and -NH-; wherein only one of **X¹** and **X²** is -NH-;
p% of the residues R' and R" independently of each other are selected from -L'-Y'-BP' and -L"-Y"-BP"; and
up to p% of **R'** and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and R" are -H;
R‴ and Rʺʺ represent independently of each other -H, -CH₃, -CH₂CH₃, or -F;
**L'** and L" represent -CH₂-, -CH₂CH₂-, -CH₂CH₂CH₂-, -CH₂CH₂CH₂CH₂-, or -CH₂CH₂CH₂CH₂CH₂-;
Y' and Y" independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, and -NHCONHCH₂-.
**BP'** and BP" represent independently of each other 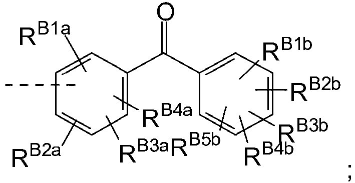
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 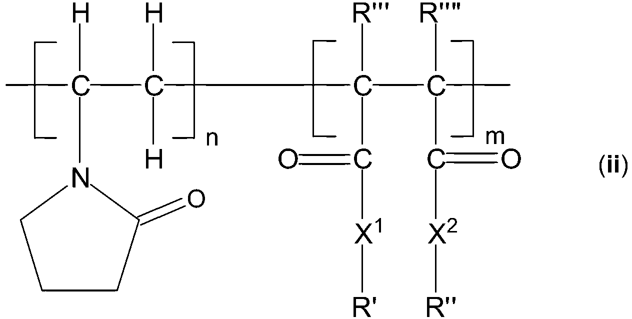
wherein m = 0.1 - 10.0 mol%; and n = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -OCOCH₂-, -NHCOCH₂-, -COOCH₂-, and -CONHCH₂-;
**BP'** and **BP"** represent independently of each other 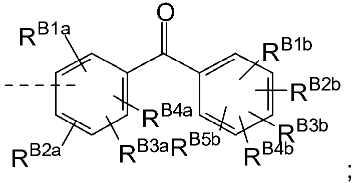
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 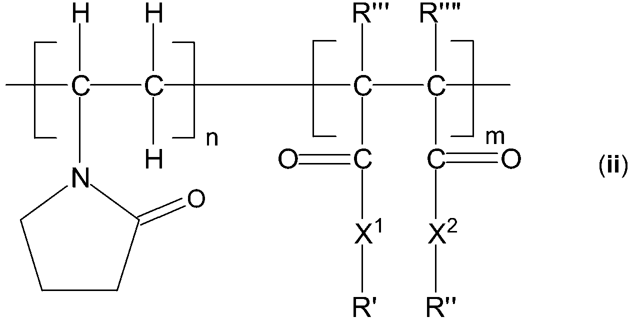
wherein **m** = 0.1 - 10.0 mol% and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -NHCO-, -CONH-, -NHCOCH₂-, and -CONHCH₂-;
BP' and BP" represent independently of each other 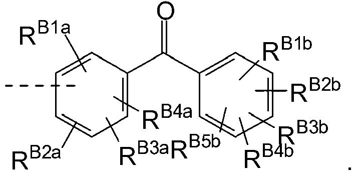
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 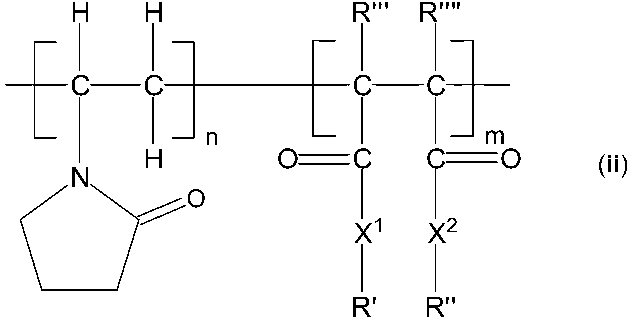
wherein m = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, and -NHCONHCH₂-.
**BP'** and **BP"** represent independently of each other 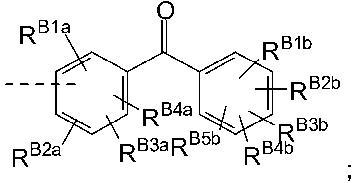
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 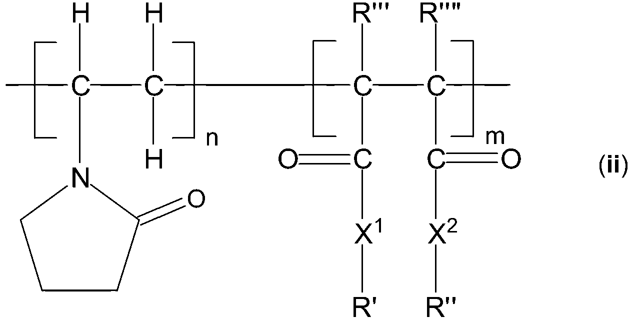
wherein m = 0.1 - 10.0 mol% and n = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -OCOCH₂-, -NHCOCH₂-, -COOCH₂-, or -CONHCH₂-;
**BP'** and **BP"** represent independently of each other 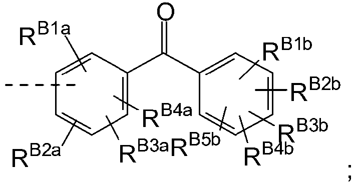
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 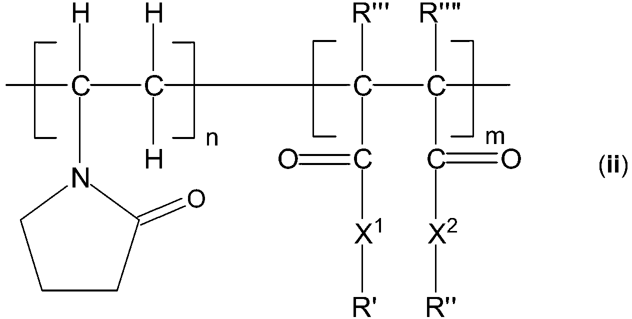
wherein **m** = 0.1 - 10.0 mol% and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to up to p% of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -NHCO-, -CONH-, -NHCOCH₂-, and -CONHCH₂-;
**BP'** and **BP"** represent independently of each other 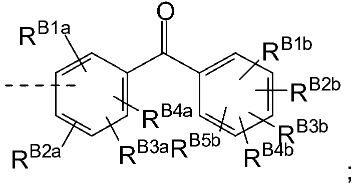
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 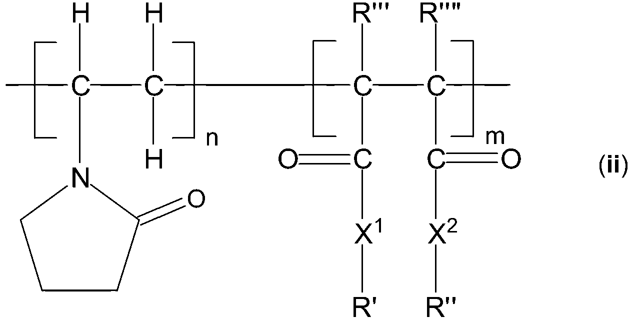
wherein m = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, and -NHCONHCH₂-.
**BP'** and **BP"** represent 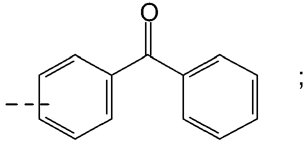
**p** represents a number from 1 - 50; and wherein **q** is an integer between 35 and 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein
**m** = 1.5 - 2.5 mol%, more preferably 2 mol%; and
**n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 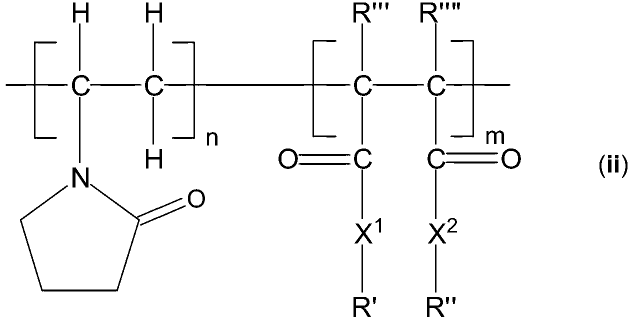
wherein
**m** = 0.1 - 10.0 mol% and
**n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -OCOCH₂-, -NHCOCH₂-, -COOCH₂-, or -CONHCH₂-;
**BP'** and **BP"** represents 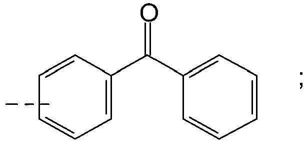
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 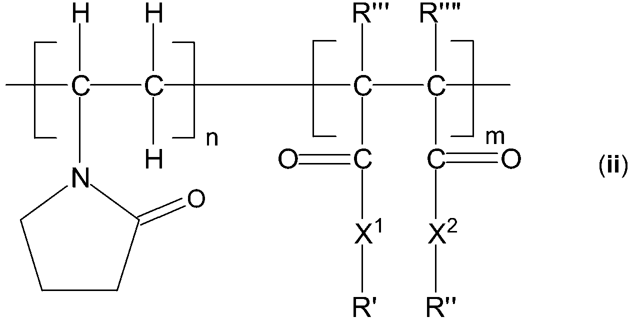
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -NHCO-, -CONH-, -NHCOCH₂-, and -CONHCH₂-;
**BP'** and **BP"** represent 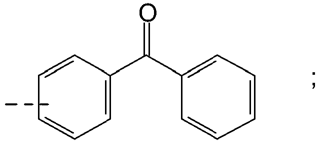
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 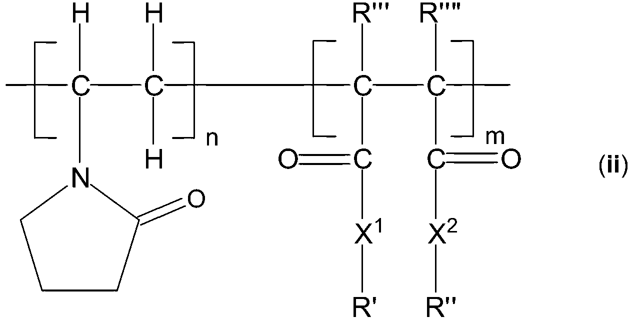
wherein **m** = 0.1 - 10.0 mol% and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 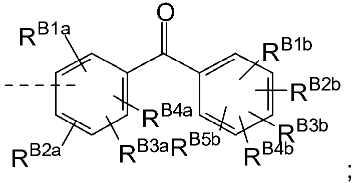
**R^{B1a}** _ **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 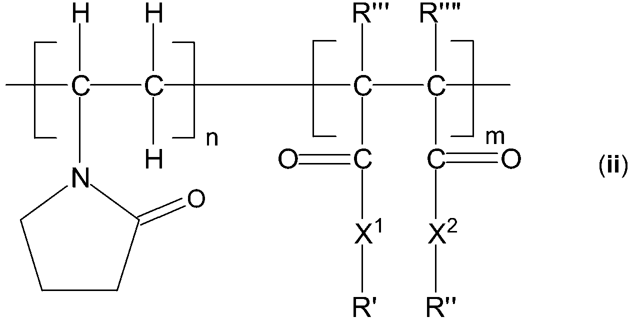
wherein **m** = 0.1 - 10.0 mol% and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 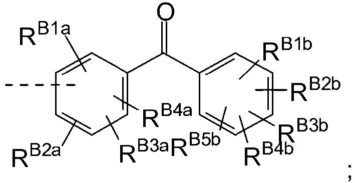
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein m = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 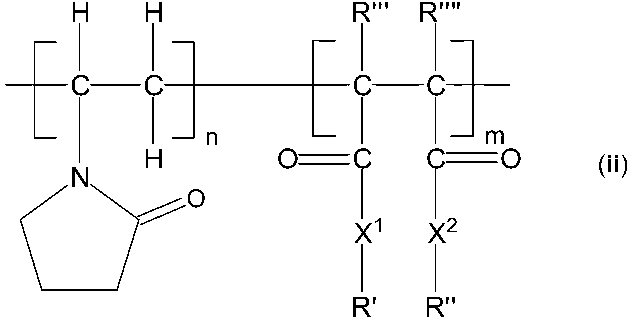
wherein **m** = 0.1 - 10.0 mol% and n = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 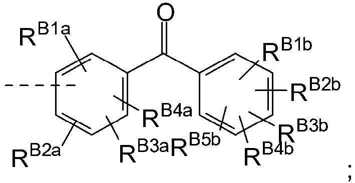
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 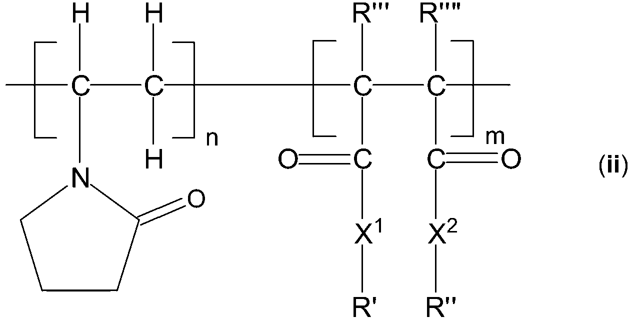
wherein **m** = 0.1 - 10.0 mol% and **n** = 100 mol% - m;
**X¹** and **X²** independently of each are selected other from -O- and -NH-; wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent 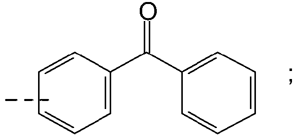
**p** represents a number from 1 - 50; and wherein **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 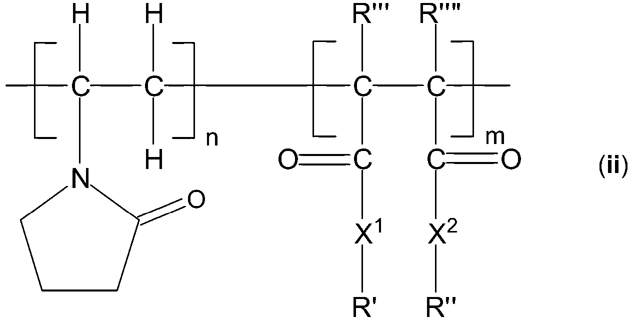
wherein m = 0.1 - 10.0 mol%, and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
**BP'** and **BP"** represent independently of each other 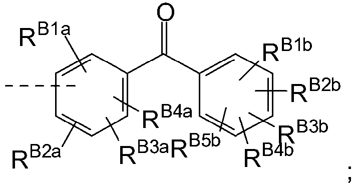
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 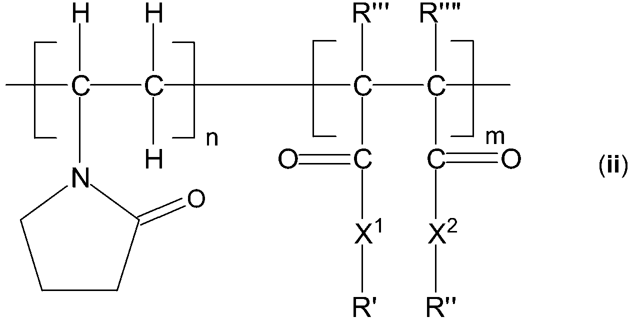
wherein m = 0.1 - 10.0 mol%, and n = 100 mol% - m;
**X¹** and **X²** independently of each are selected other from -O- and -NH-; wherein only one of **X¹** and **X²** is -NH-;
p% **of the** residues **R'** and **R"** independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and R"" represent independently of each other -H, -CH₃, -CH₂CH₃, or -F;
L' and L" represent -CH₂-; Y' and Y" represent a bond;
BP' and BP" represent independently of each other 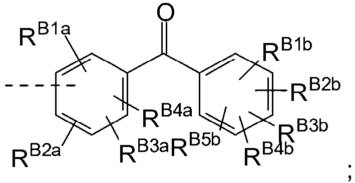
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 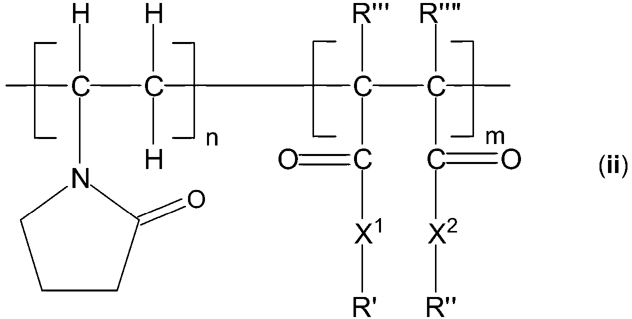
wherein m = 0.1 - 10.0 mol%. and n = 100 mol% - m;
**X¹** and **X²** independently of each are selected other from -O- and -NH-; preferably wherein only one of **X¹** and **X²** is -NH-;
p% **of the residues** R' and R" independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and Rʺʺ represent independently of each other -H, -CH₃, -CH₂CH₃, or -F; **L'** and L" represent -CH₂-; **Y'** and Y" represent a bond;
**BP'** and **BP"** represent independently of each other 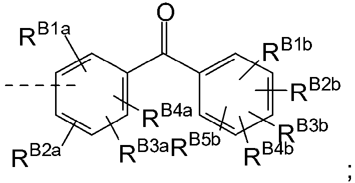
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 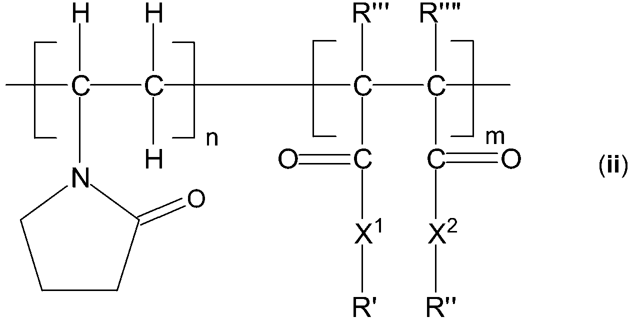
wherein m = 0.1 - 10.0 mol%, and n = 100 mol% - m;
**X¹** and **X²** independently of each are selected other from -O- and -NH-; preferably wherein only one of **X¹** and **X²** is -NH-;
p% of the residues R' and R" independently of each other are selected from -L'-Y'-BP' and -L"-Y"-BP"; and
up to p% of R' and R" are -(CH₂CH₂O)_{q}-H; and the remaining residues R' and R" are -H;
R‴ and R"" represent independently of each other -H, -CH₃, -CH₂CH₃, or -F;
L' and L" represent -CH₂-; Y' and Y" represent a bond;
BP' and BP" represent independently of each other 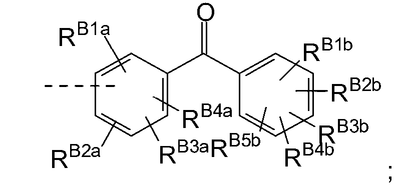
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 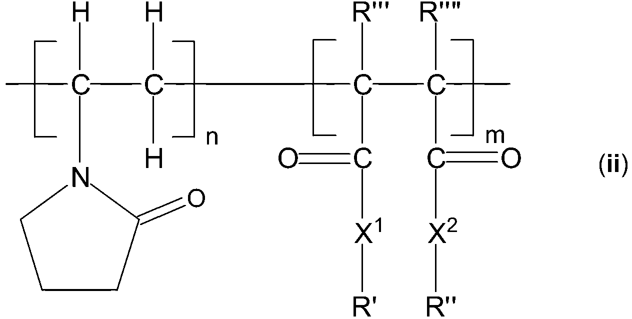
wherein m = 0.1 - 10.0 mol%, and n = 100 mol% - m;
**X¹** and **X²** independently of each are selected other from -O- and -NH-; wherein only one of **X¹** and **X²** is -NH-.
p% of the residues R' and R" independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and R"" represent independently of each other -H, -CH₃, -CH₂CH₃, or -F;
L' and L" represent -CH₂-, -CH₂CH₂-, -CH₂CH₂CH₂-, -CH₂CH₂CH₂CH₂-, or -CH₂CH₂CH₂CH₂CH₂-;
Y' and Y" represent a bond;
**BP'** and **BP"** represent 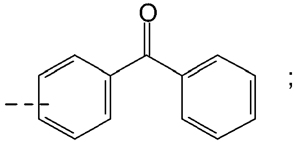
p represents a number from 1 - 50; and wherein q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 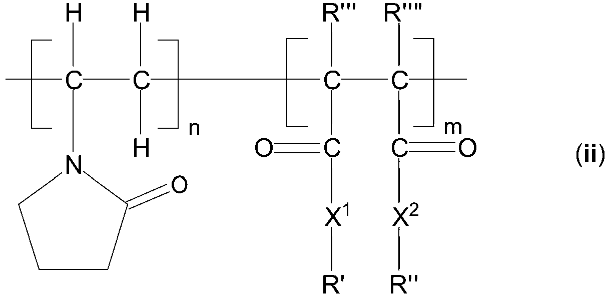
wherein m = 0.1 - 10.0 mol%, and n = 100 mol% - m;
**X¹** and **X²** independently of each are selected other from -O- and -NH-; p% **of the** residues **R'** and **R"** independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and R"" represent -H; L' and L" represent -CH₂-;
Y' and Y" independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
**BP'** and **BP"** represent independently of each other 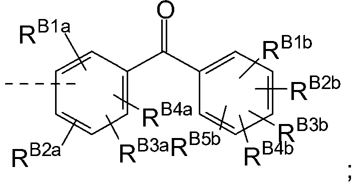
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 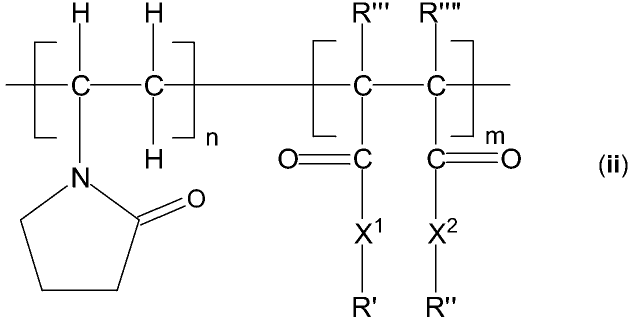
wherein m = 0.1 - 10.0 mol%, and n = 100 mol% - m;
**X¹** and **X²** independently of each are selected other from -O- and -NH-; p% of the residues R' and R" independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and R"" represent -H; L' and L" represent -CH₂-;
Y' and Y" independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
BP' and BP" represent independently of each other 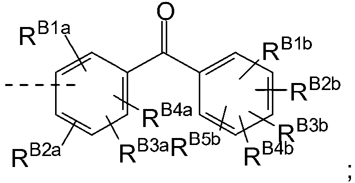
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 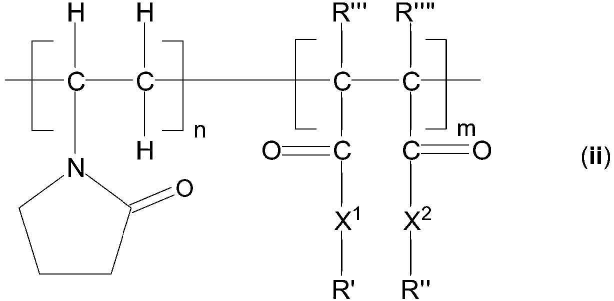
wherein m = 0.1 - 10.0 mol%, and n = 100 mol% - m;
**X¹** and **X²** independently of each are selected other from -O- and -NH-; wherein only one of **X¹** and **X²** is -NH-.
p% of the residues R' and R" independently of each other are selected from -L'-Y'-BP' and -L"-Y"-BP"; and
up to p% of R' and R" independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues R' and R" are -H;
R‴ and R"" represent -H; **L'** and L" represent -CH₂-; **Y'** and Y" represent a bond;
**BP'** and **BP"** represent independently of each other 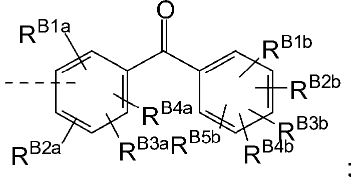
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
p is a number from 1 - 50; and q is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, p is (about) 25. In most preferred embodiments p is (about) 50.

Preferred are vinylpyrrolidone copolymers of the general formula (ii), wherein
m = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (ii), wherein m = 1.5 - 2.5 mol%, more preferably 2 mol%; and n = 100 mol% - m.

The present invention further relates to a vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 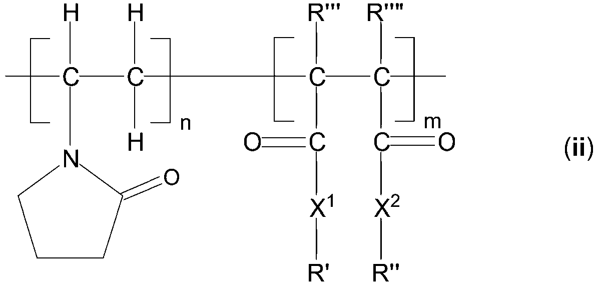
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent independently of each other 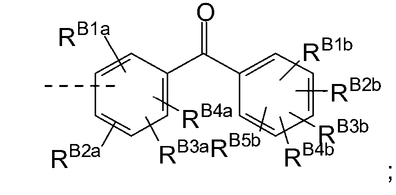
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 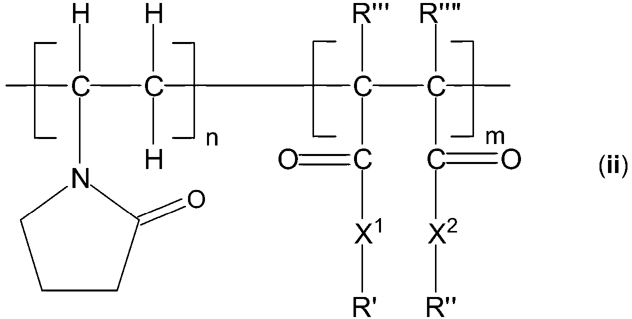
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent independently of each other 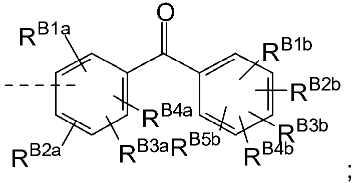
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**ii**): 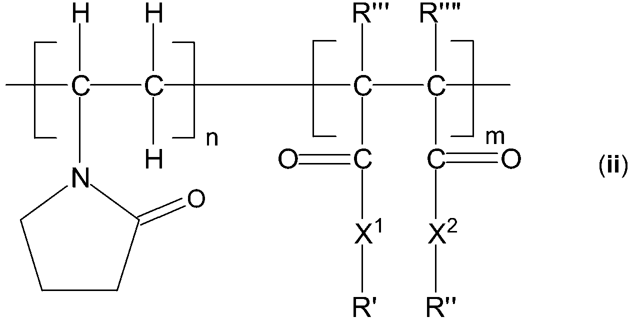
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent 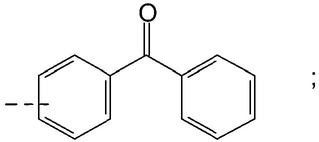
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**ii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**ii**), wherein **m** = 1.5 - 2.5 mol%, more preferably 2 mol%; and **n** = 100 mol% - m.

### Variant 3 of preferred N-vinylpyrrolidone copolymers according to the invention

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-.

Preferably, **L** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

Preferably, the benzophenone substituent **BP** is optionally substituted benzoylphenyl, more preferably benzoylphenyl.

Preferably, **Y** represents a bond, -OCOCH₂-, -NHCOCH₂-, -COOCH₂-, -CONHCH₂-, -NHCOOCH₂-, or -OCONHCH₂-.

More preferably, **Y** represents a bond, -OCOCH₂-, -NHCOCH₂-, -COOCH₂-, or -CONHCH₂-.

In preferred embodiments, up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

Thus, the present invention preferably relates to a vinylpyrrolidon copolymer of general formula (**ii**): 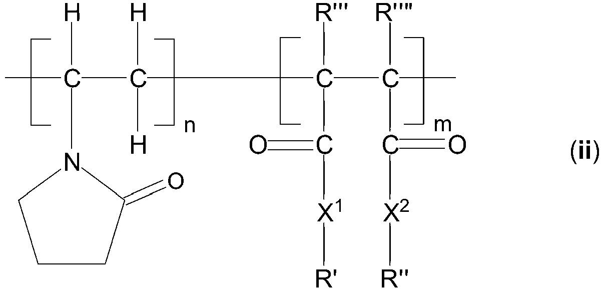
wherein **m** = 0.1 - 10.0 mol% and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 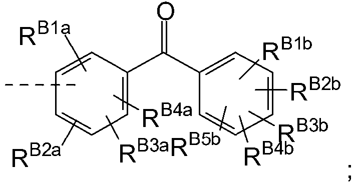
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**ii**), wherein only one of **X¹** and **X²** is -NH-. Thus, preferred are vinylpyrrolidone copolymers of the general formula (**ib**i), wherein **X¹** and **X²** are not both -NH-.

Preferred are vinylpyrrolidone copolymers of the general formula (**ii**), wherein up to **p%** of **R'** and **R"** are -(CH₂CH₂O)_{q}-H and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**ii**), wherein **Y'** and **Y"** represent a bond.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, or -CONHCH₂-; and
wherein the benzophenone substituent **BP** is optionally substituted benzoylphenyl, preferably benzoylphenyl.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-; and
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group
comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H, ; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H, ; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COOCH₂-, -CONHCH₂-, -NHCOOCH₂-, or -OCONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent;
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 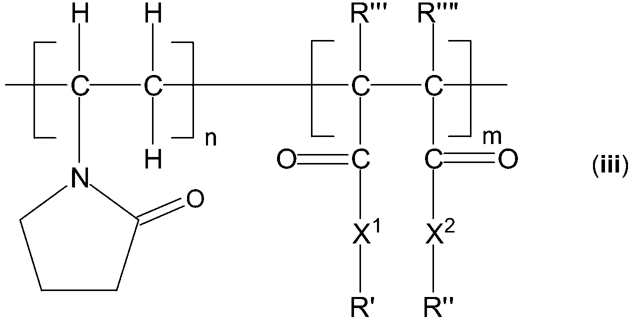
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 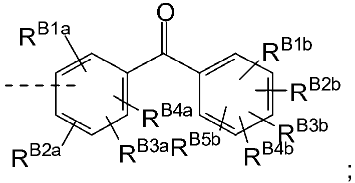
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 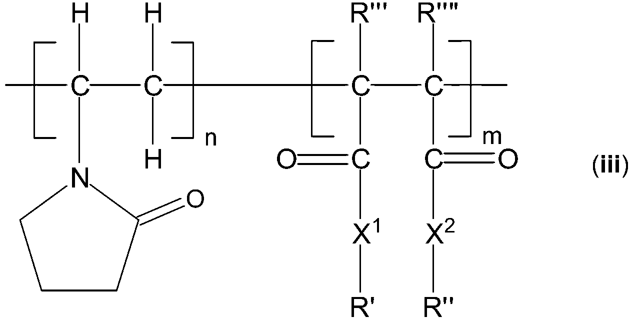
wherein **m** = 0.1 - 10.0 mol% and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 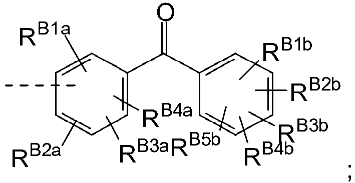
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 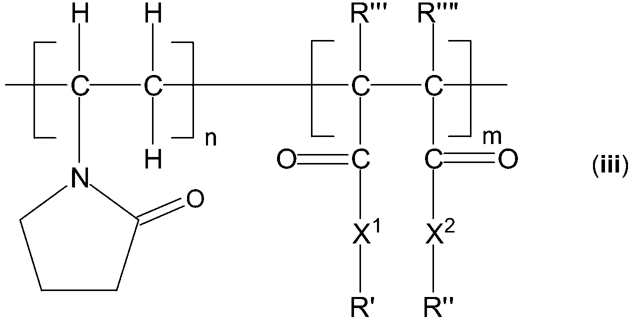
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 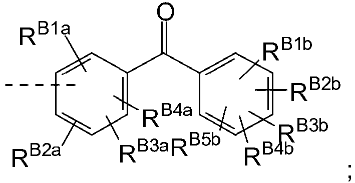
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**ii**): 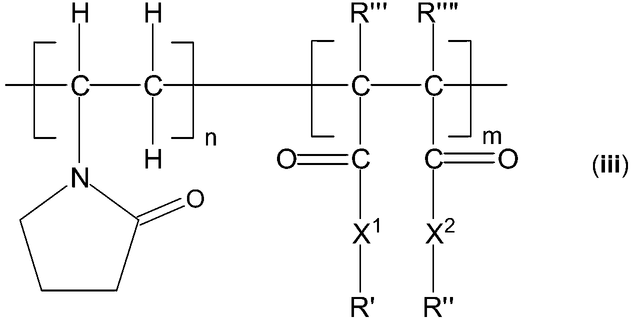
wherein **m** = 0.1 - 10.0 mol%, and **n** = 100 mol% - m;
**X¹** and **X²** independently of each are selected other from -O- and -NH-; preferably
wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, -CONHCH₂-, -NHCOOCH₂-, and -OCONHCH₂-;
**BP'** and **BP"** represent independently of each other 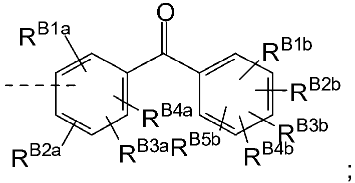
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**ii**): 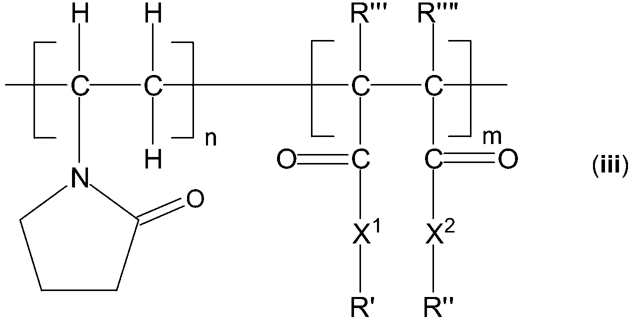
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, and -CONHCH₂-;
**BP'** and **BP"** represent independently of each other 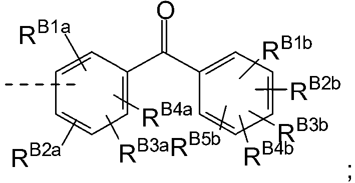
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (iii), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 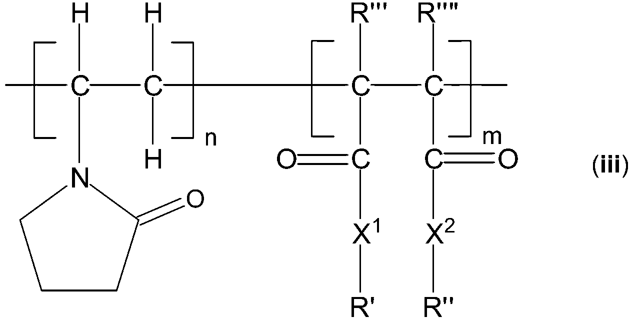
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or
-CH₂CH₂CH₂CH₂CH₂-; **Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 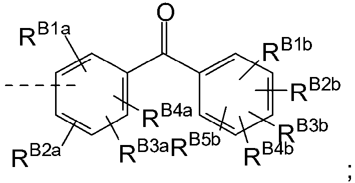
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein
**m** = 1.5 - 2.5 mol%, more preferably 2 mol%; and
**n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 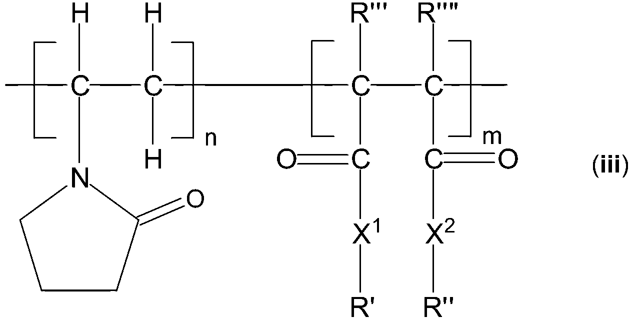
wherein **m** = 0.1 - 10.0 mol% and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, -CONHCH₂-, -NHCOOCH₂-, and -OCONHCH₂-;
**BP'** and **BP"** represent independently of each other 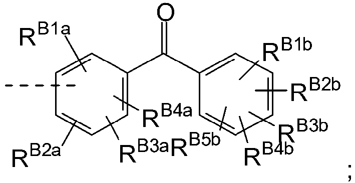
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 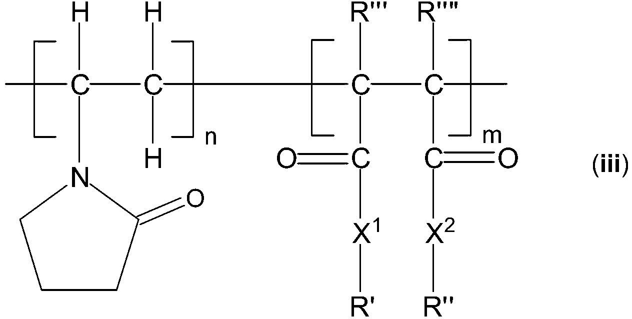
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, and -CONHCH₂-;
**BP'** and **BP"** represent independently of each other 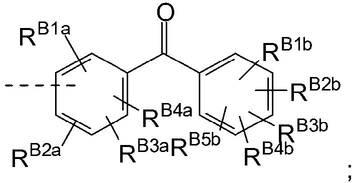
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 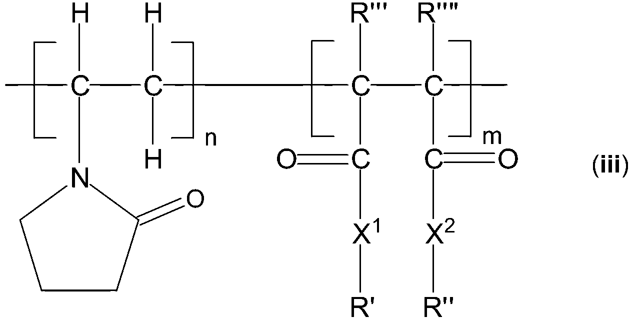
wherein **m** = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent 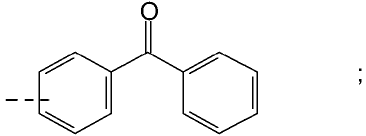
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 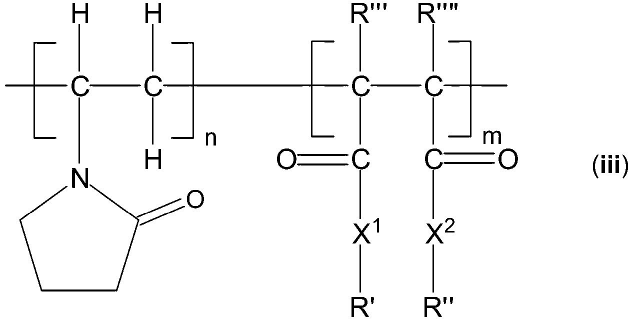
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, -CONHCH₂-, -NHCOOCH₂-, and -OCONHCH₂-;
**BP'** and **BP"** represent 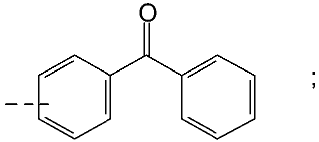
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (iii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 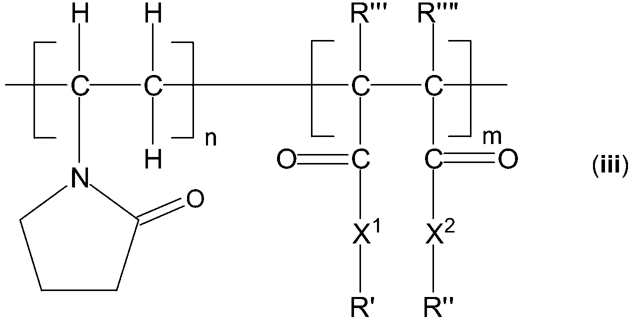
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R‴** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COOCH₂-, and -CONHCH₂-;
**BP'** and **BP"** represent 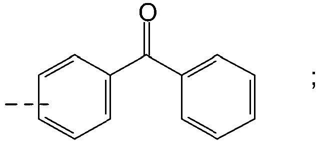
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 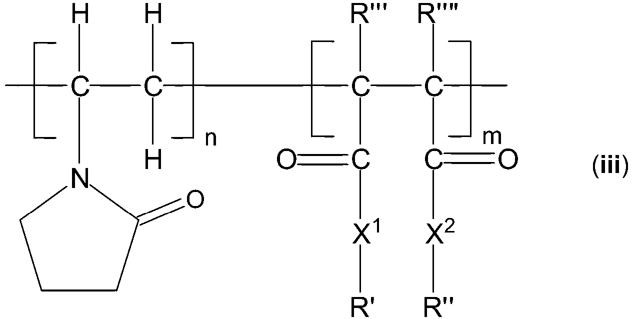
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 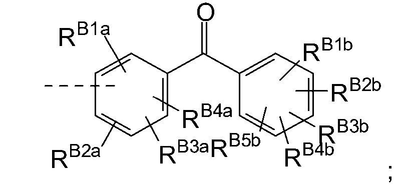
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 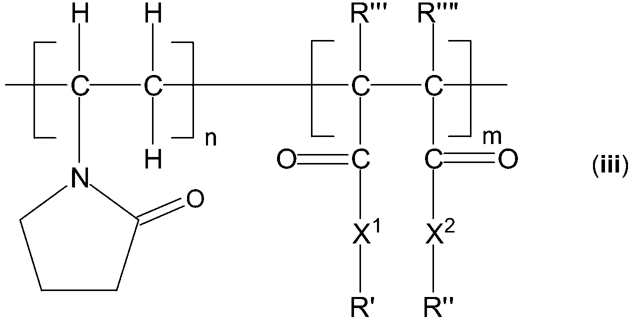
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 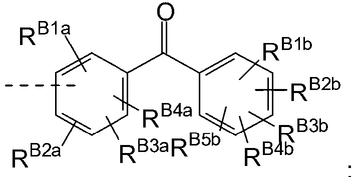
**R^{B1a} - R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 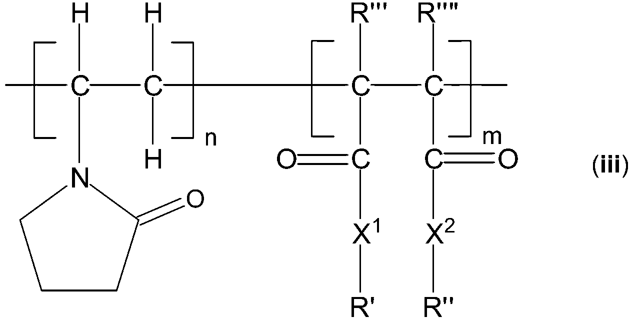
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 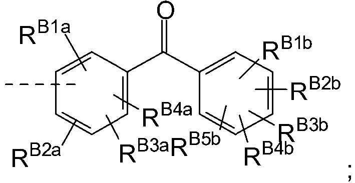
**R^{B1a} - R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein
**m** = 1.5 - 2.5 mol%, more preferably 2 mol%; and
**n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 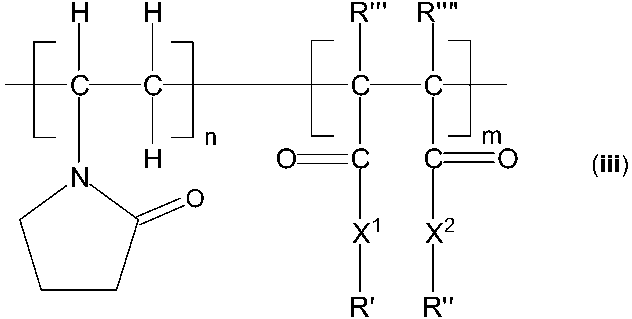
wherein **m** = 0.1 - 10.0 mol%, and n = 100 mol% - m;
**X¹** and **X²** independently of each are selected other from -O- and -NH-; wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent 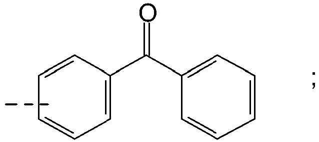
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 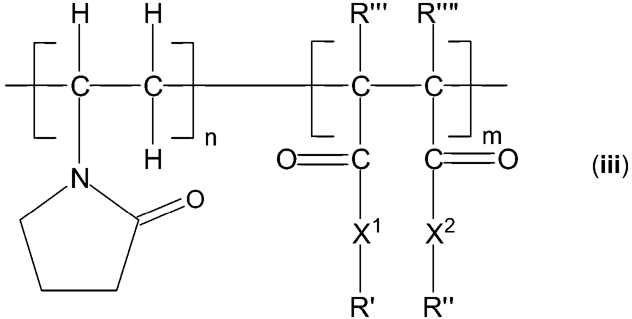
wherein **m** = 0.1 - 10.0 mol%, and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 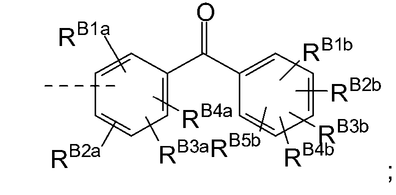
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 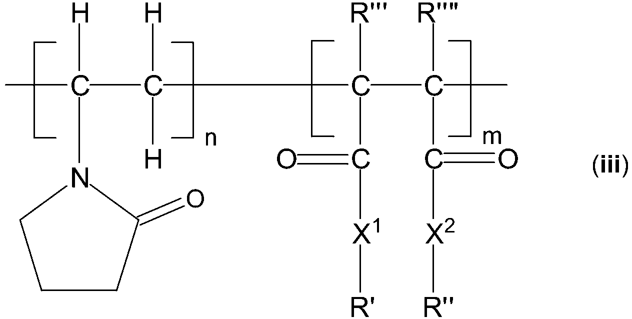
wherein **m** = 0.1 - 10.0 mol%, and **n** = 100 mol% - m;
**X¹** and **X²** independently of each are selected other from -O- and -NH-; wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-; **Y'** and **Y"** represent a bond;
**BP'** and **BP"** represent independently of each other 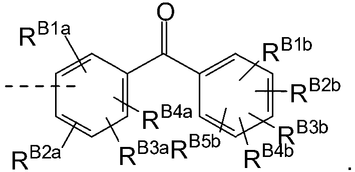
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and n = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 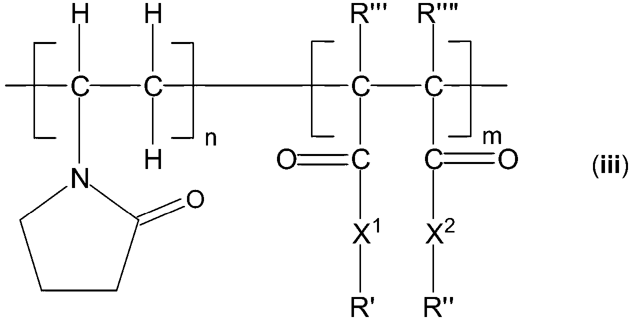
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-; **Y'** and **Y"** represent a bond;
**BP'** and **BP"** represent independently of each other 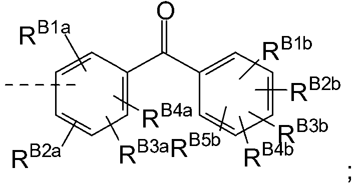
**R^{B1a} - R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 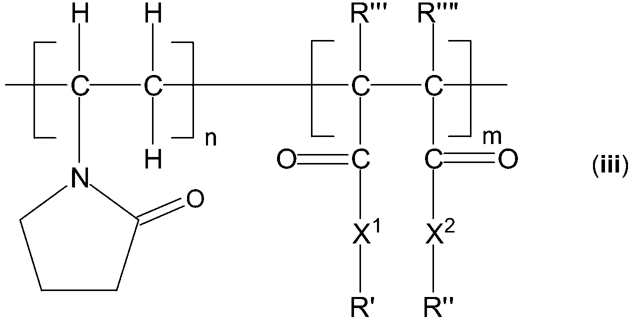
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **R""** represent independently of each other -H, -CH₃, -CH₂CH₃, or -F;
**L'** and **L"** represent -CH₂-; **Y'** and **Y"** represent a bond;
**BP'** and **BP"** represent independently of each other 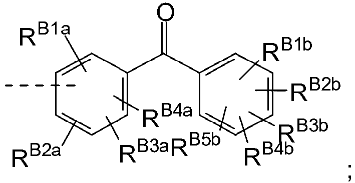
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 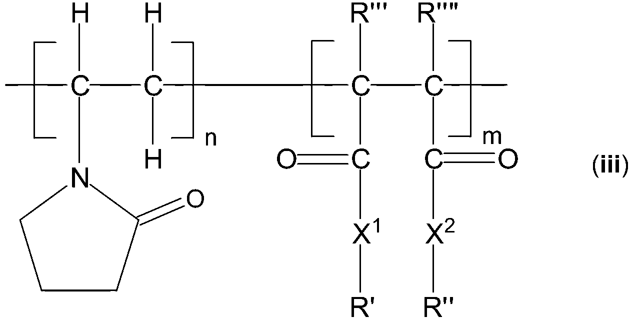
wherein **m** = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent 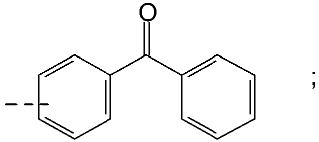
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 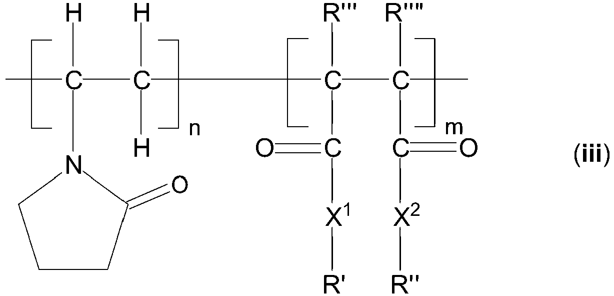
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L'** and **L"** represent -CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
**BP'** and **BP"** represent independently of each other 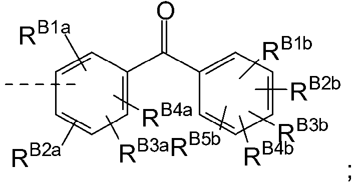
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 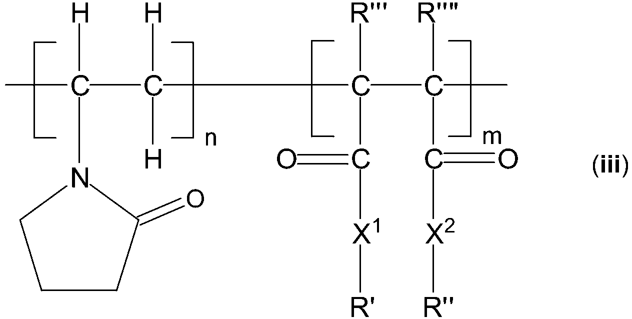
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L'** and **L"** represent -CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.
**BP'** and **BP"** represent independently of each other 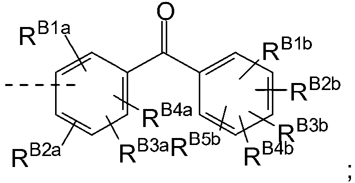
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 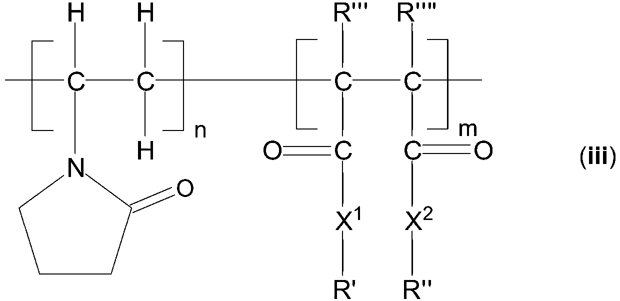
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L** represents -CH₂-; **Y'** and **Y"** represent a bond;
**BP'** and **BP"** represent independently of each other 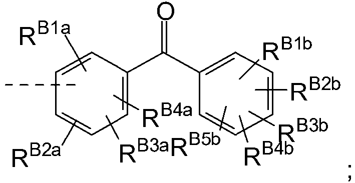
**R^{B1a} - R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 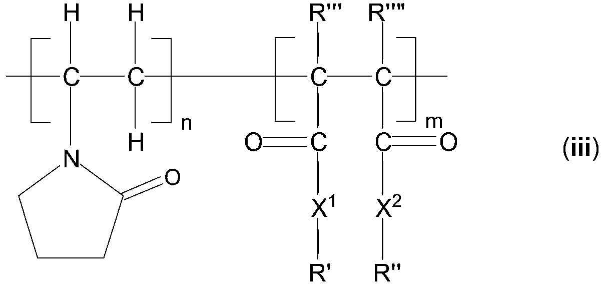
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L'** and L" represent -CH₂-; **Y'** and **Y"** represent a bond;
**BP'** and **BP"** represent independently of each other 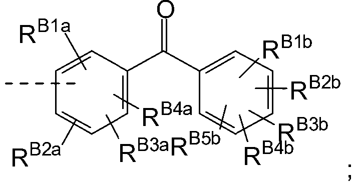
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 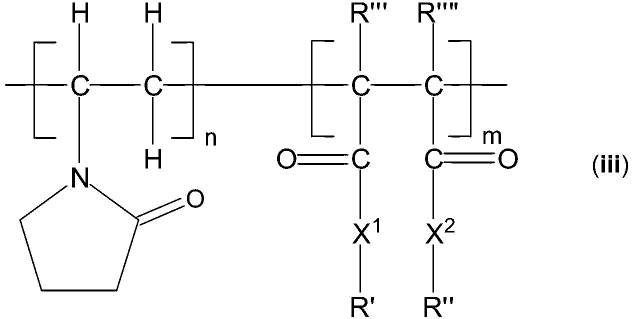
wherein **m** = 0.1 - 10.0 mol%, and **n** = 100 mol% - m;
**X¹** and **X²** independently of each are selected other from -O- and -NH-; preferably wherein only one of **X¹** and **X²** is -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴ and Rʺʺ** represent -H; **L'** and **L"** represent -CH₂-; **Y'** and **Y"** represent a bond;
**BP'** and **BP"** represent independently of each other 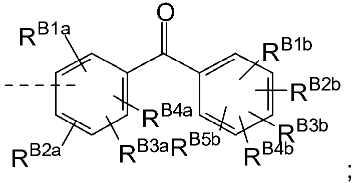
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

The present invention further relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (**iii**): 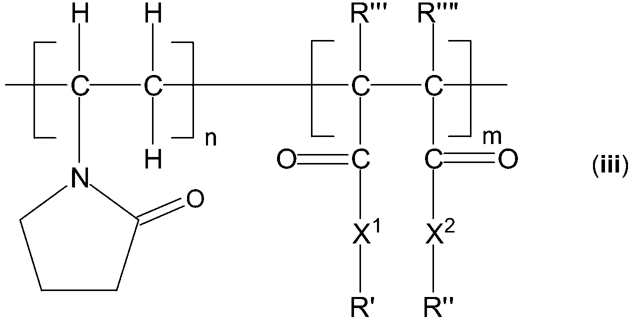
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L'** and **L"** represent -CH₂-; **Y'** and **Y"** represent a bond;
**BP'** and **BP"** represent 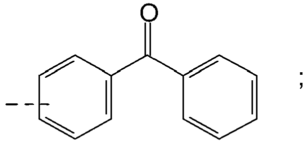
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**iii**), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (**iii**), wherein **m** = 1.5 - 2.5 mol%, more preferably 2 mol%; and **n** = 100 mol% - m.

### Variant 4 of preferred N-vinylpyrrolidone copolymers according to the invention

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent (**BP**) via a C₁-C₅ alkyl linker (**L**), wherein the benzophenone substituent (**BP**) is an optionally substituted benzoylphenyl, preferably benzoylphenyl, and
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention preferably relates to a vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent (**BP**) via a C₁-C₅ alkyl linker (**L**), wherein the benzophenone substituent (**BP**) is an optionally substituted benzoylphenyl, preferably benzoylphenyl, and
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

Preferably, the C₁-C₅ alkyl linker (**L**) is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

In preferred embodiments, the C₁-C₅ alkyl linker (**L**) is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the linker (**L**) not linked to the maleic acid moiety has a functional group **Y,** wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

Preferably, **Y** represents -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.

Preferably, **Y** represents -OCO-, -NHCO-, -COO-, -CONH-, -OCOCH₂-, -NHCOCH₂-, -COOCH₂-, or -CONHCH₂-.

Preferably, **Y** represents -NHCO-, -CONH-, -NHCOCH₂-, or -CONHCH₂-.

In further preferred embodiments, up to 100% of the functional groups of all maleic acid moieties in the vinylpyrrolidone copolymer that are **not** functionalized with a benzophenone substituent (**BP**) via a C₁-C₅ alkyl linker (**L**) independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50; and
wherein the ratio of benzophenone substituent (**BP**) to non-benzophenone substituent is ≥ 1, and the remaining functional groups of all maleic acid moieties in the vinylpyrrolidone copolymer have the residue -H.

In still further preferred embodiments, up to 100% of the functional groups of all maleic acid moieties in the vinylpyrrolidone copolymer that are **not** functionalized with a benzophenone substituent (**BP**) via a C₁-C₅ alkyl linker (**L**) have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50; and
wherein the ratio of benzophenone substituent (**BP**) to non-benzophenone substituent is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

Thus, the present invention preferably relates to a vinylpyrrolidone copolymer of the general formula (**iv**): 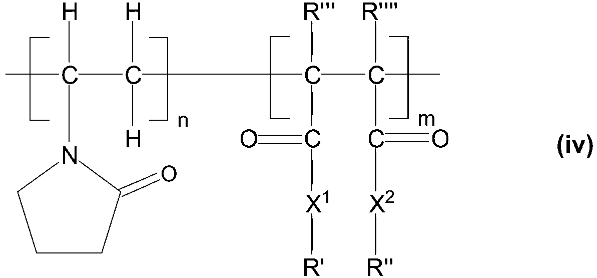 wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L** represents -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 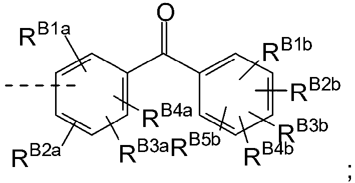
**R^{B1a}** - **R^{B4a}** and **R^{B1b}** - **R^{B5b}** represent independently of each other -H, -COOH, -CH₃ , -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (**iv**), wherein **m** = 1.5 - 2.5 mol%, more preferably 2 mol%; and **n** = 100 mol% - m.

The present invention preferably relates to vinylpyrrolidone copolymers of the general formula (**iv**): 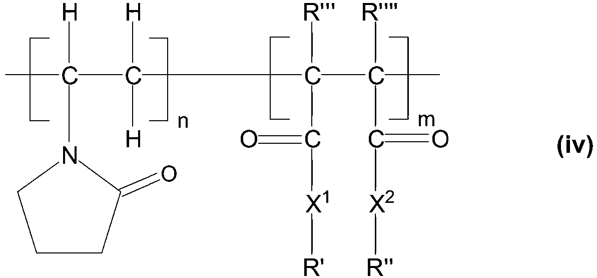 wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%;
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 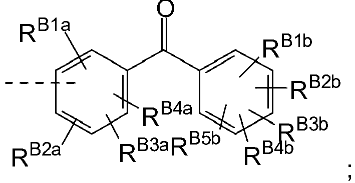
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (**i**), wherein only one of **X¹** and **X²** is -NH-. Thus, preferred are vinylpyrrolidone copolymers of the general formula (**i**), wherein **X¹** and **X²** are not both -NH-.

Preferred are vinylpyrrolidone copolymers of the general formula (**i**), wherein **Y'** and **Y"** represent a bond.

Preferred are vinylpyrrolidone copolymers of the general formula (**i**), wherein **BP'** and **BP"** represent

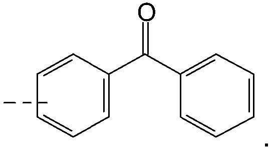

Preferred are vinylpyrrolidone copolymers of the general formula (**i**), wherein up to **p%** of **R'** and **R"** are -(CH₂CH₂O)_{q}-H, and **p** is a number from 1 - 50, wherein **q** is an integer between 35 and 50.

The present invention therefore also relates to vinylpyrrolidone copolymers of the general formula (**iv**): 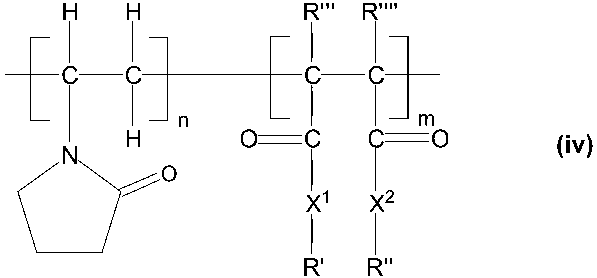 wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 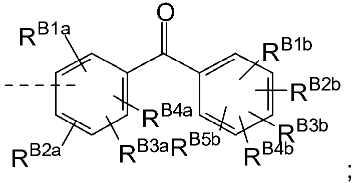
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n =** 100 mol% - m.

The present invention therefore also relates to vinylpyrrolidone copolymers of the general formula (**iv**): 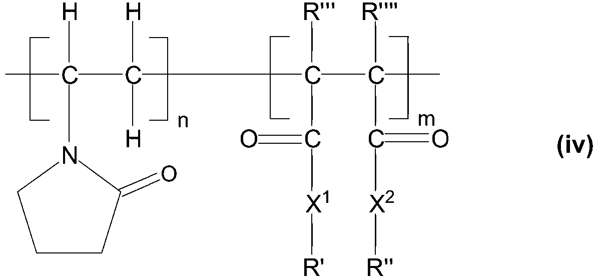 wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **R""** represent independently of each other -H, -CH₃, -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 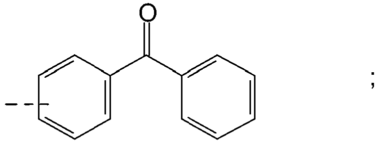
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein **m** = 1.5 - 2.5 mol%, more preferably 2 mol%; and **n** = 100 mol% - m.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent (**BP**) via a C₁-C₅ alkyl linker (**L**), wherein the benzophenone substituent (**BP**) is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker (L) is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention preferably relates to a vinylpyrrolidone copolymer composed of blocks of N-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent (BP) via a C₁-C₅ alkyl linker (L), wherein the benzophenone substituent (BP) is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker (L) is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C_{1'}C₅ alkyl linker (L) not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention preferably relates to a vinylpyrrolidone copolymer composed of blocks of N-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein up to 100% of the functional groups of all maleic acid moieties in the vinylpyrrolidone copolymer that are **not** functionalized with a benzophenone substituent **(BP)** via a C₁-C₅ alkyl linker (L) have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50; wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of N-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl; wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C_{1'}C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C_{1'}C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C_{1'}C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C_{1'}C₅ alkyl linker **(L)** not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein up to 100% of the functional groups of all maleic acid moieties in the vinylpyrrolidone copolymer that are **not** functionalized with a benzophenone substituent **(BP)** via a C₁-C₅ alkyl linker **(L)** have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50; wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl; wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C_{1'}C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C_{1'}C₅ alkyl linker **(L)** not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein up to 100% of the functional groups of all maleic acid moieties in the vinylpyrrolidone copolymer that are **not** functionalized with a benzophenone substituent **(BP)** via a C₁-C₅ alkyl linker (L) have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl; wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C_{1'}C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C_{1'}C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of N-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker (L),
wherein the vinylpyrrolidone copolymer has the general formula (iv): 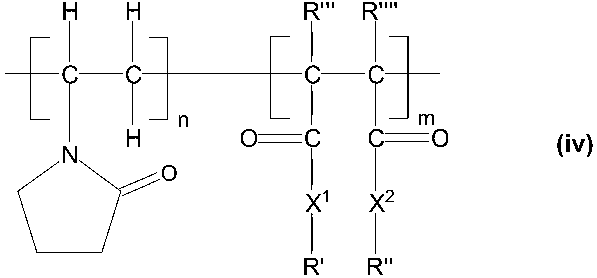
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 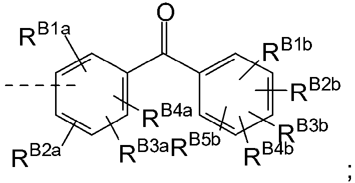
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein **m** = 1.5 - 2.5 mol%, more preferably 2 mol%; and **n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(iv):** 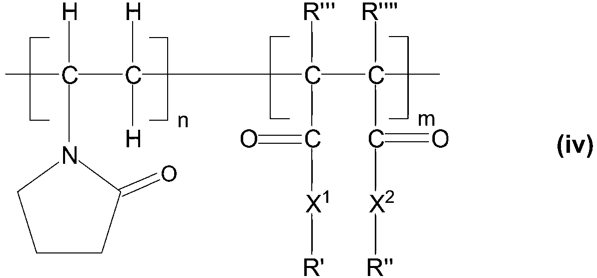
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R"'** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 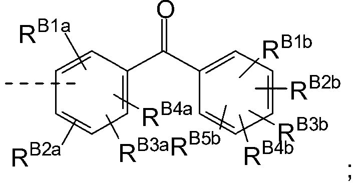
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula **(i),** wherein
**m** = 1.5 - 2.5 mol%, more preferably 2 mol%; and
**n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(iv):** 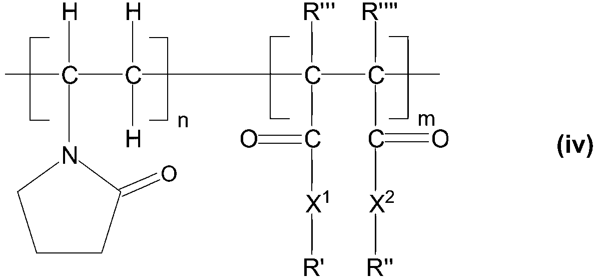
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 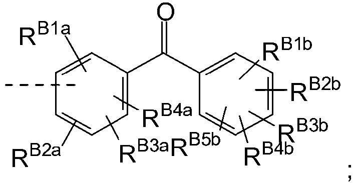
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(iv):** 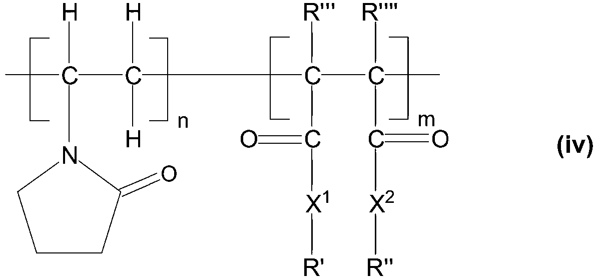
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 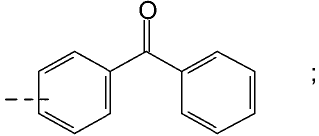
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention preferably relates to a vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(iv):** 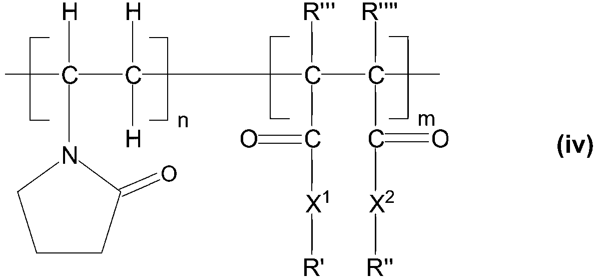
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent 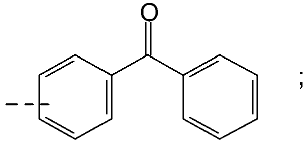
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

### Particularly preferred N-vinylpyrrolidone copolymers according to the invention

Herein particularly preferred are vinylpyrrolidone copolymers selected from the group consisting of: 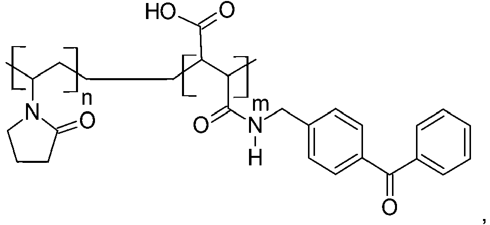 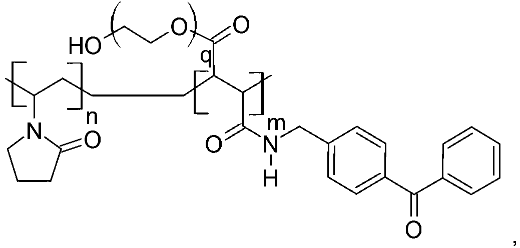 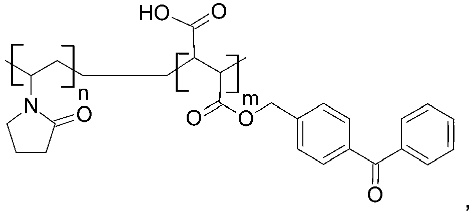 and 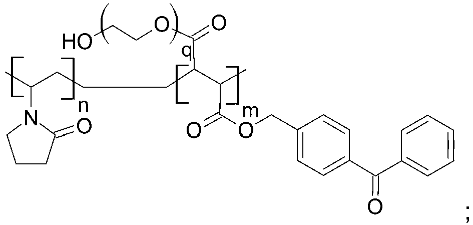 wherein
m = 0.1 - 10.0 mol% and
**n** = 100 mol% - m;
**q** is an integer between 35 and 50.

Preferred are embodiments, wherein
**m** = 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%; and
**n** = 100 mol% - m.

Further preferred are embodiments, wherein
**m** = 1.5 - 2.5 mol%, more preferably 2 mol%; and
**n** = 100 mol% - m.

### Synthesis of vinylpyrrolidone copolymers of the present invention

The present invention further relates to a method for preparing a vinylpyrrolidone copolymer comprising the following steps:
a) reacting *N*-vinylpyrrolidone (VP) and 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol% of a maleic acid comonomer: 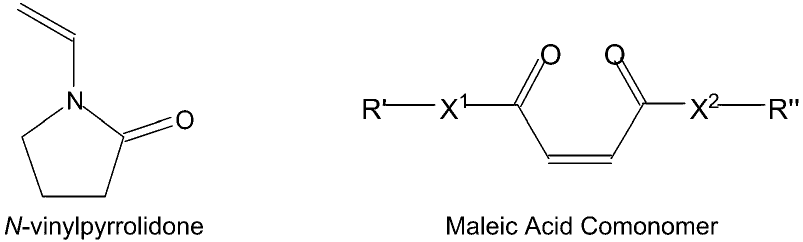 wherein
   **X¹** and **X²** are independently of each other -O- or -NH-; and **R'** and **R'** are as defined herein,
   wherein the reaction is carried out by solution polymerization in a solution or solution mixture wherein the *N*-vinylpyrrolidone (NVP), the at least one maleic acid comonomer and the resulting polyvinylpyrrolidone copolymer are freely soluble, and
   wherein the monomers are fed to the reaction mixture as (premixed) monomer solution during a predetermined period of time.

Suitable organic solvents are common solvents and their mixtures: tetrahydrofuran, acetone, ethanol, isopropanol, ether, methanol, hexane, heptane, n-octanol, isooctanol, dimethylformamide, dimethyl sulfoxide, chloroform, cyclohexane, 1,4-butanediol, ethyl acetate, petroleum ether, toluene, diethylene glycol, ethylene glycol, dichloromethane, etc.

The selection is usually based on the solubility of the reactants and the solubility of the copolymer. It likewise has an influence on the polymerization reaction and thus also on the molecular weight average (M_{w}) of the copolymer.

In combination with monitoring and control of the pot temperature during the reaction, the copolymer chain distribution can be influenced and the reaction process to form the copolymer can be carried out in a safe and controllable manner.

The resulting product is a molecular colloidal copolymer, which in a next step can be activated for further reactions via light of suitable wavelength and/or heat in the appropriate temperature range.

### Coated Medical Devices

The present invention relates to a medical device having a coating of a **cross-linked** vinylpyrrolidone copolymer of the present invention.

Manufacturing process of the medical devices

The coating of surfaces is preferably carried out from an immersion solution, but can also be applied by spraying, pipetting, brushing, tattooing, spherical, droplet dragging, filling of molds, etc., as well as in combinations of the processes. The choice of processes depends on the requirements for optimal and functional coating results, whether special coating thicknesses, partial coatings, uniformity or nonuniformity of coating thicknesses, interior or exterior surface coatings, filling of cavities or negatives are required.

The coating of the medical device is performed as follows in four successive simple steps, although steps 1-3) can also be performed on site and immediately before use.

In an application in which the components of the coating are only mixed shortly before use ("to go"), the sterilization (step 4) of the individual solutions has necessarily been carried out in advance for each separately.
1) one-time dipping of the possibly pre-treated (conditioned) surface to be coated into the coating solution,
   or
   Coating/pipetting/spotting of a surface, e.g. when partial coating is applied (e.g. only inside or outside, folds, corners, ends, center, recesses, i.e. only certain specified areas).
2) Irradiation with UV/VIS light max. 90 sec. or temperature increase or both or even longer (possibly also by exposure to daylight until the solvent has evaporated in the ideal case).
3) Drying to remove solvents and residues, if necessary.
4) Sterilization, package

In the simplest case, the coating solution consisting of at least the copolymer according to the invention is applied to the surface of the medical device by a single immersion. Subsequently, the radical formation is activated briefly with UV light or and temperature increase, so that the subsequent radical polymerization of the components takes place directly on the surface and leads to crosslinking and a bond to the surface can take place as well.

The irradiation time depends on the component and therefore varies due to its adsorption maximum. As a rule, a UV irradiation of max. 90 sec. is completely sufficient. In the case of VIS activation, the irradiation may well be longer, which can conveniently be combined with evaporation of the solvent.

The surface to be coated can consist of a wide variety of common materials. Examples include polyamides (such as PA 12, PA 6, PA 66), stainless steel, nitinol, Pebax, polyethylenes, polypropylenes, polyvinyls, polystyrenes, polyimides, polyamides, PTFE, silicones, polyurethanes, polyesters such as polymethacrylates, polyacrylates, polyacrylamides, polycarbonates, polylactides, polyglycolic acids, polyglycolic acids, and many others.e.g. polymethacrylates, polyacrylates, polyacrylamides, polycarbonates, polylactides, polyglycolides, polylactic acids, polyacetates, polycarbonates etc. and their copolymers and derivatives. Depending on the component mixture, a different preference for bonding to the material surface can be identified, which means that the optimum composition must be found experimentally for different substrates.

Depending on the material, prior conditioning of the surface may be advantageous to achieve the desired adhesion with the molecular colloidal coating. Depending on the template, conditioning can be achieved in different ways, e.g. by wetting the surface with a suitable solvent, by plasma treatment, heat or cold, irradiation or other suitable conditioning.

It is just as easy to fill the copolymers of the invention - optionally with further additives - into molds and then crosslink them by irradiation or heat, whereby the molecular colloid either remains in the mold - which is necessarily biostable or biodegradable if it remains - which then serves as a container, or is removed again after the crosslinking reaction so that it can be put to its intended use under the given shape or existing plasticity.

The present invention further relates to the use of a vinylpyrrolidone copolymer according to the invention as a medical device, component of a medical device, coating substance, coating of a medical device such as catheters of all kinds, balloon catheters, catheter shafts, stents, guide wires, needles, cannulas, additive in cosmetics, care products, hygiene products, ointments, gels, pads, medical aid, lubricant, for moisture regulation or absorption and for friction reduction of surfaces.

The present invention further relates to the use of a vinylpyrrolidone copolymer according to the invention as a medical device, component of a medical device, coating substance, coating of a medical device such as catheters, balloon catheters, catheter shafts, stents, guide wires, needles, cannulas, additive in cosmetics, care products, hygiene products, ointments, gels, pads, medical aids, lubricants, for moisture regulation or absorption, for friction reduction of surfaces, containing at least one pharmaceutically active substance which can be released in a controlled manner.

A further aspect relates to a composition comprising at least one vinylpyrrolidone copolymer according to the invention. Due to their unique properties and association with PVP, copolymers of the invention find their application in various market areas that utilize PVP homopolymers. These applications include: personal care - hair and skin care and color cosmetics, pharmaceuticals - biomaterials for delivery systems, excipients and coatings, and industrial applications - gas hydrate inhibition, paper coatings, textile coatings, ink additive, suspending aid and adhesives. Additional applications include: membrane applications (controlled gas and water permeation), lubricious coatings, compatibilizers, adhesives, polymeric surfactants and antimicrobial and antifouling coatings.

Provided is a personal care composition comprising a vinylpyrrolidone copolymer according to the invention. The personal care composition can be a hair care composition, like a hair fixative product, hair styling product such as hair gels and hair mousses, hair conditioning product or hair protectant product.

A vinylpyrrolidone copolymer according to the invention can be also advantageously used in a skin care composition, such as in water-in-oil or oil-in-water skin creams, day and night creams, eye creams, antiwrinkle creams, moisturizers, bleaching creams, vitamin creams, skin lotions, care lotions, hand and skin disinfectants and/or sanitizers, moisturizing lotions, personal hygiene care compositions, soaps, syndets, liquid washing, shower or bath preparations, nail care compositions, foot care compositions, sunscreens, repellents, shaving compositions, depilatories and anti-acne compositions.

Other uses include the incorporation of a vinylpyrrolidone copolymer according to the invention into a preparation for decorative cosmetics, preferably selected from the group consisting of makeup, mascara, lipsticks, eye shadows, kohl pencils, eyeliners, blushers, powders and eyebrow pencils.

Also provided herein is a pharmaceutical composition comprising vinylpyrrolidone copolymer according to the invention.

### Variant 1 of medical devices having a coating of a cross-linked vinylpyrrolidone copolymer according to the invention

The present invention relates to a medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl.

Preferably, the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

In preferred embodiments, the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the linker **(L)** not linked to the maleic acid moiety has a functional group **Y,** wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

In further preferred embodiments, up to 100% of the functional groups of all maleic acid moieties in the vinylpyrrolidone copolymer that are **not** functionalized with a benzophenone substituent **(BP)** via a C₁-C₅ alkyl linker (L) independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

In still further preferred embodiments, up to 100% of the functional groups of all maleic acid moieties in the vinylpyrrolidone copolymer **that** are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

The present invention relates to a medical device having a coating of a **cross-linked** vinylpyrrolidone copolymer of the general formula **(i):** 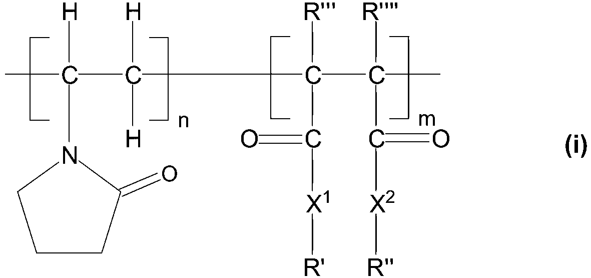
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 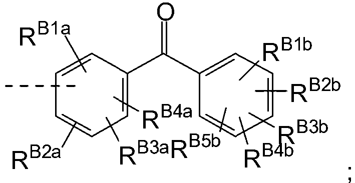
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m.

Further preferred are vinylpyrrolidone copolymers of the general formula (i), wherein **m** = 1.5 - 2.5 mol%, more preferably 2 mol%; and n = 100 mol% - m.

The present invention preferably relates to vinylpyrrolidone copolymers of the general formula (i): 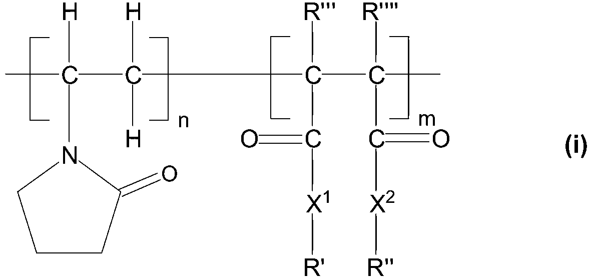 wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%;
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 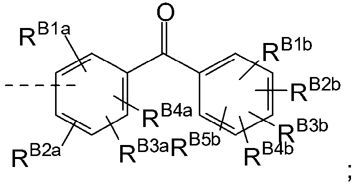
**R^{B1a}** - **R^{B4a}** and **R^{B1b}** - **R^{B5b}** represent independently of each other -H, -COOH, -CH₃ , -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein only one of **X¹** and **X²** is -NH-. Thus, preferred are vinylpyrrolidone copolymers of the general formula **(i),** wherein **X¹** and **X²** are not both -NH-.

Preferred are vinylpyrrolidone copolymers of the general formula **(i),** wherein **Y'** and **Y"** represent a bond.

Preferred are vinylpyrrolidone copolymers of the general formula (i), wherein **BP'** and **BP"** represent

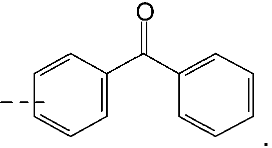

Preferred are vinylpyrrolidone copolymers of the general formula (i), wherein up to **p%** of **R'** and **R"** are -(CH₂CH₂O)_{q}-H, and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

The present invention therefore also relates to vinylpyrrolidone copolymers of the general formula (i): 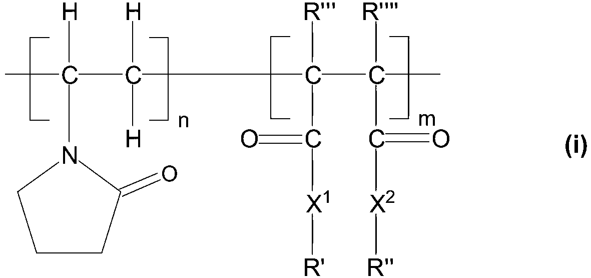
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to p% of R' and R" are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 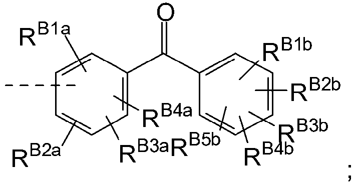
**R^{B1a}** - **R^{B4a}** and **R^{B1b}** - **R^{B5b}** represent independently of each other -H, -COOH, -CH₃ , -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention therefore also relates to vinylpyrrolidone copolymers of the general formula **(i):** 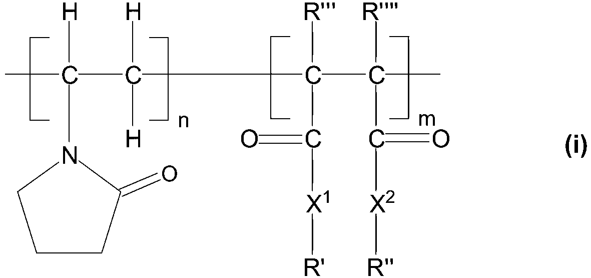
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -F; **L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 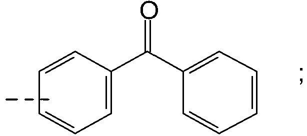
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker (L), wherein the benzophenone substituent **(BP)** is an optionally substituted benzoylphenyl, preferably benzoylphenyl;
wherein the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C₁-C₅ alkyl linker **(L)** not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein up to 100% of the functional groups of all maleic acid moieties in the vinylpyrrolidone copolymer that are **not** functionalized with a benzophenone substituent **(BP)** via a C₁-C₅ alkyl linker **(L)** have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
wherein up to 100% of the functional groups of all maleic acid moieties in the vinylpyrrolidone copolymer that are **not** functionalized with a benzophenone substituent **(BP)** via a C₁-C₅ alkyl linker (L) independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the vinylpyrrolidone copolymer has the general formula (i):
formula **(i):** 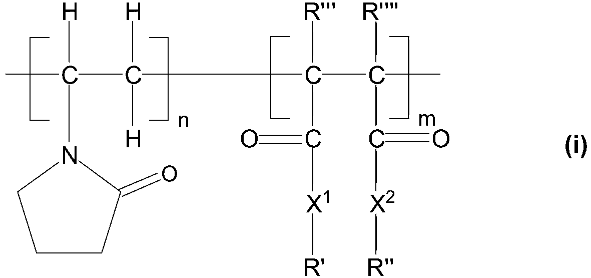
wherein **m** = 0.1 - 10.0 mol%, and **n** = 100 mol% - m;
**X¹** and **X²** independently of each are selected other from -O- and -NH-;
**p% of the residues** R' and R" independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 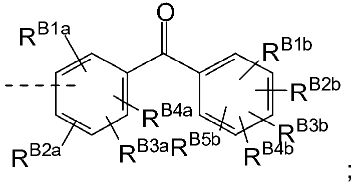
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 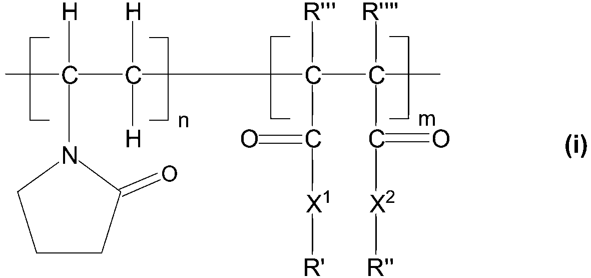
wherein **m** = 0.1 - 10.0 mol%, and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 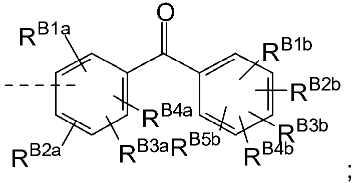
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 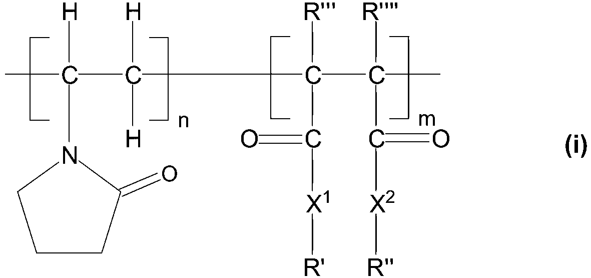
wherein **m** = 0.1 - 10.0 mol%, and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 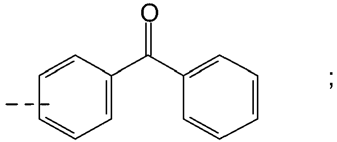
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(i),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(i):** 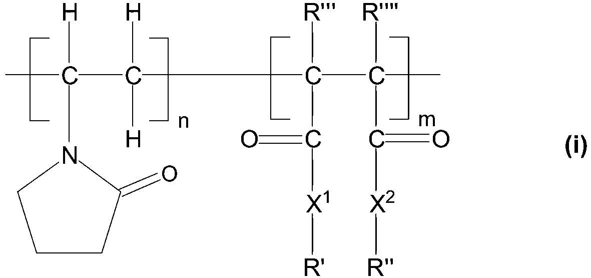
wherein **m** = 0.1 - 10.0 mol%, and **n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent -H; **L** represents -CH₂-; **Y'** and **Y"** represent a bond;
**BP'** and **BP"** represent 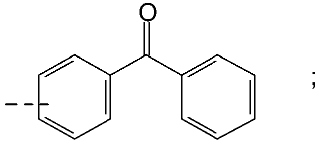
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (i), wherein **m** = 1.5 - 2.5 mol%, more preferably 2 mol%; and **n** = 100 mol% - m.

### Variant 2 of medical devices having a coating of a cross-linked vinylpyrrolidone copolymer according to the invention

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.

Preferably, **L** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

Preferably, the benzophenone substituent **BP** is optionally substituted benzoylphenyl, more preferably benzoylphenyl.

Preferably, **Y** represents a bond, -OCO-, -NHCO-, -COO-, -CONH-, -OCOCH₂-, -NHCOCH₂-, -COOCH₂-, or -CONHCH₂-.

More preferably, **Y** represents a bond, -NHCO-, -CONH-, -NHCOCH₂-, or -CONHCH₂-.

In preferred embodiments, up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

The present invention relates to a medical device having a coating of a **cross-linked** vinylpyrrolidone copolymer of general formula (ii): 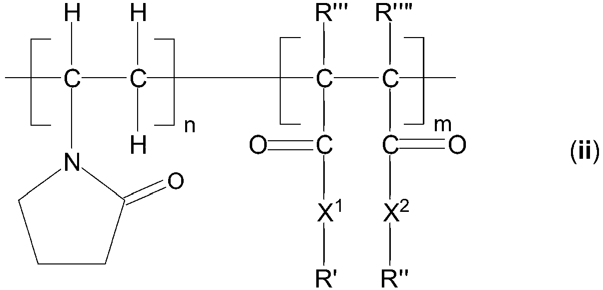
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-;
**BP'** and **BP"** represent independently of each other 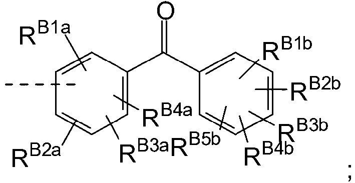
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferably, only one of **X¹** and **X²** is -NH-.

Preferred are vinylpyrrolidone copolymers of the general formula **(ii),** wherein only one of **X¹** and **X²** is -NH-. Thus, preferred are vinylpyrrolidone copolymers of the general formula (ii), wherein **X¹** and **X²** are not both -NH-.

Preferred are vinylpyrrolidone copolymers of the general formula (ii), wherein up to **p%** of **R'** and **R"** are -(CH₂CH₂O)_{q}-H and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein **Y'** and **Y"** represent a bond.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl.

The present invention relates to a medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein the amount of maleic acid moieties in relation to N-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-; preferably
**Y** represents a bond, -OCO-, -NHCO-, -COO-, -CONH-, -OCOCH₂-, -NHCOCH₂-, -COOCH₂-, or -CONHCH₂-; more preferably
**Y** represents a bond, -NHCO-, -CONH-, -NHCOCH₂-, or -CONHCH₂; and
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 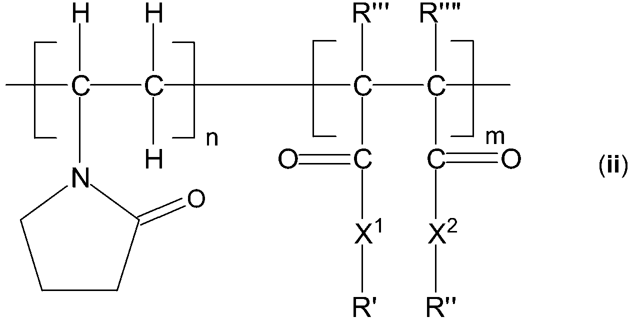
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-, preferably
**Y'** and **Y"** independently of each other are selected from a bond, -OCO-, -NHCO-, -COO-, -CONH-, -OCOCH₂-, -NHCOCH₂-, -COOCH₂-, or -CONHCH₂-, preferably
**Y'** and **Y"** independently of each other are selected from a bond, -NHCO-, -CONH-, -NHCOCH₂-, and -CONHCH₂-;
**BP'** and **BP"** represent independently of each other 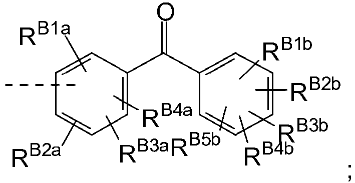
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (ii), wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 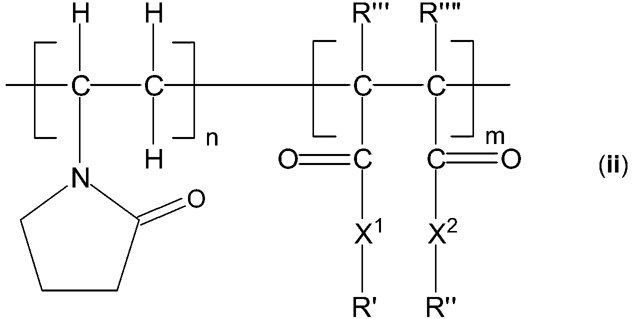
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-, preferably
**Y'** and **Y"** independently of each other are selected from a bond, -OCO-, -NHCO-, -COO-, -CONH-, -OCOCH₂-, -NHCOCH₂-, -COOCH₂-, or -CONHCH₂-, preferably
**Y'** and **Y"** independently of each other are selected from a bond, -NHCO-, -CONH-, -NHCOCH₂-, and -CONHCH₂-;
**BP'** and **BP"** represent independently of each other 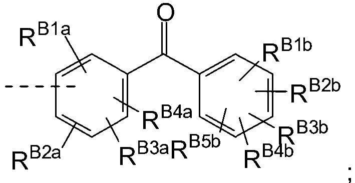
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 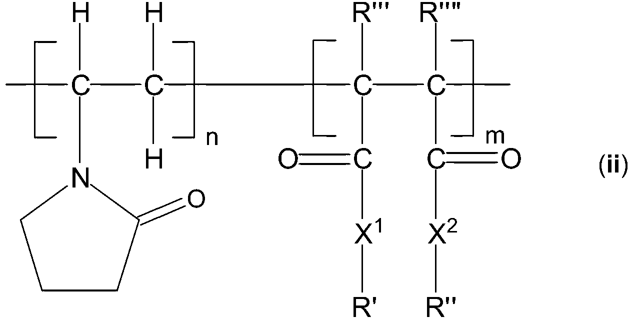
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-, preferably
**Y'** and **Y"** independently of each other are selected from a bond, -OCO-, -NHCO-, -COO-, -CONH-, -OCOCH₂-, -NHCOCH₂-, -COOCH₂-, or -CONHCH₂-, preferably
**Y'** and **Y"** independently of each other are selected from a bond, -NHCO-, -CONH-, -NHCOCH₂-, and -CONHCH₂-;
**BP'** and **BP"** represent 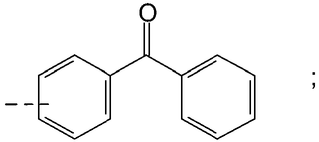
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 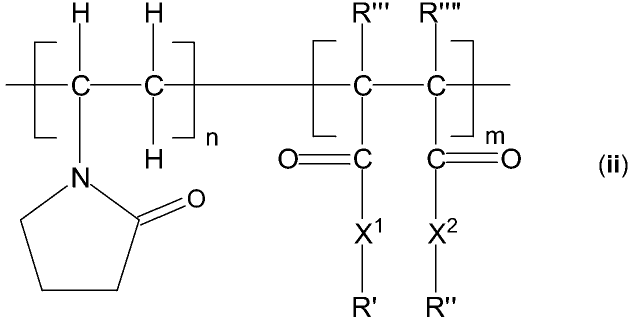
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to p% of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent independently of each other 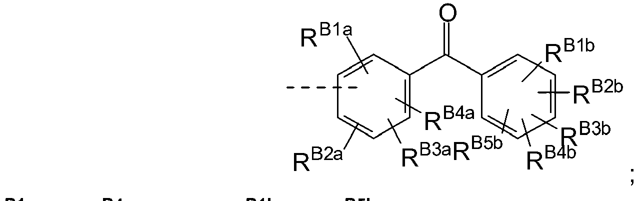
**R^{B1a}** - **R^{B4a}** and **R^{B1b} - R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 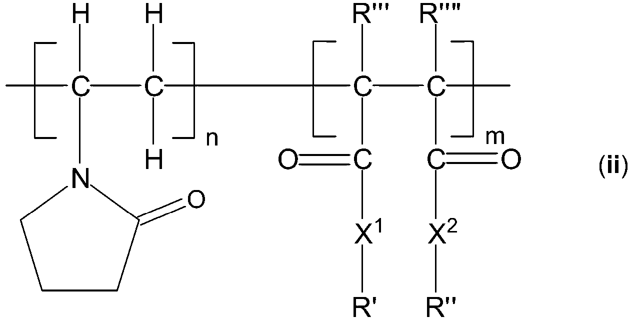
wherein **m** = 0.1 - 10.0 mol%, and **n** = 100 mol% - m;
**X¹** and **X²** independently of each are selected other from -O- and -NH-; wherein only one of **X¹** and **X²** is -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-; **Y'** and **Y"** represent a bond;
**BP'** and **BP"** represent independently of each other 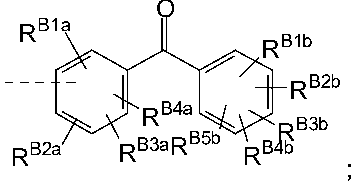
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (ii): 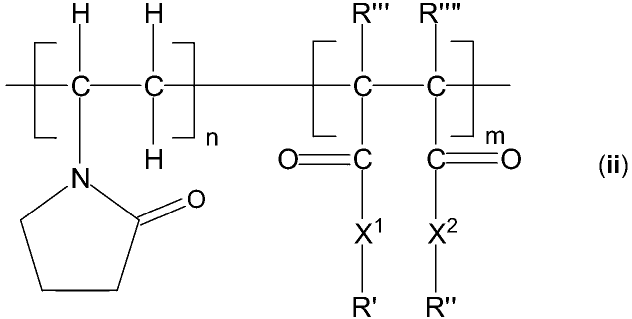
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** represent a bond;
**BP'** and **BP"** represent 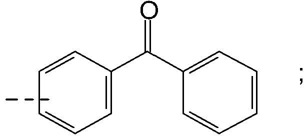
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 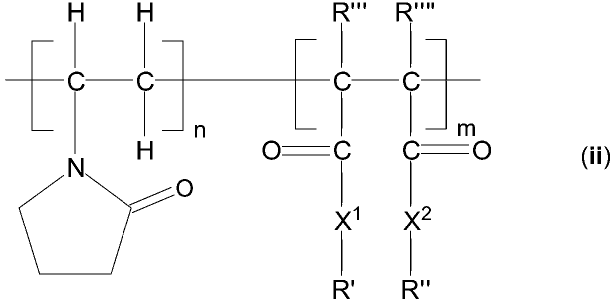
wherein **m** = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent independently of each other 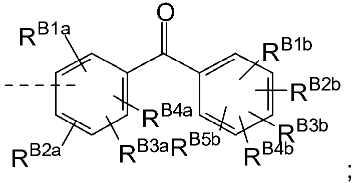
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (ii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(ii):** 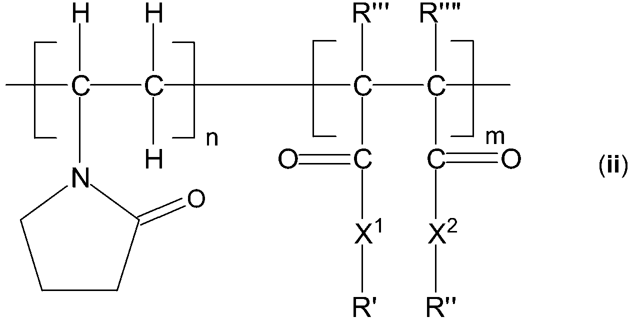
wherein **m** = 0.1 - 10.0 mol%, and **n** = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent -H; **L'** and **L"** represent -CH₂-; **Y'** and **Y"** represent a bond;
**BP'** and **BP"** represent 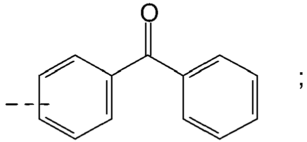
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(ii),** wherein **m** = 1.5 - 2.5 mol%, more preferably 2 mol%; and **n** = 100 mol% - m.

### Variant 3 of medical devices having a coating of a cross-linked vinylpyrrolidone copolymer according to the invention

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-.

Preferably, **L** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

Preferably, the benzophenone substituent **BP** is optionally substituted benzoylphenyl, more preferably benzoylphenyl.

Preferably, **Y** represents a bond, -OCOCH₂-, -NHCOCH₂-, -COOCH₂-, -CONHCH₂-, -NHCOOCH₂-, or -OCONHCH₂-.

More preferably, **Y** represents a bond, -OCOCH₂-, -NHCOCH₂-, -COOCH₂-, or -CONHCH₂-.

In preferred embodiments, up to 100% of the functional groups of all maleic acid moieties in the vinylpyrrolidone copolymer that are **not** functionalized with **-L-Y-BP,** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone substituent is ≥ 1, and the remaining functional groups of all maleic acid moieties in the vinylpyrrolidone copolymer have the residue -H.

In preferred embodiments, up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

The present invention relates to a medical device having a coating of a **cross-linked** vinylpyrrolidone copolymer of general formula (ii): 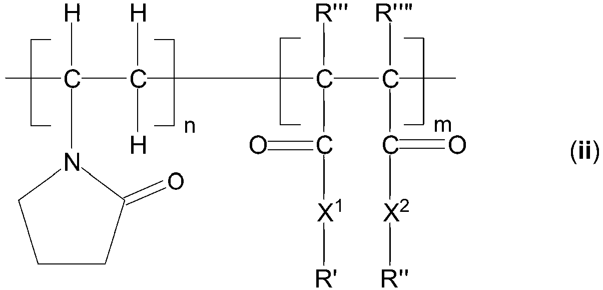 wherein
**m** = 0.1 - 10.0 mol%, and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-;
**BP'** and **BP"** represent independently of each other 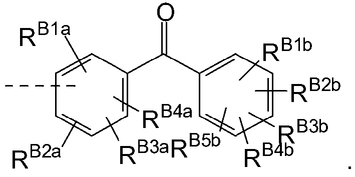
**R^{B1a} - R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein only one of **X¹** and **X²** is -NH-. Thus, preferred are vinylpyrrolidone copolymers of the general formula **(ii),** wherein **X¹** and **X²** are not both -NH-.

Preferred are vinylpyrrolidone copolymers of the general formula **(ii),** wherein up to **p%** of **R'** and **R"** are -(CH₂CH₂O)_{q}-H and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(ii),** wherein **Y'** and **Y"** represent a bond.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl.

The present invention relates to a medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the amount of maleic acid moieties in relation to *N*-vinylpyrrolidone moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl; and
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with

**-L-Y-BP**

wherein
**L** represents a C₁-C₅ alkyl linker;
**BP** represents a benzophenone substituent; and
**Y** represents a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, or -NHCONHCH₂-; preferably
**Y** represents a bond, -OCOCH₂-, -NHCOCH₂-, -COOCH₂-, -CONHCH₂-, -NHCOOCH₂-, or -OCONHCH₂-; preferably
**Y** represents a bond, -OCOCH₂-, -NHCOCH₂-, -COOCH₂-, or -CONHCH₂-;
wherein the benzophenone substituent **BP** is optionally substituted benzoylphenyl, preferably benzoylphenyl;
wherein up to 100% of the functional groups of all maleic acid moieties in the vinylpyrrolidone copolymer that are **not** functionalized with **-L-Y-BP,** independently of each other have a non-benzophenone substituent selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H; and
wherein the molar ratio of *N*-vinylpyrrolidone moieties to maleic acid moieties in the vinylpyrrolidone copolymer is between 10:1 and 1000:1

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 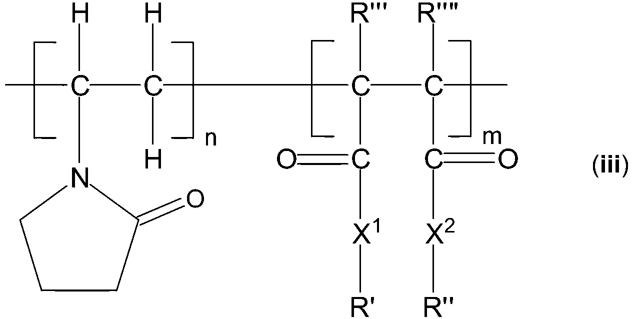
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent independently of each other 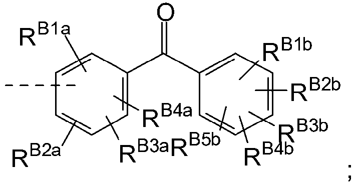
**R^{B1a} - R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(iii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(iii),** wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 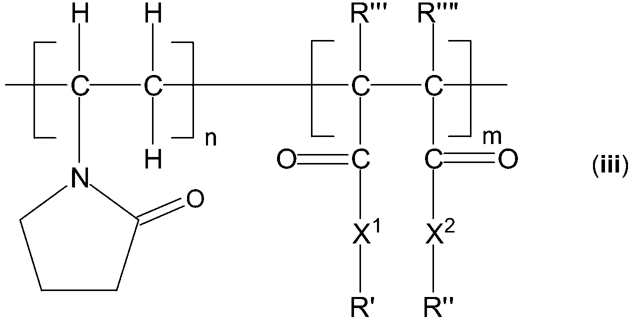
wherein **m** = 0.1 - 10.0 mol% and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent independently of each other 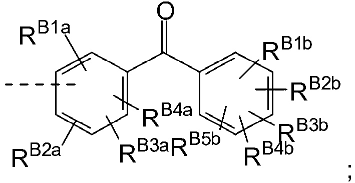
**R^{B1a} - R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (iii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (iii), wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 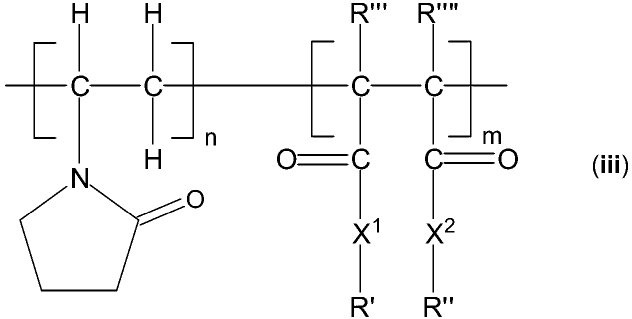
wherein **m** = 0.1 - 10.0 mol% and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent 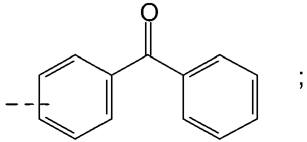
**p** represents a number from 1 - 50; and wherein **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (iii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

The present invention relates to a medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula (iii): 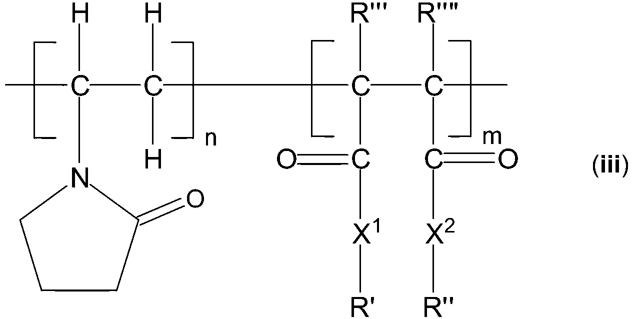
wherein m = 0.1 - 10.0 mol%, and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent independently of each other 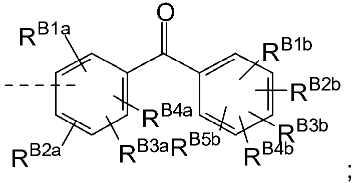
**R^{B1a}** - **R^{B4a}** and **R^{B1b}** - **R^{B5b}** represent independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and q is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (iii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula **(iii),** wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(iii):** 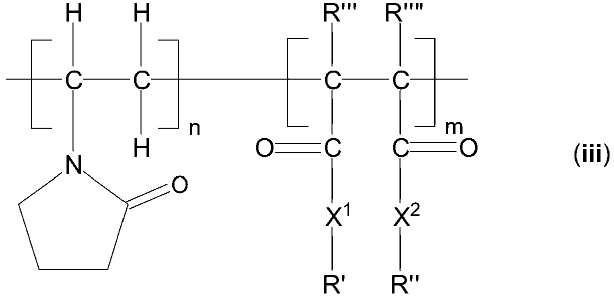
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent -H; **L'** and **L"** represent -CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent independently of each other 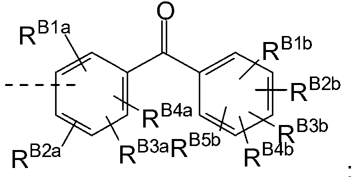
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(iii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (iii), wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(iii):** 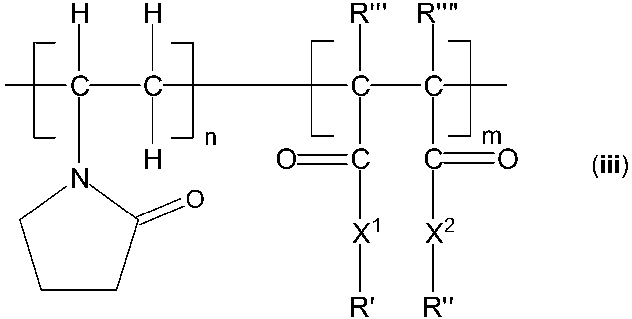
wherein **m** = 0.1 - 10.0 mol%, and **n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L'** and **L"** represent -CH₂-;
**Y'** and **Y"** independently of each other are selected from a bond, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂-, and -NHCONHCH₂-, preferably
**Y'** and **Y"** independently of each other are selected from a bond, -OCOCH₂-, -NHCOCH₂-, -COOCH₂-, -CONHCH₂-, -NHCOOCH₂-, and -OCONHCH₂-, preferably
**Y'** and **Y"** independently of each other are selected from a bond, -OCOCH₂-, -NHCOCH₂-, -COOCH₂-, and -CONHCH₂-;
**BP'** and **BP"** represent independently of each other 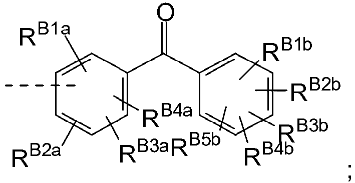
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (iii), wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (iii), wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with **-L-Y-BP;**
wherein the vinylpyrrolidone copolymer has the general formula **(iii):** 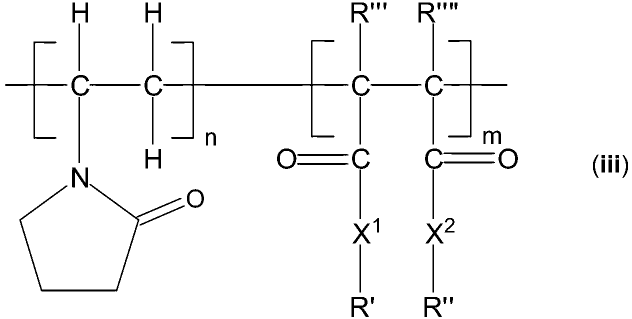
wherein m = 0.1 - 10.0 mol%, and n = 100 mol% - m;
**X¹** and **X²** independently of each are selected other from -O- and -NH-; wherein only one of **X¹** and **X²** is -NH-.
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent -H; **L** represents -CH₂-; **Y'** and **Y"** represent a bond;
**BP'** and **BP"** represent 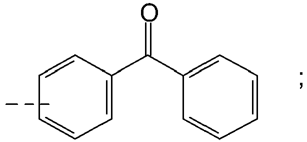
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula **(iii),** wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m.

Further preferred are vinylpyrrolidone copolymers of the general formula (iii), wherein
**m** = 1.5 - 2.5 mol%, more preferably 2 mol%; and
**n** = 100 mol% - m.

### Variant 4 of medical devices having a coating of a cross-linked vinylpyrrolidone copolymer according to the invention

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl, and
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.

The present invention relates to a medical device having a coating of a **cross-linked** vinylpyrrolidone copolymer of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl, and
wherein the molar ratio of N-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

Preferably, the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

In preferred embodiments, the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), the respective end of the linker **(L)** not linked to the maleic acid moiety has a functional group **Y,** wherein **Y** is selected from the group comprising or consisting of:
-OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

Preferably, **Y** represents -OCO-, -NHCO-, -COO-, -CONH-, -NHCOO-, -OCONH-, -OCOO-, -NHCONH-, -OCOCH₂-, -NHCOCH₂-, -COOCH₂-, -CONHCH₂-, -NHCOOCH₂-, -OCONHCH₂-, -OCOOCH₂-, or -NHCONHCH₂-.

Preferably, **Y** represents -OCO-, -NHCO-, -COO-, -CONH-, -OCOCH₂-, -NHCOCH₂-, -COOCH₂-, or -CONHCH₂-.

Preferably, **Y** represents -NHCO-, -CONH-, -NHCOCH₂-, or -CONHCH₂-.

In further preferred embodiments, up to 100% of the functional groups of all maleic acid moieties in the vinylpyrrolidone copolymer that are **not** functionalized with a benzophenone substituent **(BP)** via a C₁-C₅ alkyl linker **(L)** independently of each other have a non-benzophenone substituent selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

In still further preferred embodiments, up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide and polyacrylate, wherein **q** is preferably an integer between 35 and 50; and
wherein the ratio of benzophenone substituent **(BP)** to non-benzophenone substituent is ≥ 1, and the remaining functional groups of all maleic acid moieties in the vinylpyrrolidone copolymer have the residue -H.

Preferably, the vinylpyrrolidone copolymer has an average molecular weight of from 1,000 to 1,000,000 g/mol, preferably from 10,000 to 700,000 g/mol, and more preferably between 5,000 to 500,000 g/mol.

The present invention relates to a medical device having a coating of a **cross-linked** vinylpyrrolidone copolymer of the general formula **(iv):** 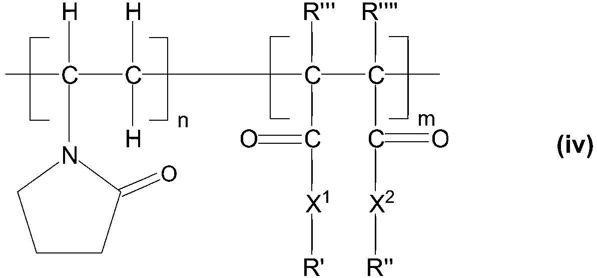
wherein **m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 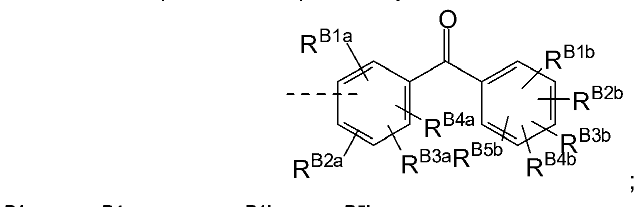
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

Further preferred are vinylpyrrolidone copolymers of the general formula (i), wherein
**m** = 1.5 - 2.5 mol%, more preferably 2 mol%; and
**n** = 100 mol% - m.

The present invention relates to a medical device having a coating of a **cross-linked** vinylpyrrolidone copolymer of the general formula **(iv):** 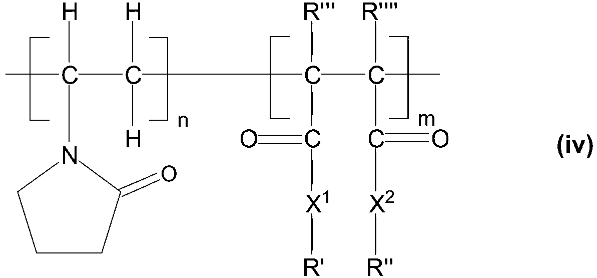 wherein
**m** = 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%;
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **R""** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 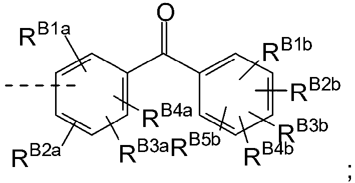
**R^{B1a}** - **R^{B4a}** and **R^{B1b}** - **R^{B5b}** represent independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Preferred are vinylpyrrolidone copolymers of the general formula (i), wherein only one of **X¹** and **X²** is -NH-. Thus, preferred are vinylpyrrolidone copolymers of the general formula (i), wherein **X¹** and **X²** are not both -NH-.

Preferred are vinylpyrrolidone copolymers of the general formula (i), wherein **Y'** and **Y"** represent a bond.

Preferred are vinylpyrrolidone copolymers of the general formula (i), wherein **BP'** and **BP"** represent

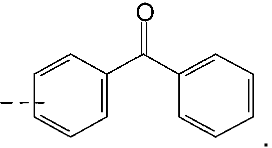

Preferred are vinylpyrrolidone copolymers of the general formula (i), wherein up to **p%** of **R'** and **R"** are -(CH₂CH₂O)_{q}-H, and **p** is a number from 1 - 50, wherein **q** is an integer between 35 and 50. In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

The present invention relates to a medical device having a coating of a **cross-linked** vinylpyrrolidone copolymer of the general formula **(iv):** 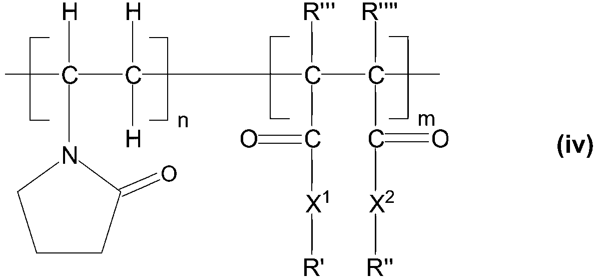 wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 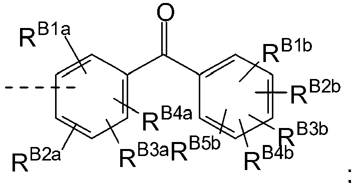
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments p is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n** = 100 mol% - m.

The present invention relates to a medical device having a coating of a **cross-linked** vinylpyrrolidone copolymer of the general formula (iv): wherein 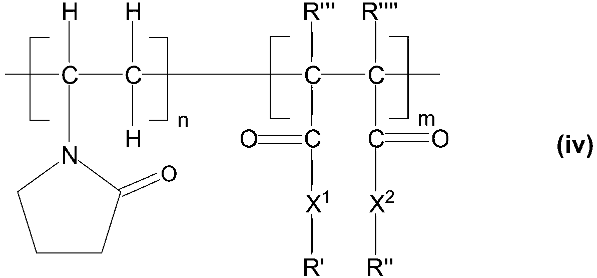
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
n = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H;
**R‴** and **Rʺʺ** represent independently of each other -H, -CH₃, -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 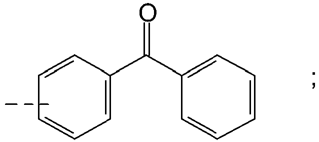
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m** = 1.5 - 2.5 mol%, more preferably 2 mol%; and
**n** = 100 mol% - m.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-); and wherein the respective end of the C₁-C₅ alkyl linker **(L)** not linked to the maleic acid moiety has a functional group **Y,** wherein **Y** is selected from the group comprising or consisting of: -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein up to 100% of the functional groups of all maleic acid moieties in the vinylpyrrolidone copolymer that are **not** functionalized with a benzophenone substituent **(BP)** via a C₁-C₅ alkyl linker **(L)** have independently of each other a non-benzophenone substituent selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl; wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-), and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker (L) independently of each other have a non-benzophenone moiety, selected from the group consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the C₁-C₅ alkyl linker **(L)** is selected from the group comprising or consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-),
and wherein the respective end of the C₁-C₅ alkyl linker **(L)** not linked to the maleic acid moiety has a functional group **Y,** wherein **Y** is selected from the group comprising or consisting of:
   -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
   wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
   wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl; wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%.
wherein up to 100% of the functional groups of all maleic acid moieties in the vinylpyrrolidone copolymer that are **not** functionalized with a benzophenone substituent **(BP)** via a C₁-C₅ alkyl linker **(L)** have a non-benzophenone substituent selected from the group comprising or consisting of polyethylene glycol -(CH₂CH₂O)_{q}-H, polylactide, polyglycolide, and polyacrylate, wherein **q** is an integer between 35 and 50;
wherein the ratio of benzophenone substituent **(BP)** to non-benzophenone substituent is ≥ 1, and the remaining functional groups of all maleic acid moieties in the vinylpyrrolidone copolymer have the residue -H.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the benzophenone substituent **(BP)** is an optionally substituted benzoylphenyl, preferably benzoylphenyl;
wherein the amount of maleic acid moieties in the vinylpyrrolidone copolymer is between 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%;
wherein the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1;
wherein the C₁-C₅ alkyl linker (L) is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),** wherein the vinylpyrrolidone copolymer has the general formula **(iv):** 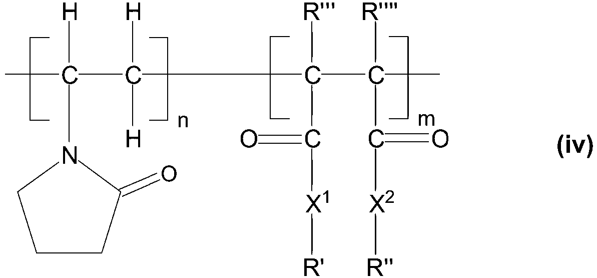 wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
**p% of the residues R'** and **R"** independently of each other are selected from -L'-Y'-BP' and -L"-Y"-BP"; and
up to **p%** of **R'** and **R"** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent independently of each other 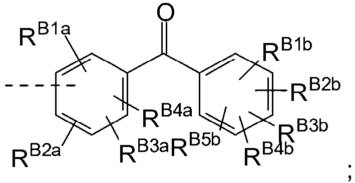
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula **(i),** wherein
**m** = 1.5 - 2.5 mol%, more preferably 2 mol%; and
**n** = 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker (L),
wherein the vinylpyrrolidone copolymer has the general formula (iv): 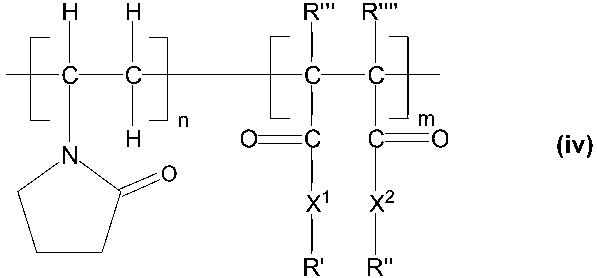
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and **Rʺʺ** represent independently of each other -H, -CH₃, -CH₂CH₃, or -F;
**L'** and **L"** represent -CH₂-, -CH₂CH₂-, -CH₂CH₂CH₂-, -CH₂CH₂CH₂CH₂-, or -CH₂CH₂CH₂CH₂CH₂-;
**Y'** and **Y"** are independently of each other selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-;
**BP'** and **BP"** represent 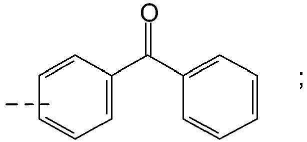
**p** represents a number from 1 - 50; and wherein **q** is an integer between 35 and 50.

In preferred embodiments, p is a number from 25 - 50. In further preferred embodiments **p** is a number from 25 - 30, more preferably 30 - 35, more preferably 35 - 40, more preferably, 40 - 45, even more preferably 45 - 50. In particular preferred embodiments, **p** is (about) 25. In most preferred embodiments **p** is (about) 50.

Further preferred are vinylpyrrolidone copolymers of the general formula (i), wherein
**m =** 1.5 - 2.5 mol%, more preferably 2 mol%; and
**n =** 100 mol% - m.

Preferably, the molar ratio of *N*-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

The present invention relates to a medical device having a coating of a **cross-linked** vinylpyrrolidone copolymer composed of blocks of *N*-vinylpyrrolidone moieties divided by maleic acid moieties, wherein each maleic acid moiety has as functional groups one carboxy group (-COO-) and one carbamoyl group (-CONH-) or two carboxy groups (-COO-) or two carbamoyl groups (-CONH-), and 1% to 50% of the functional groups of all maleic acid moieties in the vinylpyrrolidone copolymer are functionalized with a benzophenone substituent **(BP)** via a C₁-C₅ alkyl linker **(L),**
wherein the vinylpyrrolidone copolymer has the general formula **(iv):** 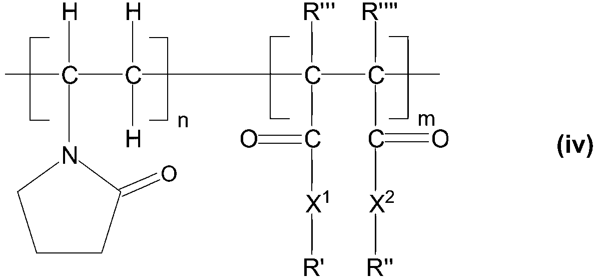
wherein
**m** = 0.1 - 10.0 mol%, preferably 0.1 - 5.0 mol%, preferably 0.1 to 2.0 mol%, more preferably 0.1 - 0.95 mol%, more preferably 0.1 - 0.9 mol%, more preferably 0.2 - 0.88 mol%, still more preferably 0.5 - 0.85 mol%; and
**n** = 100 mol% - m;
**X¹** and **X²** independently of each are selected other from -O- and -NH-;
wherein **X¹** and **X²** are not both -NH-;
**p% of the residues R'** and **R"** independently of each other are selected from -L'-Y'-BP' and -L"-Y"-BP"; and
up to **p%** of **R'** and **R"** are -(CH₂CH₂O)_{q}-H; and the remaining residues **R'** and **R"** are -H; and the remaining residues **R'** and **R"** are -H;
**R‴** and R"" represent -H; **L'** and **L"** represent -CH₂-; **Y'** and **Y"** represent a bond;
**BP'** and **BP"** represent 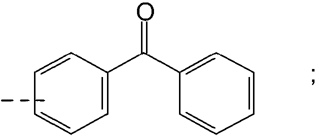
**p** represents a number from 1 - 50; and wherein **q** is an integer between 35 and 50.

Further preferred are vinylpyrrolidone copolymers of the general formula (i), wherein
**m** = 1.5 - 2.5 mol%, more preferably 2 mol%; and
**n** = 100 mol% - m.

Preferably, the molar ratio of N-vinylpyrrolidone moieties to maleic acid moieties in the vinylpyrrolidone copolymer is between 10:1 and 1000:1, preferably between 10:1 and 200:1, more preferably between 20:1 and 1000:1, further preferably between 20:1 and 200:1, more preferably between 50:1 and 150: 1, more preferably at 50:1, also preferably between 80:1 and 130:1.

### Particularly preferred medical devices having a coating of a cross-linked vinylpyrrolidone copolymer according to the invention

The present invention relates to a medical device having a coating of a **cross-linked** vinylpyrrolidone copolymer selected from the group consisting of: 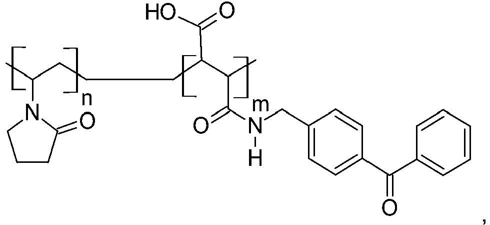 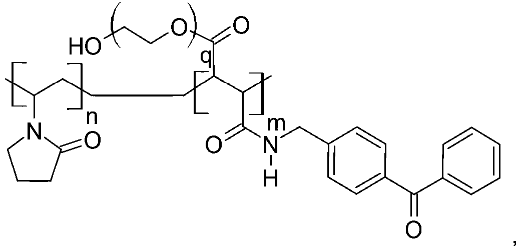 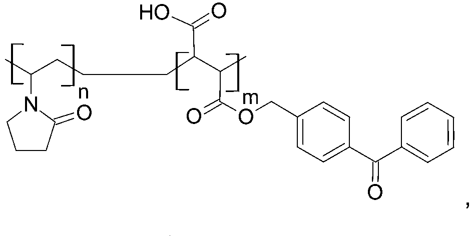 and 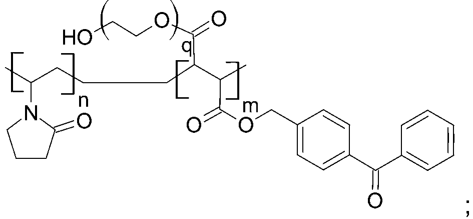 wherein
m = 0.1 - 10.0 mol% and
**n** = 100 mol% - m;
**q** is an integer between 35 and 50.

Preferred are embodiments, wherein
**m** = 0.1 - 5.0 mol%, preferably 0.1 to 2.0 mol%, more preferably 0.1 - 0.95 mol%, more preferably 0.1 - 0.9 mol%, more preferably 0.1 - 0.85 mol%, still more preferably 0.5 -0.85 mol%; and
**n** = 100 mol% - m.

Further preferred are embodiments, wherein
**m** = 1.5 - 2.5 mol%, more preferably 2 mol%; and
**n** = 100 mol% - m.

### Method for Coating of Medical Devices

The present invention therefore also relates to a method of coating a medical device comprising the following steps:
(a) providing a coating solution comprising a polyvinylpyrrolidone copolymer of the present invention;
(b) coating the medical device with the coating solution by dipping, spraying, pipetting, brushing, dropping or brushing.

The copolymers of the present invention can be used as or in all medical devices and medical aids that can be used in or on the body for short or long periods of time, e.g., in the form of coatings for tubes and catheters that must be inserted into the body, such as catheters of any kinds e.g. balloon catheters, bladder catheters, catheter shafts, guide wires, stomach tubes, endoscopes, colonoscopes, needles, cannulas, endotracheal tubes, tracheal tubes, and the like, implants such as stents, eye lenses, joint prostheses, screws, fixations, artificial exits, nasal implants, vocal cords, larynx, artificial and native valves, bone substitutes, respiratory tubes, etc., but also as cartilage substitutes, nerve sheaths, occlusion of venous valves, seals ex corpora and in corpora, subcutaneous or intramuscular implants, as moisturizing and/or active ingredient gel, micro- and macrocapsules, wound protection, breast implants, generally as filling material, plasters, pads, etc., or as a component of the exemplarily mentioned products. Thus, an enormously diverse range of products is made possible.

The possible addition of active ingredients that can be applied or covalently bound over, in or under the layer comprising a copolymer of the present invention allows for therapeutic diversity that, in symbiosis with the properties of the copolymer, provide optimal healing conditions. Importantly, they can exert the desired effect as intended. The choice of active ingredients is based on the physical and chemical interactions with the components of the copolymer as well as with the other additives such as solvents, i.e. with the matrix containing the active ingredient, and they can come from any group of active ingredients.

The combination of the compounds of the invention with other polymers also offers a wide range of possibilities. Suitable polymers include all biocompatible, biodegradable, bioresorbable and biostable polymers. The selection of the added polymer(s) is based on the function that the medical device is to fulfill. In this way, for example, the hydrophilicity and hydrophobicity can be controlled as desired, e.g. by simply mixing the solution with hydrophobic polymers or also by co-polymerization with more hydrophobic monomer components, but other properties such as dimensional stability, the degradation period, tear resistance, flexibility, pressure stability, temperature and light resistance of the end product, hemo- and biocompatibility, etc. can also be predetermined.

It should therefore be noted that the coating composition comprising the copolymers according to the invention, are characterized by particular properties, with the help of which the functionality of both invasive and non-invasive medical devices is substantially improved.

In addition to their versatility and biocompatibility as well as hemocompatibility, they possess excellent and adjustable hydrophilicity, high flexibility and deformability, very low frictional resistance, durability, abrasion resistance, high adhesion, pressure stability, storability and sterilization resistance. short- and long-term usable in or on the body. In addition, all these properties can be modified as desired and as needed.

Surprisingly, the copolymers according to the invention show a substantial improvement in the required properties over polymers produced with the aid of polymerization initiators known from the literature, beyond the previously achieved and known level, and thus lead to a likewise substantial improvement in the medical product compared with the prior art. This advantage also applies in terms of economy in the production of the monomers as well as for the end product.

To prepare a coating, *N*-vinylpyrrolidone is first reacted with the α,β-unsaturated maleic acid comonomer to form a copolymer, and these copolymers exhibit good hydrophilic properties.

Overall, the following general procedure for preparing the copolymers of the invention and coatings with these copolymers is obtained:
1. Synthesis of photosensitive α,β-unsaturated maleic acid comonomers.
2. Preparation of a copolymer of ***N***-vinylpyrrolidone and at least one photosensitive α,β-unsaturated maleic acid comonomer.
3. Optional: Pre-conditioning of surfaces with solvents, heat and or light.
4. Irradiation with light of the optimum wavelength (UV-VIS preferred) or heating in the appropriate temperature range and for the necessary duration of irradiation or heating to initiate a crosslinking reaction.
8. Drying or resting of the final product and, if necessary, removal from shaping production material.
9. package and sterilize

The polymerization reaction is preferably a radical reaction and preferably solution polymerization, but is not limited to this polymerization process. The monomers used for polymerization are preferably polymerized in a "one-pot" process involving a radical initiator (peroxides such as peroxodisulfates, dibenzoyl peroxide, etc. or azo compounds such as AIBN (2,2'-azo-bis(2-methylpropionitrile)), resulting in a linear or nearly linear molecular colloidal product with good hydrophilic properties.

With the aid of polymerization initiators and under suitable reaction conditions and solvents, the unsaturated compounds are interpolymerized with each other.

If monomers are used, random copolymers are obtained; if oligomers are used, random segment copolymers are obtained, as expected. It is also possible to activate one monomer first and add the second or the desired number of different monomers only after a certain reaction time.

### Further Monomers and Polymers

Other monomers or oligomers of biodegradable, bioabsorbable or biostable or very slowly biodegrading polymers can be added and participate in the polymerization reaction and be incorporated into the polymer chain forming a binary, ternary, quaternary, etc. mixed polymer.

Biodegradable, biodegradable or bioabsorbable polymers include. Polyvalerolactones, poly-ε-decalactones, polylactonic acid, polyglycolic acid polylactides, polyglycolides, copolymers of polylactides and polyglycolides, poly-ε-caprolactone, polyhydroxybutyric acid, polyhydroxybutyrates, polyhydroxybutyrate-co-valerates, poly(1,4-dioxane-2,3-diones), poly(1,3-dioxane-2-ones), poly-p-dioxanones, polyan-hydrides such as polymaleic anhydrides, polyhydroxymethacrylates, fibrin, polycyanoacrylates, polycaprolactone dimethylacrylates, poly-b-maleic acid polycaprolactone butyl acrylates, multiblock polymers such as.e.g., of oligocaprolactone diols and oligodioxanone diols, polyether ester multiblock polymers such as PEG and polybutylene terephthalate. polypivotolactones, polyglycolic acid trimethyl carbonates, polycaprolactone glycolides, poly-g-ethylglutamate, poly(DTH-iminocarbonate), poly(DTE-co-DT-carbonate), poly(bisphenol A-iminocarbonate), polyorthoester, polyglycolic acid trimethyl carbonate, polytrimethyl carbonate polyiminocarbonate, poly(N-vinyl) pyrolidone, polyvinyl alcohols, polyesteramides, glycolated polyesters, polyphosphoesters, polyphosphazenes, poly[p-carboxyphenoxy)propane], polyhydroxypentanoic acid, polyanhydrides, polyethylene oxide-propylene oxide, soft polyurethanes, polyurethanes with amino acid residues in the backbone, polyether esters such as polyethylene oxide, polyalkenoxalates, polyorthoesters and their copolymers, lipids, carrageenans, fibrinogen, starch, collagen, protein-based polymers, polyamino acids, synthetic polyamino acids, zein, modified zein, polyhydroxyalkanoates, pectic acid, actinic acid, modified and unmodified fibrin and casein, carboxymethyl sulfate, albumin, furthermore hyaluronic acid, heparan sulfate, heparin, chondroitin sulfate, dextran, b-cyclodextrins, and copolymers with PEG and polypropylene glycol, gum arabic, guar, gelatin, collagen, collagen-N-hydroxysuccinimide, lipids, phospholipids, modifications and copolymers and/or mixtures of the aforementioned substances are used herein. Preferred biodegradable, polymers are povidones, such as Povidone 90 and Povidone 90. Also preferred herein as biodegradable polymers are the commercially available povidones Kollidon^{®} 90F with a molecular weight of 1000kDa - 1500kDa and Kollidon^{®} 30 with a molecular weight of 44kDa - 54 kDa.

Biostable or extremely slowly degradable polymers may also be present or admixed.

The following can be used as biostable substances for the biostable layer(s): polyacrylic acid, polyacrylates such as polymethyl methacrylate, polybutyl methacrylate, polyacrylamide, polyacrylonitrile, polyamides, polyetheramides, polyethyleneamine, polyimides, polycarbonates, polycarbourethanes, polyvinyl ketones, polyvinyl halides, polyvinylidene halides, polyvinyl ethers, polyisobutylenes, polyvinyl aromatics, polyvinyl esters, polyvinyl pyrollidones, polyoxymethylenes, polytetramethylene oxide, polyethylene, polypropylene, polytetrafluoroethylene, polyurethanes, polyetherurethanes silicone-polyetherurethanes, silicone-polyurethanes, silicone-polycarbonate urethanes, polyolefin elastomers, polyisobutylenes, EPDM rubbers, fluorosilicones, carboxymethyl chitosans, polyarylether ether ketones, polyether ether ketones, polyethylene terephthalate, polyvalerates, carboxymethyl cellulose, cellulose, Rayon, Rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM rubbers, silicones such as polysiloxanes, polydimethylsiloxanes, polyvinyl halogens and copolymers, cellulose ethers, cellulose triacetates, chitosans and copolymers and/or mixtures of these substances.

By adding further monomers, oligomers or polymers, the properties of the copolymer can be controlled according to desired specifications. Thus, hydrophilicity or hydrophobicity, frictional resistance, solubility, strength, flexibility, etc. can be influenced in a targeted manner in the presence of thermo- or photoactive compounds, and thus the chemical and physical properties of the molecular colloid network produced as the end product, such as the degree of crosslinking, elasticity, adhesion, strength, "lifetime", durability, sterilizability, etc.. Thus, the possibilities are manifold up to the tailor-made individualized molecular colloidal copolymer.

It is also possible to incorporate unsaturated compounds, which cannot form macropolymers on their own, as a component in a copolymer and thus form new copolymers. Examples of such compounds include α-methylstyrene, allyl chloride, stilbene, allyl alcohols, dichloroethylene, maleic acid, maleic anhydride and trichlorostyrene and their derivatives.

The addition of further additives can provide additional options to further shape the properties of the intended end product, wether in the form of coatings, pads, plasters, fillings, seals, etc., and optimally adjust them to the desired result in the end product.

Biostable, biodegradable, biocompatible, biodegradable, bioresistant polymers and oligomers, low molecular weight compounds, active ingredients, metals, metal compounds, complexes, other compounds activatable in the UV or and in the VIS range, thermosensitive compounds, stabilizers, dyes, salts, glycosaminoglycans, carbohydrates, polysaccharides, proteins and peptides, etc., their mixtures and derivatives can be added as additives. For example, higher or lower molecular weight polymers (HMW and LMW) having the same repeating unit and differing only in molecular weight average (M_{w}) or different polymers with different monomers at similar M_{w} or far different M_{w} can be added as well.

It is important that the additives are accompanied by further optimization of the end product. The number and type of additives depends on what one wants to achieve. For example, it has been shown that the addition of polyvinylpyrrolidone LMW and HMW and subsequent activation via UV also leads to a high degree of hydrophilicity.

In general, all additives are suitable which are biocompatible and useful, do not decompose by short-term irradiation with light or exposure to even short-term temperatures below 30°C,if the decomposition products can no longer contribute to the optimization of the final product.

The component(s) are added to the solution of copolymer according to the invention (which may already have been provided in advance with additives such as active ingredients, polymers, etc.) and, if a homogeneous solution is desired, are ideally also soluble in the solvent (mixture) present or they are soluble in the copolymer.

Suspensions, emulsions as well as layers of the components as phases of different consistency, color, density, miscibility, etc. can also be represented if the final product requires it.

The present invention further relates to coating compositions comprising a vinylpyrrolidone copolymer of the present invention. The present invention further relates to coating compositions comprising a vinylpyrrolidone copolymer of the present invention and a crosslinker of the present invention.

The present invention further relates to coating compositions comprising a vinylpyrrolidone copolymer of the present invention (Component A), a crosslinker (Component B), and a first biostable and biodegradable polymer (Component C) and a second biostable and biodegradable polymer (Component D), wherein Component C is preferably PVP LMW, such as Kollidon^{®} 30, and Component D is preferably PVP HMW, such as Kollidon^{®} 90F.

The possible addition of active ingredients, e.g. applied over, in or under the coating according to the invention or covalently bonded to the coating, enables a therapeutic diversity which, in symbiosis with the properties of the coating, ensure optimal healing conditions. It is important that the active ingredients can develop the desired and intended effect. The choice of active ingredients is based on the physical and chemical interactions with the coating or the matrix containing the coating. These can come from any group of active ingredients and there is a wide choice depending on the therapeutic need, although many active ingredients can be assigned to several groups based on their mode of action.

### Active Agent

Thus, the coating of the present invention preferably contains at least one anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agent, or a mixture thereof. Also preferred is a coating containing at least one antiproliferative, immunosuppressive, antiangiogenic, antiinflammatory, fungicidal, antibiotic, antimigratory, athrombogenic or antithrombotic active agent or a mixture thereof, which may also be covalently bonded to maleic acid and subsequently released by hydrolysis.

The following anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agents may be preferred: sirolimus (rapamycin) all limus derivatives such as everolimus, biolimus, zotarolimus, temsirolimus, omatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoboside, teniposide, nimustine, carmustine, lomustine, cyclophosphamide, 4-hydroxyoxycyclophosphamide, estramustine, melphalan, ifosfamide, tropfosfamide, chlorambucil, bendamustine, dacarbazine, busulfan, procarbazine, treosulfan, tremozolomide, thiotepa, daunorubicin, doxorubicin, aclarubicin, epirubicin, mitoxantrone, idarubicin, bleomycin, mitomycin, dactinomycin, methotrexate, fludarabine, fludarabine 5'-dihydrogen phosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegasparase, anastrozole, exemestane, letrozole, formestane, aminoglutethemide, adriamycin, Azithromycin, spiramycin, cepharantine, 8-ergolines, dimethylergolines, agroclavin, 1-allylisuride, 1-allylterguride, bromerguride, bromocriptine (ergotaman-3',6',18-trione, 2-bromo-12'-hydroxy-2'-(1-methylethyl)-5'-(2-methylpropyl)-, (5'alpha)-), elymoclavine, ergocristine (ergotaman-3',6',18-trione, 12'-hydroxy-2'-(1-methylethyl)-5'-(phenylmethyl)-, (5'-alpha)-), ergocristinine, ergocornine (ergotaman-3',6',18-trione, 12'-hydroxy-2',5'-bis(1-methylethyl), (5'-alpha)-), ergocorninine, ergocryptin (ergotaman-3',6',18-trione, 12'-hydroxy-2'-(1-methylethyl)-5'-(2-methylpropyl)-, (5'alpha)- (9CI)), ergocryptinine, ergometrine, ergonovine (ergobasin, INN: Ergometrine, (8-beta(S))-9,10-didehydro-N-(2-hydroxy-1-methylethyl)-6-methyl-ergoline-8-carboxamide), ergosine, ergosinine, ergotmetrinine, ergotamine (ergotaman-3',6',18-trione, 12'-hydroxy-2'-methyl-5'-(phenylmethyl)-, (5'-alpha)-(9CI)), ergotaminin, ergovalin (ergotaman-3',6',18-trione, 12'-hydroxy-2'-methyl-5'-(1-methylethyl)-, (5'alpha)-), lergotril, lisuride (CAS no.: 18016-80-3, 3-(9,10-didehydro-6-methylergolin-8alpha-yl)-1,1-diethylurea), lysergol, lysergic acid (D-lysergic acid), lysergic acid amide (LSA, D-lysergic acid amide), lysergic acid diethylamide (LSD, D-lysergic acid diethylamide, INN: Lysergamide, (8β)-9,10-didehydro-N,N-diethyl-6-methyl-ergoline-8-carboxamide), isolysergic acid (D-isolysergic acid), Isolysergic acid amide (D-isolysergic acid amide), isolysergic acid diethylamide (D-isolysergic acid diethylamide), Mesulergin, Metergoline, Methergine (INN: Methylergometrin, (8-ßβ)(S))-9,10-didehydro-N-(1-(hydroxymethyl)propyl)-6-methyl-ergoline-8-carboxamide), methylergometrin, methysergide (INN: Methysergide, (8-β)-9,10-didehydro-N-(1-(hydroxymethyl)propyl)-1,6-dimethyl-ergoline-8-carboxamide), pergolide ((8β)-8-((methylthio)methyl)-6-propyl-ergoline), proterguride and terguride, celecoxip, thalidomide, fasudil^{®},cyclosporine, SMC proliferation inhibitor-2w, epothilone A and B, mitoxanthrone, azathioprine, mycophenolate mofetil, c-myc antisense, b-myc antisense, betulinic acid, camptothecin, PI-88 (sulfated oligosaccharide), melanocyte-stimulating hormone (α -MSH), activated protein C, IL1-β inhibitor, thymosinα -1, fumaric acid and its esters, calcipotriol, tacalcitol, lapachol, β-lapachone,podophyllotoxin, betulin, podophyllic acid-2-ethylhydrazide, molgramostim (rhuGM-CSF), peginterferon α-2b, lanograstim (r-HuG-CSF), filgrastim, macrogol, dacarbazine, basiliximab, daclizumab, Selectin (cytokine antagonist), CETP inhibitor, cadherins, cytokine inhibitors, COX-2 inhibitor, NFkB, angiopeptin, ciprofloxacin, camptothecin, fluroblastine, monoclonal antibodies that inhibit muscle cell proliferation, bFGF antagonists, probucol, prostaglandins, 1,11-dimethoxycanthin-6-one, 1-hydroxy-11-methoxycanthin-6-one, scopolectin, colchicine, NO donors such as pentaerythrityl tetranitrate, and syndnoeimines, S-nitroso derivatives, tamoxifen, staurosporine, β-estradiol,α -estradiol, estriol, estrone, ethinylestradiol, fosfestrol, medroxyprogesterone, estradiolcypionate, estradiolbenzoate, tranilast, camebacaurine and other terpenoids used in cancer therapy, verapamil, tyrosine kinase inhibitors (tyrphostins), cyclosporine A and B, paclitaxel and its derivatives such as 6-α -hydroxy-paclitaxel, baccatin, taxotere, synthetically produced as well as macrocyclic oligomers of carbon suboxide (MCS) obtained from native sources and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, Auranofin, sodium aurothiomalate, oxaceprol, celecoxib, β-sitosterol, ademetionin, myrtecaine, polidocanol, nonivamide, levomenthol, benzocaine, aescin, ellipticin, D-24851 (Calbiochem), colcemid, cytochalasin A-E, indanocins, nocadazoles, S 100 protein, bacitracin, vitronectin receptor antagonists, azelastine, guanidyl cyclase stimulator tissue inhibitor of metalloproteinase-1 and 2, free nucleic acids, Nucleic acids incorporated into viral carriers, DNA and RNA fragments, plaminogen activator inhibitor-1, plasminogen activator inhibitor-2, antisense oligonucleotides, VEGF inhibitors, IGF-1, agents from the group of antibiotics such as cefadroxil, cefazolin, cefaclor, cefotixin, tobramycin, gentamycin, penicillins such as dicloxacillin, oxacillin, sulfonamides, metronidazole, antithrombotics such as argatroban, Aspirin, abciximab, synthetic antithrombin, bivalirudin, coumadin, enoxoparin, desulfated and N-reacetylated heparin, tissue plasminogen activator, Gpllb/llla platelet membrane receptor, Factor Xₐ inhibitor antibody, interleukin inhibitors, heparin, hirudin, r-hirudin, PPACK, protamine, sodium salt of 2-methylthiazolidine-2,4-dicarboxylic acid prourokinase, streptokinase, warfarin, urokinase, vasodilators such as dipyramidol, trapidil, nitroprusside, PDGF antagonists such as triazolopyrimidine and seramine, ACE inhibitors such as captopril, cilazapril, lisinopril, enalapril, losartan, thioprotease inhibitors, Prostacyclin, vasiprost, interferonα , β and γ, histamine antagonists, serotonin blockers, apoptosis inhibitors, apoptosis regulators such as p65, NF-kB, or Bcl-xL antisense oligonucleotides, halofuginone, nifedipine, tocopherol, vitamins B1, B2, B6, and B12, folic acid, tranilast, molsidomine, tea polyphenols, epicatechin gallate, epigallocatechin gallate, boswellic acids and their derivatives, leflunomide, Anakinra, etanercept, sulfasalazine, etoposide, dicloxacylline, tetracycline, triamcinolone, mutamycin, procainimide, D24851, SC-58125, retinoic acid, quinidine, disopyrimide, flecainide, propafenone, sotolol, amidoron, natural and synthetically produced steroids such as bryophyllin A, inotodiol, maquiroside A, ghalakinoside, mansonin, strebloside, hydrocortisone, betamethasone, dexamethasone, non-steroidal anti-inflammatory drugs (NSAIDS) such as fenoporfen, ibuprofen, indomethacin, naproxen, phenylbutazone and other antiviral agents such as acyclovir, ganciclovir and zidovudine, antifungal agents such as clotrimazole, flucytosine, griseofulvin, ketoconazole, miconazole, nystatin, terbinafine, antiprozoal agents like chloroquine, mefloquine, quinine, furthermore natural terpenoids like hippocaesculin, barringtogenol-C21-angelat, 14-dehydroagrostistachin, agroskerin, agroskerin, agrostistachin, 17-hydroxyagrostistachin, ovatodiolides, 4,7-oxycycloanisomelic acid, baccharinoids B1, B2, B3 and B7, tubeimoside, bruceanols A, B and C, bruceantinosides C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, furthermore cymarin, apocymarin, aristolochic acid, anopterin, hydroxyanopterin, anemonin, protoanemonin, berberin, cheliburin chloride, cictoxin, sinococulin, bombrestatin A and B, cudraisoflavone A, curcumin, dihydronitidine, nitidine chloride, 12-beta-hydroxypregnadiene-3,20-dione, bilobol, ginkgol, ginkgolic acid, helenalin, indicin, indicin-N-oxide, lasiocarpin, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetin, pancratistatin, liriodenin, oxoushinsunin, aristolactam-AII, bisparthenolidin, periplocoside A, ghalakinoside, ursolic acid, deoxypsorospermine, psycorubin, ricin A, sanguinarin, manwuweizic acid, methylsorbifolin, sphatheliachromene, stizophyllin, mansonin,strebloside, akagerin, dihydrousambaraensin, hydroxyusambarin, strychnopentamine, strychnophyllin, usambarin, usambarensin, berberin, liriodenin, oxoushinsunin, daphnoretin, lariciresinol, methoxylariciresinol, syringaresinol, umbelliferon, afromoson, acetylvismion B, desacetylvismion A, ismion A and B, and sulfur-containing amino acids such as cystine, as well as salts, vhydrates, solvates, enantiomers, racemates, enantiomeric mixtures, diastereomeric mixtures; Metabolites, prodrugs and mixtures of the active ingredients. Other possible additives with a therapeutic effect include halogens, minerals, metals, metal salts, hydrophilic and hydrophobic polymers or oligomers, polysaccharides such as glycosaminoglycans, carragenans, chitosans, oligosaccharides, alginates, peptides, proteins, etc., wherein derivatives and combinations with each other and with the active ingredients lead to possible variations, thus enabling an individually adapted therapy.

### Crosslinkers

The present invention relates to a medical device having a coating of a **cross-linked** vinylpyrrolidone copolymer of the present invention, wherein the coating further comprises a crosslinker of the general formula **(I):**

**BP¹-L¹-Z-L²-BP²** **(I)**

wherein **BP¹** and **BP²** are benzophenone substituents covalently linked to each other via a linker **Z,** wherein the linker **Z** is selected from an aryl linker, an alkyl linker and an amino acid linker, and wherein the linker **Z** has two linkers **L¹** and **L²** each functionalized with one of the two benzophenone substituents **BP¹** and **BP²,** wherein **L¹** and **L²** independently of each other include or consist of: -O-CO-, -NH-CO-, -CO-, -O-, -NH-, -CO-O-, -CO-NH-, -NH-CO-O-, -O-CO-NH-, -O-CO-O-, and -NH-CO-NH-, or wherein the linker **Z** is -O-CO-, -NH-CO-, -CO-, -O-, -NH-, -CO-O-, -CO-NH-, -NH-CO-O-, -O-CO-NH-, -O-CO-O-, or -NH-CO-NH-, wherein **BP¹** and **BP²** are preferably optionally substituted benzoylphenyl.

In preferred embodiments, **L¹** and **L²** are independently of each other selected from the group comprising or consisting of 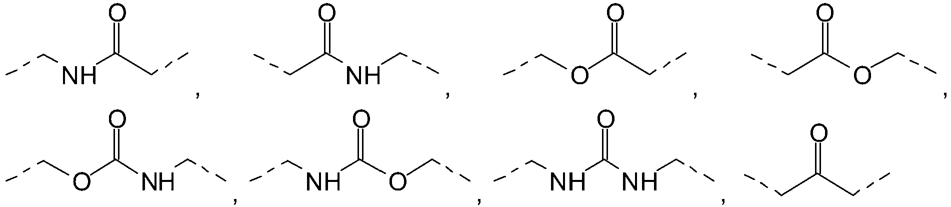 and

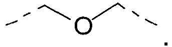

Amide linkages can be prepared by reacting an organic compound with a carboxy group and an organic compound with an amino group:

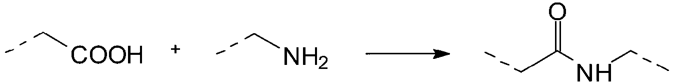

Synthesis conditions, protecting group strategies, alternative synthetic routes for the synthesis of amide-linked compounds are well known from the prior art.

Ester linkages can be prepared by reacting an organic compound with a carboxy group and an organic compound with a hydroxy group:

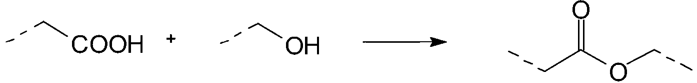

Synthesis conditions, protecting group strategies, alternative synthetic routes for the synthesis of ester-linked compounds are well known from the prior art.

Carbamate linkages can be prepared by reacting an organic compound having an isocyanate group with an organic compound having a hydroxyl group:

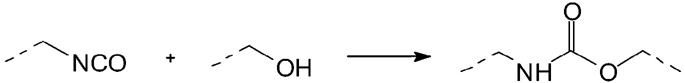

Synthesis conditions, protecting group strategies, alternative synthetic routes for the synthesis of carbamate-linked compounds are well known from the prior art.

Urea linkages can be prepared by reacting an organic compound having an isocyanate group with an organic compound having an amino group:

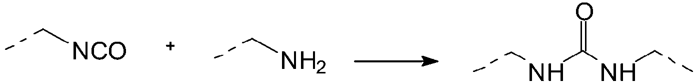

Synthesis conditions, protecting group strategies, alternative synthetic routes for the synthesis of urea-linked compounds are well known from the prior art.

Ether linkages can be prepared by reacting alcoholate with a compound having a suitable leaving group, such as haloalkanes or epoxides, in a nucleophilic substitution reaction. Synthesis conditions, protecting group strategies, alternative synthetic routes for the synthesis of ether-linked compounds are well known from the prior art.

The present invention preferably relates to crosslinkers of the general formula (1):

**BP¹-L¹-Z-L²-BP²** **(I)**

wherein **BP¹** and **BP²** are benzophenone substituents covalently linked to each other via a linker **Z,** wherein the linker **Z** is selected from an aryl linker, an alkyl linker and an amino acid linker, and wherein the linker **Z** has two linkers **L¹** and **L²** each functionalized with one of the two benzophenone substituents **BP¹** and **BP²,** wherein **L¹** and **L²** independently of each other include or consist of: -OCO-, -NHCO-, - CONH-, -NHCOO-, -OCONH-, -OCOO-, and -NHCONH-, or wherein the linker Z is an amino acid linker, -OCO-, -NHCO-, -COO-, -CONH-, -NHCOO-, -OCONH-, -OCOO-, or -NHCONH-, wherein **BP¹** and **BP²** are preferably optionally substituted benzoylphenyl.

In preferred embodiments, **L¹** and **L²** are therefore preferably independently of each other selected from the group comprising or consisting of:

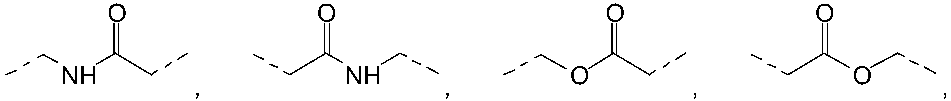

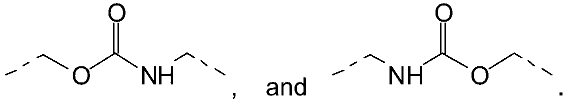

The present invention relates particularly preferably to crosslinkers of the general formula **(I):**

**BP¹-L¹-Z-L²-BP²** **(I)**

wherein **BP¹** and **BP²** are benzophenone substituents covalently linked to each other via a linker **Z,** wherein the linker **Z** is selected from an aryl linker, and an alkyl linker, and wherein the linker **Z** has two linkers **L¹** and **L²** each functionalized with one of the two benzophenone substituents **BP¹** and **BP²,** wherein **L¹** and **L²** independently of each other include or consist of: -NHCO-, or -CONH-, or wherein the linker **Z** is - NHCO-, or -CONH-, wherein **BP¹** and **BP²** are preferably optionally substituted benzoylphenyl.

In particularly preferred embodiments, **L¹** and **L²** are therefore preferably selected independently of each other from the group comprising or consisting of:

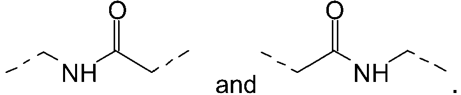

The present invention preferably relates to crosslinkers of the general formula **(Ia):**

**BP¹-L¹-Z-L²-BP²** **(Ia)**

wherein
**Z** represents an optionally substituted aryl linker, an optionally substituted alkyl linker, or
**L¹** represents -R¹-NHCO-R²-, -R¹-CONH-R²-, -R¹-OCO-R²-, -R¹-COO-R²-, -R¹-NHCOO-R²-,-R¹-OCONH-R²-, -R¹-OCOO-R²-, or -R¹-NHCONH-R²-;
**L²** represents -R³-NHCO-R⁴-, -R³-CONH-R⁴-, -R³-OCO-R⁴-, -R³-COO-R⁴- , -R³-NHCOO-R⁴-, -R³-OCONH-R⁴-, -R³-OCOO-R⁴-, or -R³-NHCONH-R⁴-;
   or
**L¹-Z-L²** together form an optionally substituted amino acid linker, -OCO-, -NHCO-, -CO-, -O-, -NH-, -COO-, -CONH-, -NHCOO-, -OCONH-, -OCOO-, or -NHCONH-;
**R¹ - R⁴** independently of each other represent a bond or -CH₂-;
**BP¹** and **BP²** independently of each other represent 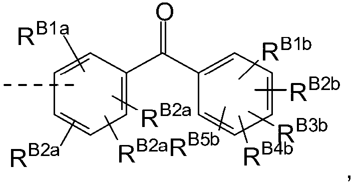
**R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention preferably relates to crosslinkers of the general formula (**Ia**):

**BP¹-L¹-Z-L²-BP²** **(Ia)**

wherein
**Z** represents an optionally substituted aryl linker, an optionally substituted alkyl linker, or an optionally substituted amino acid linker;
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
   or
**L¹-Z-L²** together form an optionally substituted amino acid linker, -OCO-, -NHCO-, -CO-, -O-, -NH-, -COO-, -CONH-, -NHCOO-, -OCONH-, -OCOO-, or -NHCONH-;
**R¹ - R⁴** independently of each other represent a bond or -CH₂-;
**BP¹** and **BP²** independently of each other represent 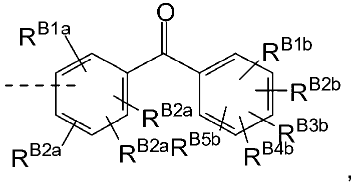
**R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

The present invention preferably relates to crosslinkers of the general formula (Ib):

**BP¹-L¹-Z-L²-BP²** **(Ib)**

wherein
**L¹-Z-L²** represents -OCO-, -COO-, -CONH-, -NHCO-, -NHCOO-, -OCONH-, -CH₂OCO-, -CH₂COO-, -CH₂CONH-, -CH₂NHCO-, -CH₂NHCOO-, -CH₂OCONH-, -OCOCH₂-, -COOCH₂-, -CONHCH₂-, -NHCOCH₂-, -NHCOOCH₂-, -CH₂OCOCH₂-, -CH₂COOCH₂-, -CH₂CONHCH₂-, -CH₂NHCOCH₂-, -CH₂NHCOO CH₂-, or -COR^{AS}NH-, or
wherein
**Z** represents -CH₂-, -C₂H₄-, -CH₂CH₂-, -C₃H₆-, -CH₂CH₂CH₂-, -CH(CH₃)-, -C[(CH₃)₂]- -CH₂-CH(CH₃)-, -CH(C₂H₅), -CH(CH₃)-CH₂-, -C₄H₈-, -CH₂CH₂CH₂CH₂-, -CH₂-C[(CH₃)₂]-, -C[(CH₃)₂]-CH₂-, -CH(CH₃)-C₂H₄-, -C₂H₄-CH(CH₃)-, -CH(CH₃)CH₂CH₂, -CH₂-CH(C₂H₅)-, -CH₂-CH(CH₃)-CH₂-, -CH(CH₃)-CH(CH₃)-, -C[(C₂H₅)(CH₃)]-, -CH(C₃H₇)-, -C₅H₁₀-, -CH₂CH₂CH₂CH₂CH₂-, -CH(CH₃)-C₃H₆-, -C(CH₃)₂-C₂H₄-, -C(C₂H₅)₂-, -CH₂CH(CH₃)-C₂H₄-, -CH(CH₃)-C(CH₃)₂-, -C₂H₄-C(CH₃)₂-, -C₆H₁₂-, -CH₂CH₂CH₂CH₂CH₂CH₂-, -C₃H₆-CH(CH₃)₂-, -C₂H₄-CH(CH₃)-C₂H₅-, -CH₂-CH(CH₃)-C₃H₇-, -CH(CH₃)-C₄H₉-, -CH(CH₃)-CH₂-C(CH₃)₂-, -C(CH₃)₂-C₃H₇-, -CH(CH₃)-CH(CH₃)-C₂H₅-, -CH₂-CH(CH₃)-CH(CH₃)₂-, -CH₂-C(CH₃)₂-C₂H₅-, -C(CH₃)₂-C(CH₃)₂-, -CH₂-C(C₂H₅)₂-, -CH₂CH₂CH₂CH₂CH₂CH₂CH₃-, -C₆H₁₂-, -C₇H₁₄-, -C₈H₁₆-, -C₉H₁₈-, -C₁₀H₂₀-, -CH₂CH₂CH₂CH₂CH₂CH₂CH₂-, -C₇H₁₄-, -C₈H₁₆-, -C₉H₁₈-, -C₁₀H₂₀-, -CR^{1a}R^{1b}-, -CR^{1a}R^{1b}-CR^{2a}R^{2b}-, -CR^{1a}R^{1b}-CR^{2a}R^{2b}-CR^{3a}R^{3b}-, -CR^{1a}R^{1b}-CR^{2a}R^{2b}-CR^{3a}R^{3b}-CR^{4a}R^{4b}-, -CR^{1a}R^{1b}-CR^{2a}R^{2b}-CR^{3a}R^{3b}-CR^{4a}R^{4b}-CR^{5a}R^{5b}- -CR^{1a}R^{1b}-CR^{2a}R^{2b}-CR^{3a}R^{3b}-CR^{4a}R^{4b}-CR^{5a}R^{5b}-CR^{6a}R^{6b}-, -CR^{1a}R^{1b}-V-CR^{2a}R^{2b}-, -CR^{1a}R^{1b}-V-CR^{2a}R^{2b}-CR^{3a}R^{3b}-, -CR^{1a}R^{1b}-CR^{2a}R^{2b}-V-CR^{3a}R^{3b}-, -CR^{1a}R^{1b}-V-CR^{2a}R^{2b}-CR^{3a}R^{3b}-CR^{4a}R^{4b}-, -CR^{1a}R^{1b}-CR^{2a}R^{2b}-V-CR^{3a}R^{3b}-CR^{4a}R^{4b}- -CR^{1a}R^{1b}-CR^{2a}R^{2b}-CR^{3a}R^{3b}-V-CR^{4a}R^{4b}-, -CR^{1a}R^{1b}-V-CR^{2a}R^{2b}-CR^{3a}R^{3b}-V-CR^{4a}R^{4b}-, -CR^{1a}R^{1b}-V-CR^{2a}R^{2b}-CR^{3a}R^{3b}-CR^{4a}R^{4b}-CR^{5a}R^{5b}-, -CR^{1a}R^{1b}-CR^{2a}R^{2b}-V-CR^{3a}R^{3b}-CR^{4a}R^{4b}-CR^{5a}R^{5b}-, -CR^{1a}R^{1b}-CR^{2a}R^{2b}-CR^{3a}R^{3b}-V-CR^{4a}R^{4b}-CR^{5a}R^{5b}-, -CR^{1a}R^{1b}-CR^{2a}R^{2b}-CR^{3a}R^{3b}-CR^{4a}R^{4b}-V-CR^{5a}R^{5b}- -CR^{1a}R^{1b}-V-CR^{2a}R^{2b}-CR^{3a}R^{3b}-V-CR^{4a}R^{4b}-CR^{5a}R^{5b}-, -CR^{1a}R^{1b}-CR^{2a}R^{2b}-V-CR^{3a}R^{3b}-CR^{4a}R^{4b}-V-CR^{5a}R^{5b-}, -CR^{1a}R^{1b}-V-CR^{2a}R^{2b}-CR^{3a}R^{3b}-CR^{4a}R⁴-V-CR^{5a}R^{5b}-, -CR^{1a}R^{1b}-V-CR^{2a}R^{2b}-CR^{3a}R^{3b}-CR^{4a}R^{4b}-CR^{5a}R^{5b}-CR^{6a}R^{6b}-, -CR^{1a}R^{1b}-CR^{2a}R^{2b}-V-CR^{3a}R^{3b}-CR^{4a}R^{4b}-CR^{5a}R^{5b}-CR^{6a}R^{6b}-, -CR^{1a}R^{1b}-CR^{2a}R^{2b}-CR^{3a}R^{3b}-V-CR^{4a}R^{4b-}CR^{5a}R^{5b}-CR^{6a}R^{6b}-, -CR^{1a}R^{1b}-CR^{2a}R^{2b}-CR^{3a}R^{3b}-CR^{4a}R^{4b}-V-CR^{5a}R^{5b}-CR^{6a}R^{6b}-, -CR^{1a}R^{1b}-CR^{2a}R^{2b}-CR^{3a}R^{3b}-CR^{4a}R^{4b}-CR^{5a}R⁵-V-CR^{6a}R^{6b}-, -CR^{1a}R^{1b}-V-CR^{2a}R^{2b}-CR^{3a}R^{3b}-V-CR^{4a}R^{4b}-CR^{5a}R^{5b}-CR^{6a}R^{6b}-, -CR^{1a}R^{1b}-V-CR^{2a}R^{2b}-CR^{3a}R^{3b}-CR^{4a}R^{4b}-V-CR^{5a}R^{5b}-CR^{6a}R^{6b}-, -CR^{1a}R^{1b}-V-CR^{2a}R^{2b}-CR^{3a}R^{3b}-CR^{4a}R^{4b}-CR^{5a}R^{5b}-V-CR^{6a}R^{6b}-, -CR^{1a}R^{1b}-CR^{2a}R^{2b}-V-CR^{3a}R^{3b}-CR^{4a}R^{4b}-V-CR^{5a}R^{5b}-CR^{6a}R^{6b}- , -CR^{1a}R^{1b}-CR^{2a}R^{2b}-V-CR^{3a}R^{3b}-CR^{4a}R^{4b}-CR^{5a}R^{5b}-V-CR^{6a}R^{6b}-, -CR^{1a}R^{1b}-CR^{2a}R^{2b}-CR^{3a}R^{3b}-V-CR^{4a}R^{4b}-CR^{5a}R^{5b}-V-CR^{6a}R^{6b}- ,-CR^{1a}R^{1b}-V-CR^{2a}R^{2b}-CR^{3a}R^{3b}-V-CR^{4a}R^{4b}-CR^{5a}R^{5b}-V-CR^{6a}R^{6b}-, 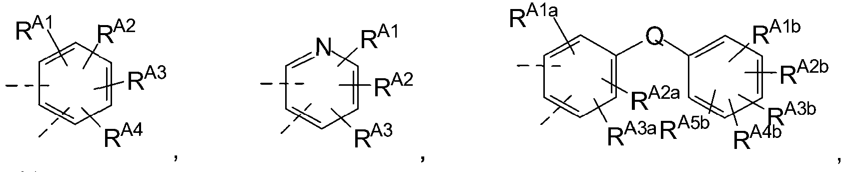 or 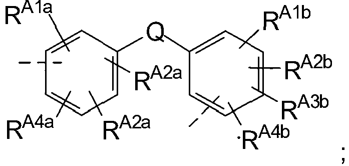
**V** represents -O-, -NH-, -NR^{N}-, -CO-, -O-CO-, -NH-CO-, -CO-O-, -CO-NH-, -NH-CO-O-, -O-CO-NH-, or -NH-CO-NH-;
**R^{N}** represents -CH₃, -C₂H₅, -C₃H₇, -CH(CH₃)₂, -cyclo-C₃H₅, -C₄H₉, -CH₂-CH(CH₃)₂, -CH(CH₃)-C₂H₅, -C(CH₃)₃, -cyclo-C₄H₇, -CH₂-cyclo-C₃H₅, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₅H₉, -CH₂-Cyclo-C₄H₇, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -cyclo-C₆H₁₁, -CH₂-cyclo-C₅H₉, -C₇H₁₅, -cyclo-C₇H₁₃, -CH₂-cyclo-C₆H₁₁, or -C₈H₁₇ or -cyclo-C₈H₁₅;
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
**R¹- R⁴** independently of each other represent a bond or -CH₂-;
**BP¹** and **BP²** independently of each other represent 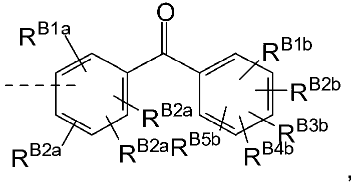
**R^{1a} - R^{6a} , R^{1b} - R^{6b}, R^{A1} - R^{A4}, R^{A1a}** - **R^{A4a}, R^{A1b}**- **R^{A4b}, R^{B1a}** - **R^{B4a}** and **R^{B1b}** - **R^{B5b}** independently of each other represent
-F, -CI, -Br, -I, -CN, -N₃, -OCN, -NCO, -SCN, -NCS, -COOH, -COCN, -OH, -CHO, -NO₂, -NH₂, -COCH₃, -CONH₂, -SO₃H, -SO₂NH₂, -NHCONH₂, -NHC(=NH)NH₂, -OCONH₂ -CH₃ , alkyl substituents such as -CH₃, -C₂H₅, -C₃H₇, -CH(CH₃)₂, -cyclo-C₃H₅, -C₄H₉, -CH₂CH(CH₃)₂, -CH(CH₃)C₂H₅, -C(CH₃)₃, -cyclo-C₄H₇, -CH₂-cyclo-C₃H₅, -C₅H₁₁, -CH(CH₃)C₃H₇, -CH₂CH(CH₃)C₂H₅, -CH(CH₃)CH(CH₃)₂, -C(CH₃)₂-C₂H₅, -CH₂C(CH₃)₃, -CH(C₂H₅)₂, -C₂H₄CH(CH₃)₂, -cyclo-C₅H₉, -CH₂-cyclo-C₄H₇, -C₆H₁₃, -C₃H₆CH(CH₃)₂, -C₂H₄CH(CH₃)-C₂H₅, -CH(CH₃)C₄H₉, -CH₂CH(CH₃)-C₃H₇, -CH(CH₃)CH₂CH(CH₃)₂, -CH(CH₃)CH(CH₃)C₂H₅, -CH₂CH(CH₃)CH(CH₃)₂, -CH₂C(CH₃)₂C₂H₅, -C(CH₃)₂C₃H₇, -C(CH₃)₂CH(CH₃)₂, -C₂H₄C(CH₃)₃, -CH(CH₃)C(CH₃)₃, -cyclo-C₆H₁₁, -CH₂-cyclo-C₅H₉, -C₇H₁₅, -cyclo-C₇H₁₃, -CH₂-cyclo-C₆H₁₁, -C₈H₁₇, -cyclo-C₈H₁₅, haloalkyl substituents such as -CH₂F, -CHF₂, -CF₃, -CH₂Cl, -CH₂CH₂F, -CH₂CHF₂, -CH₂CF₃, -CH₂Br, -CH₂CH₂Cl, -CH₂CH₂Br, arylalkyl substituents such as -CH₂Ph, -Ph, -C₂H₄Ph, -PhPh, alkoxy substituents such as -OCH₃, -OC₂H₅, -OC₃H₇, -OCH(CH₃)₂, -OC₄H₉, -OCH₂CH(CH₃)₂, -CH(CH₃)C₂H₅, -OC(CH₃)₃, -OCH₂-cyclo-C₃H₅, -OC₅H₁₁, -OCH(CH₃)-C₃H₇, -OCH₂CH(CH₃)-C₂H₅, -OCH(CH₃)CH(CH₃)₂, -OC(CH₃)₂-C₂H₅, -OCH₂-C(CH₃)₃, -OCH(C₂H₅)₂, -OC₂H₄CH(CH₃)₂, -OCH₂-cyclo-C₄H₇, -OC₆H₁₃, -OC₃H₆CH(CH₃)₂, -OC₂H₄CH(CH₃)C₂H₅, -OCH(CH₃)C₄H₉, -OCH₂CH(CH₃)C₃H₇, -OCH(CH₃)CH₂CH(CH₃)₂, -OCH(CH₃)CH(CH₃)C₂H₅, -OCH₂CH(CH₃)CH(CH₃)₂, -OCH₂C(CH₃)₂C₂H₅, -OC(CH₃)₂C₃H₇, -OC(CH₃)₂CH(CH₃)₂, -OC₂H₄C(CH₃)₃, -OCH(CH₃)C(CH₃)₃, -OCH₂-cyclo-C₅H₉, -OC₇H₁₅, -OCH₂-cyclo-C₆H₁₁, -OC₈H₁₇, -OCF₃, -OC₂F₅, -OCH₂Ph, -OPh; -OC₂H₄Ph, -OC₂H₅OH, -OC₃H₇OH, -OC₄H₉OH, -OC₅H₁₁OH, -OC₆H₁₃OH, -OC₂H₅COOH, -OC₃H₇COOH, -OC₄H₉COOH, -OC₅H₁₁COOH, -OC₆H₁₃COOH, -OC₂H₅NH₂, -OC₃H₇ NH₂, -OC₄H₉NH₂, -OC₅H₁₁NH₂, -OC₆H₁₃NH₂, -OC₂H₅OC₂H₅OH, -OC₂H₅OC₂H₅OC₂H₅OH, -OC₂H₅OC₂H₅OC₂H₅OC₂H₅OH, alkylamine substituents such as -NHCH₃, -NHC₂H₅, -NHC₃H₇, -NHC₄H₉, -NH-cyclo-C₃H₅, -NH[CH(CH₃)_{2]}, -NH[C(CH₃)_{3]}, -N(CH₃)₂, -N(C₂H₅)₂, -N(C₃H₇)₂, -N(C₄H₉)₂, -N(cyclo-C₃H₅)₂, -N[CH(CH₃)_{2]2}, -N[C(CH₃)_{3]2}, ester substituents such as -COOC(CH₃)₃, -COOCH₃, -COOCH₂CH₃, -COOCH₂CH₂CH₃, -COOCH(CH₃)₂, -COOCH₂Ph, -OCOCH₃, -OCOC₂H₅, -OCOC₃H₇, -OCO-cyclo-C₃H₅, -OCOC₄H₉, -OCOCH(CH₃)₂, -OCOCH₂Ph, -OCOC(CH₃)₃, amide substituents such as -NHCOCH₃, -NHCOC₂H₅, -NHCOC₃H₇, -NHCOCH₃, -NHCOC₂H₅, -NHCOC₃H₇, -NHCOC₄H₉, -NHCO-cyclo-C₃H₅, -NHCO[CH(CH₃)_{2]}, -NHCO[C(CH₃)_{3]}, sulfonic substituents such as -SO₂CH₃, -SO₂C₂H₅, -SO₂C₃H₇, -SO₂Ph, -SO₂-cyclo-C₃H₅, -SO₂CH(CH₃)₂, -SO₂C₄H₉, -SO₂C(CH₃)₃, sulfonamide substituents such as -NHSO₂CH₃, -NHSO₂C₂H₅, -NHSO₂C₃H₇, -CH₂SO₂NH₂, -C₂H₄SO₂NH₂, -C₃H₆SO₂NH₂, sulfonoxide substituents such as -SOCH₃, -SOC₂H₅, -SOC₃H₇, -SO-cyclo-C₃H₅, -SOCH(CH₃)₂, -SOC(CH₃)₃, carbonic ester substituents such as -OCOOCH₃, -OCOOC₂H₅, -OCOOC₃H₇, -OCOO-cyclo-C₃H₅, -OCOOC₄H₉, -OCOOCH(CH₃)₂, -OCOOCH₂Ph, -OCOOC(CH₃)₃, alkyl urea substituents such as -NHCONHCH₃, -NHCONHC₂H₅, -NHCONHC₃H₇, -NHCONHC₄H₉, -NHCONH-cyclo-C₃H₅, -NHCONH[CH(CH₃)_{2]}, -NHCONH[C(CH₃)_{3]}, -NHCON(CH₃)₂, -NHCON(C₂H₅)₂, -NHCON(C₃H₇)₂, -NHCON(C₄H₉)₂, -NHCON(cyclo-C₃H₅)₂, -NHCON[CH(CH₃)₂]₂, -NHCON[C(CH₃)₃]₂, alkylguanidine substituents such as -NHC(=NH)NHCH₃, -NHC(=NH)NHC₂H₅, -NHC(=NH)NHC₃H₇, -NHC(=NH)NHC₄H₉, -NHC(=NH)NH-cyclo-C₃H₅, -NHC(=NH)NH[CH(CH₃)₂], -NHC(=NH)NH[C(CH₃)₃], -NHC(=NH)N(CH₃)₂, -NHC(=NH)N(C₂H₅)₂, -NHC(=NH)N(C₃H₇)₂, -NHC(=NH)-N(cyclo-C₃H₅)₂, -NHC(=NH)N(C₄H₉)₂, -NHC(=NH)N[CH(CH₃)₂]₂, -NHC(=NH)N[C(CH₃)₃]₂, alkyl carbamate substituents such as -OCONHCH₃, -OCONHC₂H₅, -OCONHC₃H₇, -OCONHC₄H₉, -OCONH-cyclo-C₃H₅, -OCONH[CH(CH₃)_{2]}, -OCONH[C(CH₃)_{3]}, -OCON(CH₃)₂, -OCON(C₂H₅)₂, -OCON(C₃H₇)₂, -OCON(C₄H₉)₂, -OCON(cyclo-C₃H₅)₂, -OCON[CH(CH₃)₂]₂, -OCON[C(CH₃)₃]₂, unsaturated alkoxy substituents such as -OC₂H₄CH=CH₂, -OCH₂-CH=CHCH₃, -OC₂H₄C≡CH, -OCH₂C≡CH, -OCH₂C≡CCH₃, -OC₃H₆CH=CH₂, -OC₂H₄CH=CH-CH₃, -OCH₂CH=CHC₂H₅, -OCH₂CH=CHCH=CH₂, -OC(CH₃)=CH-CH=CH₂, -OCH₂CH=C(CH₃)₂, -OC(CH₃)=C(CH₃)₂, -OC₃HeC≡CH, -OC₂H₄C≡CCH₃, -OCH₂C≡CC₂H₅, -OCH₂C≡CCH=CH₂, -OCH₂CH=CHC≡CH, -OCH₂C≡CC≡CH, -OC₄H₈CH=CH₂, -CH=CHC₄H₉, -OC₃H₆CH=CHCH₃, -OCH₂CH=CHC₃H₇, -OC₂H₄-CH=CHC₂H₅, -OCH₂C(CH₃)=C(CH₃)₂, -OC₂H₄CH=C(CH₃)₂, -OC₄H₈C≡CH, -OC≡CC₄H₉, -OC₃HₑC≡CCH₃, -OCH₂C≡CC₃H₇, -OC₂H₄C≡CC₂H₅, alkenyl substituents and alkynyl substituents such as -C₂H₄CH=CH₂, -CH=CHC₂H₅, -CH=C(CH₃)₂, -CH₂CH=CHCH₃, -CH=CHCH=CH₂, -C₂H₄C≡CH, -C≡CC₂H₅, -CH₂C≡CCH₃, -C≡CCH=CH₂, -CH=CHC=CH, -C≡CC≡CH, -C₃H₆CH=CH₂, -CH=CHC₃H₇, -C₂H₄CH=CHCH₃, -CH₂CH=CHC₂H₅, -CH₂CH=CHCH=CH₂, -CH=CHCH=CHCH₃, -CH=CHCH₂CH=CH₂, -C(CH₃)=CHCH=CH₂, -CH=C(CH₃)-CH=CH₂, -CH=CHC(CH₃)=CH₂, -CH₂CH=C(CH₃)₂, -C(CH₃)=C(CH₃)₂, -C₃HeC≡CH, -C≡CC₃H₇, -C₂H₄C≡CCH₃, -CH₂C≡CC₂H₅, -CH₂C≡CCH=CH₂, -CH₂CH=CHC≡CH, -CH_{2C}≡CC≡CH, -C≡CCH=CHCH₃, -CH=CHC≡CCH₃, -C≡CC≡CCH₃, -C≡CCH₂CH=CH₂, -CH=CHCH₂C≡CH, -C≡CCH₂C≡CH, -C(CH₃)=CHCH=CH₂, -CH=C(CH₃)CH=CH₂, -CH=CHC(CH₃)=CH₂, -C(CH₃)=CHC≡CH, -CH=C(CH₃)C≡CH, -C≡CC(CH₃)=CH₂, -C₄H₈CH=CH₂, -CH=CHC₄H₉, -C₃H₆CH=CH-CH₃, -CH₂CH=CH-C₃H₇, -C₂H₄CH=CH-C₂H₅, -CH₂C(CH₃)=C(CH₃)₂, -C₂H₄CH=C(CH₃)₂, -C₄H₈C≡CH, -C≡CC₄He, -C₃H₆C≡CCH₃, -CH₂C≡CC₃H₇, -C₂H₄C≡CC₂H₅, -CH=CHPh or -C=CPh;
Q represents -CH₂-, or -CO-; and
**R^{AS}** is selected from

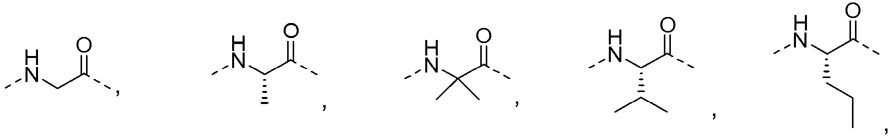

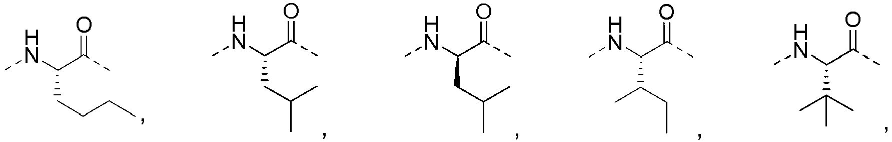

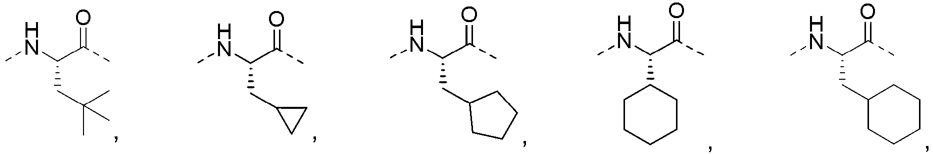

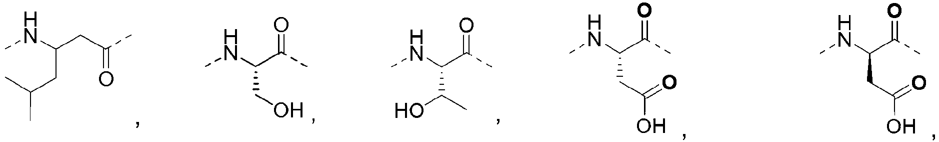

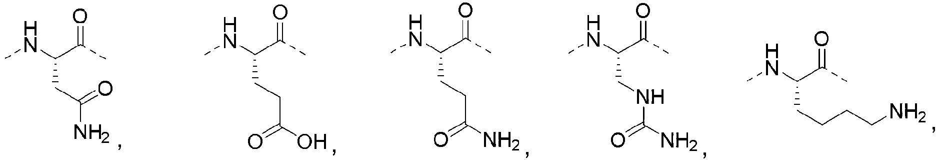

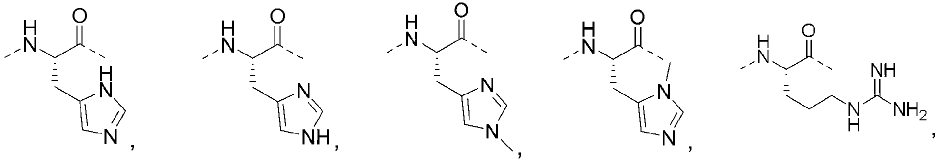

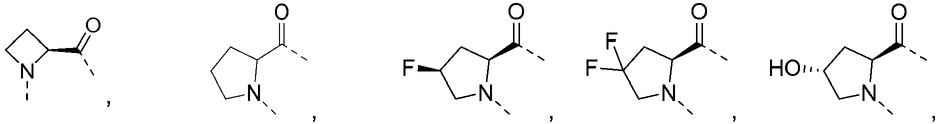

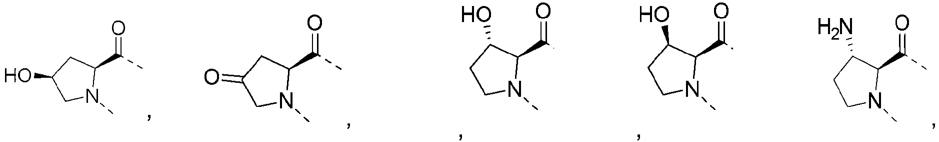

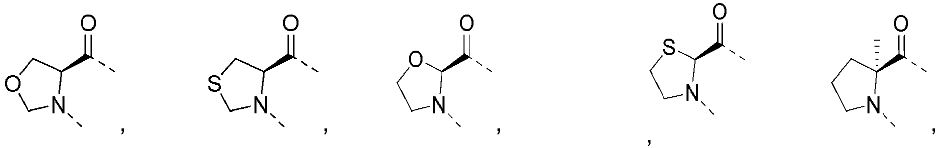

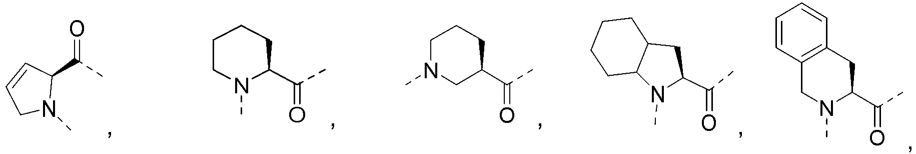

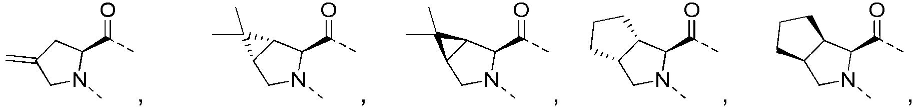

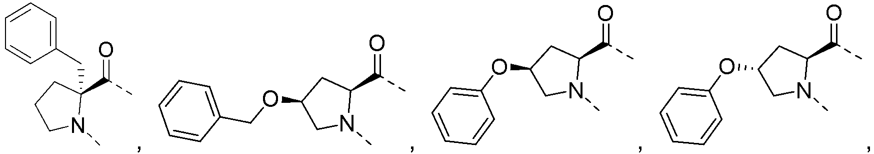

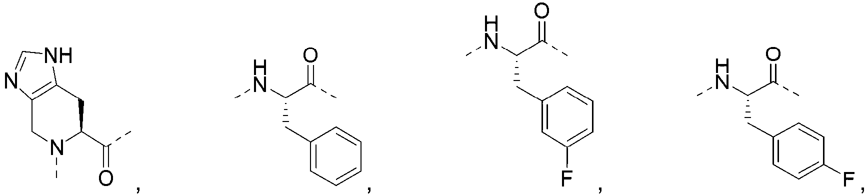

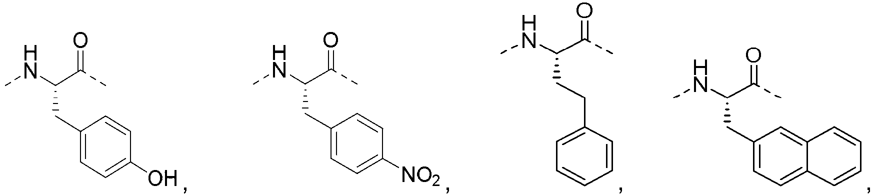

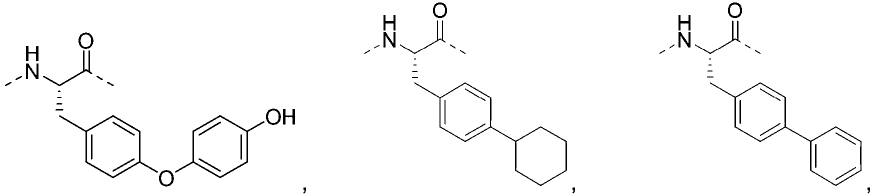

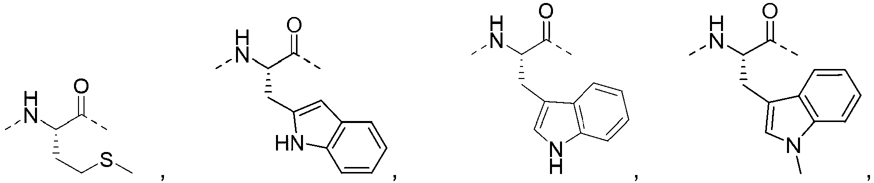

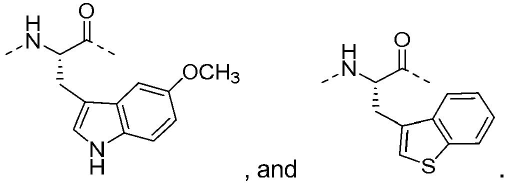

In preferred embodiments, **R^{1a}** - **R^{6a}, R^{1b} - R^{6b}, R^{A1} - R^{A4}, R^{A1a}** - **R^{A4a}, R^{A1b}** - **R^{A4b}, R^{B1a}** - **R^{B4a}** and **R^{B1b}** - **R^{B5b}** independently of each other represent -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

In preferred embodiments, **R^{1a} - R^{6a}, R^{1b} - R^{6b}, R^{A1} - R^{A4}, R^{A1a} - R^{A4a}, R^{A1b} - R^{A4b}, R^{B1a}** - **R^{B4a}** and **R^{B1b}** - **R^{B5b}** independently of each other represent -H, -OH, -NO₂, -COCH₃, -COOH, -CONH₂, -SO₃H, -SO₂NH₂, -OCH₃, -OCF₃, -NH₂, -NH(CH)₃, -N(CH₃)₂, or -SOCH₃.

The present invention preferably relates to crosslinkers of the general formula **(Ib):**

**BP¹-L¹-Z-L²-BP²** **(Ib)**

wherein
**Z** represents 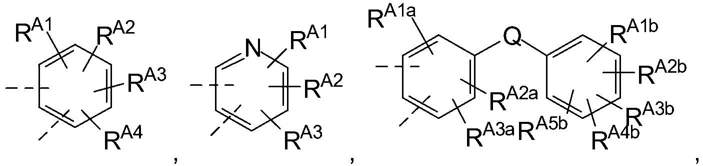 or 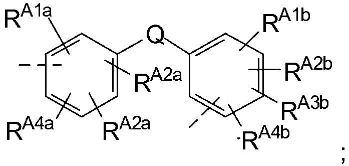
**L¹** represents -R¹-NHCO-R²-, -R¹-CONH-R²-, -R¹-OCO-R²-, -R¹-COO-R²-, -R¹-NHCOO-R²-, or -R¹-OCONH-R²-;
**L²** represents -R³-NHCO-R⁴-, -R³-CONH-R⁴-, -R³-OCO-R⁴-, -R³-COO-R⁴-, -R³-NHCOO-R⁴-, or -R³-OCONH-R⁴-;
**R¹** - **R⁴** independently of each other represent a bond or -CH₂-;
**BP¹** and **BP²** independently of each other represent 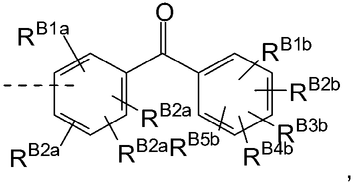
**R^{A1}** - **R^{A4}, R^{A1a}** - **R^{A4a}, R^{A1b}** - **R^{A4b}, R^{B1a}** - **R^{B4a}** and **R^{B1b}** - **R^{B5b}** independently of
each other represent -H, -COOH, -CH₃ , -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.
**Q** represents -CH₂-, or -CO-; and

In preferred embodiments, **R^{1a}** - **R^{6a}, R^{1b}** - **R^{6b}, R^{A1} - R^{A4}, R^{A1a}** - **R^{A4a}, R^{A1b}** - **R^{A4b}, R^{B1a}** - **R^{B4a}** and **R^{B1b}** - **R^{B5b}** independently of each other represent -H, -OH, -NO₂, -COCH₃, -COOH, -CONH₂, -SO₃H, -SO₂NH₂, -OCH₃, -OCF₃, -NH₂, -NH(CH)₃, -N(CH₃)₂, or -SOCH₃.

Thus, the present invention relates to crosslinkers of the general formula **(Id):**

**BP¹-L¹-Z-L²-BP²** **(Id)**

wherein
**L¹-Z-L²** represents -OCO-, -COO-, -CONH-, -NHCO-, -NHCOO-, -OCONH-, -CH₂OCO-, -CH₂COO-, -CH₂CONH-, -CH₂NHCO-, -CH₂NHCOO-, -CH₂OCONH-, -OCOCH₂-, -COOCH₂-, -CONHCH₂-, -NHCOCH₂-, -NHCOOCH₂-, -CH₂OCOCH₂-, -CH₂COOCH₂-, -CH₂CONHCH₂-, -CH₂NHCOCH₂-, or -CH₂NHCOOCH₂-,
**BP¹** and **BP²** independently of each other represent 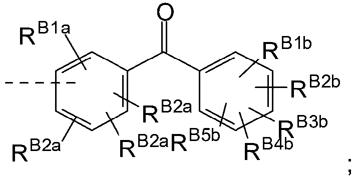 and **R^{B1a}** - **R^{B4a}** and **R^{B1b}** - **R^{B5b}** are as defined herein.

In preferred embodiments, **R^{B1a}** - **R^{B4a}** and **R^{B1b}** - **R^{B5b}** are independently of each other -H, -F, -Cl, -Br, -I; -CN, -N₃, -OCN, -NCO, -SCN, -NCS, -COOH, -COCN, -OH, -CHO, -NO₂, -NH₂, -COCH₃, -CONH₂, -SO₃H, -SO₂NH₂, -NH-CO-NH₂, -NH-C(=NH)-NH₂, -O-CO-NH₂, -CH₃, -OCH₃, -OC₂H₅, -OC₃H₇, -OCH(CH₃)₂, -OC(CH₃)₃, -OC₄H₉, -O-cyclo-C₃H₅, -OCH₂-cyclo-C₃H₅, -O-C₂H₄-cyclo-C₃H₅; -OCH₂CH(CH₃)₂, -OC₂H₄OCH₃, -CH₂OCH₃, -CH₃, -CF₃, -C₂H₅, or -C₃H₇.

In further preferred embodiments, **R^{B1a}** - **R^{B4a}** and **R^{B1b}** - **R^{B5b}** are independently of each other -H, OH, -NO₂, -COCH₃, -COOH, -CONH₂, -SO₃H, -SO₂NH₂, -OCH₃, -OCF₃, -NH₂, -NH(CH)₃, -N(CH₃)₂, or -SOCH₃.

In particularly preferred embodiments, **R^{B1a}** - **R^{B4a}** and **R^{B1b}** - **R^{B5b}** are -H.

In some embodiments, crosslinkers of the following formulas are preferred: 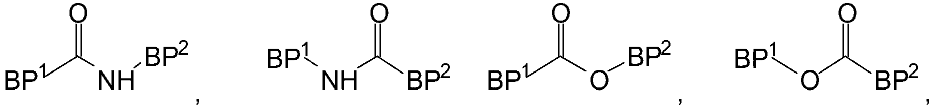 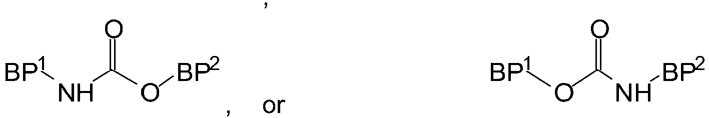 and **BP¹** and **BP²** are as defined herein.

In some further embodiments, crosslinkers of the following formulas are preferred: 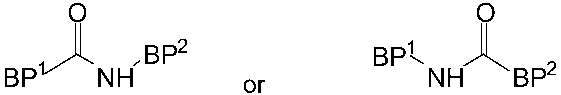 and **BP¹** and **BP²** are as defined herein.

The present invention thus relates to crosslinkers of the general formula **(Id):**

**BP¹-L¹-Z-L²-BP²** **(Id)**

wherein **-L¹-Z-L²-** is selected from 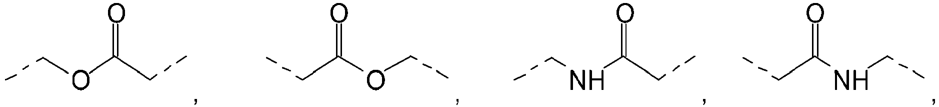 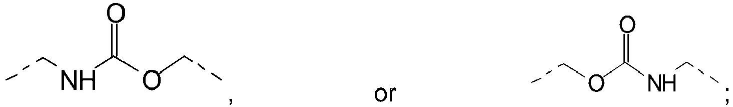
and **BP¹** and **BP²** are as defined herein.

Examples of some of these preferred crosslinkers crosslinkers of the general formula **(Id)** are: 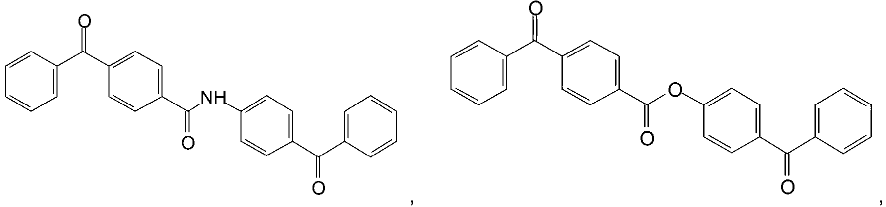 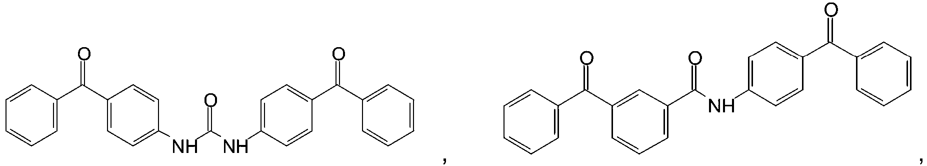 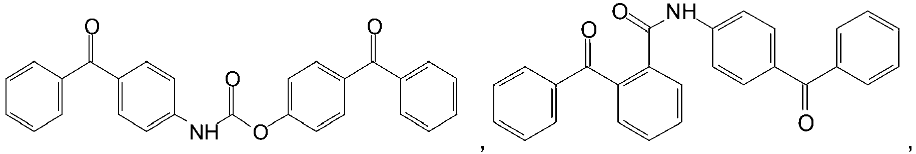 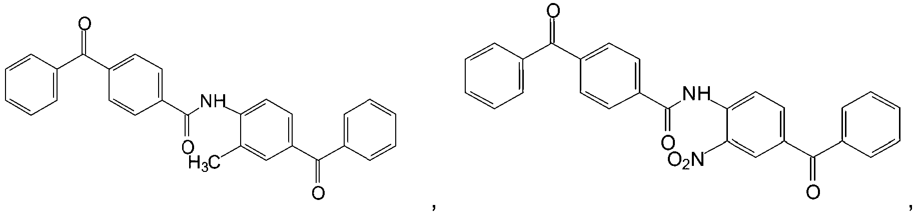 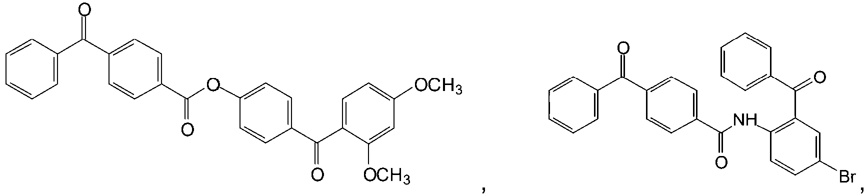 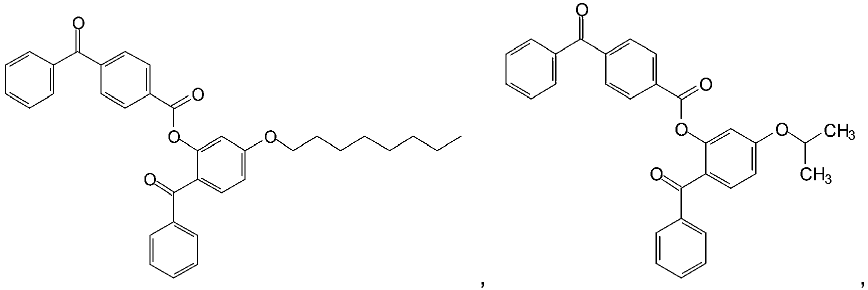 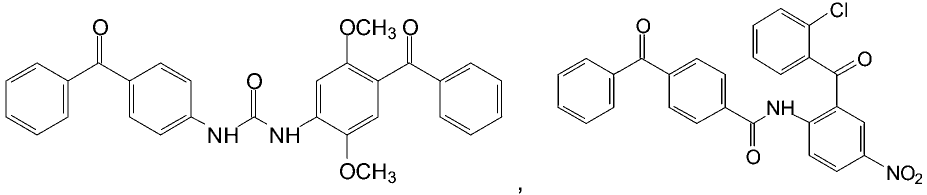 and

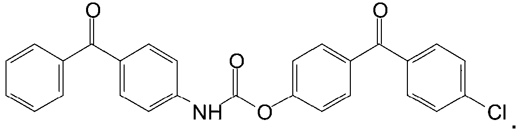

The present invention thus relates to crosslinkers of the general formula **(Ie):**

**BP¹-L¹-Z-L²-BP²** **(Ie)**

wherein
**-L¹-Z-L²-** represents -CO-R^{AS}-NH-;
and **R^{AS}, BP¹,** and **BP²** are as defined herein.

As defined herein, -CO-R^{AS}-NH- has the following structure:

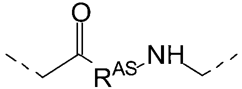

In general, R^{AS} has the following structure:

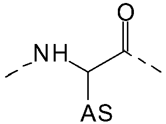

Preferably, -CO-R^{AS}-NH- has therefore the following structure:

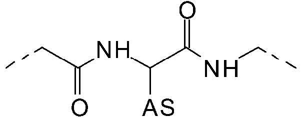

**R^{AS}** is preferably selected from amino acids such as glycine, alanine, leucine, isoleucine, valine, proline, tyrosine, phenylanaline, histidine, arginine, lysine, glutamic acid, aspartic acid, glutamine, asparagine, cysteine, tryptophan, methionine, serine, threonine, and derivatives thereof. In some embodiments, **R^{AS}** is preferably selected from histidine, arginine, or lysine. In some embodiments, **R^{AS}** is preferably selected from glutamic acid or aspartic acid. In some embodiments, **R^{AS}** is preferably selected tyrosine, threonine, glutamine, glycine, serine, cysteine, or asparagine. In some embodiments, **R^{AS}** is preferably selected from alanine, valine, methionine, leucine, isoleucine, proline, tryptophan, or phenylalanine.

Examples of some of these preferred crosslinkers of the general formula **(Ie)** are: 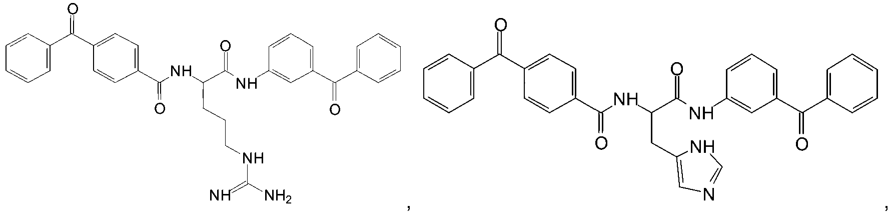 and

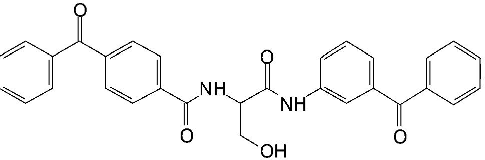

Preferably, the present invention relates to crosslinkers of general formula (I):

**BP¹-L¹-Z-L²-BP²** **(I)**

wherein **-L¹-Z-L²-** represents
-R¹-OCO-R²-Z-R³-COO-R ⁴- -R¹-COO-R²-Z-R³-OCO-R⁴-, -R¹-OCO-R² -Z-R³-OCO-R⁴- -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴- , -R¹-CONH-R²-Z-R³-CONH-R⁴-, -R¹-NHCOO-R²-Z-R³-OCONH-R⁴- , -R¹-OCONH-R²-Z-R³-NHCOO-R⁴-, -R¹-NHCOO-R²-Z-R³-NHCOO-R⁴- , -R¹-OCONH-R²-Z-R³-OCONH-R⁴-, -R¹-OCO-R²-Z-R³-NHCO-R⁴- , -R¹-OCO-R²-Z-R³-CONH-R⁴--R¹-OCO-R²-Z-R³-NHCOO-R⁴- , -R¹-OCO-R²-Z-R³-OCONH-R⁴-, -R¹COO-R²-Z-R³-NHCO-R⁴- , -R¹-COO-R²-Z-R³-CONH-R⁴-, -R¹-COO-R²-Z-R³-NHCOO-R⁴-, -R¹-COO-R²-Z-R³-OCO-NH-R⁴-, -R¹-NHCO-R²-Z-R³-OCO-R⁴-, -R¹-NHCO-R²-Z-R³-COO-R⁴-, -R¹-NHCO-R²-Z-R³-NHCOO-R⁴- , -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R² -Z-R³-OCO-R⁴- -R¹-NHCOO-R²-Z-R³-COO-R⁴-, -R¹-NHCOO-R²-Z-R³-NHCO-R⁴- , -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴- , -R¹-OCONH-R²-Z-R³-CONH-R⁴-, -R¹-NHCONH-R²-Z-R³-NHCONH-R⁴-, -R¹-OCO-R²-Z-R³-NHCONH-R⁴-, -R¹-NHCO-R²-Z-R³-NHCONH-R⁴-, -R¹-COO-R²-Z-R³-NHCONH-R⁴-, -R¹-CONH-R²-Z-R³-NHCONH-R⁴-, -R¹-NHCOO-R²-Z-R³-NHCONH-R⁴-, -R¹-OCONH-R²-Z-R³-NHCONH-R⁴-, -R¹-NHCONH-R²-Z-R³-OCO-R⁴-, -R¹-NHCONH-R²-Z-R³-COO-R⁴-, -R¹-NHCONH-R²-Z-R³-NHCO-R⁴-, -R¹-NHCONH-R²-Z-R³-CONH-R⁴-, -R¹-NHCONH-R²-Z-R³-NHCOO-R⁴-, -R¹NHCONH-R²-Z-R³-OCONH-R⁴- , -R¹-OCOO-R²-Z-R³-OCOO-R⁴-, -R¹-OCO-R²-Z-R³-OCONH-R⁴- , -R¹-NHCO-R²-Z-R³-OCOO-R⁴-, -R¹-COO-R²-Z-R³-OCOO-R⁴- , -R¹-CONH-R² -Z-R³-OCOO-R⁴--R¹-NHCOO-R²-Z-R³-OCOO-R⁴- , -R¹-OCONH-R²-Z-R³-OCOO-R⁴-, -R¹-OCOO-R²-Z-R³-OCO-R⁴-, -R¹-OCOO-R²-Z-R³-COO-R⁴-, -R¹-OCOO-R²-Z-R³-NHCO-R⁴-, -R¹-OCOO-R²-Z-R³-CONH-R⁴-, -R¹-OCOO-R²-Z-R³-NHCOO-R⁴- , or -R¹-OCOO-R²-Z-R³-OCONH-R²-; and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-COO-R⁴- -R¹-COO-R²-Z-R³-OCO-R⁴-, -R¹-OCO-R ²-Z-R³-OCO-R⁴- -R¹-COO-R²-Z-R³-COO-R⁴--R¹-NHCO-R²-Z-R³-CONH-R⁴-, -R¹-CONH-R²-Z-R³-NHCO-R⁴-, -R¹-NHCO-R²-Z-R³-NHCO-R⁴- , -R¹-CONH-R²-Z-R³-CONH-R⁴-, -R¹-NHCOO-R²-Z-R³-OCONH-R⁴- , -R¹-OCONH-R²-Z-R³-NHCOO-R⁴-, -R¹-NHCOO-R²-Z-R³-NHCOO-R⁴- , -R¹-OCONH-R²-Z-R³-OCONH-R⁴-, -R¹-OCO-R²-Z-R³-NHCO-R⁴- , -R¹-OCO-R²-Z-R³-CONH-R⁴-, -R¹-OCO-R²-Z-R³-NHCOO-R⁴- , -R¹-OCO-R²-Z-R³-OCONH-R⁴-, -R¹-COO-R²-Z-R³-NHCO-R⁴- -R¹-COO-R²-Z-R³-CONH-R⁴-, -R¹-COO-R²-Z-R³-NHCOO-R⁴-, -R¹-COO-R²-Z-R³-OCONH-R⁴-, -R¹-NHCO-R²-Z-R³-OCO-R⁴-, -R¹-NHCO-R²-Z-R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴- , -R¹-CONH-R²-Z-R³-OCONH-R⁴-, -R¹-NHCOO-R²-Z-R³-OCO-R⁴-, -R¹-NHCO-O-R²-Z-R³-COO-R⁴-, -R¹-NHCOO-R²-Z-R³-NHCO-R⁴- , -R¹-NHCO-O-R²-Z-R³-CONH-R⁴-, -R¹-OCONH-R²-Z-R³-OCO-R⁴-, -R¹-OCO-NH-R²-Z-R³-COO-R⁴-, -R¹OCONH-R²-Z-R³-NHCO-R⁴- , or -R¹-OCONH-R²-Z-R³-CONH-R⁴-, and **R¹, R², R³, R⁴, BP¹, BP², and Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-COO-R⁴- , -R¹-COO-R²-Z-R³-OCO-R⁴-, -R¹-OCO-R²-Z-R³-OCO-R⁴-, -R¹-COO-R²-Z-R³-COO-R⁴-, -R¹-NHCO-R²-Z-R³-CONH-R⁴-, -R¹-CONH-R²-Z-R³-NHCO-R⁴-, -R¹-NHCO-R²-Z-R³-NHCO-R⁴- , -R¹-CONH-R²-Z-R³-CONH-R⁴-, -R¹-NHCOO-R²-Z-R³-OCONH-R⁴- , -R¹-OCONH-R²-Z-R³-NHCOO-R⁴-, -R¹-NHCOO-R²-Z-R³-NHCOO-R⁴-, or -R¹-OCONH-R²-Z-R³-OCONH-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-COO-R⁴- , -R¹-COO-R²-Z-R³-OCO-R⁴-, -R¹-NHCO-R²-Z-R³-CONH-R⁴-, -R¹-CONH-R²-Z-R³-NHCO-R⁴-, -R¹-NHCOO-R²-Z-R³-OCONH-R⁴-, or -R¹-OCONH-R²-Z-R³-NHCOO-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-COO-R⁴-, -R¹-COO-R²-Z-R³-OCO-R⁴-, -R¹-OCO-R²-Z-R³-OCO-R⁴-, or -R¹-COO-R²-Z-R³-COO-R²;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCO-R²-Z-R³-CONH-R⁴-, -R¹-CONH-R²-Z-R³-NHCO-R⁴-, -R¹-NHCO-R²-Z-R³-NHCO-R⁴-, or -R¹-CONH-R²-Z-R³-CONH-R⁴-;
and **R¹, R², R³, R⁴, BP¹, BP², and Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCOO-R²-Z-R³-OCONH-R⁴-, -R¹-OCONH-R²-Z-R³-NHCOO-R⁴-, -R¹-NHCOO-R²-Z-R³-NHCOO-R⁴-, or -R¹-OCONH-R²-Z-R³-OCONH-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-COO-R⁴-, or -R¹-COO-R²-Z-R³-OCO-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCO-R²-Z-R³-CO-NH-R⁴-, or -R¹-CO-NH-R²-Z-R³-NH-CO-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCOO-R²-Z-R³-OCONH-R⁴-, or -R¹-OCONH-R²-Z-R³-NHCOO-R⁴-;
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCO-R²-Z-R³-NHCO-R⁴- , -R¹-OCO-R²-Z-R³-CONH-R⁴-, -R¹-OCO-R²-Z-R³-NHCOO-R⁴-, -R¹-OCO-R²-Z-R³-OCONH-R⁴--R¹-COO-R²-Z-R³-NHCO-R⁴- , -R¹-COO-R²-Z-R³-CONH-R⁴-, -R¹-COO-R²-Z-R³-NHCOO-R⁴- , -R¹-COO-R²-Z-R³-OCONH-R⁴-, -R¹-NHCO-R²-Z-R³-OCO-R⁴-, -R¹-NHCO-R²-Z-R³-COO-R⁴--R¹-NHCO-R²-Z-R³-NHCOO-R⁴-, -R¹-NHCO-R²-Z-R³-OCONH-R⁴-, -R¹-CONH-R²-Z-R³-OCO-R⁴- , -R¹-CONH-R²-Z-R³-COO-R⁴-, -R¹-CONH-R²-Z-R³-NHCOO-R⁴-, -R¹-CONH-R²-Z-R³-OCONH-R⁴-, -R¹-NHCOO-R²-Z-R³-OCO-R⁴-, -R¹-NHCOO-R ²-Z-R³COO-R⁴--R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴--R¹-OCONH-R²-Z-R³-NHCO-R⁴-, or -R¹-OCONH-R²-Z-R³-CONH-R⁴-,
and **R¹, R², R³, R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-OCOR²-Z-R³-NHCO-R⁴-, -R¹-OCO-R²-Z-R³-CONH-R⁴-, -R¹-OCO-R²-Z-R³-NHCOO-R⁴-, -R¹-OCO-R²-Z-R³-O-CONH-R⁴-, -R¹-COO-R²-Z-R³-NHCO-R⁴- , -R¹-COO-R²-Z-R³-CONH-R⁴-, -R¹-COO-R²-Z-R³-NHCOO-R⁴-, or -R¹-COO-R²-Z-R³-OCONH-R⁴- ,
and **R¹, R², R³,** a **R⁴, BP¹, BP²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCO-R²-Z-R³-OCO-R⁴-, -R¹-NHCO-R²-Z-R³-COO-R⁴-, -R¹-NHCO-R²-Z-R³-NHCOO-R⁴-, -R¹-NHCO-R²-Z-R³-OCONH-R⁴-, -R¹-CONH-R²-Z-R³-OCO-R⁴-, -R¹-CONH-R²-Z-R³-COO-R⁴-, -R¹-CONH-R²-Z-R³-NHCOO-R⁴-, or -R¹-CONH-R²-Z-R³-OCONH-R⁴-;
and **R¹, R², R³, R⁴, Bp¹, Bp²,** and **Z** are as defined herein.

Preferably, the present invention relates to crosslinkers of general formula **(I):**

**BP¹-L¹-Z-L²-BP²** **(I)**

wherein **-L¹-Z-L²-** represents
-R¹-NHCOO-R²-Z-R³-OCO-R⁴-, -R¹-NHCOO-R²-Z-R³-COO-R⁴-, -R¹-NHCOO-R²-Z-R³-NHCO-R⁴-, -R¹-NHCOO-R²-Z-R³-CONH-R⁴-, -R¹-OCONH-R²-Z-R³-OCO-R⁴-, -R¹-OCONH-R²-Z-R³-COO-R⁴-, -R¹-OCONH-R²-Z-R³-NHCO-R⁴-, and -R¹-OCONH-R²-Z-R³-CONH-R⁴-,
and **R¹, R², R³, R⁴, Bp¹, Bp²,** or **Z** are as defined herein.

In preferred embodiments, **Z** is an unsubstituted C₁₋₁₀ alkenylene selected from the group consisting of -C₂H₄-, -C₃H₆-, -C₄H₈-, -C₅H₁₀-, -C₆H₁₂-, -C₇H₁₄-, -C₈H₁₆-, -C₉H₁₈- or -C₁₀H₂₀-; more preferably, an unsubstituted C₁₋₆ alkenylene selected from the group consisting of aus -C₂H₄-, -C₃H₆-, -C₄H₈-, -C₅H₁₀-, -C₆H₁₂-. In other words, it is preferred in some embodiments that **Z** is a straight or branched unsubstituted C₁₋₆ alkenylene selected from the group comprising or consisting of -CH₂-, -CH₂CH₂-, -CH₂CH₂CH₂-, CH₂CH₂CH₂CH₂-, -CH₂CH₂CH₂CH₂CH₂-, -CH₂CH₂CH₂CH₂CH₂CH₂-, -CH₂CH₂CH₂CH₂CH₂CH₂CH₂-, -CH(CH₃)-, -C[(CH₃)₂]-, -CH₂-CH(CH₃)-, -CH(C₂H₅), -CH(CH₃)-CH₂-, -CH₂-C[(CH₃)₂]-, -C[(CH₃)₂]-CH₂-, -CH(CH₃)-C₂H₄-_{,} -C₂H₄-CH(CH₃)-, -CH(CH₃)CH₂CH₂, -CH₂-CH(C₂H₅)-, -CH₂-CH(CH₃)-CH₂-, -CH(CH₃)-CH(CH₃)-, -C[(C₂H₅)(CH₃)]-, -CH(C₃H₇)-, -CH(CH₃)-C₃H₆-, -C(CH₃)₂-C₂H₄-, -C(C₂H₅)₂-, -CH₂CH(CH₃)-C₂H₄-, -CH(CH₃)-C(CH₃)₂-, -C₂H₄-C(CH₃)₂-, -C₃H₆-CH(CH₃)₂-, -C₂H₄-CH(CH₃)-C₂H₅-, CH₂-CH(CH₃)-C₃H₇-, -CH(CH₃)-C₄H₉-, -CH(CH₃)-CH₂-C(CH₃)₂-, -C(CH₃)₂-C₃H₇-, -CH(CH₃)-CH(CH₃)-C₂H₅-, -CH₂-CH(CH₃)-CH(CH₃)₂-, -CH₂-C(CH₃)₂-C₂H₅-, -C(CH₃)₂-C(CH₃)₂-, and -CH₂-C(C₂H₅)₂-;. In preferred embodiments, **Z** is an unsubstituted straight C₁₋₆ alkylene selected from the group comprising or consisting of -CH₂-, -CH₂CH₂-, -CH₂CH₂CH₂-, CH₂CH₂CH₂CH₂-, -CH₂CH₂CH₂CH₂CH₂-, -CH₂CH₂CH₂CH₂CH₂CH₂-, and -CH₂CH₂CH₂CH₂CH₂CH₂CH₂-.

In preferred embodiments, **Z** is an unsubstituted branched C₁₋₆ alkylene selected from the group comprising or consisting of -CH(CH₃)-, -C[(CH₃)₂]- -CH₂-CH(CH₃)-, -CH(C₂H₅), -CH(CH₃)-CH₂-, -CH₂-C[(CH₃)₂]-, -C[(CH₃)₂]-CH₂-, -CH(CH₃)-C₂H₄-, -C₂H₄-CH(CH₃)-, -CH(CH₃)CH₂CH₂, -CH₂-CH(C₂H₅)-, -CH₂-CH(CH₃)-CH₂-, -CH(CH₃)-CH(CH₃)-, -C[(C₂H₅)(CH₃)]-, -CH(C₃H₇)-, -CH(CH₃)-C₃H₆-, -C(CH₃)₂-C₂H₄-, -C(C₂H₅)₂-, -CH₂CH(CH₃)-C₂H₄-, -CH(CH₃)-C(CH₃)₂-, -C₂H₄-C(CH₃)₂-, -C₃H₆-CH(CH₃)₂-, -C₂H₄-CH(CH₃)-C₂H₅-, CH₂-CH(CH₃)-C₃H₇-, -CH(CH₃)-C₄H₉-,-CH(CH₃)-CH₂-C(CH₃)₂-, -C(CH₃)₂-C₃H₇-, -CH(CH₃)-CH(CH₃)-C₂H₅-,-CH₂-CH(CH₃)-CH(CH₃)₂-, -CH₂-C(CH₃)₂-C₂H₅-, -C(CH₃)₂-C(CH₃)₂-, and -CH₂-C(C₂H₅)₂-.

In preferred embodiments, **Z** is a substituted C₁₋₆ -alkenylene selected from the group consisting of -CR^{1a}R^{1b}-, -CR^{1a}R^{1b}-CR^{2a}R^{2b}-, -CR^{1a}R^{1b}-CR^{2a}R^{2b}-CR^{3a}R^{3b}-, -CR^{1a}R^{1b}-CR^{2a}R^{2b}-CR^{3a}R^{3b}-CR^{4a}R^{4b}-, -CR^{1a}R^{1b}-CR^{2a}R^{2b}-CR^{3a}R^{3b}-CR^{4a}R^{4b}-CR^{5a}R^{5b}-, and -CR^{1a}R^{1b}-CR^{2a}R^{2b}-CR^{3a}R^{3b}-CR^{4a}R^{4b}-CR^{5a}R^{5b}-CR^{6a}R^{6b}-.

In preferred embodiments, **L²** is selected from the group comprising or consisting of -CR^{1a}R^{1b}-V-CR^{2a}R^{2b}-, -CR^{1a}R^{1b}-V-CR^{2a}R^{2b}-CR^{3a}R^{3b}-, -CR^{1a}R^{1b}-CR^{2a}R^{2b}-V-CR^{3a}R^{3b}-, -CR^{1a}R^{1b}-V-CR^{2a}R^{2b}-CR^{3a}R^{3b}-CR^{4a}R^{4b}-, -CR^{1a}R^{1b}-CR^{2a}R^{2b}-V-CR^{3a}R^{3b}-CR^{4a}R^{4b}-, -CR^{1a}R^{1b}-CR^{2a}R^{2b}-CR^{3a}R^{3b}-V-CR^{4a}R^{4b}-, -CR^{1a}R^{1b}-V-CR^{2a}R^{2b}-CR^{3a}R^{3b}-V-CR^{4a}R^{4b}-, -CR^{1a}R^{1b}-V-CR^{2a}R^{2b}-CR^{3a}R^{3b}-CR^{4a}R^{4b}-CR^{5a}R^{5b}-, -CR^{1a}R^{1b}-CR^{2a}R^{2b}-V-CR^{3a}R^{3b}-CR^{4a}R^{4b}-CR^{5a}R^{5b}- -CR^{1a}R^{1b}-CR^{2a}R^{2b}-CR^{3a}R^{3b}-V-CR^{4a}R^{4b}-CR^{5a}R^{5b}-, -CR^{1a}R^{1b}-CR^{2a}R^{2b}-CR^{3a}R^{3b}-CR^{4a}R^{4b}-V-CR^{5a}R^{5b}-, -CR^{1a}R^{1b}-V-CR^{2a}R^{2b}-CR^{3a}R^{3b}-V-CR^{4a}R^{4b}-CR^{5a}R^{5b}- -CR^{1a}R^{1b}-CR^{2a}R^{2b}-V-CR^{3a}R^{3b}-CR^{4a}R^{4b}-V-CR^{5a}R^{5b}-, -CR^{1a}R^{1b}-V-CR^{2a}R^{2b}-CR^{3a}R^{3b}-CR^{4a}R⁴-V-CR^{5a}R^{5b}-, -CR^{1a}R^{1b}-V-CR^{2a}R^{2b}-CR^{3a}R^{3b}-CR^{4a}R^{4b}-CR^{5a}R^{5b}-CR^{6a}R^{6b}-, -CR^{1a}R^{1b}-CR^{2a}R^{2b}-V-CR^{3a}R^{3b}-CR^{4a}R^{4b}-CR^{5a}R^{5b}-CR^{6a}R^{6b}-, -CR^{1a}R^{1b}-CR^{2a}R^{2b}-CR^{3a}R^{3b}-V-CR^{4a}R^{4b}-CR^{5a}R^{5b}-CR^{6a}R^{6b}-, -CR^{1a}R^{1b}-CR^{2a}R^{2b}-CR^{3a}R^{3b}-CR^{4a}R^{4b}-V-CR^{5a}R^{5b}-CR^{6a}R^{6b}-, -CR^{1a}R^{1b}-CR^{2a}R^{2b}-CR^{3a}R^{3b}-CR^{4a}R^{4b}-CR^{5a}R⁵-V-CR^{6a}R^{6b}-, -CR^{1a}R^{1b}-V-CR^{2a}R^{2b}-CR^{3a}R^{3b}-V-CR^{4a}R^{4b}-CR^{5a}R^{5b}-CR^{6a}R^{6b}-, -CR^{1a}R^{1b}-V-CR^{2a}R^{2b}-CR^{3a}R^{3b}-CR^{4a}R^{4b}-V-CR^{5a}R^{5b}-CR^{6a}R^{6b}-, -CR^{1a}R^{1b}-V-CR^{2a}R^{2b}-CR^{3a}R^{3b}-CR^{4a}R^{4b}-CR^{5a}R^{5b}-V-CR^{6a}R^{6b}-, -CR^{1a}R^{1b}-CR^{2a}R^{2b}-V-CR^{3a}R^{3b}-CR^{4a}R^{4b}-V-CR^{5a}R^{5b}-CR^{6a}R^{6b}-, -CR^{1a}R^{1b}-CR^{2a}R^{2b}-V-CR^{3a}R^{3b}-CR^{4a}R^{4b}-CR^{5a}R^{5b}-V-CR^{6a}R^{6b}-, -CR^{1a}R^{1b}-CR^{2a}R^{2b}-CR^{3a}R^{3b}-V-CR^{4a}R^{4b}-CR^{5a}R^{5b}-V-CR^{6a}R^{6b}-, and -CR^{1a}R^{1b}-V-CR^{2a}R^{2b}-CR^{3a}R^{3b}-V-CR^{4a}R^{4b}-CR^{5a}R^{5b}-V-CR^{6a}R^{6b}-, wherein **V** is -O-, -NH-, -NRⁿ-, -CO-, -O-CO-, -NH-CO-, -CO-O-, -CO-NH-, -NH-CO-O-, -O-CO-NH-, or -NH-CO-NH-.

In further preferred embodiments, it is preferred that **V** is -O- and **Z** is selected from the group comprising or consisting of -CR^{1a}R^{1b}-O-CR^{2a}R^{2b}-, -CR^{1a}R^{1b}-O-CR^{2a}R^{2b}-CR^{3a}R^{3b}-, -CR^{1a}R^{1b}-CR^{2a}R^{2b}-O-CR^{3a}R^{3b}-, -CR^{1a}R^{1b}-V-CR^{2a}R^{2b}-CR^{3a}R^{3b}-CR^{4a}R^{4b}-, -CR^{1a}R^{1b}-CR^{2a}R^{2b}-O-CR^{3a}R^{3b}-CR^{4a}R^{4b}-, -CR^{1a}R^{1b}-CR^{2a}R^{2b}-CR^{3a}R^{3b}-O-CR^{4a}R^{4b}-, -CR^{1a}R^{1b}-O-CR^{2a}R^{2b}-CR^{3a}R^{3b}-O-CR^{4a}R^{4b}-, -CR^{1a}R^{1b}-V-CR^{2a}R^{2b}-CR^{3a}R^{3b}-CR^{4a}R^{4b}-CR^{5a}R^{5b}-, -CR^{1a}R^{1b}-CR^{2a}R^{2b}-V-CR^{3a}R^{3b}-CR^{4a}R^{4b}-CR^{5a}R^{5b}-, -CR^{1a}R^{1b}-CR^{2a}R^{2b}-CR^{3a}R^{3b}-O-CR^{4a}R^{4b}-CR^{5a}R^{5b}-, -CR^{1a}R^{1b}-CR^{2a}R^{2b}-CR^{3a}R^{3b}-CR^{4a}R^{4b}-O-CR^{5a}R^{5b}-, -CR^{1a}R^{1b}-O-CR^{2a}R^{2b}-CR^{3a}R^{3b}-O-CR^{4a}R^{4b}-CR^{5a}R^{5b}-, -CR^{1a}R^{1b}-CR^{2a}R^{2b}-O-CR^{3a}R^{3b}-CR^{4a}R^{4b}-O-CR^{5a}R^{5b}-, -CR^{1a}R^{1b}-O-CR^{2a}R^{2b}-CR^{3a}R^{3b}-CR^{4a}R⁴-O-CR^{5a}R^{5b}-, -CR^{1a}R^{1b}-O-CR^{2a}R^{2b}-CR^{3a}R^{3b}-CR^{4a}R^{4b}-CR^{5a}R^{5b}-CR^{6a}R^{6b}-, -CR^{1a}R^{1b}-CR^{2a}R^{2b}-V-CR^{3a}R^{3b}-CR^{4a}R^{4b}-CR^{5a}R^{5b}-CR^{6a}R^{6b}-, -CR^{1a}R^{1b}-CR^{2a}R^{2b}-CR^{3a}R^{3b}-O-CR^{4a}R^{4b}-CR^{5a}R^{5b}-CR^{6a}R^{6b}-, -CR^{1a}R^{1b}-CR^{2a}R^{2b}-CR^{3a}R^{3b}-CR^{4a}R^{4b}-O-CR^{5a}R^{5b}-CR^{6a}R^{6b}-, -CR^{1a}R^{1b}-CR^{2a}R^{2b}-CR^{3a}R^{3b}-CR^{4a}R^{4b}-CR^{5a}R⁵-O-CR^{6a}R^{6b}-, -CR^{1a}R^{1b}-O-CR^{2a}R^{2b}-CR^{3a}R^{3b}-O-CR^{4a}R^{4b}-CR^{5a}R^{5b}-CR^{6a}R^{6b}-, -CR^{1a}R^{1b}-O-CR^{2a}R^{2b}-CR^{3a}R^{3b}-CR^{4a}R^{4b}-O-CR^{5a}R^{5b}-CR^{6a}R^{6b}-, -CR^{1a}R^{1b}-O-CR^{2a}R^{2b}-CR^{3a}R^{3b}-CR^{4a}R^{4b}-CR^{5a}p^{5b}-O-CR^{6a}R^{6b}-, -CR^{1a}R^{1b}-CR^{2a}R^{2b}-O-CR^{3a}R^{3b}-CR^{4a}R^{4b}-O-CR^{5a}R^{5b}-CR^{6a}R^{6b}-, -CR^{1a}R^{1b}-CR^{2a}R^{2b}-O-CR^{3a}R^{3b}-CR^{4a}R^{4b}-CR^{5a}R^{5b}-O-CR^{6a}R^{6b}-, -CR^{1a}R^{1b}-CR^{2a}R^{2b}-CR^{3a}R^{3b}-O-CR^{4a}R^{4b}-CR^{5a}R^{5b}-O-CR^{6a}R^{6b}-, and -CR^{1a}R^{1b}-O-CR^{2a}R^{2b}-CR^{3a}R^{3b}-O-CR^{4a}R^{4b}-CR^{5a}R^{5b}-O-CR^{6a}R^{6b}-, in some of these embodiments **Z** is preferably selected from the group comprising or consisting of -CH₂OCH₂-, -CH₂OCH₂CH₂-, -CH₂CH₂OCH₂-, -CH₂CH₂OCH₂CH₂-, and -CH₂CH₂OCH₂CH₂OCH₂CH₂-.

In preferred embodiments, **V** is -NR^{N}- and **Z** is selected from the group comprising or consisting of -CR^{1a}R^{1b}-NR⁷-CR^{2a}R^{2b}-, -CR^{1a}R^{1b}-NR⁷-CR^{2a}R^{2b}-CR^{3a}R^{3b}-, -CR^{1a}R^{1b}-CR^{2a}R^{2b}-NR⁷-CR^{3a}R^{3b}-, -CR^{1a}R^{1b}-NR⁷-CR^{2a}R^{2b}-CR^{3a}R^{3b}-CR^{4a}R^{4b}-, -CR^{1a}R^{1b}-CR^{2a}R^{2b}-NR⁷-CR^{3a}R^{3b}-CR^{4a}R^{4b}-, -CR^{1a}R^{1b}-CR^{2a}R^{2b}-CR^{3a}R^{3b}-NR⁷-CR^{4a}R^{4b}-, -CR^{1a}R^{1b}-NR⁷-CR^{2a}R^{2b}-CR^{3a}R^{3b}- NR⁷-CR^{4a}R^{4b}-, -CR^{1a}R^{1b}-NR⁷-CR^{2a}R^{2b}-CR^{3a}R^{3b}-CR^{4a}R^{4b}-CR^{5a}R^{5b}-, -CR^{1a}R^{1b}-CR^{2a}R^{2b}-NR⁷-CR^{3a}R^{3b}-CR^{4a}R^{4b}-CR^{5a}R^{5b}-, -CR^{1a}R^{1b}-CR^{2a}R^{2b}-CR^{3a}R^{3b}-NR⁷-CR^{4a}R^{4b}-CR^{5a}R^{5b}-, -CR^{1a}R^{1b}-CR^{2a}R^{2b}-CR^{3a}R^{3b}-CR^{4a}R^{4b}-NR⁷-CR^{5a}R^{5b}-, -CR^{1a}R^{1b}-O-CR^{2a}R^{2b}-CR^{3a}R^{3b}-NR⁷-CR^{4a}R^{4b}-CR^{5a}R^{5b}-, -CR^{1a}R^{1b}-CR^{2a}R^{2b}-NR⁷-CR^{3a}R^{3b}-CR^{4a}R^{4b}-NR⁷-CR^{5a}R^{5b}-, -CR^{1a}R^{1b}-NR⁷-CR^{2a}R^{2b}-CR^{3a}R^{3b}-CR^{4a}R⁴-NR⁷-CR^{5a}R^{5b}-, -CR^{1a}R^{1b}-NR⁷-CR^{2a}R^{2b}-CR^{3a}R^{3b}-CR^{4a}R^{4b}-CR^{5a}R^{5b}-CR^{6a}R^{6b}-, -CR^{1a}R^{1b}-CR^{2a}R^{2b}-NR⁷-CR^{3a}R^{3b}-CR^{4a}R^{4b}-CR^{5a}R^{5b}-CR^{6a}R^{6b}-, -CR^{1a}R^{1b}-CR^{2a}R^{2b}-CR^{3a}R^{3b}-NR⁷-CR^{4a}R^{4b}-CR^{5a}R^{5b}-CR^{6a}R^{6b}-, -CR^{1a}R^{1b}-CR^{2a}R^{2b}-CR^{3a}R^{3b}-CR^{4a}R^{4b}-NR⁷-CR^{5a}R^{5b}-CR^{6a}R^{6b}-, -CR^{1a}R^{1b}-CR^{2a}R^{2b}-CR^{3a}R^{3b}-CR^{4a}R^{4b}-CR^{5a}R⁵-NR⁷-CR^{6a}R^{6b}-, -CR^{1a}R^{1b}-NR⁷-CR^{2a}R^{2b}-CR^{3a}R^{3b}-NR⁷-CR^{4a}R^{4b}-CR^{5a}R^{5b}-CR^{6a}R^{6b}-, -CR^{1a}R^{1b}-NR⁷-CR^{2a}R^{2b}-CR^{3a}R^{3b}-CR^{4a}R^{4b}-NR⁷-CR^{5a}R^{5b}-CR^{6a}R^{6b}-, -CR^{1a}R^{1b}-NR⁷-CR^{2a}R^{2b}-CR^{3a}R^{3b}-CR^{4a}R^{4b}-CR^{5a}R^{5b}-NR⁷-CR^{6a}R^{6b}-, -CR^{1a}R^{1b}-CR^{2a}R^{2b}-NR⁷-CR^{3a}R^{3b}-CR^{4a}R^{4b}-NR⁷-CR^{5a}R^{5b}-CR^{6a}R^{6b}-, -CR^{1a}R^{1b}-CR^{2a}R^{2b}-NR⁷-CR^{3a}R^{3b}-CR^{4a}R^{4b}-CR^{5a}R^{5b}-NR⁷-CR^{6a}R^{6b}-, -CR^{1a}R^{1b}-CR^{2a}R^{2b}-CR^{3a}R^{3b}-NR⁷-CR^{4a}R^{4b}-CR^{5a}R^{5b}-NR⁷-CR^{6a}R^{6b}-, and -CR^{1a}R^{1b}-NR⁷-CR^{2a}R^{2b}-CR^{3a}R^{3b}-NR⁷-CR^{4a}R^{4b}-CR^{5a}R^{5b}-NR⁷-CR^{6a}R^{6b}-; and
**R^{N}** is -CH₃, -C₂H₅, -C₃H₇, -CH(CH₃)₂, -cyclo-C₃H₅, -C₄H₉, -CH₂-CH(CH₃)₂, -CH(CH₃)-C₂H₅, -C(CH₃)₃, -cyclo-C₄H₇, -CH₂-cyclo-C₃H₅, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₅H₉, -CH₂-cyclo-C₄H₇, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -cyclo-C₆H₁₁, -CH₂-cyclo-C₅H₉, -C₇H₁₅, -cyclo-C₇H₁₃, -CH₂-cyclo-C₆H₁₁, -C₈H₁₇, or -cyclo-C₈H₁₅. In some of these embodiments, **L²** is preferably selected from the group comprising or consisting of -CH₂CH₂NHCH₂-, -CH₂CH₂N(CH₃)CH₂-, and -CH₂CH₂N(CH₃)CH₂CH₂-.

In some preferred embodiments, **V** is -O-, -NH-, -NR^{N}-, -CO-, or -S(O)₂-, and **Z** is -CH₂SO₂CH₂-, and in some embodiments it is further preferred that V is -OCO- or -COO-, and **Z** is -CH₂CO₂CH₂- or -CH₂CO₂CH₂CH₂-.

In preferred embodiments, **R^{1b} - R^{6b}** are -H; wherein in some still further preferred embodiments, it is preferred that only one of **R^{1a}** - **R^{6a}** and **R^{1b}** - **R^{6b}** is not -H.

Examples of some of these preferred crosslinkers are: 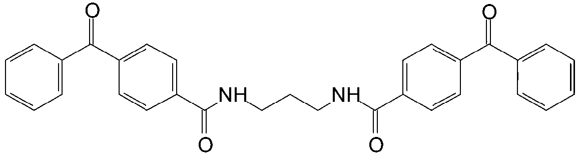 and

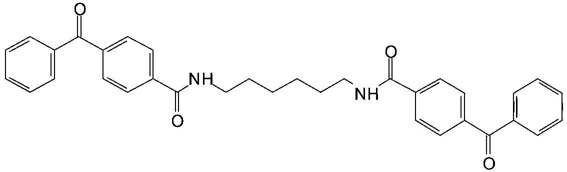

Some embodiments relate to crosslinkers of the general formula (**I**), wherein **Z** is 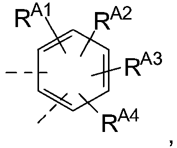 and wherein **R**^{A1} - **R^{A4}** are as defined herein, wherein in some of these embodiments it is preferred that **L¹-Z-L²** is -Ph-, -CH₂-Ph-, or -CH₂-Ph-CH₂.

Some embodiments relate to crosslinkers of the general formula (**I**), wherein **Z** is 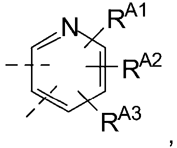 and wherein **R**^{A1} - **R^{A4}** are as defined herein, wherein in some of these embodiments it is preferred that **L¹-Z-L²** -Py-, -CH₂-Py-, or -CH₂-Py-CH₂

Some embodiments relate to crosslinkers of the general formula (I), wherein **Z** is, 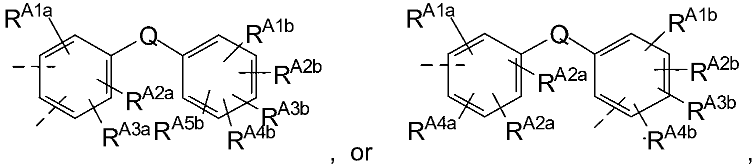 wherein **Q** represents -CH₂- or -CO-; and wherein **R^{A1a}** - **R^{A4a}, R^{A1b}** - **R^{A4b}** and **R^{B1a}** - are as defined herein.

Some embodiments relate to crosslinkers of the general formula (**I**), wherein **Z** is 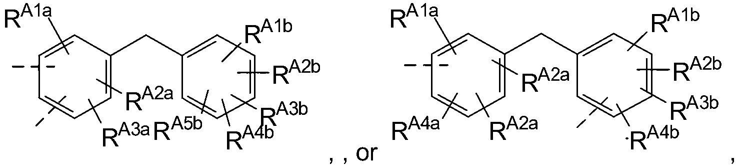 and wherein **R^{A1a}** - **R^{A4a}** , **R^{A1b}** - **R^{A4b}** and **R^{B1a}** - **R^{B4a}** are as defined herein.

Preferably, **R^{A1a}** - **R^{A4a}, R^{A1b}** - **R^{A4b}** and **R^{B1a}** - **R^{B4a}** are independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF_{3,} -C₂H₅, -C₃H₇, -NH₂, -NH(CH)₃, -N(CH₃)₂, or -SOCH₃.

Some further preferred embodiments relate to crosslinkers of the general formula (**I**), wherein **Z** is 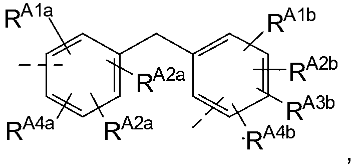 and wherein **R^{A1a}** - **R^{A4a}, R^{A1b}** - **R^{A4b},** and **R^{B1a}** - **R^{B4a}** are as defined herein.

Preferably, **R^{A1a}** - **R^{A4a}, R^{A1b}** - **R^{A4b},** and **R^{B1a}** - **R^{B4a}** are independently of each other -H, -COOH, -CH₃ , -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Some embodiments relate to crosslinkers of the general formula (**I**), wherein **Z** is 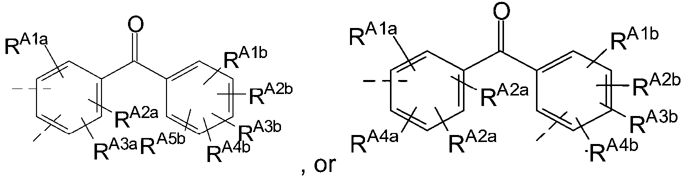 and wherein **R^{A1a}** - **R^{A4a}, R^{A1b}** - **R^{A4b},** and **R^{B1a}** - **R^{B4a}** are as defined herein.

Preferably, **R^{A1a}** - **R^{A4a}, R^{A1b}** - **R^{A4b},** and **R^{B1a}** - **R^{B4a}** are independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Some further preferred embodiments relate to crosslinkers of the general formula (**I**), wherein **Z** is 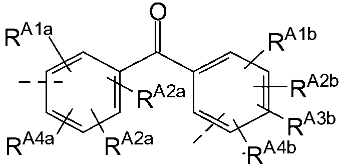 and wherein **R^{A1a}** - **R^{A4a}, R^{A1b}** - **R^{A4b}** and **R^{B1a}** - **R^{B4a}** are as defined herein.

Preferably, **R^{A1a}** - **R^{A4a}, R^{A1b}** - **R^{A4b}** and **R^{B1a}** - **R^{B4a}** are independently of each other -H, -COOH, -CH₃, -F, -Cl, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃.

Examples of some of these preferred crosslinkers are: 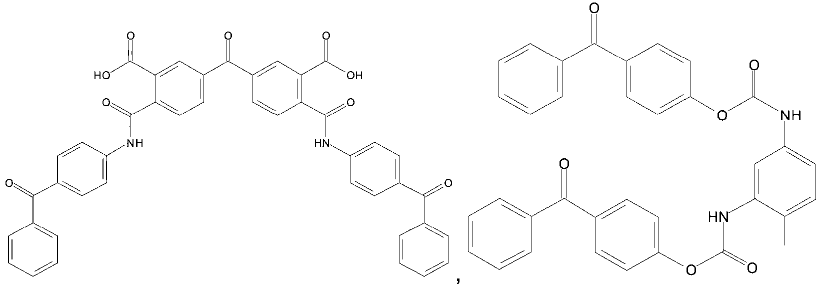 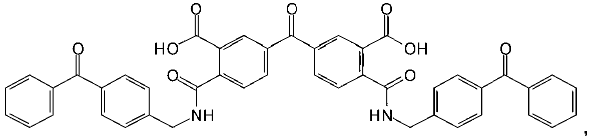 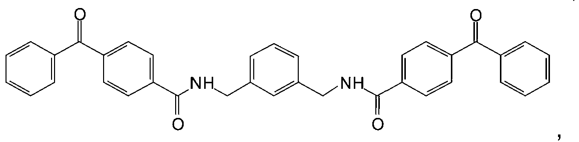 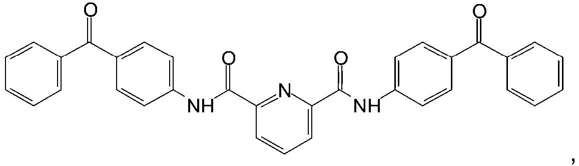 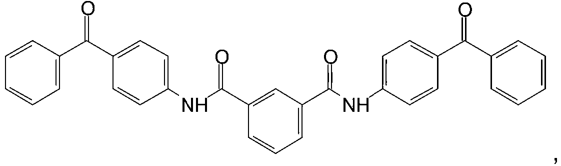 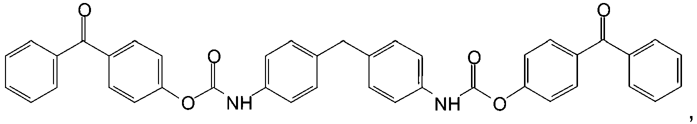 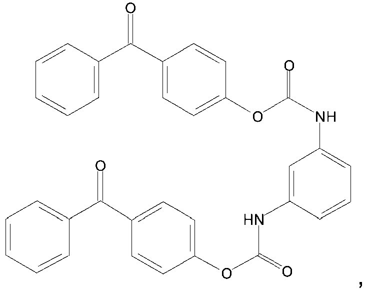 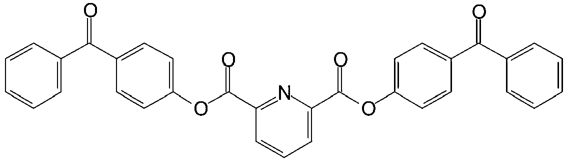 and

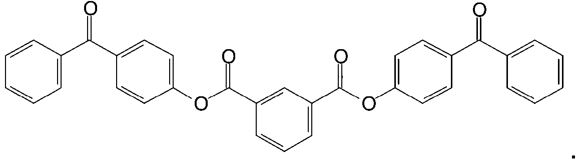

In some embodiments, the crosslinker is preferably selected from one of the crosslinkers of the group comprising or consisting of: 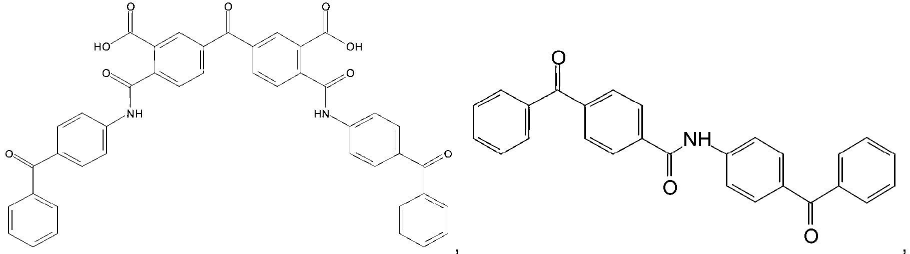 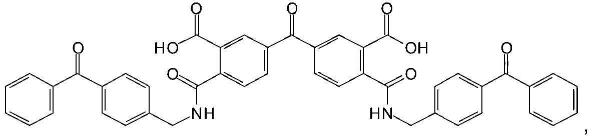 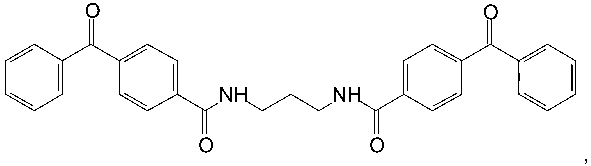 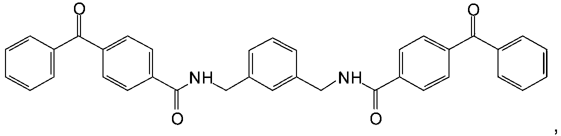 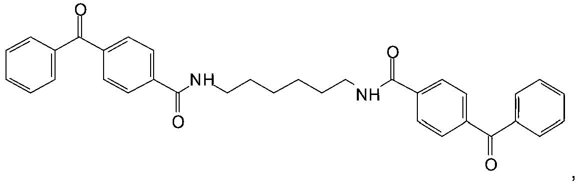 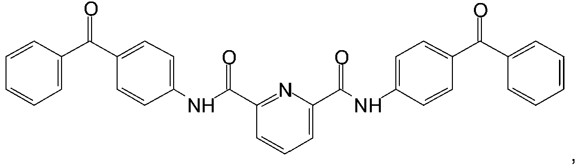 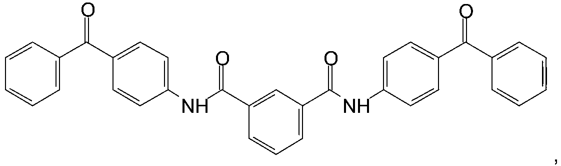 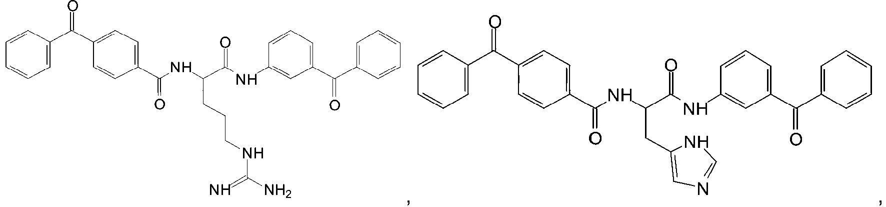 and

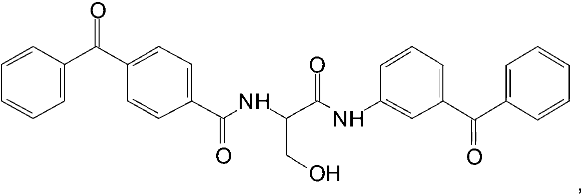

In some embodiments, the crosslinker is preferably selected from one of the crosslinkers of the group comprising or consisting of: 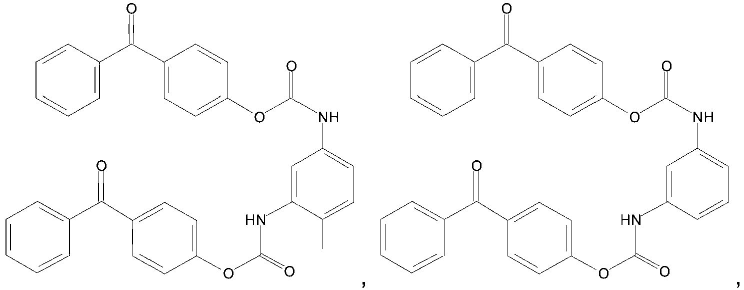 and

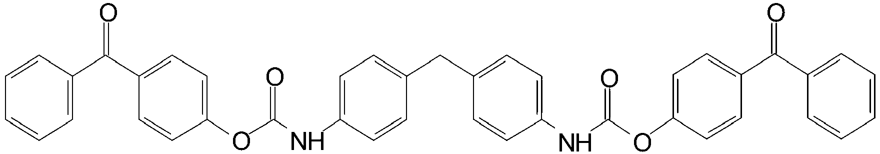

In some embodiments, the crosslinker is preferably selected from one of the crosslinkers of the group comprising or consisting of: 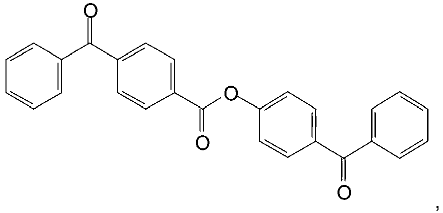 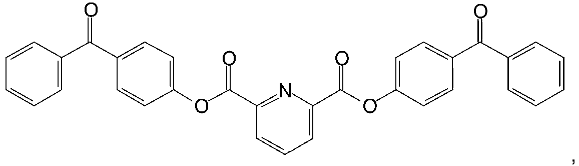 and

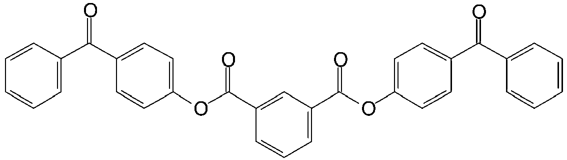

Particularly preferred crosslinkers include, but are not limited to, carbonyl bis-((4-benzoylphenyl)carbamoyl)-benzophenone, benzophenone dicarboxyl di-N-(4-(benzoylphenyl)methylaminocarboxylate), di-benzoylbenzoic acid-1,3-diamidopropane, 4,4'-{N-[N'-(4-benzoylphenyl)-hydroxycarbonyl]} diaminomethylene bisphenol, 1,3-{N-[N'-(4-benzoylphenyl)-hydroxycarbonyl]}-diaminobenzene, 1,3-{N-[N'-(4-benzoylphenyl)-carbonyl]}-diaminoxylylene, 1,6--{N-[N'-(4-benzoylphenyl)-carbonyl]}-diaminohexane, 4-benzoylbenzoic acid -4-benzylphenyl ester, 4-benzoylbenzoic acid-4-benzoylphenyl amide, pyridine-2,6-dicarboxylicacid-bis-(4-benzoylphenyl)amide, pyridine-2,6-dicarboxylicacid-bis-(4-benzoylphenyl)ester, isophtalic acid-bis-(4-benzoyl-phenyl)amide, isophtalic acid-bis-(4-benzoylphenyl)ester, benzoylphenyl-Arg-(4-benzoylphenyl)amide, benzoylphenyl-His-(4-benzoylphenyl)amide, benzoylphenyl-Ser-(4-benzoylphenyl)amide 2,4-{N-[N'-(4-hydroxymethylbenzoylphenyl)-carbonyl]}-diaminotoluene, mixtures thereof and derivatives.

Di-carboxyl-di-N-(4-benzoylphenyl)aminocarboxylate (BCBAC), benzophenone dicarboxyl di-N-(4-(benzoylphenyl) methyl aminocarboxylate) (BCBMC) and dibenzoyl benzoic acid-1,3-diamidopropane (BPAP) are particularly preferred crosslinkers.

The synthesis of the crosslinkers according to the invention is preferably carried out in a simple one-pot procedure by selection of suitable solvents. In the simplest case, the selection of the solvent is based on the solubility of the reactants and the insolubility of the resulting product in a solvent for isolation and purification.

To produce a polymer network, the at least one vinylpyrrolidine copolymer is dissolved with a crosslinker in a suitable solvent. After activation of the component mixture of vinylpyrrolidine copolymer and crosslinker by means of heat and/or light of suitable wavelength ranges in the UV and/or VIS range, reaction occurs between the components after the formation of radicals, producing a crosslinked vinylpyrrolidine copolymer whose degree of crosslinking depends on the proportion of compounds capable of forming radicals and the necessary activation energy in the mixture of vinylpyrrolidine copolymer and crosslinker. The crosslinker serves to stabilize the radicals of the vinylpyrrolidine copolymer formed by photoactivation, which, however, simultaneously leads to crosslinking of the linear vinylpyrrolidine copolymers with each other and crosslinking of vinylpyrrolidine copolymer with the crosslinker.

As a result, there is a noticeable increase in mechanical, physical and chemical properties. The resulting vinylpyrrolidine copolymer network has improved flexibility, lower frictional resistance, higher stability and improved moisture retention, which in turn means an increase in hydrophilicity as well as other properties.

The light-activated reaction of the crosslinker with the vinylpyrrolidine copolymer can, depending on the procedure and wavelength range, also lead to reaction with materials present that are not part of the mixture, such as a surface to which the vinylpyrrolidine copolymer and the crosslinker are applied, thereby increasing adhesion to that surface by forming covalent bonds whereby stronger adhesion also enables longer usage time, including repeatable application. This reaction with the substrate is facilitated by conditioning the material that comes into contact with the component mixture. This is done, for example, by pre-wetting with a suitable solvent, but can also be achieved by irradiation with light, heat, plasma process, etc.

### Description of the figures:

- **Figure 1:**: shows a diagram of the measurement for the determination of the coefficient of friction (CoF) of nylon substrate coated with Kollidon 30K and Kollidon 90F in the mixing ratio 2:1 (w/w) and uncoated nylon substrate as reference over 25 cycles.
- **Figure 2:**: shows a graph of the measurement to determine the coefficient of friction (CoF) of nylon substrate coated with Kollidon 90F and uncoated nylon substrate as a reference over 25 cycles.
- **Figure 3:**: shows a diagram of the measurement to determine the coefficient of friction (CoF) of polypropylene (PP) substrate coated with NVP-co-MBBM (single coating) and uncoated polypropylene substrate as a reference over 25 cycles.
- **Figure 4:**: shows a diagram of the measurement for determining the coefficient of friction (CoF) of nylon substrate coated with NVP-co-MBBM (single coating) and uncoated nylon substrate as a reference over 25 cycles.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Example 1

### Synthesis of N-[(4-benzoylbenzyl)] maleic acid monoamide (MBBM)

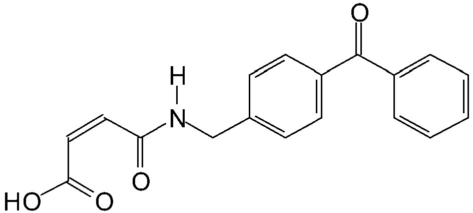

In a 250 mL single-neck flask, 1.521 g (0.0155 mol) of maleic anhydride and 3.062 g (0.0155 mol, or 1.0 moleq) of 4-benzoylbenzylamine hydrochloride were weighed and suspended or partially dissolved in 75 mL of ethyl acetate at room temperature (rt). Then, the solution was gassed with inert gas (argon) for 2 min and stirred further at rt for 2 h. Subsequently, the resulting solid was separated from the solvent by vacuum filtration. The resulting white to pale yellow dry residue (i.e. solid) was then washed 3 times in 100 mL n-heptane. Vacuum filtration was used to separate the solid from the solvent or precipitant. The resulting white solid was then dried overnight (approximately 18 hours).

### Example 2

### Synthesis of PEG maleic acid monoester

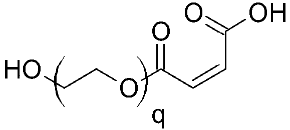

In a 250 mL single-neck flask, 1.525 g (set value: 1.520 g, corresponds to: 0.0155 mol) of maleic anhydride and 31.000 g (set value: 31.000 g, corresponds to: 0.0155 mol, or 1.0 moleq) of PEG 2000 (q ≈ 40) were weighed and dissolved in 150 mL of THF at rt. After complete dissolution of the reactants, 0.213 g (set value: 0.200 g, corresponds to: 1.0514 mmol, or 0.068 moleq) para-toluenesulfonic acid monohydrate was added to the solution. The solution was then gassed with inert gas (argon) for 2 min and refluxed to 60° C for 5 hours. The solution was then added to 250 mL of n-heptane, resulting in precipitation of the reaction product. Vacuum filtration was used to separate the solid obtained from the solvent/precipitate mixture. The obtained white solid was then dried by rotary vane pump vacuum overnight.

### Example 3

### Synthesis of O-PEG-N-[(4-benzoylbenzyl)]maleic acid monoamide

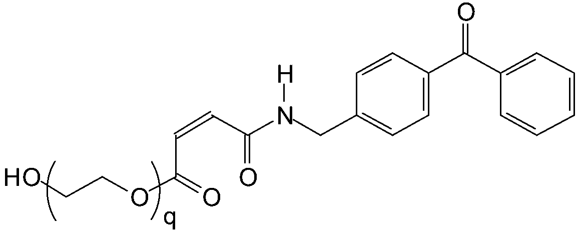

2.500 g of the solid obtained from Example 1 (0.0085 mol) was dissolved in 50 mL of THF to which 1.450 mL of diisopropylcarbodiimide (0.0094 mol, or 1.1 moleq) was added. Subsequently, 17.000 g PEG 2000 (q ≈ 40) (0.0085 mol, or 1 moleq) was added to the solution at rt. After 5 hours of reaction time, the reaction was terminated. The solution was added to 250 mL of n-heptane, resulting in precipitation of the reaction product. Vacuum filtration was used to separate the solid obtained from the solvent/precipitate mixture. The obtained white solid was then dried by rotary vane pump vacuum overnight.

### Example 4

### Preparation of poly(VP-co-MBBM) from NVP and N-[4-(benzoylbenzyl)]maleic acid monoamide.

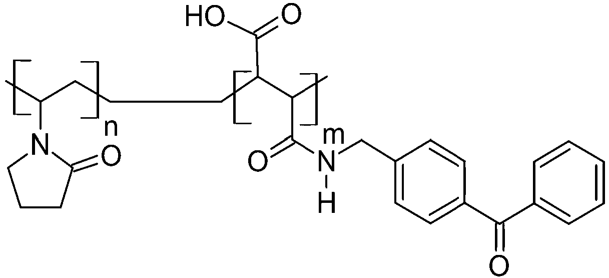

In a 250 mL three-neck flask equipped with an internal thermometer, 0.315 g of the solid obtained from Synthesis 1 (set value: 0.314 g, or 0.001065 mol, (0.75 mol%)) was weighed. Under argon introduction, 15.0 mL of N-vinyl-2-pyrrolidone (15.6 g, or 0.1420 mol) and 10 mL of degassed dimethylformamide were then added. The flask was then sonicated for 15 min., gassed again 150 mg (0.926 mmol) of azobis(isobutyronitrile) (AIBN) was weighed and added to the monomer solution. The temperature of the solution was slowly increased to about 60° C. After a total of 75 min reaction time, the solution had become so viscous that the magnetic stirring core got stuck. The viscous polymer dimethylformamide solution was then mixed with 40 mL deionized water and heated to about 90° C. A milky turbid solution was obtained, which after cooling to rt was transferred to dialysis tubes (MWCO: 6 to 8 kD) and dialyzed overnight against approx. 50 L of deionized water. After dialysisthe polymer solution was frozen and finally freeze-dried.

### Example 5

### Production of coating solutions

1.750 g PVP-co-MBBM is dissolved in isopropanol/water (70:30 v/v) to give a 7% solution. After a 10 min treatment in the ultrasonic bath, the solution is stored under nitrogen atmosphere until use.

Coating solution A) was prepared with PVP-co-MBBM containing 5mol% maleic acid.

Coating solution B) was prepared with PVP-co-MBBM containing 1mol% maleic acid.

Coating solution C) was prepared using PVP-co-MBBM containing 0.75mol% maleic acid.

Coating solution D) was prepared using PVP-co-MBBM containing 0.5mol% maleic acid.

### Example 6

### Coating of nylon

Nylon samples were coated with coating solutions A to D from Example 5 using the following procedure.

A catheter (nylon) is fully immersed in the solution at a speed of 20mm/s, then left in the solution for 10s and then withdrawn again at a speed of 5mm/s. The wetted catheter is then exposed to UV radiation for 3 min in the UV chamber and then dried at 70° C for 15min. The coating procecedure can be repeated as often as required.

### Coating method:

1) Immersion speed: 20mm/s
2) Incubation time: 10 s
3) Emergence speed: 5mm/s
4) Curing in UV oven: 3.0 min
5) Drying: 15 min at 70°C

To produce a double coating, steps 1-5 of the coating procedure were repeated.

Lubricity was measured by determining the coefficients of friction (CoF) using Harland FTS 6000 (Harland Medical System, U.S.A.) according to the program "5 cm 25 c 5 N". In each case, 25 measurements were taken at the 0° angle and 25 measurements were taken at the 90° angle. The measurement distance was 5 cm and the measurement force was 5 N. Uncoated nylon was used as a reference.

### Measurement of lubricity

with Harland FTS 6000
Program " 5cm 25c 5N"
25 measurements each 0° angle
and 25 measurements rotated by 90

| | |
|---|---|
| Measuring distance: | 5 cm |
| Measuring force: | 5 N |
| Repetition: | 25 x |

1 uncoated sample per material

The results for a single coating and a double coating with coating solution A (5 mol% maleic acid content) are shown in Table 1 below:

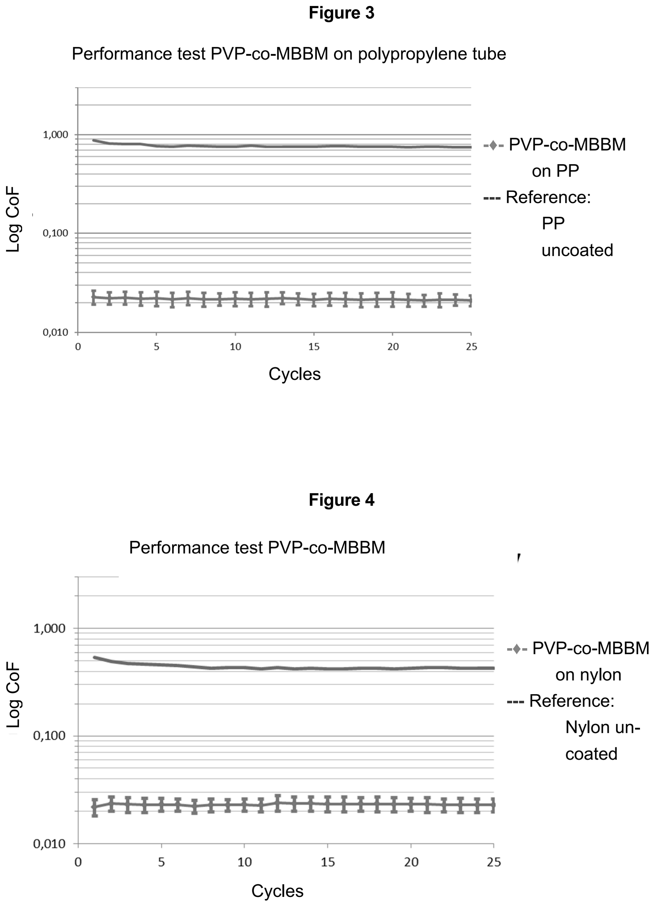

**Table 1: Results of the coefficient of friction (CoF) determination for coating solution A for a single coating and a double coating (Fig. 4).**

| | **Single coating** | | **Double coating** | |
|---|---|---|---|---|
| **Measurement** | **Ø-CoF coated** | **Ø-CoF uncoated** | **Ø-CoF coated** | **Ø-CoF uncoated** |
| 1 | 0.192 | 0.542 | 0.171 | 0.496 |
| 2 | 0.158 | 0.491 | 0.178 | 0.483 |
| 3 | 0.149 | 0.470 | 0.172 | 0.471 |
| 4 | 0.147 | 0.464 | 0.174 | 0.469 |
| 5 | 0.145 | 0.456 | 0.172 | 0.470 |
| 6 | 0.143 | 0.451 | 0.169 | 0.467 |
| 7 | 0.142 | 0.438 | 0.169 | 0.460 |
| 8 | 0.140 | 0.427 | 0.172 | 0.462 |
| 9 | 0.137 | 0.431 | 0.169 | 0.467 |
| 10 | 0.138 | 0.431 | 0.168 | 0.461 |
| 11 | 0.134 | 0.423 | 0.169 | 0.464 |
| 12 | 0.133 | 0.432 | 0.168 | 0.462 |
| 13 | 0.134 | 0.424 | 0.166 | 0.457 |
| 14 | 0.135 | 0.425 | 0.170 | 0.462 |
| 15 | 0.135 | 0.422 | 0.170 | 0.467 |
| 16 | 0.138 | 0.421 | 0.173 | 0.462 |
| 17 | 0.135 | 0.424 | 0.172 | 0.462 |
| 18 | 0.140 | 0.429 | 0.174 | 0.461 |
| 19 | 0.140 | 0.422 | 0.174 | 0.460 |
| 20 | 0.140 | 0.428 | 0.175 | 0.460 |
| 21 | 0.142 | 0.431 | 0.176 | 0.462 |
| 22 | 0.142 | 0.431 | 0.176 | 0.462 |
| 23 | 0.143 | 0.426 | 0.180 | 0.457 |
| 24 | 0.145 | 0.429 | 0.179 | 0.466 |
| 25 | 0.144 | 0.429 | 0.179 | 0.465 |
| Ø | **0.137** | **0.440** | **0.173** | **0.465** |

The results for a single coating with coating solution B (1 mol% maleic acid content) are shown in Table 2 below:

**Table 2: Results of the coefficient of friction (CoF) determination for coating solution B for a single coating.**

| **Measurement** | **Ø-CoF coated** | **Ø-CoF uncoated** |
|---|---|---|
| 1 | 0.024 | 0.542 |
| 2 | 0.024 | 0.491 |
| 3 | 0.023 | 0.470 |
| 4 | 0.023 | 0.464 |
| 5 | 0.023 | 0.456 |
| 6 | 0.023 | 0.451 |
| 7 | 0.023 | 0.438 |
| 8 | 0.023 | 0.427 |
| 9 | 0.023 | 0.431 |
| 10 | 0.023 | 0.431 |
| 11 | 0.024 | 0.423 |
| 12 | 0.024 | 0.432 |
| 13 | 0.024 | 0.424 |
| 14 | 0.024 | 0.425 |
| 15 | 0.025 | 0.422 |
| 16 | 0.025 | 0.421 |
| 17 | 0.026 | 0.424 |
| 18 | 0.026 | 0.429 |
| 19 | 0.027 | 0.422 |
| 20 | 0.026 | 0.428 |
| 21 | 0.027 | 0.431 |
| 22 | 0.028 | 0.431 |
| 23 | 0.028 | 0.426 |
| 24 | 0.029 | 0.429 |
| 25 | 0.029 | 0.429 |
| Ø | **0.025** | **0.440** |

The results for a single coating with coating solution C (0.75mol% maleic acid content) are shown in Table 3 below:

**Table 3: Results of the coefficient of friction (CoF) determination for coating solution C for a single coating.**

| **Measurement** | **Ø-CoF coated** | **Ø-CoF uncoated** |
|---|---|---|
| 1 | 0.022 | 0.496 |
| 2 | 0.022 | 0.483 |
| 3 | 0.022 | 0.471 |
| 4 | 0.021 | 0.469 |
| 5 | 0.021 | 0.470 |
| 6 | 0.021 | 0.467 |
| 7 | 0.021 | 0.460 |
| 8 | 0.021 | 0.462 |
| 9 | 0.021 | 0.467 |
| 10 | 0.021 | 0.461 |
| 11 | 0.021 | 0.464 |
| 12 | 0.021 | 0.462 |
| 13 | 0.022 | 0.457 |
| 14 | 0.022 | 0.462 |
| 15 | 0.022 | 0.467 |
| 16 | 0.023 | 0.462 |
| 17 | 0.023 | 0.462 |
| 18 | 0.024 | 0.461 |
| 19 | 0.024 | 0.460 |
| 20 | 0.024 | 0.460 |
| 21 | 0.025 | 0.462 |
| 22 | 0.025 | 0.462 |
| 23 | 0.026 | 0.457 |
| 24 | 0.027 | 0.466 |
| 25 | 0.026 | 0.465 |
| Ø | **0.023** | **0.465** |

The results for a single coating with coating solution D (0.5mol% maleic acid content) are shown in Table 4 below:

**Table 4: Results of the coefficient of friction (CoF) determination for coating solution D for a single coating.**

| **Measurement** | **Ø-CoF coated** | **Ø-CoF uncoated** |
|---|---|---|
| 1 | 0.012 | 0.542 |
| 2 | 0.014 | 0.491 |
| 3 | 0.013 | 0.470 |
| 4 | 0.013 | 0.464 |
| 5 | 0.013 | 0.456 |
| 6 | 0.013 | 0.451 |
| 7 | 0.012 | 0.438 |
| 8 | 0.013 | 0.427 |
| 9 | 0.013 | 0.431 |
| 10 | 0.013 | 0.431 |
| 11 | 0.013 | 0.423 |
| 12 | 0.014 | 0.432 |
| 13 | 0.014 | 0.424 |
| 14 | 0.014 | 0.425 |
| 15 | 0.013 | 0.422 |
| 16 | 0.013 | 0.421 |
| 17 | 0.013 | 0.424 |
| 18 | 0.013 | 0.429 |
| 19 | 0.013 | 0.422 |
| 20 | 0.013 | 0.428 |
| 21 | 0.013 | 0.431 |
| 22 | 0.013 | 0.431 |
| 23 | 0.013 | 0.426 |
| 24 | 0.013 | 0.429 |
| 25 | 0.013 | 0.429 |
| Ø | **0.013** | **0.440** |

### Result

A reduced coefficient of friction (CoF) was observed with coating solution A (5 mol% MBBM content) compared with uncoated substrate. The average measured CoF (Ø CoF) was 0.173 compared to the CoF of uncoated nylon of 0.440.

Excellent coefficients of friction (CoF) were observed with coating solutions B, C and D (1 mol% / 0.75 mol% / 0.5 mol% MBM content) compared to uncoated substrate as well as compared to the results for coating solution A. An average CoF (Ø CoF) of less than 0.1 was obtained for all three coating solutions. In particular, for coating solution D, an excellent CoF of 0.013 was obtained.

These results show that a molar content of maleic acid copolymer below 1 mol% in the NVP copolymer is particularly advantageous for coatings of medical devices. With a content below 1 mol%, especially with a content about 0.5 mol%, a significantly reduced frictional resistance is observed with good adhesion to the surface.

### Example 7

### Coating of nylon, Pebax 6333, Pebax 7233, pellethane and polypropylene subtrates.

### 7.1 Substrates tested

1) Nylon tube material, dimension: 0.95 x 0.75 x 320mm (polyamide).
2) Pebax 6333 tube material (thermoplastic polyether blockamide)
3) Pebax 7233 tube material (thermoplastic polyether blockamide)
4) Pellethane extrusion material (thermoplastic polyurethane elastomer).
5) Polypropylene tube material, Fluidfix PP tube, 1.6 x 3.2 mm (inner diameter 0.8 mm).

### 7.2 Coating solutions

### Composition A

Coating solution with 7.175% (mass/volume) total solids containing 1.00% [mass/volume] PVP-co-MBBM with 2.5 mol% maleic acid content. Solvent: isopropanol/water (85:15).

### Composition B

Coating solution with 7.175% (mass/volume) total solids containing 1.00% [mass/volume] PVP-co-MBBM with 5 mol% maleic acid content. Solvent: isopropanol/water (85:15).

### Composition C

Coating solution with 7.175% (mass/volume) total solids containing 1.00% [mass/volume] PVP-co-MBBM with 1.0 mol% maleic acid content. Solvent: Isopropanol/water (85:15).

### Composition D

Coating solution with 7.175% (mass/volume) total solids containing 1.00% [mass/volume] PVP-co-MBBM with 0.75 mol% maleic acid content. Solvent: isopropanol/water (85:15).

### Composition E

Coating solution with 3.34% (mass/volume) total solids containing 0.50% [mass/volume] PVP-co-MBBM with 1.0 mol% maleic acid content. Solvent: isopropanol/water (85:15).

### Composition F

Coating solution with 3.34% (mass/volume) total solids containing 0.50% [mass/volume] PVP-co-MBBM with 0.75 mol% maleic acid content. Solvent: isopropanol/water (85:15).

### 7.3 Optical investigations of the solubility behavior

The coating solutions with compositions A - F were stirred for 16 h.

### Composition A

After 16 h under stirring, transparent insoluble solid was observed and gave a slightly turbid coating solution.

### Composition B

After 16 h under stirring, yellow insoluble solid was observed and gave a slightly turbid coating solution.

### Composition C

After 16 h under stirring, yellow insoluble solid was observed and gave almost clear coating solution.

### Composition D

After 16 h under stirring, little yellow insoluble solid was observed and gave an almost clear coating solution.

### Composition E

After 16 h under stirring, a faint yellow clear coating solution was obtained.

### Composition F

After 16 h under stirring, a faint yellow clear coating solution was obtained.

### 7.4 Coating process

1) Immersion speed: 10 mm/s
2) Incubation time: 30 s
3) Emergence speed: 10 mm/s
4) Curing in UV oven: 2.0 - 3.0 min
5) Drying

Lubricity was measured by determining the coefficients of friction (CoF) with Harland FTS 6000. 25 measurements were taken at 0° angles and 25 measurements at 90° angles. The measurement distance was 5 cm with a tensile speed of 20 mm/s. The clamping force was 250 g and the clamping material was silicone, 80A. Uncoated substrate was used as reference.

### Measurement of lubricity

with Harland FTS 6000
25 measurements each 0° angle
and 25 measurements rotated by 90

| | |
|---|---|
| Measuring distance: | 5 cm |
| Train speed | 20 mm/s |
| Clamping force | 250 g |
| Repetition: | 25 x |

1 uncoated sample per material

### 7.5 CoF measurement of coated nylon tubular material

Composition A
   Ø-CoF, uncoated: 0.478 (n=2)
   Ø-CoF, coated: 0.178 ± 0.083 (n=10)
Composition C
   Ø-CoF, uncoated: 0.478 (n=2)
   Ø-CoF, coated: 0.009 ± 0.002 (n=10)
Composition D
   Ø-CoF, uncoated: 0.478 (n=2)
   Ø-CoF, coated: 0.015 ± 0.009 (n=10)

The coating solutions of compositions A, C and D gave good CoF results on nylon tubular material. The coating solution of composition A with 2.5 mol% maleic acid content showed insufficient CoF performance (Fig. 4).
Composition E
   Ø-CoF, uncoated: 0.478 (n=2)
   Ø-CoF, coated: 0.023 ± 0.009 (n=10)
Composition F
   Ø-CoF, uncoated: 0.478 (n=2)
   Ø-CoF, coated: 0.025 ± 0.008 (n=10)

Composition E and F coating solutions showed excellent results on nylon for single coatings.

### 7.6 CoF measurement of coated PEBAX 6333 tube material

Composition B
   Ø-CoF, uncoated: 0.839 (n=2)
   Ø-CoF, coated: 0.107 ± 0.083 (n=10).
Composition C
Single coating:
   Ø-CoF, uncoated: 0.839 (n=2)
   Ø-CoF, coated: 0.014 ± 0.009 (n=10)
Double coating:
   Ø-CoF, uncoated: 0.839 (n=2)
   Ø-CoF, coated: 0.019 ± 0.006 (n=10)
Composition D
Single coating:
   Ø-CoF, uncoated: 0.839 (n=2)
   Ø-CoF, coated: 0.037 ± 0.023 (n=10)
Double coating:
   Ø-CoF, uncoated: 0.839 (n=2)
   Ø-CoF, coated: 0.031 ± 0.007 (n=10)

The coating solutions of compositions C and D gave very good CoF results on PEBAX 6333 tubular material. The coating solution of composition B with 5 mol% maleic acid content showed still insufficient CoF performance.

### 7.7 CoF measurement of coated PEBAX 7233 tube material

Composition C
Single coating:
   Ø-CoF, uncoated: 0.908 (n=2)
   Ø-CoF, coated: 0.024 ± 0.009 (n=10)
Composition E
Single coating:
   Ø-CoF, uncoated: 0.908 (n=2)
   Ø-CoF, coated: 0.080 ± 0.020 (n=10)
Composition F
Single coating:
   Ø-CoF, uncoated: 0.908 (n=2)
   Ø-CoF, coated: 0.076 ± 0.018 (n=10)

The coating solutions of compositions C, E and F gave good CoF results on PEBAX 7233 tubular material.

### 7.8 CoF measurement of coated pellethane tube material

Composition C
Single coating:
   Ø-CoF, uncoated: 0.688 (n=2)
   Ø-CoF, coated: 0.012 ± 0.010 (n=6)
Composition D
Single coating:
   Ø-CoF, uncoated: 0.688 (n=2)
   Ø-CoF, coated: 0.011 ± 0.003 (n=6)

The coating solutions of compositions C, E and F resulted in good CoF yields on pellethane tubular material.

### 7.9 CoF measurement of coated polypropylene tube material

Composition E
Single coating:
   Ø-CoF, uncoated: 0.768 (n=2)
   Ø-CoF, coated: 0.018 ± 0.003 (n=10)
Composition F
Single coating:
   Ø-CoF, uncoated: 0.768 (n=2)
   Ø-CoF, coated: 0.017 ± 0.003 (n=10)

The coating solutions of compositions E and F gave good CoF results on polypropylene tubing (Figure 3).

### Example 8

### Synthesis of dibenzotirone (BBBD; 4,5-bis(4-benzoylphenylmethyleneoxy) benzene-1,3-disulfonate (Reference Example)

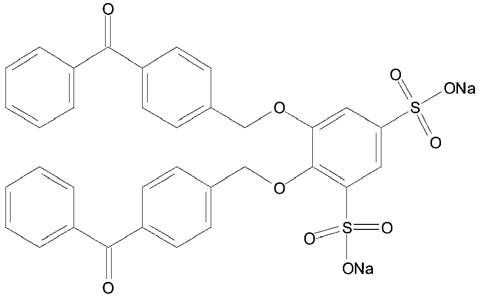

9.0 g of tiron (pyrocatechol-3,5-disulfonic acid disodium salt) and 2.167 g of sodium hydroxide were dissolved in 45 mL of deionized water and tempered to about 75° C under reflux. 15.670 g of 4-(bromomethyl)benzophenone (96%) was weighed and dissolved in 45 mL of 1,4 dioxane under slight heating (<50° C) and added to the basic tiron solution heated to 75° C. The solution was then refluxed. The solution turbid for a short time and turned deep dark green and within 1 hour returned to yellow and completely clear. After 3 hours of reaction, post-dosing was then carried out with 4.015 g of 4-(bromomethyl)benzophenone (96%) in 10 mL of 1,4 dioxane.

Then 0.880 g sodium hydroxide dissolved in deionized water was added. After 20 h boiling under reflux, the solution was concentrated *in vacuo* to dryness. The yellowish solid was suspended in approximately 50 mL of chloroform. After filtration 67 mL of deionized water was added. Upon heating to approx. 80° C, the solid dissolved completely to form a yellow solution to which 67 mL of methanol was added. The solution was slowly cooled with stirring until a white solid crystallized at about 50° C. The solid (dibenzotirone) was separated by vacuum filtration, washed 2x with 100 mL methanol/VE water (1:1) and dried at rt followed by further post-drying with rotary vane pump vacuum.

### Example 9

### Production of solutions as well as coating of surfaces with these solutions

### Example 9.1:

Preparation of a coating solution with 4 components and coating of catheter outer tube pieces.

### Solution:

2.50 g PVP-co-MBBM (component A)
0.438 g BBBD (component B)
5.0 g Kollidon 90F (component C)
10.0 g Kollidon 30K (component D
are dissolved in isopropanol/water (70:30 v/v).

### Coating:

Catheter outer tubing is cut into 12 cm long pieces (5 samples) and then pulled on matching stylet wire. After wiping with isopropanol for conditioning, the pieces are dipped into the coating solution.
Immersion speed: 20 mm/sec
Incubation time: 10 sec
Emergence speed: 5 mm/sec
UV curing: 3 min
Drying: 5 min at RT

### Example 9.2.

Preparation of a coating solution of 4 components and coating of nylon tubing by immersion method with subsequent measurement of the coefficient of friction.

### Solution:

22 g Kollidon 30K (component D)
11 g Kollidon 90F (component C)
5.5 g PVP-co-MBBM (component A)
0.963 g BBBD (component B)
are dissolved in deionized water/isopropanol (70:30 v/v) ).

### Coating:

Immersion speed: 20 mm/sec
Incubation time: 30 sec
Plunge speed: 10 mm/sec
Curing in UV oven: 2 min
Drying: 15 min at 50°C

### Example 10

### Adhesion of the coating to a surface

The adhesion is shown in the measurement of the coefficient of friction and results from the measurement of the coefficient of friction depending on the cycles performed. The higher the value of the coefficient of friction becomes in the course of the cycles, the more the coating detaches, the adhesion decreases.

If the coefficient of friction does not change or hardly changes over the successive cycles (measured up to 25 cycles), this indicates a stable coating with good adhesion to the surface of the coated object.

### Example 11

### Shelf Life (storage life)

To determine the shelf life and durability of the hydrophilic coating material, a number of samples scheduled for the planned time period are coated, dried and packaged according to instructions. During a specified time frame, at least two samples are taken, visually inspected and the coefficient of friction and adhesion are measured.

| | |
|---|---|
| Stock solution: | 2-propanol 60 ml |
| | Deionized water 340 ml |
| | Kollidon 90F (2% w/v) 8 g |
| | Kollidon 30K (4% w/v) 16 g |
| Immersion solution: | Stock solution 140 ml |
| | Poly(VP-co-MBBM) (1% w/v) 1400 mg |
| | Dibenzotirone (0.175% w/v) 245 mg |

The nylon catheters used here are threaded onto a stylet and then immersed in the solution at a speed of 20 mm/s. The fully immersed nylon catheter remains in the solution for 30 s and is then pulled out again at a speed of 10 mm/s. This is followed by UV irradiation for 2 min. The final drying is carried out in the drying oven for 15 min.

The parts are cured for 4h at 177°C, cleaned and packed.

### Example 12

Synthesis of N-[3-(4-benzoylbenzamido)propyl]maleic acid

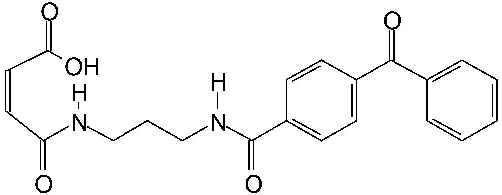

5 g of Boc-protected N-(3-aminopropyl)maleic acid amide were suspended in 35 mL of chloroform with stirring. The suspension was then cooled to approximately 0° C. To the cooled suspension, 7.265 g of 4-benzoylbenzoic acid chloride was added. Then, 2.5 mL of chloroform and 8.624 mL of triethylamine were slowly dropped into the suspension. After 1.5 hours, the cooling bath was removed. The golden yellow solution was then stirred further for 2.5 hours. It was then washed 3x with conc. HCl in a separatory funnel. The slightly turbid solution was then dried over sodium sulfate. The clear golden yellow solution was then filtered and the solvent was removed to dryness at low temperature *in vacuo.* Next, 20 mL of chloroform and a spatula tip of phenothiazine were added to the dry residue. 80 mL of toluene was then added and the suspension was briefly heated to 75° C. The suspension was cooled to room temperature. An additional 100 mL of toluene-chloroform solution (4:1) was added to the suspension. The solid was then filtered off by vacuum filtration, and dried overnight with rotary vane pump vacuum. The Boc protecting group was then removed according to standard procedures.
Solubility in THF: Very soluble
Solubility in N-vinylpyrrolidone: Freely soluble

### Example 13

### Synthesis of crosslinkers

### Example 13.1: Crosslinker B1 (reference example)

Synthesis of dibenzotirone (BBBD; 4,5-bis(4-benzoylphenylmethyleneoxy) benzene-1,3-disulfonate.

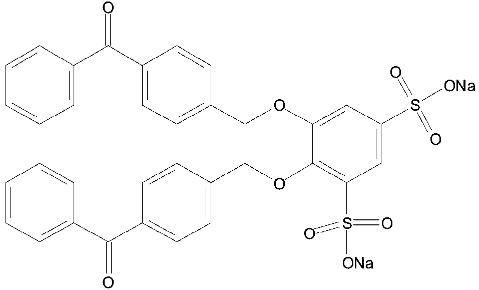

9.0 g of tiron (pyrocatechol-3,5-disulfonic acid disodium salt) and 2.167 g of sodium hydroxide were dissolved in 45 mL of deionized water and tempered to about 75° C under reflux. A clear yellow solution was obtained. 15.670 g of 4-(bromomethyl)benzophenone (96% strength) was weighed and dissolved in 45 mL of 1,4 dioxane under slight heating (<50° C) and added to the basic tiron solution heated to 75° C. The solution was then refluxed. After 3 hours of reaction, post-dosing was then carried out with 4.015 g of 4-(bromomethyl)benzophenone (96%) in 10 mL of 1,4 dioxane. Then 0.880 g sodium hydroxide dissolved in deionized water was added. After 20 h under reflux, the solution was i.v. concentrated to dryness. The yellowish solid was suspended in approximately 50 mL of chloroform. After filtration, the solid was transferred to a 200 mL single-neck flask and 67 mL of deionized water was added. Upon heating to approximately 80° C, the solid dissolved completely to form a yellow solution to which 67 mL of methanol was added. The solution was then slowly cooled with stirring until a white solid crystallized at about 50° C. The solid (dibenzotirone) was separated by vacuum filtration, washed 2x with 100 mL methanol/VE water (1:1) and dried at rt followed by further post-drying with rotary vane pump vacuum.

### Example 13.2: Crosslinker B2

### Synthesis of 2,4-{N-[N'-(4-benzoylphenyl)-hydroxycarbonyl]}-diaminotoluene (BPHAT).

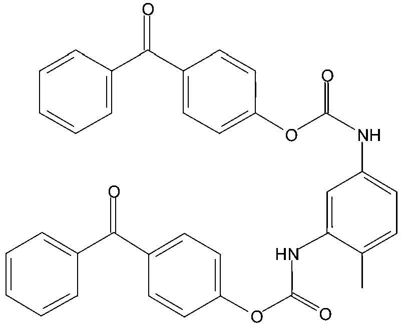

In a 250 mL single-neck flask, 11.130 g of 4-hydroxybenzophenone, 65 mg of 1,4-diazabicyclo[2.2.2]octane (DABCO), and 10 µL of dibutyltin dilaurate were dissolved in 50 mL of tetrahydrofuran with stirring at rt. Then, 4.000 mL of toluene diisocyanate was added to the solution. The solution was then stirred for 5h at about 75°C and continued to stir overnight at 40°C. The next day, the solution was transferred to 400 mL of n-heptane, whereupon a yellowish colored amorphous solid spontaneously precipitated. The solvent mixture was tempered to about 60° C and then cooled back to rt. The solid was then separated from the solvent mixture by vacuum filtration and redissolved in 100 mL acetone. A milky white solution was obtained. Subsequently, 300 mL of n-heptane was added to the solution, whereupon a white solid precipitated. Using vacuum filtration, the solid was again separated from the solvent mixture and dried.

### Example 13.3: Crosslinker B3

### Synthesis of carbonyl-bis-((4-benzoylphenyl)carbamoyl)-benzophenone [BCBAC].

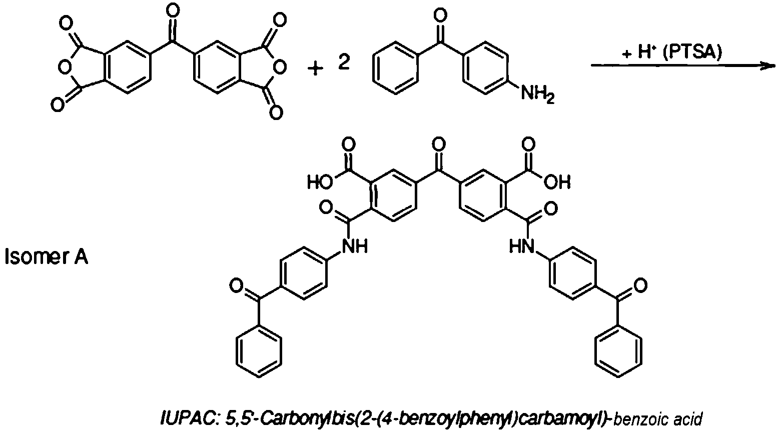

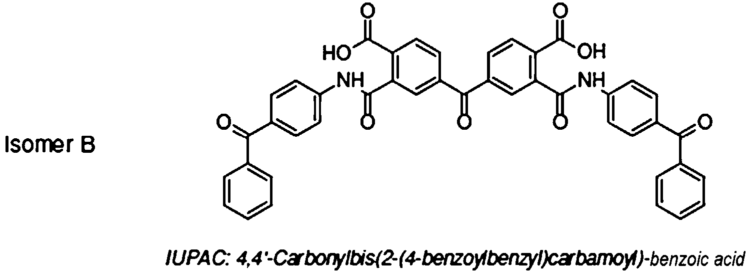

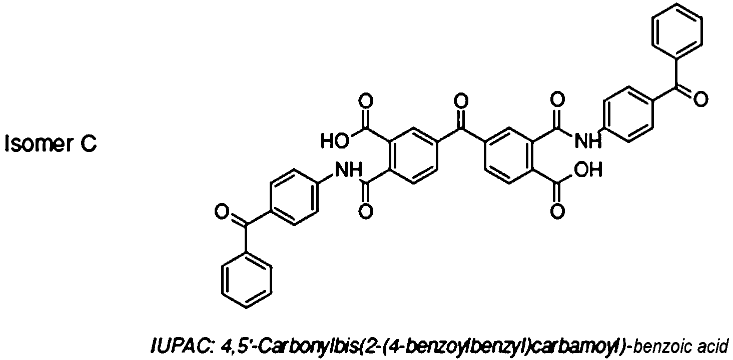

5.009 g of benzophenone-3,3',4,4' tetracarboxyl dianhydride and 6.128 g of 4-aminobenzophenone were dissolved in 100 mL of THF + 30 mL of acetone. 0.213 g of para-toluenesulfonic acid monohydrate was added. It was then gassed with nitrogen and refluxed to 60° C overnight andthe resulting golden yellow solution was then concentrated *in vacuo* to dryness. The resulting dry residue was taken up in 50 mL chloroform + 10 mL acetone, and then precipitated in 300 mL n-heptane. Vacuum filtration was used to separate the solid from the solvent, and the resulting white, very fine powdery solid was then dried under vacuum overnight.

### Example 13.4: Crosslinker B4

### Benzophenone di-carboxyl di-N-(4-(benzoylphenyl)-methylaminocarboxylate) [BCBMC].

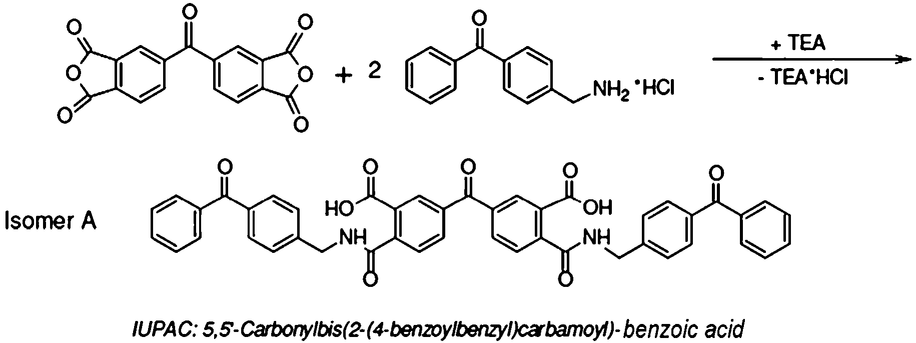

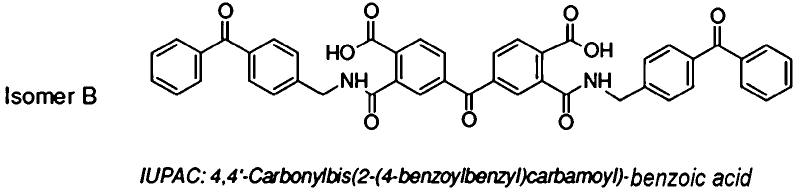

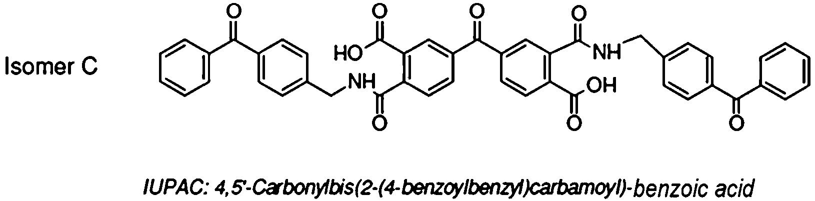

In a 250 mL single-neck flask, 5.000 g of 4-benzoylbenzylamine hydrochloride and 3.260 g of benzophenone-3,3',4,4' tetracarboxyldianhydride were dissolved in 150 mL of tetrahydrofuran (THF). 2.825 mL of triethylamine was also added to the reaction solution and tempered at reflux to 60 °C for 3 days. Then, the yellow and slightly turbid solution was cooled to rt. Triethylammonium chloride precipitated from the cooled solution and was separated. To precipitate the solid, 600 mL of n-heptane was added to the yellow solution and after decantation, the precipitated solid was separated from the solvent. The milky white solvent mixture was concentrated to dryness. Both solids were then dried with rotary vane pump vacuum. The solubility of both solids was first investigated before further purification. It was found that the dry residue of the solvent mixture was insoluble or only partially soluble in chloroform, whereas the precipitated yellow solid dissolved readily in chloroform. The yellow solid was dissolved in chloroform for further purification and precipitated again by adding n-heptane. The solvent mixture became milky turbid and after centrifugation, the milky turbid solution was separated again. The milky turbid solution was concentrated to dryness, and the resulting white dry residue was washed 2x with about 100 mL of acetone-ethanol (1:1), adding first the acetone portion and then the ethanol portion.

The washed solid was separated from the solvent mixture by vacuum filtration using a glass suction filter (Por. 5) and transferred to a tared 100 mL single-neck flask. The solid remaining in the falcon tubes was dissolved in chloroform, transferred to a tared single-neck flask, and concentrated to dryness. Then, rotary vane pump vacuum was used to dry for 24 hours.

### Example 13.5: Crosslinker B5

### Synthesis of di-benzoylbenzoic acid-1,3-diamidopropane (BPAP).

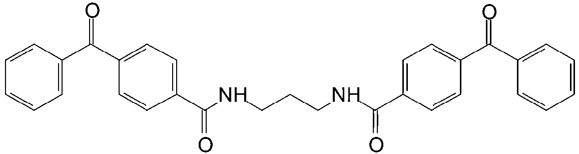

0.871 g of 1,3 diaminopropane was dissolved in 25 mL of THF. 5.317 g of benzoylbenzoic acid was dissolved under heating in 50 mL of THF at rt and a drop of triethylamine and 4 mL of diisopropylcarbodiimide were added. The diaminopropane solution was then added, whereupon a white solid spontaneously crystallized. After about 2 hours, the reaction was stopped and the solid was separated from the solvent by vacuum filtration. The solid was then washed in 50 mL THF and filtered off again. The crude product obtained was insoluble in chloroform, acetone and ethanol, soluble in water, methanol and water-alcohol. For recrystallization, the crude product was suspended in ethanol and heated to boiling temperature. Deionized water was added, whereupon the solid gradually dissolved and then cooled, precipitating the recrystallized solid. After vacuum filtration, BPAP was dried.

### Example 13.6 Crosslinker B6

### Synthesis of 4,4'-{N-[N'-(4-benzoylphenyl)-hydroxycarbonyl]} diaminomethylene bisphenol.

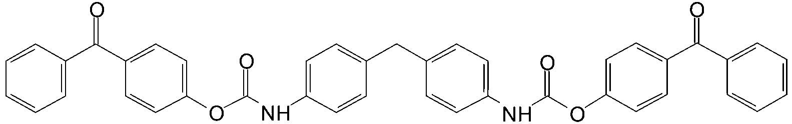

### a) Preparation with chloroform as solvent and addition of DBTDL

5.062 g methylene bis(phenyl isocyanate) and 8.025 g 4-hydroxybenzophenone were dissolved in 100 mL chloroform with stirring at rt. The solution was then stirred for about 48 h. 50 mL of the solution was transferred to a 100 mL single-neck flask and 10 µL of dibutyltin dilaurate was added. Both solutions were then further stirred for another 3 days. In contrast to the original solution, the solution to which tin catalyst was added was highly turbid. The reaction was stopped and the solution was slowly transferred to a beaker filled with 200 mL of n-heptane, precipitated and decanted. The precipitated sticky solid was slurried again with 100 mL of n-heptane and decanted again. The solid was then dried.

The product is soluble in acetone.

### b) Preparation with chloroform as solvent

5.027 g of methylene bis(phenyl isocyanate) and 7.973 g of 4-hydroxybenzophenone were dissolved in 100 mL of chloroform with stirring at rt and continued stirring for 5 days. The solution was slowly transferred to 400 mL of n-heptane, precipitated and decanted. The precipitated sticky solid was dissolved in 150 mL acetone (milky turbid solution) and slowly precipitated by adding 50 mL n-hexane several times (total 250 mL). The precipitated solid was filtered off, washed with n-hexane and dried.

### Example 13.7: Crosslinker B7

### Synthesis of 1,3-{N-[N'-(4-benzoylphenyl)-hydroxycarbonyl]}-diaminobenzene (BPHAB).

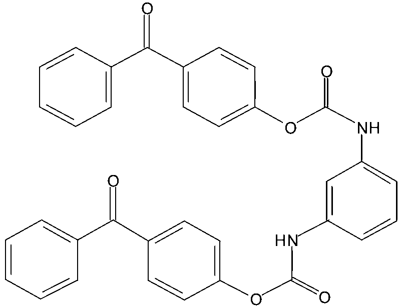

### a) Chloroform as solvent, DABCO as acyl transfer reagent

2.009 g of 1,3-phenylene diisocyanate, 4.953 g, 4-hydroxybenzophenone and 0.107 g of 1,4diazabicyclo-[2.2.2]octane were dissolved in 100 mL of chloroform with stirring at rt. A milky turbid solution was formed, which was heated to about 30° C at reflux. Within a few minutes, a solid flocculated. After 10 min, it was completely precipitated subsequently suspended in 300 mL of chloroform. The chloroform suspension was heated to about 55° C with stirring, whereupon the solid dissolved almost completely. The insoluble solid portions were filtered off and dried, and the chloroform solution was cooled to rt., a white voluminous solid had again precipitated from the chloroform solution, which was separated and dried. The dried solid was dissolved in 45 mL of acetone at rt. 15 mL of the acetone solution was then purified by column chromatography using a solvent mixture of chloroform-acetone (9:1).

The product is highly soluble in THF, soluble in acetone and DMF, poorly soluble in ethanol A photoinitiator with good sliding friction performance especially on nylon tubing material.

### b) Chloroform as solvent, with DABCO as acyl transfer reagent (larger solvent volume and modified addition sequence).

4.953 g of 4-hydroxybenzophenone and 0.102 g of 1,4-diazabicyclo[2.2.2]octane were dissolved in 150 mL of chloroform with stirring at rt. 2.003 g of 1,3-phenylene diisocyanate was dissolved in 50 mL of chloroform and dropped into the hydroxybenzophenone solutionand stirred overnight, then cooled in a freezer. The precipitated solid was then filtered off and dried. The chloroform solution was processed separately. The solid was then recrystallized in 60 mL ethanol + 5 mL acetone. The chloroform-soluble fractions were precipitated by addition of 150 mL n-heptane, dried, and analyzed by IR spectroscopy. Subsequently, the solid (crude yield: approx. 6.6 g) was completely dissolved in 45 mL ethanol-acetone (95:5) with gentle heating. The solution was then re-compressed to dryness using a rotary evaporator at 40° C bath temperature) until only a yellowish gel remained in the flask. The yellow gel was dissolved in 50 mL of ethanol with heating. Then, a total of 75 mL of deionized water was slowly added to the warm ethanolic solution. Upon cooling to rt, a white solid gradually precipitated.

### Example 13.8: Crosslinker B8

### Synthesis of 1,3--{N-[N'-(4-benzoylphenyl)-carbonyl]}-diaminoxylylene (BPDX).

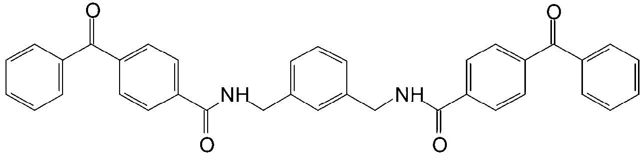

2.26 g of benzoylbenzoic acid and 60 mg of DMAP dissolved in 22 ml of THF, after the addition of 660 µl of xylylenediamine, a white precipitate formed immediately. DIC was then added to the mixture and stirred overnight. The solvent was removed in vacuo.

### Example 14.9: Crosslinker B9

### Synthesis of 1,6--{N-[N'-(4-benzoylphenyl)-carbonyl]}-diaminohexane (BPDH).

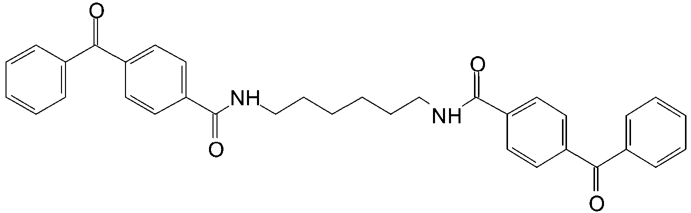

2.26 g of benzoylbenzoic acid and 60 mg of dimethylaminopyridine (DMAP) were dissolved in 20 ml of DMF and 660 µl of m-1,6 diaminohexane was added. After removal of DMF, the white precipitate was taken up in ethyl acetate (EE) / sat. NaHCO₃ solution and extracted with the alkaline solution, then washed neutrally with water. Subsequently, it was washed several more times with 5% KHSO₄ solution and then washed neutrally with water. After drying over Na₂SO₄ the solution was concentrated and precipitated from EE with n-heptane.

### Example 13.10: Crosslinker B10

### Synthesis of 4-benzoylbenzoic acid 4-benzylphenyl ester (PBBPE).

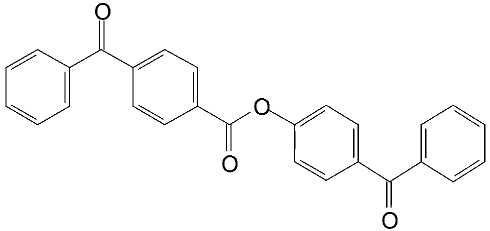

2.26 g benzoylbenzoic acid, 2.22 g 4-hydroxybenzophenone and 60 mg DMAP were dissolved in 22 ml THF and 1700 µl DIC was added. After about 1 min, a white precipitate was formed. The THF was then removed in vacuo. The white residue did not dissolve in EE / sat. NaHCO₃ solution. The solid was filtered off, washed with water and dried.

### Example 13.11: Crosslinker B11

### 4-Benzoylbenzoic acid-4-benzylphenylamide (PBPBA)

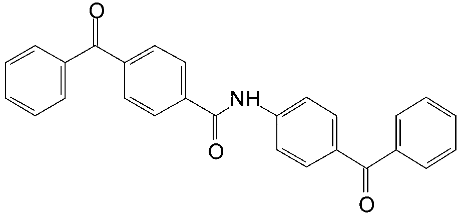

2.26 g benzoylbenzoic acid, 986 mg 4-aminobenzophenone and 60 mg DMAP were dissolved in 20 ml THF and 1700 µl DIC was added. After about 3 min, a white precipitate formed and was almost dissolved the next day. The THF was removed in vacuo. The white residue did not dissolve in EE / water. The solid was filtered off, washed with water and dried. After drying over Na₂SO₄, the EE was removed in vacuo. The white residue was no longer soluble in EE at rt and was filtered off.

### Example 13.12: Crosslinker B12

### Synthesis of pyridine-2,6-dicarboxylicacid-bis-(4-benzoylphenyl)amide (PCBPA).

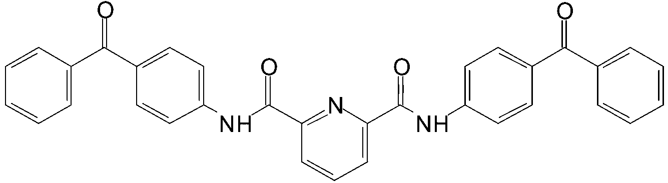

835 mg of pyridinedicarboxylic acid was dissolved in 15 ml of DMF. 4-aminobenzophenone and DMAP were dissolved in 10 ml DMF and added to the solution of pyridinedicarboxylic acid. Then, 1.7 ml of DIC was added. The mixture was stirred overnight. Then, DMF was removed in vacuo. The residue was taken up with EE/water and washed with NaHCO₃ solution and water. This precipitated white solid that was acetone soluble. The EE phase was extracted with KHSO₄ solution, washed with water, dried over Na₂SO₄. After concentration, further product could be isolated.

### Example 13.13: Crosslinker B13

### Synthesis of pyridine-2,6-dicarboxylicacid-bis-(4-benzoylphenyl)ester (PCBPE)

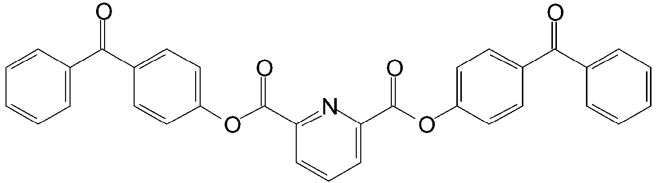

2,6-Pyridinedicarboxylic acid was dissolved in 10 ml DMF, and a white precipitate was formed after the addition of 120 mg DMAP. 2.19 g of 4-hydroxybenzophenone was dissolved in 10 ml of DMF and added to the 2,6-pyridinedicarboxylic acid. After addition of 5 ml DMF, it was slightly heated until the solution was clear. After cooling, 1.7 ml of DIC was added; after 5 min, a white precipitate was formed. After stirring overnight, the DMF was removed in vacuo. The residue was taken up in EE/5% KHSO₄ solution. The remaining solid was vacuumed. The solid taken up was insoluble in acetone, isopropanol, EtOH, THF and CMA. The EE phase was washed with water, dried over Na₂SO₄ and concentrated. The residue was dissolved in EE and precipitated with n-heptane. The precipitated product (PCBPE solid II) was soluble in THF and DC pure.

### Example 13.14: Crosslinker B14

### Synthesis of isophtalic acid-bis-(4-benzoyl-phenyl)amide (IPBPA)

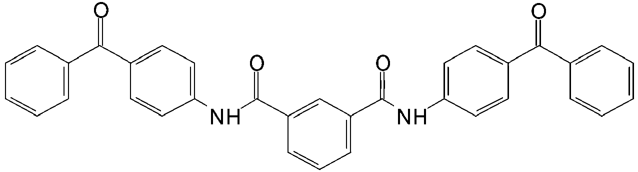

835 mg isophthalic acid was almost dissolved in 20 ml THF. 2.27 g 4-aminobenzophenone and 120 mg DMAP were dissolved in 10 ml THF. The solutions were combined, DIC was added and after stirring overnight, the reaction mixture was concentrated and taken up in EE/water. The EE phase was washed successively with KHSO₄, water and NaHCO₃ /H₂O and dried.

### Example 13.15: Crosslinker B15

### Synthesis of isophtalic acid-bis-(4-benzoyl-phenyl)ester (IPBPE).

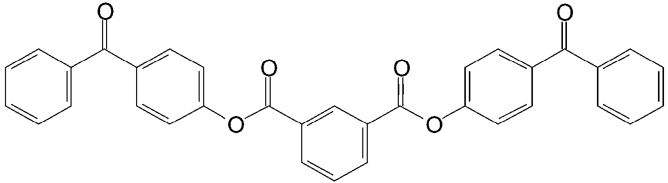

Isophthalic acid, DMAP and 4-hydroxybenzophenone were dissolved in 15 ml THF and 1.5 ml DMF. After addition of 1.7 ml DIC, a white solid precipitated . The solvent was removed in vacuo. The residue was taken up in a lot of EE/ 5% KHSO₄, dissolving the solid completely. The EE phase was washed with NaHCO₃ /H₂O and dried over Na₂SO₄ and after the solution was concentrated, the product precipitated from EE. The product formed white needles.

### Example 13.16: Crosslinker B16

### Synthesis of benzoylphenyl-Arg-(4-benzoylphenyl)amide (BP-Arg-BPA).

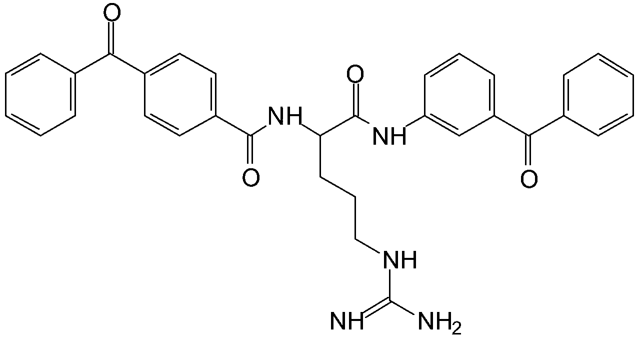

1. Preparation of Boc-Arg-BPA
   3.75 g Boc-His was suspended in 25 ml DMF. No solution occurred even after addition of 2.1 ml Et₃N. For activation, 1.96 ml of chloroformic acid isobutyl ester formed an additional white precipitate, Et₃N-HCl. 2.96 g of 4-aminobenzophenone was dissolved in 11 ml of DMF and added to the suspension of Boc-His. Additional Et₃N was added to make the reaction solution basic. The mixture was stirred overnight. The DMF was removed under oil pump vacuum. The residue was taken up with EE/H₂O. The EE solution was washed with KHSO₄ solution and with NaHCO₃ solution and with water; then dried over Na₂SO₄. Boc-His-BPA was precipitated from EE with n-heptane.
2. Coupling of H-Arg-BPA with 4-benzoylbenzoic acid to benzoylphenyl-Arg (4-benzoylphenyl) amide (BP-Arg-BPA).

### 1. Variant:

Boc-Arg-BPA was deblocked with TFA in 60 min and then withdrawn via vacuum. The residue was taken up with THF and neutralized with 1.2 ml Et₃N. 452 mg of 4-benzoylbenzoic acid and 760 mg of HBTU were dissolved in THF, then the neutralized solution of H-Arg-BPA was added and the "pH" was adjusted to about pH 8 by further addition of 200 µl of Et₃N. The mixture was stirred overnight and then concentrated in vacuo. The oily residue was taken up with EE/H₂O. EE solution was washed with KHSO₄ solution and with NaHCO₃ solution and with water; then dried over Na₂SO₄ and recrystallized from EE /n-heptane.

### 2. Variant (mixed anhydride synthesis)

550 mg of Boc-Arg-BPA was dissolved in TFA and deblocked in 45 min and then the TFA was removed. The residue was taken up with 6 ml DMF, added with ca.164 µl Et₃N and then activated with 152 µl chloroformic acid isobutyl ester. Then 268 mg of 4-benzoylbenzoic acid dissolved in DMF was added and stirred overnight. The reaction solution was concentrated in vacuo. The oily residue was taken up with EE/H₂O. The EE solution was washed with KHSO₄ solution and with NaHCO₃ solution and with water and dried over Na₂SO₄.

The product was recrystallized from EE/n-heptane.

### Example 13.17:

### Benzoylphenyl-His-(4-benzoylphenyl)amide (BP-His-BPA)

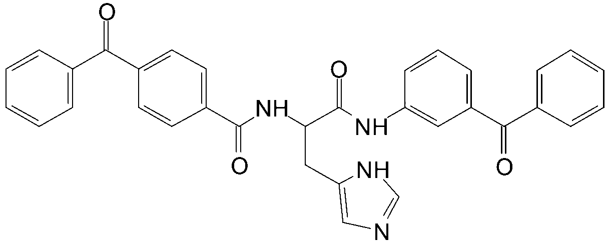

1. Preparation of Boc-His-BPA
   3.75 g Boc-His was suspended in 25 ml DMF. No solution occurred even after addition of 2.1 ml Et₃N. For activation, 1.96 ml of chloroformic acid isobutyl ester formed an additional white precipitate, Et₃N-HCl. 2.96g of 4-aminobenzophenone was dissolved in 11 ml of DMF and added to the suspension of Boc-His. Additional Et₃N was added to make the reaction solution basic. The mixture was stirred overnight. The DMF was removed under oil pump vacuum. The residue was taken up with EE/H₂O. The EE solution was washed with KHSO₄ solution and with NaHCO₃ solution and with water; then dried over Na₂SO₄. Boc-His-BPA was precipitated from EE with n-heptane.
2. Coupling of H-His-BPA with 4-benzoylbenzoic acid to benzoylphenyl-His (4-benzoylphenyl) amide (BP-His-BPA).

870 mg of Boc-His-BPA was dissolved in TFA and deblocked in 45 min. The TFA was removedn and the residue was taken up 3x with butyl methyl ether and concentrated. The oily residue was then taken up in 6 ml DMF and neutralized with 880 µl Et₃N. 390 mg 4-benzoylbenzoic acid dissolved in DMF was neutralized with 280µl Et₃N and then activated with 223 µl chloroformic acid isobutyl ester within 3 min. The neutralized solution of H-His-BPA was then added. After pH control, the mixture was stirred overnight and concentrated in vacuo. The oily residue was taken up with EE/H₂O and washed with KHSO₄ solution, NaHCO₃ solution and with water. After drying over Na₂SO₄ was recrystallized from EE/n-heptane.

### Example 13.18:

### Synthesis of benzoylphenyl-ser-(4-benzoylphenyl)amide (BP-Ser-BPA).

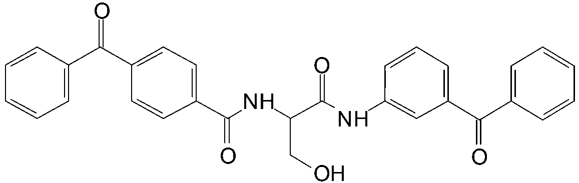

1. Production of H-Ser-BPA
   b) 1.025 g of Boc-Ser was dissolved in 10 ml of THF, then 4-aminobenzophenone and DMAP were added. A clear solution was formed and a white solid was formed after the addition of DIC. The mixture was stirred overnight, retaining the solid. The THF was removed in vacuo and the solid was aspirated, then washed with KHSO4 solution and with water.
   b) 2.05 g of Boc-Ser was dissolved in 12 ml of DMF, added with 1.4 ml of Et₃N and then activated with 1.3 ml of chloroformic acid isobutyl ester for 3 min. Then, 1.97 ml of ABP was added and stirred overnight. THF was removed in vacuo and the residue was taken up in EE and KHSO₄ solution and washed with NaHCO₃ solution and with water. After drying and concentration, recrystallization was performed from EE/n-heptane.
2. Coupling of H-Ser-BPA with 4-benzoylbenzoic acid to benzoylphenyl-ser (4-benzoylphenyl) amide (BP-Ser-BPA).

620 mg Boc-Ser-BPA was dissolved in TFA and deblocked in 45 min. After TFA was withdrawn, the residue was taken up with 6 ml DMF and neutralized with Et₃N. Then 370 mg of 4-benzoylbenzoic acid was dissolved in 2 ml of DMF and activated with 210 µl of chloroformic acid isobutyl ester for 3 min. Then the neutralized solution of H-Ser-BPA was added and stirred overnight. The reaction solution was concentrated in vacuo. The oily residue was taken up with EE/H₂O and washed with KHSO₄ solution, NaHCO₃ solution and with water; then dried over Na₂ SO₄ and recrystallized from EE/n- heptane.

### Example 14

### Example 14.1

Preparation of a coating solution without and with additives and application of a hydrophilic coating on balloon catheters (05x60-850) by dip coating.

### 1. Preparation of the coating solutions

### Solution 1 (solid concentration 51.00 mg/mL):

5.0 g PVP-co-MBBM (component A)
0.1 g BPHAT (component B from example 13.2)

Before use, this solution was diluted 1:1 with ethanol, i.e., the final concentration of the coating solution was 25.5 mg/mL.

### Solution 2: (solid concentration 71.75 mg/mL) in ethanol

1.0 g PVP-co-MBBM (component A)
0.175 g Photoinitiator BPHAT (component B from Example 13.2)
2.0 g Kollidon 90 F (component C)
4.0 g Kollidon 30K (component D)
dissolved in 100 mL ethanol

This solution was used undiluted.

### 2. Execution

A 15 mL falcon tube was filled with immersion solution 1. Prior to dip coating, the respective balloon catheter was fitted with a three-way valve at the designated connector. The balloon protective sheath and stylet were first peeled off, and a 0.075-inch inner diameter balloon protective sheath was slipped on. The stylet was then slid back on, and the protective sheath was pushed toward the proximal end of the catheter until approx. 2 cm beyond the end of the balloon. Using a 1 mL syringe, the balloon was now slowly expanded through the three-way valve. The valve was then closed and the syringe removed. The expanded balloon was cleaned with a cleanroom wipe, soaked in isopropanol and then submerged. The immersion time was 3 s. (for 6 cm length this corresponds to: 2.0 cm/s.), the balloon remained in the immersion solution for 10 sec. and was then withdrawn from the immersion solution within 12 s (for 6 cm length this corresponds to: 0.5 cm/s.). The coated balloon catheter was then UV-exposed for 2 min and post-dried in a drying oven at 50° C for 5 min. The coated balloon was now folded back. With a slow rotating movement in the direction of the fold, the protective balloon cover was now slid over the balloon.

### Example 14.2.

Preparation of a coating solution of 5 components and coating of nylon tubing by immersion method with subsequent determination of the coefficient of friction on nylon tubing and polypropylene tubing.

### Solution :

5.4 g PVP-co-MBBM (component A)
0.105 g BCBAC (component B from example 13.3.)
0.6 g VP-co-maleate (component E)
12.0 g PVP 90 F (component C)
24.0 g PVP 30 K (component D)

The components are dissolved in 600 ml isopropanol/acetone (85:15 vol/vol).

The coating is applied by immersion (30sec) followed by UV irradiation for 3 min. After drying for 5 min at room temperature, the sliding ability was tested (with Harland FTS6000).
**Log CoF: MW = 0.041 +/- 0.008 nylon**
**Log CoF: MW = 0.055 +/- 0.019 PP tube**

### Example 14.3.

Preparation of a coating solution from 4 components and coating of polypropylene hose in the dipping process with subsequent measurement of the coefficient of friction.

### Solution:

5.4 g PVP-co-MBBM (component A)
0.105 g BCBAC (component B from example 13.3.)
0.5 g Kollidon 90F (component C)
1.0 g Kollidon 30K (component D)
are dissolved in isopropanol/acetone (85:15 v/v).

### Coating

Immersion speed: 10 mm/sec
Incubation time: 10 sec
Emergence speed: 5 mm/sec
Curing in UV oven: 3 min
Drying: 5 min at room temperature
**Log CoF: MW = 0.039 +/- 0.009**

### Example 14.4.

Preparation of a coating solution from 4 components and coating of nylon tubing in the dipping process with subsequent measurement of the coefficient of friction.

### Solution:

0.25 g PVP-co-MBBM (component A)
0.963 g BCBAC (component B from example 13.3.)
0.5 g Kollidon 90F (component C)
1.0 g Kollidon 30K (component D)
are dissolved in isopropanol/0.067 mol/l NaOHaqu. (85:15 v/v).

### Coating:

Immersion speed: 20 mm/sec
Incubation time: 30 sec
Emergence speed: 10 mm/sec
Curing in UV oven: 3 min
Drying: 5 min at 50°C
**CoF** : **0.054 +/- 0.011**

### Example 14.5.

Preparation of a coating solution from 4 components and coating of nylon tubing in the dipping process with subsequent measurement of the coefficient of friction.

### Solution:

4.0 g Kollidon 30K (component D)
2.0 g Kollidon 90F (component C)
0.25 g PVP-co-MBBM (component A) (2 mol% maleic acid moiety)
0.175 g BCBAC (component B from example 13.3.)
are dissolved in isopropanol/ demineralized water (85:15 vol./vol.) and filtered.

### Coating:

Immersion speed: 20 mm/sec
Incubation time: 30 sec
Emergence speed: 10 mm/sec
Curing in UV oven: 3 min
Irradiation dose: 4055.80 mJ/cm2
Drying: 15 min (rt)
**CoF : 0.007 +/- 0.002**

### Example 14.6.

Preparation of a coating solution from 3 components and coating of nylon tubing in the dipping process with subsequent measurement of the coefficient of friction.

### Solution:

0.5 g PVP-co-MBBM (component A) (1 mol% maleic acid moiety)
0.04 g BCBAC (component B from example 13.3.)
2.8 g Kollidon 90F
are dissolved in isopropanol/VE water (85:15 v/v) and filtered.

### Coating:

Immersion speed: 20 mm/sec
Incubation time: 30 sec
Emergence speed: 10 mm/sec
Curing in UV oven: 3 min
Irradiation dose: 4055.80 mJ/cm2
Drying: 15 min (rt)
**CoF** : **0.012 +/- 0.002**

### Example 14.7.

Preparation of a coating solution of 4 components and coating of silicone hose by immersion method with subsequent measurement of the coefficient of friction.

### Primer solution:

The silicone tubes are pretreated with plasma. They are then placed in the primer solution for 60 min:
ethanol (70%), HEMA (13.6%, PVP 90F (7%), aminosilane (10%), methacrylic anhydride (6.5%).

Then dry at rt for 20 minutes and subsequently at 135° C for 2h.

### Immersion solution:

0.25 g PVP-co-MBBM (component A) (1 mol% maleic acid moiety)
0.044 g BCBAC (component B from example 13.3.)
0.5 g Kollidon 90F (component C)
1.0 g Kollidon 30K (component D)
are dissolved in isopropanol/VE water (85:15 v/v) and filtered.

### Coating:

Immersion speed: 20 mm/sec
Incubation time: 30 sec
Emergence speed: 10 mm/sec
Curing in UV oven: 3 min
Drying: 5 min at 50°C

### Example 14.8.

Preparation of a coating solution from 4 components and coating of silicone hose in the dipping process.

### Primer solution:

The silicone tubes are added to a primer solution of n-heptane (60%), 6670 Part A (20%) and 6670 Part B (20%).

### Immersion solution:

0.25 g PVP-co-MBBM (component A) (1 mol% maleic acid moiety)
0.044 g BCBAC (component B from example 13.3.)
0.5 g Kollidon 90F (component C)
1.0 g Kollidon 90F (component D)
are dissolved in isopropanol/NaOH_{aqu.} (85:15 v/v) and filtered.

### Coating:

Immersion speed: 20 mm/sec
Incubation time: 30 sec
Emergence speed: 10 mm/sec
Curing in UV oven: 3 min
Drying: 5 min at 50°C

### Example 14.9 Preparation of a coating solution from 3 components and coating of nylon, pellethane and pebax hose by immersion process

### Immersion solution:

0.25 g PVP-co-MBBM (component A) (0.5 mol% maleic acid moiety)
0.01 g BCBAC (component B from example 13.3.)
0.7 g Kollidon 90F (component C)
are dissolved in isopropanol/VE water (85:15 vol/vol).

### Coating:

Immersion speed: 20 mm/sec
Incubation time: 30 sec
Emergence speed: 10 mm/sec
Curing in UV oven: 3.0 min
Drying: 5 min at RT and 15 min at 50°C

### Coefficient of friction:

**CoF (nylon): 0.018 +/-0.004**
**CoF (pellethane)** : **0.015 +/-0.004**
**CoF (Pebax): 0.017 +/-0.005**

## Claims

1. A medical device having a coating of a **cross-linked** vinylpyrrolidone copolymer composed of blocks of N-vinylpyrrolidone moieties divided by maleic acid moieties, wherein each maleic acid moiety has as functional groups a carboxy group (-COO-) and a carbamoyl group (-CONH-), or two carboxy groups (-COO-), or two carbamoyl groups (-CONH-), and 1% to 50% of the functional groups of all maleic acid moieties in the vinylpyrrolidone copolymer are functionalized with
**-L-Y-8P**
wherein
**L** represents a C₁-C₅ alkyl linker;
**BP** represents a benzophenone substituent; and
**Y** represents a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
with the proviso that when **Y** is -O-, the C₁-C₅ alkyl linker (**L**) is not ethylene (-CH₂CH₂-),
wherein the coating contains at least one anti-inflammatory, cytostatic, cytotoxic, antiproliferative, antimicrotubule, antiangiogenic, antiviral, antibacterial, antibiotic, antimitotic, antirestenotic, antifungal antineoplastic, antimigrative, anticancerogenic, vasodilator, athrombogenic, and antithrombogenic agent, or a mixture thereof.

2. The medical device according to claim 1, wherein the anti-inflammatory, cytostatic, cytotoxic, antiproliferative, antimicrotubule, antiangiogenic, antiviral, antibacterial, antibiotic, antimitotic, antirestenotic, antifungal, antineoplastic, antimigrative, anticancerogenic, vasodilator, athrombogenic, and antithrombogenic agent is selected from the group comprising or consisting of: sirolimus (rapamycin), everolimus, biolimus, zotarolimus, temsirolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoboside, teniposide, nimustine, carmustine, lomustine, cyclophosphamide, 4-hydroxyoxycyclophosphamide, estramustine, melphalan, ifosfamide, tropfosfamide, chlorambucil, bendamustine, dacarbazine, busulfan, procarbazine, treosulfan, tremozolomide, thiotepa, daunorubicin, doxorubicin, aclarubicin, epirubicin, Mitoxantrone, idarubicin, bleomycin, mitomycin, dactinomycin, methotrexate, fludarabine, fludarabine 5'-dihydrogen phosphate, cladribine, mercaptopurine, thioguanine, cytarabine, fluorouracil, gemcitabine, capecitabine, docetaxel, Carboplatin, cisplatin, oxaliplatin, amsacrine, irinotecan, topotecan, hydroxycarbamide, miltefosine, pentostatin, aldesleukin, tretinoin, asparaginase, pegasparase, anastrozole, exemestane, letrozole, formestane, aminoglutethemide, adriamycin, Azithromycin, spiramycin, cepharantine, 8-ergolines, dimethylergolines, agroclavin, 1-allylisuride, 1-allylterguride, bromerguride, bromocriptine (ergotaman-3',6',18-trione, 2-bromo-12'-hydroxy-2'-(1-methylethyl)-5'-(2-methylpropyl)-,(5'alpha)-), elymoclavine, ergocristine (ergotaman-3',6',18-trione, 12'-hydroxy-2'-(1-methylethyl)-5'-(phenylmethyl)-, (5'-alpha)-), ergocristinine, ergocornine (ergotaman-3',6',18-trione, 12'-hydroxy-2',5'-bis(1-methylethyl)-, (5'-alpha)-), ergocorninine, ergocryptin (ergotaman-3',6',18-trione, 12'-hydroxy-2'-(1-methylethyl)-5'-(2-methylpropyl)-, (5'alpha)- (9Cl)), ergocryptinine, ergometrine, ergonovine (ergobasin, INN: Ergometrine, (8beta(S))-9,10-didehydro-N-(2-hydroxy-1-methylethyl)-6-methyl-ergoline-8-carboxamide), ergosine, ergosinine, ergotmetrinine, ergotamine (ergotaman-3',6',18-trione, 12'-hydroxy-2'-methyl-5'-(phenylmethyl)-, (5'-alpha)- (9CI)), ergotaminin, ergovalin (ergotaman-3',6',18-trione, 12'-hydroxy-2'-methyl-5'-(1-methylethyl)-, (5'alpha)-), lergotril, lisuride (CAS no.: 18016-80-3, 3-(9,10-didehydro-6-methylergolin-8alpha-yl)-1,1-diethylurea), lysergol, lysergic acid (D-lysergic acid), lysergic acid amide (LSA, D-lysergic acid amide), lysergic acid diethylamide (LSD, D-lysergic acid diethylamide, INN: lysergamide, (8β)-9,10-didehydro-N,N-diethyl-6-methyl-ergoline-8-carboxamide), isolysergic acid (D-isolysergic acid), isolysergic acid amide (D-isolysergic acid amide), isolysergic acid diethylamide (D-isolysergic acid diethylamide), mesulergin, metergoline, methergine (INN: methylergometrin, (8beta(S))-9,10-didehydro-N-(1-(hydroxymethyl)propyl)-6-methyl-ergoline-8-carboxamide), methylergometrin, methysergide (INN: methysergide, (8beta)-9,10-didehydro-N-(1-(hydroxymethyl)propyl)-1,6-dimethyl-ergoline-8-carboxamide), pergolide ((8β)-8-((methylthio)methyl)-6-propyl-ergoline), proterguride and terguride, celecoxip, thalidomide, Fasudil^{®}· cyclosporine, SMC proliferation inhibitor-2w, epothilone A and B, mitoxanthrone, azathioprine, mycophenolate mofetil, c-myc antisense, b-myc antisense, betulinic acid, camptothecin, PI-88 (sulfated oligosaccharide), melanocyte-stimulating hormone (α -MSH), activated protein C, IL1-β inhibitor, thymosinα - 1, fumaric acid and its esters, calcipotriol, tacalcitol, lapachol, β-lapachone,podophyllotoxin, betulin, podophyllic acid-2-ethylhydrazide, molgramostim (rhuGM-CSF), peginterferon α-2b, lanograstim (r-HuG-CSF), filgrastim, macrogol, dacarbazine, basiliximab, daclizumab, Selectin (cytokine antagonist), CETP inhibitor, cadherins, cytokine inhibitors, COX-2 inhibitor, NFkB, angiopeptin, ciprofloxacin, camptothecin, fluroblastine, monoclonal antibodies that inhibit muscle cell proliferation, bFGF antagonists, probucol, prostaglandins, 1,11-dimethoxycanthin-6-one, 1-hydroxy-11-methoxycanthin-6-one, scopolectin, colchicine, NO donors such as pentaerythrityl tetranitrate, and syndnoeimines, S-nitroso derivatives, tamoxifen, staurosporine, β-estradiol,α - estradiol, estriol, estrone, ethinylestradiol, fosfestrol, medroxyprogesterone, estradiolcypionate, estradiolbenzoate, tranilast, camebacaurine and other terpenoids used in cancer therapy, verapamil, tyrosine kinase inhibitors (tyrphostins), cyclosporine A and B, paclitaxel and its derivatives such as 6-α - hydroxy-paclitaxel, baccatin, taxotere, synthetically produced as well as macrocyclic oligomers of carbon suboxide (MCS) obtained from native sources and its derivatives, mofebutazone, acemetacin, diclofenac, lonazolac, dapsone, o-carbamoylphenoxyacetic acid, lidocaine, ketoprofen, mefenamic acid, piroxicam, meloxicam, chloroquine phosphate, penicillamine, tumstatin, avastin, D-24851, SC-58125, hydroxychloroquine, auranofin, sodium aurothiomalate, oxaceprol, celecoxib, β-sitosterol, ademetionin, myrtecaine, polidocanol, nonivamide, levomenthol, benzocaine, aescin, ellipticin, D-24851 (Calbiochem), colcemid, cytochalasin A-E, indanocins, nocadazoles, S 100 protein, bacitracin, vitronectin receptor antagonists, azelastine, guanidyl cyclase stimulator tissue inhibitor of metalloproteinase-1 and 2, free nucleic acids, Nucleic acids incorporated into viral carriers, DNA and RNA fragments, plaminogen activator inhibitor-1, plasminogen activator inhibitor-2, antisense oligonucleotides, VEGF inhibitors, IGF-1, Agents from the group of antibiotics such as cefadroxil, cefazolin, cefaclor, cefotixin, tobramycin, gentamycin, penicillins such as dicloxacillin, oxacillin, sulfonamides, metronidazole, antithrombotics such as argatroban, aspirin, abciximab, synthetic antithrombin, bivalirudin, coumadin, enoxoparin, desulfated and N-reacetylated heparin, tissue plasminogen activator, Gpllb/llla platelet membrane receptor, factor Xₐ inhibitor antibody, interleukin inhibitors, heparin, hirudin, r-hirudin, PPACK, protamine, sodium salt of 2-methylthiazolidine-2,4-dicarboxylic acid prourokinase, streptokinase, warfarin, urokinase, Vasodilators such as dipyramidol, trapidil, nitroprusside, PDGF antagonists such as triazolopyrimidine and seramine, ACE inhibitors such as captopril, cilazapril, lisinopril, enalapril, losartan, thioprotease inhibitors, Prostacyclin, vasiprost, interferonα, β and γ, histamine antagonists, serotonin blockers, apoptosis inhibitors, apoptosis regulators such as p65, NF-kB, or Bcl-xL antisense oligonucleotides, halofuginone, nifedipine, tocopherol, vitamins B1, B2, B6, and B12, folic acid, tranilast, molsidomine, tea polyphenols, epicatechin gallate, epigallocatechin gallate, boswellic acids and their derivatives, leflunomide, Anakinra, etanercept, sulfasalazine, etoposide, dicloxacylline, tetracycline, triamcinolone, mutamycin, procainimide, D24851, SC-58125, retinoic acid, quinidine, disopyrimide, flecainide, propafenone, sotolol, amidoron, natural and synthetically produced steroids such as bryophyllin A, inotodiol, maquiroside A, ghalakinoside, mansonin, strebloside, hydrocortisone, betamethasone, dexamethasone, non-steroidal anti-inflammatory drugs (NSAIDS) such as fenoporfen, ibuprofen, indomethacin, naproxen, phenylbutazone and other antiviral agents such as acyclovir, ganciclovir and zidovudine, antifungal agents such as clotrimazole, flucytosine, griseofulvin, ketoconazole, miconazole, nystatin, terbinafine, antiprozoal agents like chloroquine, mefloquine, quinine, natural terpenoids like hippocaesculin, barringtogenol-C21-angelat, 14-dehydroagrostistachin, agroskerin, agroskerin, agrostistachin, 17-hydroxyagrostistachin, ovatodiolides, 4,7-oxycycloanisomelic acid, baccharinoids B1, B2, B3 and B7, tubeimoside, bruceanols A, B and C, bruceantinosides C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, furthermore cymarin, apocymarin, aristolochic acid, anopterin, hydroxyanopterin, anemonin, protoanemonin, berberin, cheliburin chloride, cictoxin, sinococulin, bombrestatin A and B, cudraisoflavone A, curcumin, dihydronitidine, nitidine chloride, 12-beta-hydroxypregnadiene-3,20-dione, bilobol, ginkgol, ginkgolic acid, helenalin, indicin, indicin-N-oxide, lasiocarpin, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetin, pancratistatin, liriodenin, oxoushinsunin, aristolactam-All, bisparthenolidin, periplocoside A, ghalakinoside, ursolic acid, deoxypsorospermine, psycorubin, ricin A, sanguinarin, manwuweizic acid, methylsorbifolin, sphatheliachromene, stizophyllin, mansonin, strebloside, akagerin, dihydrousambaraensin, hydroxyusambarin, strychnopentamine, strychnophyllin, usambarin, usambarensin, berberin, liriodenin, oxoushinsunin, daphnoretin, lariciresinol, methoxylariciresinol, syringaresinol, umbelliferon, afromoson, acetylvismion B, desacetylvismion A, vismion A and B, and sulfur-containing amino acids such as cysteine, as well as salts, hydrates, solvates, enantiomers, racemates, enantiomeric mixtures, diastereomeric mixtures; metabolites, prodrugs and mixtures of the active ingredients.

3. The medical device according to claim 1 or 2, wherein the C₁-C₅ alkyl linker is selected from the group consisting of methylene (-CH₂-), ethylene (-CH₂CH₂-), propylene (-CH₂CH₂CH₂-), butylene (-CH₂CH₂CH₂CH₂-), and pentylene (-CH₂CH₂CH₂CH₂CH₂-).

4. The medical device according to any one of claims 1 - 3, wherein the benzophenone substituent **BP** is benzoylphenyl.

5. The medical device according to any one of claims 1 - 4, wherein Y represents -OCO-, -NHCO-, -COO-, -CONH-, -OCOCH₂-, -NHCOCH₂-, -COOCH₂-, or -CONHCH₂-.

6. The medical device according to any one of claims 1 - 5, wherein Y represents -NHCO-, -CONH-, -NHCOCH₂-, or -CONHCH₂-.

7. The medical device according to any one of claims 1 - 6, wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group, and an organometallic group, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

8. The medical device according to any one of claims 1 - 6, wherein up to 100% of the functional groups of all maleic acid moieties in the vinylpyrrolidone copolymer that are **not** functionalized with **-L-Y-BP,** independently of each other have a non-benzophenone residue selected from the group comprising or consisting of polyethylene glycol -(CH₂CH₂O)_{q}-H, polylactide, polyglycolide and polyacrylate, wherein **q** is an integer between 35 and 50;
wherein the ratio of **-L-Y-BP** to non-benzophenone residue is ≥ 1, and the remaining functional groups of all maleic acid moieties in the vinylpyrrolidone copolymer have the residue -H.

9. The medical device according to any one of claims 1 - 8, wherein the copolymer has the general formula (ii): 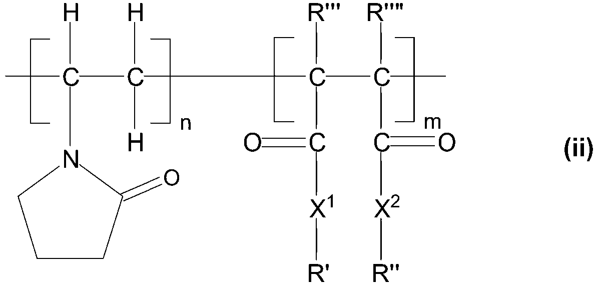 wherein
m = 0.1 - 10.0 mol% and
**n** = 100 mol% - m;
**X¹** and **X²** independently of each are selected other from -O- and -NH-;
**p% of the residues R^{'}** and **R^{"}** independently of each other are selected from -L'-Y'-BP' and -L"-Y"-BP"; and
up to p% of **R^{'}** and **R^{"}** independently of each other are selected from -(CH₂CH₂O)_{q}-H, polylactide, polyglycolide, polyacrylate, alkyl, aryl, arylalkyl, alkoxy, aryloxy, alkylhydroxy, arylhydroxy, alkylaryloxy, alkylamino, arylamino, alkylarylamino, silyl group and an organometallic group; and
the remaining residues **R^{'}** and **R^{"}** are -H;
**R‴** and **Rʺʺ** represent independently of each other -H, -CH₃, -CH₂CH₃, or -F;
**L** represents -CH₂-, -CH₂CH₂-, -CH₂CH₂CH₂-, -CH₂CH₂CH₂CH₂-, or -CH₂CH₂CH₂CH₂CH₂-;
**Y'** and **Y"** independently of each other are selected from a bond, -OCO-, -NHCO-, -CO-, -O-, -NH-, -COO-, -CONH-, -NHCOO-, -OCONH-, -OCOO-, -NHCONH-, -OCOCH₂-, -NHCOCH₂-, -COCH₂-, -OCH₂-, -NHCH₂-, -COOCH₂-, -CONHCH₂-, -NHCOOCH₂-, -OCONHCH₂-, -OCOOCH₂- and -NHCONHCH₂-,
with the proviso that when **Y** is -O-, the C₁-C₅ alkyl linker (**L**) is not ethylene (-CH₂CH₂-);
**BP** represents 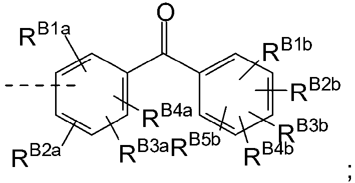
**R^{B1a}** - **R^{B4a}** and **R^{B1b}** - **R^{B5b}** represent independently of each other -H, -COOH, -CH₃, -F, -CI, -Br, -I, -OH, -CN, -N₃, -OCN, -NCO, -SCN, -NCS, -CHO, -COCN, -NHCONH₂, -NHC(=NH)NH₂, -OCONH₂, -NO₂, -COCH₃, -CONH₂, -SO₃H, -SO₂NH₂, -OCH₃, -OC₂H₅, -OC₃H₇, -OCF₃, -OCH(CH₃)₂, -OC(CH₃)₃, -OC₄H₉, -O-cyclo-C₃H₅, -OCH₂-cyclo-C₃H₅, -OC₂H₄-cyclo-C₃H₅, -OCH₂CH(CH₃)₂, -OC₂H₄OCH₃, -CH₂OCH₃, -CF₃, -C₂H₅, -C₃H₇, -NH₂, -NH(CH)₃, -N(CH₃)₂, or -SOCH₃;
**p** is a number from 1 - 50;
wherein **q** is an integer between 35 and 50.

10. The medical device according to claim 9, wherein only one of X¹ and X² is -NH- and/or wherein **Y'** and **Y"** represent a bond.

11. The medical device according to claim 9 or 10, wherein up to **p%** of **R^{'}** and **R^{"}** are -(CH₂CH₂O)_{q}-H and **p** is a number from 1 - 50, wherein **q** is an integer between 35 and 50.

12. The medical device according to any one of claims 11 - 11, wherein the vinylpyrrolidone copolymer is selected from the group consisting of: 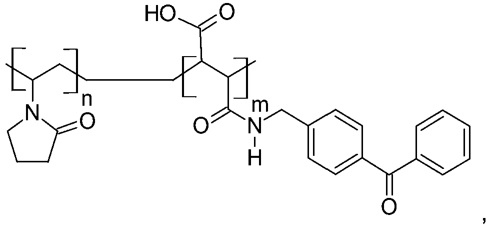 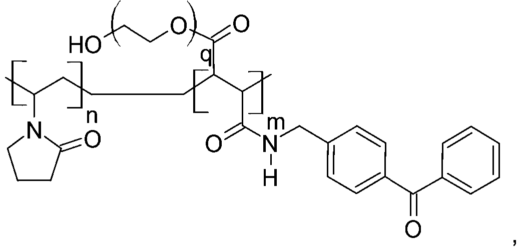 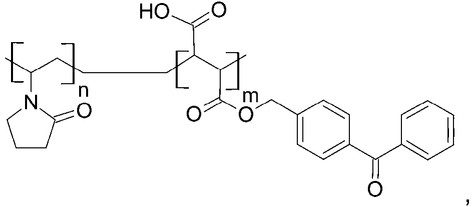 and 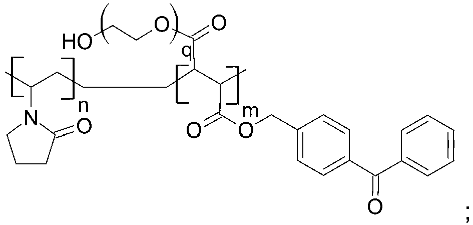 wherein
m = 0.1 - 10.0 mol% and
n = 100 mol% - m;
q is an integer between 35 and 50.

13. The medical device according to any one of the claims 1 - 12, wherein the medical device is selected from the group comprising or consisting of balloon catheters, bladder catheters, catheter shafts, guide wires, stomach tubes, endoscopes, colonoscopes, needles, cannulas, endotracheal tubes, tracheal cannulas, implants such as stents, ocular lenses, prosthetic joints, screws, fixations, artificial exits, nasal implants, vocal cords, larynx, artificial and native valves such as cardiac or venous valves, esophageal sphincter, sphincter ani, bone graft substitutes, and ventilator tubes.

14. The medical device according to any one of the claims 1 - 13, wherein the coating further comprises a crosslinker.

15. The medical device according to claim 14, wherein the crosslinker has the general formula (I):
**BP¹-L¹-Z-L²-BP²** (**I**)
wherein **BP¹** and **BP²** are benzophenone substituents covalently linked to each other via a linker **Z,** wherein the linker **Z** is selected from an aryl linker, an alkyl linker and an amino acid linker, and wherein the linker **Z** has two linkers **L¹** and **L²** each functionalized with one of the two benzophenone substituents **BP¹** and **BP²,** wherein **L¹** and **L²** independently of each other include or consist of:
-O-CO-, -NH-CO-, -CO-, -O-, -NH-, -CO-O-, -CO-NH-, -NH-CO-O-, -O-CO-NH-, -O-CO-O-, and -NH-CO-NH-, or wherein the linker **Z** is -O-CO-, -NH-CO-, -CO-, -O-, -NH-, -CO-O-, -CO-NH-, -NH-CO-O-, -O-CO-NH-, -O-CO-O-, or -NH-CO-NH-.
